# EUROPEAN PATENT SPECIFICATION

(11) **EP 1 178 986 B1**
(45) Date of publication and mention of the grant of the patent: **25.02.2004**
(21) Application number: 00929721.9
(22) Date of filing: 18.05.2000
(51) Int. Cl.: C07D 409/12, C07D 307/68, C07D 405/12, C07D 409/14, C07D 405/14, C07D 307/32, A61K 31/4015, A61K 31/365, A61K 31/40, A61K 31/381, A61P 37/02

(54) **FURANONE DERIVATIVES AS INHIBITORS OF CATHEPSIN S**
FURANONDERIVATE ALS INHIBITOREN VON CATHEPSIN S
DERIVES FURANONES INHIBITEURS DE CATHEPSINE S

(30) Priority: 18.05.1999 GB 9911417
(43) Date of publication of application: 13.02.2002
(73) Proprietor: Medivir UK Ltd, Cambridge CB1 9PT (GB); Peptimmune, Inc., Cambridge, MA 02139 (US)
(72) Inventor: QUIBELL, Martin, Cherry Hinton Cambridge CB1 9GS (GB); TAYLOR, Steven, Cambridge CB3 8SG (GB)
(74) Representative: Davies, Jonathan Mark
(86) International application number: PCT/GB2000/001894
(87) International publication number: WO 2000/069855

(56) References cited:
- EP-A- 0 603 873
- WO-A-97/40066
- WO-A-98/50533

## Description

### Field of the invention

Cathepsin S is a highly active cysteine protease belonging to the papain superfamily. Its primary structure is 57%, 41% and 45% homologous with that of the human cathepsin L and H and plant cysteine proteases papain respectively, although only 31% homologous with Cathepsin B.

It is found mainly in lymph nodes, spleen, and macrophages and this limited occurrence suggests the potential involvement of this enzyme in the pathogenesis of degenerative disease.

Moreover, it has been found that destruction of Ii by proteolysis is required for MHC class II molecules to bind antigenic peptides, and for transport of the resulting complex to the cell surface. Furthermore, it has been found that Cathepsin S is essential in B cells for effective Ii proteolysis necessary to render class II molecules competent for binding peptides. Therefore, the inhibition of this enzyme may be useful in modulating class II-restricting immune response (WO 97/40066). Selective inhibition of a single protease in a complex mixture of proteolytic enzymes and more especially over other members of the same enzyme class or family is imperative as incorrect regulation of proteolytic activity can lead to unwanted pathological conditions such as hypertension, blood clotting or worse. This has lead to the search for inhibitors that selectively inhibit only one member of a proteolytic family, a problem that is very relevant to the Cathepsin family, which have a high degree of homology.

The invention relates to novel protease inhibitors, particularly inhibitors of the cysteine proteases of the papain superfamily and more particularly to Cathepsin S. The inhibitors of the invention are selective over other members of the family and in particular show selectivity over other members of the Cathepsin family such as L and K.

### Description of the related art

In WO 97/40066, the use of inhibitors against Cathepsin S is described. The inhibition of this enzyme is suggested to prevent or treat disease caused by protease activity. WO 98/50533 describes the use of compounds according to the formula (I).

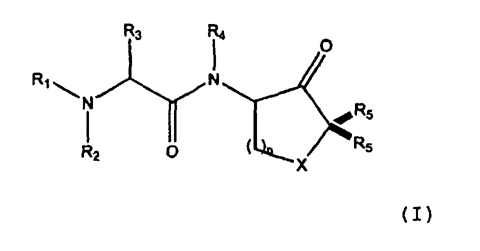

It is suggested the compounds of this formula, known as the tetrahydrofuran-3-ones, are useful as inhibitors to proteases, in particular the papain superfamily; specifically those of the Cathepsin family; and particularly Cathepsin K.

### Summary of the invention

The present invention provides compounds which inhibit the cysteine protease Cathepsin S but do not significantly inhibit other members of the papain superfamily. The compounds of the present invention are useful for the treatment of diseases caused by or enhanced by the presence or the activity of the protease enzyme.

Accordingly, the first aspect of the invention provides a compound according to formula (II): 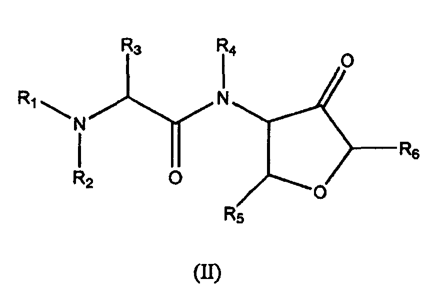 wherein:
R1 = Rⁱ, RⁱC(O), Rⁱ C (S) , Rⁱ SO₂, Rⁱ OC(O), Rⁱ NHC(O)
Rⁱ= 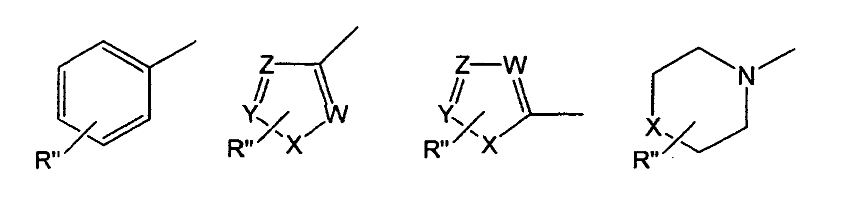
X, = O, S, NH;
W, Y, Z = CH, N;
Rⁱⁱ = single or multiple ring substitution combinations taken from:
   H, C₁₋₇-alkyl, C₁₋₇ alkyl C₃₋₆-cycloalkyl, OH, SH, amine, halogen;
   R2, R4 = H, C₁₋₇-alkyl, C₁₋₇ alkyl C₃₋₇-cycloalkyl;
   R3 = C₁₋₇-alkyl, C₁₋₇ alkyl C₃₋₇-cycloalkyl, Ar- C₁₋₇-alkyl;
   R5 = C₁₋₇-alkyl, Ar- C₁₋₇-alkyl, C₁₋₃-alkyl-CONRⁱⁱⁱ, R^{iv};
   Rⁱⁱⁱ = H, methyl;
   R^{iv}= 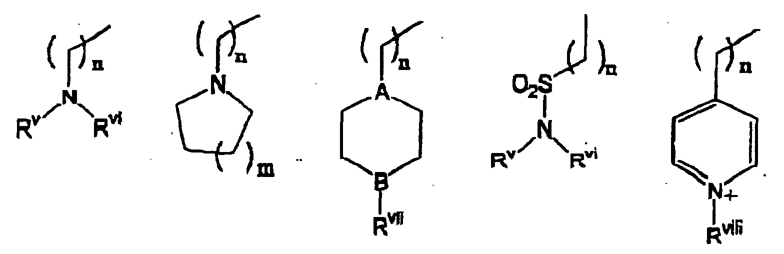
where n = 1-3, m = 1-3;
R^{v} R^{vi} = H, C₁₋₇-alkyl;
A = N, CH;
B = N, 0, S, CH;
R^{vii} - absent when B = O, S; or R^{vii} = H, C₁₋₇-alkyl when B = N, CH;
R^{viii} = O, C₁₋₇-alkyl;
R6 = H
and wherein,
C₁₋₇-alkyl or C₁₋₃ alkyl may be optionally substituted by one or two halogens and/or a heteroatom S, O, N, which if located at a chain terminus is substituted by one or two H atoms;
Ar is phenyl, pyrazolyl, imidazolyl, oxazolyl, thiazinolyl, isothiazinolyl, oxadiazolyl, 1,2,3-triazolyl, furanyl, or thienyl, which may be optionally substituted by halogen C₁₋₃-alkyl, OH, OC₁₋₃-alkyl, SH, SC₁₋₃-alkyl, or amine;
amine is -NH₂, -NHC₁₋₃-alkyl or -N(C₁₋₃-alkyl)₂
and pharmaceutically acceptable salts thereof.

'C₁₋₇-alkyl' as applied herein is meant to include straight and branched chain aliphatic carbon chains such as methyl, ethyl, n-propyl, isopropyl, n-butyl, isobutyl, t-butyl, pentyl, isopentyl, hexyl, heptyl and any simple isomers thereof. Additionally, any C₁₋₇-alkyl may optionally be substituted by one or two halogens and/or a heteroatom S, O, NH. If the heteroatom is located at a chain terminus then it is appropriately substituted with one or 2 hydrogen atoms.

'C₁₋₃-alkyl' as applied herein includes methyl, ethyl, propyl, isopropyl, cyclopropyl, any of which may be optionally substituted as described in the paragraph above.

'Amine' includes NH₂, NHC₁₋₃-alkyl or N(C₁₋₃-alkyl)₂.

'Halogen' as applied herein is meant to include F, Cl, Br, I, particularly chloro and preferably fluoro.

'Ar- C₁₋₇-alkyl' as applied herein is meant to include a phenyl, pyrazolyl, imidazolyl, oxazolyl, isoxazolyl, thiazinolyl, isothiazinolyl, thiazolyl, oxadiazolyl, 1,2,3-triazolyl, 1,2,4-triazolyl, furanyl or thienyl aromatic ring (Ar) attached through a 'C₁₋₇-alkyl' (defined above) to the dihydro-(3H)-furanone ring system or in the case of R3 linked directly to the molecule backbone. Optionally, the aromatic ring Ar may be substituted with halogen, C₁₋₃-alkyl, OH, OC₁₋₃-alkyl, SH, SC₁₋₃-alkyl, amine and the like.

'C₁₋₃-alkyl-CONRⁱⁱⁱ, R^{iv}' as applied herein is meant to include straight or branched carbon chain substituted with a 1°, 2° or 3° carboxamide wherein Rⁱⁱⁱ, R^{iv} includes H and Me.

'C₁₋₃-alkyl-SO₂-R^{ix'} , as applied herein is meant to include straight or branched carbon chain substituted with a sulphone wherein R^{ix} includes 'C₁₋₇-alkyl', 'Ar- C₁₋₇-alkyl', 'C₃₋₆-cycloalkyl'.

'C₁₋₃-alkyl-C(O)-NHR^{ix}, as applied herein is meant to include straight or branched carbon chain substituted with a secondary carboxamide wherein R^{ix} includes 'C₁₋₇-alkyl', 'Ar- C₁₋₇-alkyl', 'C₃₋₆-cycloalkyl'.

If a chiral centre is present, all isomeric forms are intended to be covered.

Suitably compounds of the present invention have
R1 = ^{Ri}C(O)
Where R' = 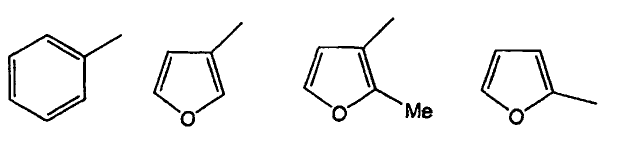 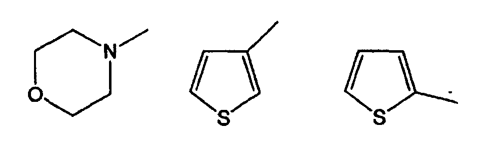 R2 and R4 = H;
R3 = n-butyl, t-butyl, 3-(2,2-dimethylpropyl), 4-(2-methylbutyl), 4-(3,3-dimethylbutyl), 4-(3,3-dimethyl-2-methylbutyl), 4-(3-methyl-2-methylbutyl), 5-(2-methyl-3-methylpentyl);
R5 = CH₃, C₂H₅, CH₂Ar, CH₂CONH₂, (CH₂)₂CONH₂, 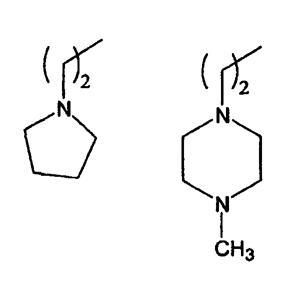 R6 = H
and wherein,
C₁₋₇-alkyl or C₁₋₃ alkyl may be optionally substituted by one or two halogens and/or a heteroatom S, O, N, which if located at a chain terminus is substituted by one or two H atoms;
Ar is phenyl, pyrazolyl, imidazolyl, oxazolyl, thiazinolyl, isothiazinolyl, oxadiazolyl, 1,2,3-triazolyl, furanyl, or thienyl, which may be optionally substituted by halogen C₁₋₃-alkyl, OH, OC₁₋₃-alkyl, SH, SC₁₋₃-alkyl, or amine;
amine is -NH₂, -NHC₁₋₃-alkyl or -N(C₁₋₃-alkyl)₂

Both (R) and (S) stereochemistries at the furan 5-position are encompassed by the invention with (S) being preferred in some cases, for instance when R5 = CH₃;

The invention may be employed in a method using the compounds of formula II for the treatment of diseases wherein cathepsin S is a factor, ie diseases or conditions alleviated or modified by inhibition of cathepsin s**,** preferably without substantial concomitant inhibition of other members of the papain superfamily. Examples of such diseases or conditions include those enumerated in WO 97/40066, such as autoimmune diseases, allergies, multiple sclerosis, rheumatoid arthritis and the like. The invention further provides the use of the compounds of formula II in the manufacture of a medicament for the treatment of diseases or conditions alleviated or moderated by inhibition of cathepsin S.

The compounds of the invention can form salts which form an additional aspect of the invention. Appropriate pharmaceutically acceptable salts of the compounds of Formula II include salts of organic acids, especially carboxylic acids, including but not limited to acetate, trifluoroacetate, lactate, gluconate, citrate, tartrate, maleate, malate, pantothenate, isethionate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-napthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids. The compounds of Formula II may in some cases be isolated as the hydrate.

While it is possible for the active agent to be administered alone, it is preferable to present it as part of a pharmaceutical formulation. Such a formulation will comprise the above defined active agent together with one or more acceptable carriers/excipients and optionally other therapeutic ingredients. The carrier(s) must be acceptable in the sense of being compatible with the other ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of Formula I or its pharmaceutically acceptable salt in conjunction or association with a pharmaceutically acceptable carrier or vehicle. If the manufacture of pharmaceutical formulations involves intimate mixing of pharmaceutical excipients and the active ingredient in salt form, then it is often preferred to use excipients which are non-basic in nature, i.e. either acidic or neutral.

Formulations for oral administration in the present invention may be presented as discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion and as a bolus etc.

With regard to compositions for oral administration (e.g. tablets and capsules), the term suitable carrier includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring or the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

The term "N-protecting group" or "N-protected" and the like as used herein refers to those groups intended to protect the N-terminus of an amino acid or peptide or to protect an amino group against undesirable reactions during synthetic procedures. Commonly used N-protecting groups are disclosed in Greene, "Protective Groups in Organic Synthesis" (John Wiley & Sons, New York, 1981), which is hereby incorporated by reference. N-protecting groups include acyl groups such as formyl, acetyl, propionyl, pivaloyl, t-butylacetyl, 2-chloroacetyl, 2-bromoacetyl, trifluoracetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl, and the like, carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butoxycarbonyl, diisopropylmethoxycarbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl, and the like; alkyl gropus such as benzyl, triphenylmethyl, benzyloxymethyl and the like; and silyl groups such as trimethylsilyl and the like. Favoured N-protecting groups include formyl, acetyl, allyl, F-moc, benzoyl, pivaloyl, t-butylacetyl, phenylsulfonyl, benzyl, t-butoxycarbonyl (BOC) and benzyloxycarbonyl (Cbz). Hydroxy and/or carboxy protecting groups are also extensively reviewed in Greene ibid and include ethers such as methyl, substituted methyl ethers such as methoxymethyl, methylthiomethyl, benzyloxymethyl, t-butoxymethyl, 2-methoxyethoxymethyl and the like, silyl ethers such as trimethylsilyl (TMS), t-butyldimethylsilyl (TBDMS) tribenzylsilyl, triphenylsilyl, t-butyldiphenylsilyl triisopropyl silyl and the like, substituted ethyl ethers such as 1-ethoxymethyl, 1-methyl-1-methoxyethyl, t-butyl, allyl, benzyl, p-methoxybenzyl, dipehenylmethyl, triphenylmethyl and the like, aralkyl groups such as trityl, and pixyl (9-hydroxy-9-phenylxanthene derivatives, especially the chloride). Ester hydroxy protecting groups include esters such as formate, benzylformate, chloroacetate, methoxyacetate, phenoxyacetate, pivaloate, adamantoate, mesitoate, benzoate and the like. Carbonate hydroxy protecting groups include methyl vinyl, allyl, cinnamyl, benzyl and the like.

Compounds are synthesised by a combination of chemistries, performed either in solution or on the solid phase. The general scheme for preparation of the furanone ring system is given in Scheme 1, commencing from either a commercially available chiral amino acid derivative or a stereoselectively prepared amino acid, for instance from Scheme 2.

The stereoselective synthesis detailed in Scheme 2 was adapted from Blaskovich, M.A., Evinder, G., Rose, N. G. W., Wilkinson, S., Luo, Y. and Lajoie, G. A. *J. Org. Chem,* 63, 3631-3646, 1998. The addition of Grignard reagent to compound **(10)** yielding the (R) isomer of compound **(11)** is applicable to a huge range of alternative Grignard reagents. This allows ready access to analogues of compound **(15)** by standard Grignard chemistry to produce R5 analogues embraced by formula (II). The R5 substituent confers many beneficial qualities to molecules of general formula (II) including improvements in potency, selectivity and offers the potential to append inhibitor molecules with a basic functionality to improve solubility and pharmacokinetic properties. Additionally, molecules of formula (II) where R5 is alkyl and not simply hydrogen show good chiral stability at the furanone alpha carbon (ring position 4).

Note particularly the presence of the substituent R5 in formula (II) in comparison with the absence of any substituent in the same position in formula (I) according to WO 98/50533.

An alternative route towards chiral β-alkyl serine amino acids is detailed in scheme 3, commencing from D-mannitol.

The addition of organocuprate reagents to the advanced oxirane intermediate **(44)** is applicable to a wide selection of reagents, giving ready access to analogues of compound **(15)** ie analogues of R5 in formula (II).

To access molecules containing potential binding elements in R6 formula (II), a number of synthetic chemistry routes are available. One example extends the basic concepts developed for the preparation of the furanone ring system depicted in schemes 1 and 8 (scheme 4). Intermediate **(51)**, which can be prepared with alternative ring stereochemistries from alternative threonine isomers, provides access to the functionalities defined in R6 formula (II).

An alternative route to access molecules containing potential binding elements in R6 from formula (II), is based upon transformation of a chiral sugar starting material (scheme 5). Intermediate **(59),** which can be prepared with alternative ring stereochemistries from alternative starting sugar isomers using conventional saccharide chemistry, provides access to many functionalities in R6 formula (II).

Many active inhibitors contain commercially available amino acid residues such as L-leucine, L-norleucine etc (see table 1). Alternatively, active inhibitors contain new and novel hydrophobic amino acids, which are prepared following the chemistry detailed in scheme 6. The synthesis detailed in Scheme 6 was adapted from Dexter, C. S. and Jackson, R. F. W. *Chem.Commun.* 1, 75-76, 1998, and allows ready access to analogues embraced by R3 in formula (II). The side chains of some of the novel, multiply branched alpha-amino acid building blocks exemplified herein can be thought of as hybrids of the properties of combinations of other amino acid side chains, such as those of norleucine and t-butylalanine and are thus referred to as "hybrids" in the tables.

The furanone building blocks (synthesis exemplified in Scheme 1) are utilised in a solid phase synthesis of inhibitor molecules (typically 5-25mg product) detailed in Scheme 7. Alternatively, for larger scale syntheses, full preparation of inhibitors by solution phase chemistry may be performed as detailed in Scheme 8.

Compounds were previously named (for instance in the priority document GB 9911417.5) using amino acid nomenclature i.e. a sidechain of 2,2-dimethylpropyl was termed the amino acid *tert*-butylalanine. The current specification contains novel amino acids for which common names are not available. Therefore, all previously exemplified and new compounds are re-named following IUPAC guidelines. For example, the compound below was previously named :-
Dihydro-(4-(S)-Amino-N-[(3-furanoyl)-*tert*-butyl-L-alanine])-5-(S)-methyl)-3(2H)-furanone

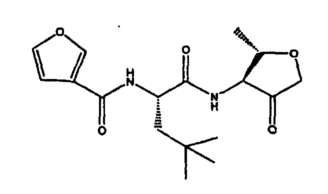

Under the new naming regime, the compound will be termed as:-
Furan-3-carboxylic acid [3,3-dimethyl-1S-(2S-methyl-4-oxo-tetrahydrofuran-3S-ylcarbamoyl)butyl]amide.

Unless otherwise specified, where a chiral centre is present in a molecule but not assigned, both R and S isomers are intended.

Further compounds of the present invention include, but are not limited to, the following examples;
Furan-3-carboxylic acid [3,3-dimethyl-1S-(2-ethyl-4-oxo-tetrahydrofuran-3-ylcarbamoyl)butyl]amide,
Furan-3-carboxylic acid [1S-(2-carbamoylmethyl-4-oxo-tetrahydrofuran-3-ylcarbamoyl)-3,3-dimethylbutyl]amide,
Furan-3-carboxylic acid {1S-[2-(2-dimethylaminoethyl)-4-oxo-tetrahydrofuran-3-ylcarbamoyl]-3,3-dimethylbutyl}amide,
Furan-3-carboxylic acid [3,3-dimethyl-1S-(4-oxo-2-pyrrolidin-1-ylmethyl-tetrahydrofuran-3-ylcarbamoyl)butyl]amide,
Furan-3-carboxylic acid [4-methyl-1S-(2S-methyl-4-oxo-tetrahydrofuran-3S-ylcarbamoyl)pentyl]amide,
Furan-3-carboxylic acid [4-methyl-1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoyl)-4-methylpentyl]amide,
Furan-3-carboxylic acid {1S-[2-(2-dimethylaminoethyl)-4-oxo-tetrahydrofuran-3-ylcarbamoyl]-4-methylpentyl}amide,
Furan-3-carboxylic acid [4-methyl-1S-(4-oxo-2-pyrrolidin-1-ylmethyl-tetrahydrofuran-3-ylcarbamoyl)pentyl]amide,
Furan-3-carboxylic acid [3,3-dimethyl-1S-(2S-methyl-4-oxo-tetrahydrofuran-3S-ylcarbamoyl)pentyl]amide,
Furan-3-carboxylic acid [3,3-dimethyl-1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoyl)-3,3-dimethylpentyl]amide,
Furan-3-carboxylic acid {1S-[2-(2-dimethylaminoethyl)-4-oxo-tetrahydrofuran-3-ylcarbamoyl]-3,3-dimethylpentyl}amide,
Furan-3-carboxylic acid [3,3-dimethyl-1S-(4-oxo-2-pyrrolidin-1-ylmethyl-tetrahydrofuran-3-ylcarbamoyl)pentyl]amide,
Furan-3-carboxylic acid [3,3,4-trimethyl-1S-(2S-methyl-4-oxo-tetrahydrofuran-3S-ylcarbamoyl)pentyl]amide,
Furan-3-carboxylic acid [3,3,4-trimethyl -1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoyl)-3,3,4-trimethylpentyl]amide,
Furan-3-carboxylic acid {1S-[2-(2-dimethylaminoethyl)-4-oxo-tetrahydrofuran-3-ylcarbamoyl]- 3,3,4-trimethylpentyl}amide,
Furan-3-carboxylic acid [3,3,4-trimethyl-1S-(4-oxo-2-pyrrolidin-1-ylmethyl-tetrahydrofuran-3-ylcarbamoyl)pentyl]amide,
Furan-3-carboxylic acid [3,4-dimethyl-1S-(2S-methyl-4-oxo-tetrahydrofuran-3S-ylcarbamoyl)pentyl]amide,
Furan-3-carboxylic acid [3,4-dimethyl-1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoyl)-3,4-dimethylpentyl]amide,
Furan-3-carboxylic acid {1S-[2-(2-dimethylaminoethyl)-4-oxo-tetrahydrofuran-3-ylcarbamoyl]-3,4-dimethylpentyl}amide,
Furan-3-carboxylic acid [3,4-dimethyl-1S-(4-oxo-2-pyrrolidin-1-ylmethyl-tetrahydrofuran-3-ylcarbamoyl)pentyl]amide,
Furan-3-carboxylic acid [4,5-dimethyl-1S-(2S-methyl-4-oxo-tetrahydrofuran-3S-ylcarbamoyl)hexyl]amide,
Furan-3-carboxylic acid [4,5-dimethyl-1S-(2-ethyl-4-oxo-tetrahydrofuran-3-ylcarbamoyl)hexyl]amide,
Furan-3-carboxylic acid [1S-(2-carbamoylmethyl-4-oxo-tetrahydrofuran-3-ylcarbamoyl)-4,5-dimethylhexyl]amide,
Furan-3-carboxylic acid {1S-[2-(2-dimethylaminoethyl)-4-oxo-tetrahydrofuran-3-ylcarbamoyl]-4,5-dimethylhexyl}amide,
Furan-3-carboxylic acid [4,5-dimethyl-1S-(4-oxo-2-pyrrolidin-1-ylmethyl-tetrahydrofuran-3-ylcarbamoyl)hexyl]amide,
Furan-3-carboxylic acid [3-methyl-1S-(2S-methyl-4-oxo-tetrahydrofuran-3S-ylcarbamoyl)-3-phenylbutyl]amide,
Furan-3-carboxylic acid [3-methyl-1S-(2-ethyl-4-oxo-tetrahydrofuran-3-ylcarbamoyl)-3-phenylbutyl]amide,
Furan-3-carboxylic acid [1S-(2-carbamoylmethyl-4-oxo-tetrahydrofuran-3-ylcarbamoyl)-3-methyl-3-phenylbutyl]amide,
Furan-3-carboxylic acid {1S-[2-(2-dimethylaminoethyl)-4-oxo-tetrahydrofuran-3-ylcarbamoyl]-3-methyl-3-phenylbutyl}amide,
Furan-3-carboxylic acid [3-methyl-1S-(4-oxo-2-pyrrolidin-1-ylmethyl-tetrahydrofuran-3-ylcarbamoyl)-3-phenylbutyl]amide,
Furan-3-carboxylic acid [3,3-dimethyl-1S-(2S-methyl-4-oxo-tetrahydrofuran-3S-ylcarbamoyl)-4-phenylbutyl]amide,
Furan-3-carboxylic acid [3,3-dimethyl-1S-(2-ethyl-4-oxo-tetrahydrofuran-3-ylcarbamoyl)-4-phenylbutyl]amide,
Furan-3-carboxylic acid [1S-(2-carbamoylmethyl-4-oxo-tetrahydrofuran-3-ylcarbamoyl)-3,3-dimethyl-4-phenylbutyl]amide,
Furan-3-carboxylic acid {1S-[2-(2-dimethylaminoethyl)-4-oxo-tetrahydrofuran-3-ylcarbamoyl]-3,3-dimethyl-4-phenylbutyl}amide,
Furan-3-carboxylic acid [3,3-dimethyl-1S-(4-oxo-2-pyrrolidin-1-ylmethyl-tetrahydrofuran-3-ylcarbamoyl)-4-phenylbutyl]amide,
Furan-3-carboxylic acid [3,3-dimethyl-1S-(2S-methyl-4-oxo-tetrahydrofuran-3S-ylcarbamoyl)-5-phenylpentyl]amide,
Furan-3-carboxylic acid [3,3-dimethyl-1S-(2-ethyl-4-oxo-tetrahydrofuran-3-ylcarbamoyl)-5-phenylpentyl]amide,
Furan-3-carboxylic acid [1S-(2-carbamoylmethyl-4-oxo-tetrahydrofuran-3-ylcarbamoyl)-3,3-dimethyl-5-phenylpentyl]amide,
Furan-3-carboxylic acid {1S-[2-(2-dimethylaminoethyl)-4-oxo-tetrahydrofuran-3-ylcarbamoyl]-3,3-dimethyl-5-phenylpentyl}amide,
Furan-3-carboxylic acid [3,3-dimethyl-1S-(4-oxo-2-pyrrolidin-1-ylmethyl-tetrahydrofuran-3-ylcarbamoyl)-5-phenylpentyl]amide,
Thiophene-3-carboxylic acid [3,3-dimethyl-1S-(2S-methyl-4-oxo-tetrahydrofuran-3S-ylcarbamoyl)butyl]amide,
Thiophene-3-carboxylic acid [3,3-dimethyl-1S-(2-ethyl-4-oxo-tetrahydrofuran-3-ylcarbamoyl)butyl]amide,
Thiophene-3-carboxylic acid [1S-(2-carbamoylmethyl-4-oxo-tetrahydrofuran-3-ylcarbamoyl)-3,3-dimethylbutyl]amide,
Thiophene-3-carboxylic acid {1S-[2-(2-dimethylaminoethyl)-4-oxo-tetrahydrofuran-3-ylcarbamoyl]-3,3-dimethylbutyl}amide,
Thiophene-3-carboxylic acid [3,3-dimethyl-1S-(4-oxo-2-pyrrolidin-1-ylmethyl-tetrahydrofuran-3-ylcarbamoyl)butyl]amide,
Thiophene-3-carboxylic acid [4-methyl-1S-(2S-methyl-4-oxo-tetrahydrofuran-3S-ylcarbamoyl)pentyl]amide,
Thiophene-3-carboxylic acid [4-methyl-1S-(2-ethyl-4-oxo-tetrahydrofuran-3-ylcarbamoyl)pentyl]amide,
Thiophene-3-carboxylic acid [1S-(2-carbamoylmethyl-4-oxo-tetrahydrofuran-3-ylcarbamoyl)-4-methylpentyl]amide,
Thiophene-3-carboxylic acid {1S-[2-(2-dimethylaminoethyl)-4-oxo-tetrahydrofuran-3-ylcarbamoyl]-4-methylpentyl}amide,
Thiophene-3-carboxylic acid [4-methyl-1S-(4-oxo-2-pyrrolidin-1-ylmethyl-tetrahydrofuran-3-ylcarbamoyl)pentyl]amide,
Thiophene-3-carboxylic acid [3,3-dimethyl-1S-(2S-methyl-4-oxo-tetrahydrofuran-3S-ylcarbamoyl)pentyl] amide,
Thiophene-3-carboxylic acid [3,3-dimethyl-1S-(2-ethyl-4-oxo-tetrahydrofuran-3-ylcarbamoyl)pentyl] amide,
Thiophene-3-carboxylic acid [1S-(2-carbamoylmethyl-4-oxo-tetrahydrofuran-3-ylcarbamoyl)-3,3-dimethylpentyl]amide,
Thiophene-3-carboxylic acid {1S-[2-(2-dimethylaminoethyl)-4-oxo-tetrahydrofuran-3-ylcarbamoyl]-3,3-dimethylpentyl}amide,
Thiophene-3-carboxylic acid [3,3-dimethyl-1S-(4-oxo-2-pyrrolidin-1-ylmethyl-tetrahydrofuran-3-ylcarbamoyl)pentyl]amide,
Thiophene-3-carboxylic acid [3,3,4-trimethyl-1S-(2S-methyl-4-oxo-tetrahydrofuran-3S-ylcarbamoyl)pentyl]amide,
Thiophene-3-carboxylic acid [3,3,4-trimethyl -1S-(2-ethyl-4-oxo-tetrahydrofuran-3-ylcarbamoyl)pentyl]amide,
Thiophene-3-carboxylic acid [1S-(2-carbamoylmethyl-4-oxo-tetrahydrofuran-3-ylcarbamoyl)-3,3,4-trimethylpentyl]amide,
Thiophene-3-carboxylic acid {1S-[2-(2-dimethylaminoethyl)-4-oxo-tetrahydrofuran-3-ylcarbamoyl]- 3,3,4-trimethylpentyl}amide,
Thiophene-3-carboxylic acid [3,3,4-trimethyl-1S-(4-oxo-2-pyrrolidin-1-ylmethyl-tetrahydrofuran-3-ylcarbamoyl)pentyl] amide,
Thiophene-3-carboxylic acid [3,4-dimethyl-1S-(2S-methyl-4-oxo-tetrahydrofuran-3S-ylcarbamoyl)pentyl]amide,
Thiophene-3-carboxylic acid [3,4-dimethyl-1S-(2-ethyl-4-oxo-tetrahydrofuran-3-ylcarbamoyl)pentyl]amide,
Thiophene-3-carboxylic acid [1S-(2-carbamoylmethyl-4-oxo-tetrahydrofuran-3-ylcarbamoyl)-3,4-dimethylpentyl]amide,
Thiophene-3-carboxylic acid {1S-[2-(2-dimethylaminoethyl)-4-oxo-tetrahydrofuran-3-ylcarbamoyl]-3,4-dimethylpentyl}amide,
Thiophene-3-carboxylic acid [3,4-dimethyl-1S-(4-oxo-2-pyrrolidin-1-ylmethyl-tetrahydrofuran-3-ylcarbamoyl)pentyl]amide,
Thiophene-3-carboxylic acid [4,5-dimethyl-1S-(2S-methyl-4-oxo-tetrahydrofuran-3S-ylcarbamoyl)hexyl]amide,
Thiophene-3-carboxylic acid [4,5-dimethyl-1S-(2-ethyl-4-oxo-tetrahydrofuran-3-ylcarbamoyl)hexyl]amide,
Thiophene-3-carboxylic acid [1S-(2-carbamoylmethyl-4-oxo-tetrahydrofuran-3-ylcarbamoyl)-4,5-dimethylhexyl]amide,
Thiophene-3-carboxylic acid {1S-[2-(2-dimethylaminoethyl)-4-oxo-tetrahydrofuran-3-ylcarbamoyl]-4,5-dimethylhexyl}amide,
Thiophene-3-carboxylic acid [4,5-dimethyl-1S-(4-oxo-2-pyrrolidin-1-ylmethyl-tetrahydrofuran-3-ylcarbamoyl)hexyl]amide,
Thiophene-3-carboxylic acid [3-methyl-1S-(2S-methyl-4-oxo-tetrahydrofuran-3S-ylcarbamoyl)-3-phenylbutyl]amide,
Thiophene-3-carboxylic acid [3-methyl-1S-(2-ethyl-4-oxo-tetrahydrofuran-3-ylcarbamoyl)-3-phenylbutyl]amide,
Thiophene-3-carboxylic acid [1S-(2-carbamoylmethyl-9-oxo-tetrahydrofuran-3-ylcarbamoyl)-3-methyl-3-phenylbutyl]amide,
Thiophene-3-carboxylic acid {1S-[2-(2-dimethylaminoethyl)-4-oxo-tetrahydrofuran-3-ylcarbamoyl]-3-methyl-3-phenylbutyl}amide,
Thiophene-3-carboxylic acid [3-methyl-1S-(4-oxo-2-pyrrolidin-1-ylmethyl-tetrahydrofuran-3-ylcarbamoyl)-3-phenylbutyl]amide,
Thiophene-3-carboxylic acid [3,3-dimethyl-1S-(2S-methyl-4-oxo-tetrahydrofuran-3S-ylcarbamoyl)-4-phenylbutyl]amide,
Thiophene-3-carboxylic acid [3,3-dimethyl-1S-(2-ethyl-4-oxo-tetrahydrofuran-3-ylcarbamoyl)-4-phenylbutyl]amide,
Thiophene-3-carboxylic acid [1S-(2-carbamoylmethyl-4-oxo-tetrahydrofuran-3-ylcarbamoyl)-3,3-dimethyl-4-phenylbutyl]amide,
Thiophene-3-carboxylic acid {1S-[2-(2-dimethylaminoethyl)-4-oxo-tetrahydrofuran-3-ylcarbamoyl]-3,3-dimethyl-4-phenylbutyl}amide,
Thiophene-3-carboxylic acid [3,3-dimethyl-1S-(4-oxo-2-pyrrolidin-1-ylmethyl-tetrahydrofuran-3-ylcarbamoyl)-4-phenylbutyl]amide,
Thiophene-3-carboxylic acid [3,3-dimethyl-1S-(2S-methyl-4-oxo-tetrahydrofuran-3S-ylcarbamoyl)-5-phenylpentyl]amide,
Thiophene-3-carboxylic acid [3,3-dimethyl-1S-(2-ethyl-4-oxo-tetrahydrofuran-3-ylcarbamoyl)-5-phenylpentyl]amide,
Thiophene-3-carboxylic acid [1S-(2-carbamoylmethyl-4-oxo-tetrahydrofuran-3-ylcarbamoyl)-3,3-dimethyl-5-phenylpentyl]amide,
Thiophene-3-carboxylic acid {1S-[2-(2-dimethylaminoethyl)-4-oxo-tetrahydrofuran-3-ylcarbamoyl]-3,3-dimethyl-5-phenylpentyl}amide,
Thiophene-3-carboxylic acid [3,3-dimethyl-1S-(4-oxo-2-pyrrolidin-1-ylmethyl-tetrahydrofuran-3-ylcarbamoyl)-5-phenylpentyl]amide,
2-Methylfuran-3-carboxylic acid [3,3-dimethyl-1S-(2S-methyl-4-oxo-tetrahydrofuran-3S-ylcarbamoyl)butyl]amide,
2-Methylfuran-3-carboxylic acid [3,3-dimethyl-1S-(2-ethyl-4-oxo-tetrahydrofuran-3-ylcarbamoyl)butyl]amide,
2-Methylfuran-3-carboxylic acid [1S-(2-carbamoylmethyl-4-oxo-tetrahydrofuran-3-ylcarbamoyl)-3,3-dimethylbutyl]amide,
2-Methylfuran-3-carboxylic acid {1S-[2-(2-dimethylaminoethyl)-4-oxo-tetrahydrofuran-3-ylcarbamoyl]-3,3-dimethylbutyl}amide,
2-Methylfuran-3-carboxylic acid [3,3-dimethyl-1S-(4-oxo-2-pyrrolidin-1-ylmethyl-tetrahydrofuran-3-ylcarbamoyl)butyl]amide,
2-Methylfuran-3-carboxylic acid [4-methyl-1S-(2S-methyl-4-oxo-tetrahydrofuran-3S-ylcarbamoyl)pentyl]amide,
2-Methylfuran-3-carboxylic acid [4-methyl-1S-(2-ethyl-4-oxo-tetrahydrofuran-3-ylcarbamoyl)pentyl]amide,
2-Methylfuran-3-carboxylic acid [1S-(2-carbamoylmethyl-4-oxo-tetrahydrofuran-3-ylcarbamoyl)-4-methylpentyl]amide,
2-Methylfuran-3-carboxylic acid {1S-[2-(2-dimethylaminoethyl)-4-oxo-tetrahydrofuran-3-ylcarbamoyl]-4-methylpentyl}amide,
2-Methylfuran-3-carboxylic acid [4-methyl-1S-(4-oxo-2-pyrrolidin-1-ylmethyl-tetrahydrofuran-3-ylcarbamoyl)pentyl}amide,
2-Methylfuran-3-carboxylic acid [3,3-dimethyl-1S-(2S-methyl-4-oxo-tetrahydrofuran-3S-ylcarbamoyl)pentyl]amide,
2-Methylfuran-3-carboxylic acid [3,3-dimethyl-1S-(2-ethyl-4-oxo-tetrahydrofuran-3-ylcarbamoyl)pentyl]amide,
2-Methylfuran-3-carboxylic acid [1S-(2-carbamoylmethyl-4-oxo-tetrahydrofuran-3-ylcarbamoyl)-3,3-dimethylpentyl]amide,
2-Methylfuran-3-carboxylic acid {1S-[2-(2-dimethylaminoethyl)-4-oxo-tetrahydrofuran-3-ylcarbamoyl]-3,3-dimethylpentyl}amide,
2-Methylfuran-3-carboxylic acid [3,3-dimethyl-1S-(4-oxo-2-pyrrolidin-1-ylmethyl-tetrahydrofuran-3-ylcarbamoyl)pentyl]amide,
2-Methylfuran-3-carboxylic acid [3,3,4-trimethyl-1S-(2S-methyl-4-oxo-tetrahydrofuran-3S-ylcarbamoyl)pentyl]amide,
2-Methylfuran-3-carboxylic acid [3,3,4-trimethyl -1S-(2-ethyl-4-oxo-tetrahydrofuran-3-ylcarbamoyl)pentyl]amide,
2-Methylfuran-3-carboxylic acid (1S-(2-carbamoylmethyl-4-oxo-tetrahydrofuran-3-ylcarbamoyl)-3,3,4-trimethylpentyl]amide,
2-Methylfuran-3-carboxylic acid {1S-[2-(2-dimethylaminoethyl)-4-oxo-tetrahydrofuran-3-ylcarbamoyl]-3,3,4-trimethylpentyl}amide,
2-Methylfuran-3-carboxylic acid [3,3,4-trimethyl-1S-(4-oxo-2-pyrrolidin-1-ylmethyl-tetrahydrofuran-3-ylcarbamoyl)pentyl]amide,
2-Methylfuran-3-carboxylic acid [3,4-dimethyl-1S-(2S-methyl-4-oxo-tetrahydrofuran-3S-ylcarbamoyl)pentyl]amide,
2-Methylfuran-3-carboxylic acid [3,4-dimethyl-1S-(2-ethyl-4-oxo-tetrahydrofuran-3-ylcarbamoyl)pentyl]amide,
2-Methylfuran-3-carboxylic acid [1S-(2-carbamoylmethyl-4-oxo-tetrahydrofuran-3-ylcarbamoyl)-3,4-dimethylpentyl]amide,
2-Methylfuran-3-carboxylic acid {1S-[2-(2-dimethylaminoethyl)-4-oxo-tetrahydrofuran-3-ylcarbamoyl]-3,4-dimethylpentyl}amide,
2-Methylfuran-3-carboxylic acid [3,4-dimethyl-1S-(4-oxo-2-pyrrolidin-1-ylmethyl-tetrahydrofuran-3-ylcarbamoyl)pentyl]amide,
2-Methylfuran-3-carboxylic acid [4,5-dimethyl-1S-(2S-methyl-4-oxo-tetrahydrofuran-3S-ylcarbamoyl)hexyl]amide,
2-Methylfuran-3-carboxylic acid [4,5-dimethyl-1S-(2-ethyl-4-oxo-tetrahydrofuran-3-ylcarbamoyl)hexyl]amide,
2-Methylfuran-3-carboxylic acid [1S-(2-carbamoylmethyl-4-oxo-tetrahydrofuran-3-ylcarbamoyl)-4,5-dimethylhexyl]amide,
2-Methylfuran-3-carboxylic acid {1S-[2-(2-dimethylaminoethyl)-4-oxo-tetrahydrofuran-3-ylcarbamoyl]-4,5-dimethylhexyl}amide,
2-Methylfuran-3-carboxylic acid [4,5-dimethyl-1S-(4-oxo-2-pyrrolidin-1-ylmethyl-tetrahydrofuran-3-ylcarbamoyl)hexyl]amide,
2-Methylfuran-3-carboxylic acid [3-methyl-1S-(2S-methyl-4-oxo-tetrahydrofuran-3S-ylcarbamoyl)-3-phenylbutyl]amide,
2-Methylfuran-3-carboxylic acid [3-methyl-1S-(2-ethyl-4-oxo-tetrahydrofuran-3-ylcarbamoyl)-3-phenylbutyl]amide,
2-Methylfuran-3-carboxylic acid [1S-(2-carbamoylmethyl-4-oxo-tetrahydrofuran-3-ylcarbamoyl)-3-methyl-3-phenylbutyl]amide,
2-Methylfuran-3-carboxylic acid {1S-[2-(2-dimethylaminoethyl)-4-oxo-tetrahydrofuran-3-ylcarbamoyl]-3-methyl-3-phenylbutyl}amide,
2-Methylfuran-3-carboxylic acid [3-methyl-1S-(4-oxo-2-pyrrolidin-1-ylmethyl-tetrahydrofuran-3-ylcarbamoyl)-3-phenylbutyl]amide,
2-Methylfuran-3-carboxylic acid [3,3-dimethyl-1S-(2S-methyl-4-oxo-tetrahydrofuran-3S-ylcarbamoyl)-4-phenylbutyl]amide,
2-Methylfuran-3-carboxylic acid [3,3-dimethyl-1S-(2-ethyl-4-oxo-tetrahydrofuran-3-ylcarbamoyl)-4-phenylbutyl]amide,
2-Methylfuran-3-carboxylic acid [1S-(2-carbamoylmethyl-4-oxo-tetrahydrofuran-3-ylcarbamoyl)-3,3-dimethyl-4-phenylbutyl]amide,
2-Methylfuran-3-carboxylic acid {1S-[2-(2-dimethylaminoethyl)-4-oxo-tetrahydrofuran-3-ylcarbamoyl]-3,3-dimethyl-4-phenylbutyl}amide,
2-Methylfuran-3-carboxylic acid [3,3-dimethyl-1S-(4-oxo-2-pyrrolidin-1-ylmethyl-tetrahydrofuran-3-ylcarbamoyl)-4-phenylbutyl]amide,
2-Methylfuran-3-carboxylic acid [3,3-dimethyl-1S-(2S-methyl-4-oxo-tetrahydrofuran-3S-ylcarbamoyl)-5-phenylpentyl]amide,
2-Methylfuran-3-carboxylic acid [3,3-dimethyl-1S-(2-ethyl-4-oxo-tetrahydrofuran-3-ylcarbamoyl)-5-phenylpentyl]amide,
2-Methylfuran-3-carboxylic acid [1S-(2-carbamoylmethyl-4-oxo-tetrahydrofuran-3-ylcarbamoyl)-3,3-dimethyl-5-phenylpentyl]amide,
2-Methylfuran-3-carboxylic acid {1S-[2-(2-dimethylaminoethyl)-4-oxo-tetrahydrofuran-3-ylcarbamoyl]-3,3-dimethyl-5-phenylpentyl}amide,
2-Methylfuran-3-carboxylic acid [3,3-dimethyl-1S-(4-oxo-2-pyrrolidin-1-ylmethyl-tetrahydrofuran-3-ylcarbamoyl)-5-phenylpentyl]amide,
1*H*-Pyrrole-3-carboxylic acid [3,3-dimethyl-1S-(2S-methyl-4-oxo-tetrahydrofuran-3S-ylcarbamoyl)butyl]amide,
1*H*-Pyrrole-3-carboxylic acid [3,3-dimethyl-1S-(2-ethyl-4-oxo-tetrahydrofuran-3-ylcarbamoyl)butyl]amide,
1*H*-Pyrrole-3-carboxylic acid [1S-(2-carbamoylmethyl-4-oxo-tetrahydrofuran-3-ylcarbamoyl)-3,3-dimethylbutyl]amide,
1*H*-Pyrrole-3-carboxylic acid {1S-[2-(2-dimethylaminoethyl)-4-oxo-tetrahydrofuran-3-ylcarbamoyl]-3,3-dimethylbutyl}amide,
1*H*-Pyrrole-3-carboxylic acid [3,3-dimethyl-1S-(4-oxo-2-pyrrolidin-1-ylmethyl-tetrahydrofuran-3-ylcarbamoyl)butyl]amide,
1*H*-Pyrrole-3-carboxylic acid [4-methyl-1S-(2S-methyl-4-oxo-tetrahydrofuran-3S-ylcarbamoyl)pentyl]amide,
1*H*-Pyrrole-3-carboxylic acid [4-methyl-1S-(2-ethyl-4-oxo-tetrahydrofuran-3-ylcarbamoyl)pentyl]amide,
1*H*-Pyrrole-3-carboxylic acid [1S-(2-carbamoylmethyl-4-oxo-tetrahydrofuran-3-ylcarbamoyl)-4-methylpentyl]amide,
1*H-*Pyrrole-3-carboxylic acid {1S-[2-(2-dimethylaminoethyl)-4-oxo-tetrahydrofuran-3-ylcarbamoyl]-4-methylpentyl}amide,
1*H*-Pyrrole-3-carboxylic acid [4-methyl-1S-(4-oxo-2-pyrrolidin-1-ylmethyl-tetrahydrofuran-3-ylcarbamoyl)pentyl]amide,
1*H*-Pyrrole-3-carboxylic acid [3,3-dimethyl-1S-(2S-methyl-4-oxo-tetrahydrofuran-3S-ylcarbamoyl)pentyl]amide,
1*H*-Pyrrole-3-carboxylic acid [3,3-dimethyl-1S-(2-ethyl-4-oxo-tetrahydrofuran-3-ylcarbamoyl)pentyl]amide,
1*H*-Pyrrole-3-carboxylic acid [1S-(2-carbamoylmethyl-4-oxo-tetrahydrofuran-3-ylcarbamoyl)-3,3-dimethylpentyl]amide,
1*H*-Pyrrole-3-carboxylic acid {1S-[2-(2-dimethylaminoethyl)-4-oxo-tetrahydrofuran-3-ylcarbamoyl]-3,3-dimethylpentyl}amide,
1*H*-Pyrrole-3-carboxylic acid [3,3-dimethyl-1S-(4-oxo-2-pyrrolidin-1-ylmethyl-tetrahydrofuran-3-ylcarbamoyl)pentyl]amide,
1*H*-Pyrrole-3-carboxylic acid [3,3,4-trimethyl-1S-(2S-methyl-4-oxo-tetrahydrofuran-3S-ylcarbamoyl)pentyl]amide,
1*H*-Pyrrole-3-carboxylic acid [3,3,4-trimethyl -1S-(2-ethyl-4-oxo-tetrahydrofuran-3-ylcarbamoyl)pentyl]amide,
1*H*-Pyrrole-3-carboxylic acid [1S-(2-carbamoylmethyl-4-oxo-tetrahydrofuran-3-ylcarbamoyl)-3,3,4-trimethylpentyl]amide,
1*H*-Pyrrole-3-carboxylic acid {1S-[2-(2-dimethylaminoethyl)-4-oxo-tetrahydrofuran-3-ylcarbamoyl]-3,3,4-trimethylpentyl}amide,
1*H*-Pyrrole-3-carboxylic acid [3,3,4-trimethyl-1S-(4-oxo-2-pyrrolidin-1-ylmethyl-tetrahydrofuran-3-ylcarbamoyl)pentyl]amide,
1*H*-Pyrrole-3-carboxylic acid [3,4-dimethyl-1S-(2S-methyl-4-oxo-tetrahydrofuran-3S-ylcarbamoyl)pentyl]amide,
1*H*-Pyrrole-3-carboxylic acid [3,4-dimethyl-1S-(2-ethyl-4-oxo-tetrahydrofuran-3-ylcarbamoyl)pentyl]amide,
1*H*-Pyrrole-3-carboxylic acid [1S-(2-carbamoylmethyl-4-oxo-tetrahydrofuran-3-ylcarbamoyl)-3,4-dimethylpentyl]amide,
1*H*-Pyrrole-3-carboxylic acid {1S-[2-(2-dimethylaminoethyl)-4-oxo-tetrahydrofuran-3-ylcarbamoyl]-3,4-dimethylpentyl}amide,
1*H*-Pyrrole-3-carboxylic acid [3,9-dimethyl-1S-(4-oxo-2-pyrrolidin-1-ylmethyl-tetrahydrofuran-3-ylcarbamoyl)pentyl]amide,
1*H*-Pyrrole-3-carboxylic acid [4,5-dimethyl-1S-(2S-methyl-4-oxo-tetrahydrofuran-3S-ylcarbamoyl)hexyl]amide,
1*H*-Pyrrole-3-carboxylic acid [4,5-dimethyl-1S-(2-ethyl-4-oxo-tetrahydrofuran-3-ylcarbamoyl)hexyl] amide,
1*H*-Pyrrole-3-carboxylic acid [1S-(2-carbamoylmethyl-4-oxo-tetrahydrofuran-3-ylcarbamoyl)-4,5-dimethylhexyl]amide,
1*H*-Pyrrole-3-carboxylic acid {1S-[2-(2-dimethylaminoethyl)-4-oxo-tetrahydrofuran-3-ylcarbamoyl]-4,5-dimethylhexyl}amide,
1*H*-Pyrrole-3-carboxylic acid [4,5-dimethyl-15-(4-oxo-2-pyrrolidin-1-ylmethyl-tetrahydrofuran-3-ylcarbamoyl)hexyl]amide,
1*H*-Pyrrole-3-carboxylic acid [3-methyl-1S-(2S-methyl-4-oxo-tetrahydrofuran-3S-ylcarbamoyl)-3-phenylbutyl]amide,
1*H*-Pyrrole-3-carboxylic acid [3-methyl-1S-(2-ethyl-4-oxo-tetrahydrofuran-3-ylcarbamoyl)-3-phenylbutyl]amide,
1*H*-Pyrrole-3-carboxylic acid [1S-(2-carbamoylmethyl-4-oxo-tetrahydrofuran-3-ylcarbamoyl)-3-methyl-3-phenylbutyl]amide,
1*H*-Pyrrole-3-carboxylic acid {1S-[2-(2-dimethylaminoethyl)-4-oxo-tetrahydrofuran-3-ylcarbamoyl]-3-methyl-3-phenylbutyl}amide,
1*H*-Pyrrole-3-carboxylic acid [3-methyl-1S-(4-oxo-2-pyrrolidin-1-ylmethyl-tetrahydrofuran-3-ylcarbamoyl)-3-phenylbutyl]amide,
1*H*-Pyrrole-3-carboxylic acid [3,3-dimethyl-1S-(2S-methyl-4-oxo-tetrahydrofuran-3S-ylcarbamoyl)-4-phenylbutyl]amide,
1*H*-Pyrrole-3-carboxylic acid [3,3-dimethyl-1S-(2-ethyl-4-oxo-tetrahydrofuran-3-ylcarbamoyl)-4-phenylbutyl]amide,
*1H*-Pyrrole-3-carboxylic acid [1S-(2-carbamoylmethyl-4-oxo-tetrahydrofuran-3-ylcarbamoyl)-3,3-dimethyl-4-phenylbutyl]amide,
1*H*-Pyrrole-3-carboxylic acid {1S-[2-(2-dimethylaminoethyl)-4-oxo-tetrahydrofuran-3-ylcarbamoyl]-3,3-dimethyl-4-phenylbutyl}amide,
1*H*-Pyrrole-3-carboxylic acid [3,3-dimethyl-1S-(4-oxo-2-pyrrolidin-1-ylmethyl-tetrahydrofuran-3-ylcarbamoyl)-4-phenylbutyl]amide,
1*H*-Pyrrole-3-carboxylic acid [3,3-dimethyl-1S-(2S-methyl-4-oxo-tetrahydrofuran-3S-ylcarbamoyl)-5-phenylpentyl]amide,
1*H*-Pyrrole-3-carboxylic acid [3,3-dimethyl-1S-(2-ethyl-4-oxo-tetrahydrofuran-3-ylcarbamoyl)-5-phenylpentyl]amide,
1*H*-Pyrrole-3-carboxylic acid [1S-(2-carbamoylmethyl-4-oxo-tetrahydrofuran-3-ylcarbamoyl)-3,3-dimethyl-5-phenylpentyl]amide,
1*H*-Pyrrole-3-carboxylic acid {1S-[2-(2-dimethylaminoethyl)-4-oxo-tetrahydrofuran-3-ylcarbamoyl]-3,3-dimethyl-5-phenylpentyl}amide,
1*H*-Pyrrole-3-carboxylic acid [3,3-dimethyl-1S-(4-oxo-2-pyrrolidin-1-ylmethyl-tetrahydrofuran-3-ylcarbamoyl)-5-phenylpentyl]amide,
*N*-[3,3-dimethyl-1S-(2S-methyl-4-oxo-tetrahydrofuran-3S-ylcarbamoyl)butyl]benzamide,
*N*-[3,3-dimethyl-1S-(2-ethyl-4-oxo-tetrahydrofuran-3-ylcarbamoyl)butyl]benzamide,
*N-*[1S-(2-carbamoylmethyl-4-oxo-tetrahydrofuran-3-ylcarbamoyl)-3,3-dimethylbutyl]benzamide,
*N*-{1S-[2-(2-dimethylaminoethyl)-4-oxo-tetrahydrofuran-3-ylcarbamoyl]-3,3-dimethylbutyl}benzamide,
*N*-[3,3-dimethyl-1S-(4-oxo-2-pyrrolidin-l-ylmethyl-tetrahydrofuran-3-ylcarbamoyl)butyl]benzamide,
*N*-[4-methyl-1S-(2S-methyl-4-oxo-tetrahydrofuran-3S-ylcarbamoyl)pentyl]benzamide,
*N*-[4-methyl-1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-4-methylpentyl]benzamide,
*N-*{1S-[2-(2-dimethylaminoethyl)-4-oxo-tetrahydrofuran-3-ylcarbamoyl]-4-methylpentyl}benzamide,
*N*-[4-methyl-1S-(4-oxo-2-pyrrolidin-1-ylmethyl-tetrahydrofuran-3-ylcarbamoyl)pentyl]benzamide,
*N*-[3,3-dimethyl-1S-(2S-methyl-4-oxo-tetrahydrofuran-3S-ylcarbamoyl)pentyl]benzamide,
*N*-[3,3-dimethyl-1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-3,3-dimethylpentyl]benzamide,
*N*-{1S-[2-(2-dimethylaminoethyl)-4-oxo-tetrahydrofuran-3-ylcarbamoyl]-3,3-dimethylpentyl}benzamide,
*N*-[3,3-dimethyl-1S-(4-oxo-2-pyrrolidin-1-ylmethyl-tetrahydrofuran-3-ylcarbamoyl)pentyl]benzamide,
*N*-[3,3,4-trimethyl-1S-(2S-methyl-4-oxo-tetrahydrofuran-3S-ylcarbamoyl)pentyl]benzamide,
*N*-[3,3,4-trimethyl -1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-3,3,4-trimethylpentyl]benzamide,
*N*-{2S-[2-(2-dimethylaminoethyl)-4-oxo-tetrahydrofuran-3-ylcarbamoyl]- 3,3,4-trimethylpentyl}benzamide,
*N*-[3,3,4-trimethyl-1S-(4-oxo-2-pyrrolidin-1-ylmethyl-tetrahydrofuran-3-ylcarbamoyl)pentyl]benzamide,
*N*-[3,4-dimethyl-1S-(2S-methyl-4-oxo-tetrahydrofuran-3S-ylcarbamoyl)pentyl]benzamide,
*N*-[3,4-dimethyl-1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-3,4-dimethylpentyl]benzamide,
*N*-{1S-[2-(2-dimethylaminoethyl)-4-oxo-tetrahydrofuran-3-ylcarbamoyl]-3,4-dimethylpentyl}benzamide,
*N*-[3,4-dimethyl-1S-(4-oxo-2-pyrrolidin-1-ylmethyl-tetrahydrofuran-3-ylcarbamoyl)pentyl]benzamide,
*N*-[4,5-dimethyl-1S-(2S-methyl-4-oxo-tetrahydrofuran-3S-ylcarbamoyl)hexyl]benzamide,
*N*-[4,5-dimethyl-1S-(2-ethyl-4-oxo-tetrahydrofuran-3-ylcarbamoyl)hexyl]benzamide,
*N*-[1S-(2-carbamoylmethyl-4-oxo-tetrahydrofuran-3-ylcarbamoyl)-4,5-dimethylhexyl]benzamide,
*N*-{1S-[2-(2-dirnethylaminoethyl)-4-oxo-tetrahydrofuran-3-ylcarbamoyl]-4,5-dimethylhexyl}benzamide,
*N-*[4,5-dimethyl-1S-(4-oxo-2-pyrrolidin-1-ylmethyl-tetrahydrofuran-3-ylcarbamoyl)hexyl]benzamide,
*N*-[3-methyl-1S-(2S-methyl-4-oxo-tetrahydrofuran-3S-ylcarbamoyl)-3-phenylbutyl]benzamide,
*N*-[3-methyl-1S-(2-ethyl-4-oxo-tetrahydrofuran-3-ylcarbamoyl)-3-phenylbutyl]benzamide,
*N*-[1S-(2-carbamoylmethyl-4-oxo-tetrahydrofuran-3-ylcarbamoyl)-3-methyl-3-phenylbutyl]benzamide,
*N*-{1S-[2-(2-dimethylaminoethyl)-4-oxo-tetrahydrofuran-3-ylcarbamoyl]-3-methyl-3-phenylbutyl}benzamide,
*N*-[3-methyl-1S-(4-oxo-2-pyrrolidin-1-ylmethyl-tetrahydrofuran-3-ylcarbamoyl)-3-phenylbutyl]benzamide,
*N*-[3,3-dimethyl-1S-(2S-methyl-4-oxo-tetrahydrofuran-3S-ylcarbamoyl)-4-phenylbutyl]benzamide,
*N*-[3,3-dimethyl-1S-(2-ethyl-4-oxo-tetrahydrofuran-3-ylcarbamoyl)-4-phenylbutyl]benzamide,
*N*-[1S-(2-carbamoylmethyl-4-oxo-tetrahydrofuran-3-ylcarbamoyl)-3,3-dimethyl-4-phenylbutyl]benzamide,
*N*-{1S-[2-(2-dimethylaminoethyl)-4-oxo-tetrahydrofuran-3-ylcarbamoyl]-3,3-dimethyl-4-phenylbutyl}benzamide,
*N*-[3,3-dimethyl-1S-(4-oxo-2-pyrrolidin-1-ylmethyl-tetrahydrofuran-3-ylcarbamoyl)-4-phenylbutyl]benzamide,
*N*-[3,3-dimethyl-1S-(2S-methyl-4-oxo-tetrahydrofuran-3S-ylcarbamoyl)-5-phenylpentyl]benzamide,
*N*-[3,3-dimethyl-1S-(2-ethyl-4-oxo-tetrahydrofuran-3-ylcarbamoyl)-5-phenylpentyl]benzamide,
*N*-[1S-(2-carbamoylmethyl-4-oxo-tetrahydrofuran-3-ylcarbamoyl)-3,3-dimethyl-5-phenylpentyl]benzamide,
*N*-{1S-[2-(2-dimethylaminoethyl)-4-oxo-tetrahydrofuran-3-ylcarbamoyl]-3,3-dimethyl-5-phenylpentyl}benzamide,
*N*-[3,3-dimethyl-1S-(4-oxo-2-pyrrolidin-1-ylmethyl-tetrahydrofuran-3-ylcarbamoyl)-5-phenylpentyl]benzamide,
Morpholine-4-carboxylic acid [3,3-dimethyl-1S-(2S-methyl-4-oxo-tetrahydrofuran-3S-ylcarbamoyl)butyl]amide,
Morpholine-4-carboxylic acid [3,3-dimethyl-1S-(2-ethyl-4-oxo-tetrahydrofuran-3-ylcarbamoyl)butyl]amide,
Morpholine-4-carboxylic acid [1S-(2-carbamoylmethyl-4-oxo-tetrahydrofuran-3-ylcarbamoyl)-3,3-dimethylbutyl]amide,
Morpholine-4-carboxylic acid {1S-[2-(2-dimethylaminoethyl)-4-oxo-tetrahydrofuran-3-ylcarbamoyl]-3,3-dimethylbutyl}amide,
Morpholine-4-carboxylic acid [3,3-dimethyl-1S-(4-oxo-2-pyrrolidin-1-ylmethyl-tetrahydrofuran-3-ylcarbamoyl)butyl]amide,
Morpholine-4-carboxylic acid [4-methyl-1S-(2S-methyl-4-oxo-tetrahydrofuran-3S-ylcarbamoyl)pentyl]amide,
Morpholine-4-carboxylic acid [4-methyl-1S-(2-ethyl-4-oxo-tetrahydrofuran-3-ylcarbamoyl)pentyl]amide,
Morpholine-4-carboxylic acid [1S-(2-carbamoylmethyl-4-oxo-tetrahydrofuran-3-ylcarbamoyl)-4-methylpentyl]amide,
Morpholine-4-carboxylic acid {1S-[2-(2-dimethylaminoethyl)-4-oxo-tetrahydrofuran-3-ylcarbamoyl]-4-methylpentyl}amide,
Morpholine-4-carboxylic acid [4-methyl-1S-(4-oxo-2-pyrrolidin-1-ylmethyl-tetrahydrofuran-3-ylcarbamoyl)pentyl]amide,
Morpholine-4-carboxylic acid [3,3-dimethyl-2S-(2S-methyl-4-oxo-tetrahydrofuran-3S-ylcarbamoyl)pentyl]amide,
Morpholine-4-carboxylic acid [3,3-dimethyl-1S-(2-ethyl-4-oxo-tetrahydrofuran-3-ylcarbamoyl)pentyl]amide,
Morpholine-4-carboxylic acid [1S-(2-carbamoylmethyl-4-oxo-tetrahydrofuran-3-ylcarbamoyl)-3,3-dimethylpentyl]amide,
Morpholine-4-carboxylic acid {1S-[2-(2-dimethylaminoethyl)-4-oxo-tetrahydrofuran-3-ylcarbamoyl]-3,3-dimethylpentyl}amide,
Morpholine-4-carboxylic acid [3,3-dimethyl-1S-(4-oxo-2-pyrrolidin-1-ylmethyl-tetrahydrofuran-3-ylcarbamoyl)pentyl]amide,
Morpholine-4-carboxylic acid [3,3,4-trimethyl-1S-(2S-methyl-4-oxo-tetrahydrofuran-3S-ylcarbamoyl)pentyl]amide,
Morpholine-4-carboxylic acid [3,3,4-trimethyl -1S-(2-ethyl-4-oxo-tetrahydrofuran-3-ylcarbamoyl)pentyl]amide,
Morpholine-4-carboxylic acid [1S-(2-carbamoylmethyl-4-oxo-tetrahydrofuran-3-ylcarbamoyl)-3,3,4-trimethylpentyl]amide,
Morpholine-4-carboxylic acid {1S-[2-(2-dimethylaminoethyl)-4-oxo-tetrahydrofuran-3-ylcarbamoyl)-3,3,4-trimethylpentyl}amide,
Morpholine-4-carboxylic acid [3,3,4-trimethyl-1S-(4-oxo-2-pyrrolidin-1-ylmethyl-tetrahydrofuran-3-ylcarbamoyl)pentyl]amide,
Morpholine-4-carboxylic acid [3,4-dimethyl-1S-(2S-methyl-4-oxo-tetrahydrofuran-3S-ylcarbamoyl)pentyl]amide,
Morpholine-4-carboxylic acid [3,4-dimethyl-1S-(2-ethyl-4-oxo-tetrahydrofuran-3-ylcarbamoyl)pentyl]amide,
Morpholine-4-carboxylic acid [1S-(2-carbamoylmethyl-4-oxo-tetrahydrofuran-3-ylcarbamoyl)-3,4-dimethylpentyl]amide,
Morpholine-4-carboxylic acid {1S-[2-(2-dimethylaminoethyl)-4-oxo-tetrahydrofuran-3-ylcarbamoyl]-3,4-dimethylpentyl}amide,
Morpholine-4-carboxylic acid [3,4-dimethyl-1S-(4-oxo-2-pyrrolidin-1-ylmethyl-tetrahydrofuran-3-ylcarbamoyl)pentyl]amide,
Morpholine-4-carboxylic acid [4,5-dimethyl-1S-(2S-methyl-4-oxo-tetrahydrofuran-3S-ylcarbamoyl)hexyl]amide,
Morpholine-4-carboxylic acid [4,5-dimethyl-1S-(2-ethyl-4-oxo-tetrahydrofuran-3-ylcarbamoyl)hexyl]amide,
Morpholine-4-carboxylic acid [1S-(2-carbamoylmethyl-4-oxo-tetrahydrofuran-3-ylcarbamoyl)-4,5-dimethylhexyl]amide, Morpholine-4-carboxylic acid {1S-[2-(2-dimethylaminoethyl)-4-oxo-tetrahydrofuran-3-ylcarbamoyl]-4,5-dimethylhexyl}amide,
Morpholine-4-carboxylic acid [4,5-dimethyl-1S-(4-oxo-2-pyrrolidin-1-ylmethyl-tetrahydrofuran-3-ylcarbamoyl)hexyl]amide,
Morpholine-4-carboxylic acid [3-methyl-1S-(2S-methyl-4-oxo-tetrahydrofuran-3S-ylcarbamoyl)-3-phenylbutyl]amide, Morpholine-4-carboxylic acid [3-methyl-1S-(2-ethyl-4-oxo-tetrahydrofuran-3-ylcarbamoyl)-3-phenylbutyl]amide, Morpholine-4-carboxylic acid [1S-(2-carbamoylmethyl-4-oxo-tetrahydrofuran-3-ylcarbamoyl)-3-methyl-3-phenylbutyl]amide,
Morpholine-4-carboxylic acid {1S-[2-(2-dimethylaminoethyl)-4-oxo-tetrahydrofuran-3-ylcarbamoyl]-3-methyl-3-phenylbutyl}amide,
Morpholine-4-carboxylic acid [3-methyl-1S-(4-oxo-2-pyrrolidin-1-ylmethyl-tetrahydrofuran-3-ylcarbamoyl)-3-phenylbutyl]amide,
Morpholine-4-carboxylic acid [3,3-dimethyl-1S-(2S-methyl-4-oxo-tetrahydrofuran-3S-ylcarbamoyl)-4-phenylbutyl]amide, Morpholine-4-carboxylic acid [3,3-dimethyl-1S-(2-ethyl-4-oxo-tetrahydrofuran-3-ylcarbamoyl)-4-phenylbutyl]amide,
Morpholine-4-carboxylic acid [1S-(2-carbamoylmethyl-4-oxo-tetrahydrofuran-3-ylcarbamoyl)-3,3-dimethyl-4-phenylbutyl]amide,
Morpholine-4-carboxylic acid {1S-[2-(2-dimethylaminoethyl)-4-oxo-tetrahydrofuran-3-ylcarbamoyl]-3,3-dimethyl-4-phenylbutyl}amide,
Morpholine-4-carboxylic acid [3,3-dimethyl-1S-(4-oxo-2-pyrrolidin-1-ylmethyl-tetrahydrofuran-3-ylcarbamoyl)-4-phenylbutyl]amide,
Morpholine-4-carboxylic acid [3,3-dimethyl-1S-(2S-methyl-4-oxo-tetrahydrofuran-3S-ylcarbamoyl)-5-phenylpentyl]amide,
Morpholine-4-carboxylic acid [3,3-dimethyl-1S-(2-ethyl-4-oxo-tetrahydrofuran-3-ylcarbamoyl)-5-phenylpentyl]amide,
Morpholine-4-carboxylic acid [1S-(2-carbamoylmethyl-4-oxo-tetrahydrofuran-3-ylcarbamoyl)-3,3-dimethyl-5-phenylpentyl]amide,
Morpholine-4-carboxylic acid {1S-[2-(2-dimethylaminoethyl)-4-oxo-tetrahydrofuran-3-ylcarbamoyl]-3,3-dimethyl-5-phenylpentyl}amide,
Morpholine-4-carboxylic acid [3,3-dimethyl-1S-(4-oxo-2-pyrrolidin-1-ylmethyl-tetrahydrofuran-3-ylcarbamoyl)-5-phenylpentyl]amide,
and pharmaceutically acceptable salts thereof.

Example molecules prepared using the general chemistries outlined above and by the methods detailed in the experimental are shown in Tables 1 and 2. Judicial combination of R1, R3 and R5 substituents in general formula (II) yields potent and selective inhibitors of cathepsin S e.g. Furan-3-carboxylic acid [3,3,4-trimethyl -1S-(2S-ethyl-4-oxo-tetrahydrofuran-3-ylcarbamoyl)pentyl]amide: 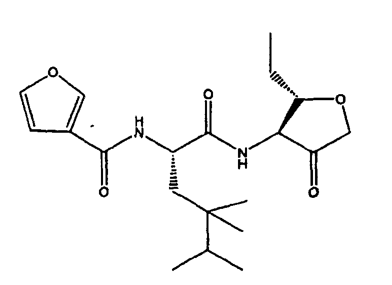 Ki mammalian cath S (15nM), murine cath S (149nM) rat cath S (271nM), cathepsin L (> 100µM), cathepsin K (5.5µM). Molecules may be chosen which show a range of activities for mammalian, murine and rat cathepsin S (see Table 2) which may exemplify many facets of an inhibitor development programme e.g. activities in murine or mammalian cell-based assays, dosing of species for disease-related animal models etc.

Molecules of general formula (II) have the potential for good oral bioavailability e.g. 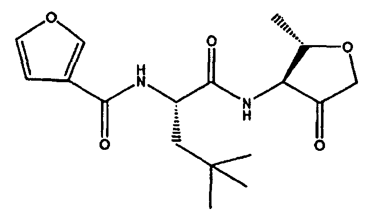 Furan-3-carboxylic acid [3,3-dimethyl-1S-(2S-methyl-4-oxo-tetrahydrofuran-3S-ylcarbamoyl)butyl]amide dosed i.v. and orally at 10mg / kg to mice gave an oral bioavailability of % *(F)* 58.

### Experimental Section

### Solution Phase Chemistry

### Example 1. Furan-3-carboxylic acid [3,3-dimethyl-1S-(2S-methyl-4-oxo-tetrahydrofuran-3S-ylcarbamoyl) butyl] amide (4)

### Following general chemistry scheme 8

### (a) General method for the synthesis of N-Boc protected diazoketones, exemplified by (2S, 3S)-N-Boc-O-t-butyl-L-threonyldiazomethane (1)

(2S, 3S)- N-Boc-O-t-butyl-L-threonine (1.2g, 4.2mmol) was dissolved in dry DCM (20mL) and N-methylmorpholine (1mL, 2.2eq) added. The reaction mixture was cooled to -15°C and stirred under an atmosphere of argon. Isobutyl chloroformate (0.56mL, 4.3mmol) was added and the mixture stirred for 10mins at -15°C. A solution of diazomethane in diethyl ether (45mL, approx 40mmol) was added and the reaction allowed to warm to room temperature over 1hr, then acetic acid was added dropwise until effervescence had ceased. The reaction mixture was diluted with DCM (100mL) and washed successively with saturated aqueous sodium bicarbonate (2 x 75mL), water (75mL) and brine (75mL) and dried over sodium sulphate. The solvent was removed *in vacuo* to give crude (2S, 3S)-N-Boc-O-t-butyl-L-threonyldiazomethane (1.2g, ~100%) as a pale yellow oil. The above synthesis was repeated 9 times and the total crude product pooled (12g) and used without purification for the next stage.

### (b) General method for the synthesis of Boc-3(2H)-furanones, exemplified by dihydro-(4S-amino-[N-Boc])-5S-methyl-3(2H)-furanone (2)

A solution of lithium chloride (13.6g, 320mmol) in 80% aqueous acetic acid (400mL) was cooled to 5°C and added to crude (2S, 3S)-N-Boc-O-t-butyl-L-threonyldiazomethane (1) (9.6g) with stirring. The oil dissolved over 10mins and stirring continued for a further 1hr slowly warming to room temperature, with evolution of gas. The solvents were removed in vacuo and the residue taken into EtOAc (250mL) and washed successively with water (250mL), saturated aqueous sodium bicarbonate (2 x 100mL) and brine (75mL), then dried over sodium sulphate. The solvent was removed *in vacuo* and the crude product purified by flash chromatography over silica gel (150g) eluting with EtOAc / heptane (1:2, v/v). Two fractions were pooled and the quicker eluting fraction reduced *in vacuo* to approx 50mL heptane and left to crystallise to give dihydro-(4S-amino-[N-Boc])-5S-methyl-3(2H)-furanone (2) as a white solid, yield 4.05g, 18.8mmol, 58%. Electrospray-MS m/z 216 (MH⁺), 160 (MH⁺ - 56), elemental analysis C₁₀H₁₇O₄N (req) %C 55.80, %H 7.96, %N 6.51, (fnd) %C 55.82, %H 7.86, %N 6.44. δ_{H} (500 MHz; CDCl₃) ; 1.41 (9H, s, C(CH₃)₃), 1.49 (3H, d, *J* 6, 5S-CH₃), 3.72 (1H, bm, furanone CHα), 3.90-4.02 (2H, 5S-H + 1 x furanone COCH₂O), 4.22 (1H, d, *J* 17.4, 1 x furanone COCH₂O), 4.85 (1H, bs, furanone, NH). δ_{c} (125 MHz; CDCl₃) ; 19.34 (5S-CH₃) , 28.45 (C(CH₃)₃), 62.79 (furanone CHα), 71.06 (furanone COCH₂O), 77.96 (5S-CHCH₃), 80.88 (C(CH₃)₃), 155.6 ( (CH₃)₃ CO-CO), 212.6 (furanone CO).

### (c) General method for N-terminal extension, exemplified by dihydro-(4S-amino-[N-Boc-L-tert-butylalanyl])-5S-methyl-3(2H)-furanone (3)

Dihydro-(4S-amino-[N-Boc])-5S-methyl-3(2H)-furanone **(2)** (1.0g, 4.6mmol) was treated with a solution of 4.0M HCl in dioxan (25mL) at room temperature for 1hr. The solvents were removed *in vacuo* and the residue azeotroped with 2 x toluene to give the hydrochloride salt as a white solid. Boc-L-*tert*-butylalanine pentafluorophenyl ester (2.0g, 1.05eq) and 1-hydroxybenzotriazole hydrate (0.735g, 1.05eq) were dissolved in DMF (20mL) and after 5mins added to the above salt. The clear solution was then treated with N-methylmorpholine (0.51g, 0.56mL, 1.1eq) and left at room temperature for 2hrs. The solvents were removed *in vacuo* and the crude product purified by flash chromatography over silica gel (50g) eluting with EtOAc / heptane (1:3, v/v), then EtOAc / heptane (1:2, v/v). Fractions were pooled and reduced *in vacuo* to give dihydro-(4S-amino-[N-Boc-L-*tert*-butylalanyl])-5S-methyl-3(2H)-furanone **(3)** as a white solid, yield 1.31g, 3.82mmol, 83%. Electrospray-MS m/z 343 (MH⁺), 287 (MH⁺ - 56).

### (d) General method for addition of capping group, exemplified by Furan-3-carboxylic acid [3,3-dimethyl-1S-(2S-methyl-4-oxo-tetrahydrofuran-3S-ylcarbamoyl)butyl]amide (4)

Dihydro-(4S-amino-[N-Boc-L-*tert*-butylalanyl])-5S-methyl-3(2H)-furanone **(3)** (1.03g, 3.0mmol)) was treated with a solution of 4.0M HCl in dioxan (25mL) at room temperature for 1hr. The solvents were removed *in vacuo* and the residue azeotroped with 2 x toluene to give the hydrochloride salt as a white solid.

Furan-3-carboxypentafluorophenyl ester (0.88g, 1.05eq) and 1-hydroxybenzotriazole hydrate (0.48g, 1.05eq) were dissolved in DMF (15mL) and after 5mins added to the above salt. The clear solution was then treated with N-methylmorpholine (0.33g, 0.36mL, 1.1eq) and left at room temperature for 2hrs, producing a dark solution. The solvents were removed *in vacuo* and the crude product purified by flash chromatography over silica gel (50g) eluting with EtOAc / heptane (3:2, v/v). Fractions were pooled and reduced *in vacuo* to give Furan-3-carboxylic acid [3,3-dimethyl-1S-(2S-methyl-4-oxo-tetrahydrofuran-3S-ylcarbamoyl)butyl]amide **(4)** as a pale tan solid, yield 0.45g, 1.35mmol, 45%. Electrospray-MS m/z 337 (MH⁺). Analytical HPLC Rt = 10.29mins (97.3%), HRMS C₁₇H₂₄O₅N₂Na requires M, 359.1583, found: MNa⁺, 359.1573. (δ - 2.85 ppm), elemental analysis C₁₇H₂₄O₅N₂ (req) %C 60.70, %H 7.19, %N 8.32, (fnd) %C 60.08, %H 7.07, %N 8.17.
δ_{H} (500 MHz; CDCl₃); 0.93 (9H, s, C(CH₃)₃), 1.35 (3H, d, *J* 6, 5S-CH₃), 1.68/1.85 (2H, m, CHCH₂C(CH₃)₃), 3.80 (1H, t, *J* 8.2, furanone CHα), 4.08 / 4.15 (2H, d, *J* 17.0, furanone COCH₂O), 4.10 (1H, bm, 5S-H), 4.75 (1H, m, *tert*-BuAla CHα), 6.59 (1H, d, *J* 1.6, furan H4), 7.35 (1H, t, *J* 1.2, furan H5), 7.87 (1H, s, furan H2), 7.95 (1H, d, *J* 7.2, tert-BuAla, NH), 8.35 (1H, d, *J* 7.4, furanone, NH). δ_{c} (125 MHz; CDCl₃); 18.67 (5S-CH₃), 29.36 (C(CH₃)₃), 30.40 (C(CH₃)₃), 44.52 (CHCH₂C(CH₃)₃), 50.93 (*tert*-BuAla CHα), 61.11 (furanone CHα), 70.92 (furanone COCH₂O), 75.76 (5S-CHCH₃), 108.4 (furan C4), 121.5 (furan C3), 143.2 (furan C2 or 5), 145.2 (furan C2 or 5), 162.5 (furan -3-CO), 174.2 (*tert*-BuAla CO), 210.9 (furanone CO).

### Example 2. 4,4-Dimethyl-2S-(furan-3-sulfonylamino)pentanoic acid (2S-methyl-4-oxo-tetrahydrofuran-3S-yl)amide (5)

### (a) General method for addition of sulphonyl capping group, exemplified by 4,4-Dimethyl-2S-(furan-3-sulfonylamino)pentanoic acid (2S-methyl-4-oxo-tetrahydrofuran-3S-yl)amide (5)

Dihydro-(4S-amino-(N-Boc-L-*tert*-butylalanyl])-5S-methyl-3(2H)-furanone **(3)** (34mg, 0.1mmol) was treated with a solution of 4.0M HCl in dioxan (5mL) at room temperature for 1hr. The solvents were removed *in vacuo* and the residue azeotroped with 2 x toluene to give the hydrochloride salt as a white solid.

Hydrochloride salt was dissolved in dry DCM (2mL) and furan-3-sulphonylchloride (33mg, 0.2mmol) added followed by diisopropylethylamine (52µL, 3eq) and catalytic N,N-dimethylaminopyridine (2mg). After 2hr at room temperature, the solution was diluted with DCM (15mL) and washed successively with 0.1N HCl (25mL), water (2 x 25mL) and brine (25mL), then dried over sodium sulphate. The solvent was removed *in vacuo* and the crude product purified by flash chromatography over silica gel (15g) eluting with EtOAc / heptane (1:1, v/v). Fractions were pooled and reduced *in vacuo* to give 4,4-Dimethyl-2S-(furan-3-sulfonylamino)pentanoic acid (2S-methyl-4-oxo-tetrahydrofuran-3S-yl)amide **(5)** as a white solid, lyophilised from 0.1%aq TFA / acetonitrile, yield 14mg, 0.038mmol, 38%. Electrospray-MS m/z 373.2 (MH⁺).
Analytical HPLC Rt = 10.80mins (97.6%), HRMS C₁₆H₂₄O₆N₂SNa requires *M*, 395.1253, found: MNa⁺, 395.1251. (δ - 0.53 ppm), elemental analysis C₁₆H₂₄O₆N₂S .1/3 TFA (req) %C 48.78, %H 5.98, %N 6.83, (fnd) %C 48.47, %H 6.11, %N 6.75. δ_{H} (500 MHz; CDCl₃); 0.87 (9H, s, C((CH₃)₃), 1.40 (3H, d, *J* 6, 5S-CH₃), 1.45 (1H, q, CHCH₂C(CH₃)₃), 1.75 (1H, q, *J* 4.1, 10.4, CHCH₂C(CH₃)₃), 3.78 (1H, m, furanone CHα), 3.82 (1H, dt, *tert*-BuAla CHδ), 4.05 (2H, d, *J* 17.0, furanone COCH₂O + 5S-CHCH₃) , 4.20 (1H, d, *J* 17.0, furanone COCH₂O), 5. 62 (1H, d, *J* 7.2, tert-BuAla, NH), 6.66 (1H, d, *J* 1.6, furan H4), 6.82 (1H, d, *J* 7.2, furanone, NH), 7.48 (1H, t, *J* 1.2, furan H5), 8.05 (1H, m, furan H2).
δ_{c} (125 MHz; CDCl₃); 18.79 (5S-CH₃) , 29.20 (C(CH₃)₃), 30.35 (C(CH₃)₃), 46.20 (CHCH₂C(CH₃)₃), 54.48 (*tert*-BuAla CHα), 61.35 (furanone CHα), 70.71 (furanone COCH₂O), 75.00 (5S-CHCH₃), 108.2 (furan C4), 126.5 (furan C3), 144.7 (furan C2 or 5), 146.0 (furan C2 or 5), 172.95 (*tert*-BuAla CO), 211.03 (furanone CO).

### Example 3. 4,4-Dimethyl-2S-(thiophene-3-sulfonylamino)pentanoic acid (2S-methyl-4-oxo-tetrahydrofuran-3S-yl)amide (6)

Prepared as detailed above for compound **(5)**, but using thiophene-3-sulphonyl chloride, to give **(6)** as a pale pink solid, lyophilised from 0.1%aq TFA / acetonitrile, yield 30mg, 0.077mmol, 77%. Electrospray-MS m/z 389.2 (MH⁺). Analytical HPLC Rt = 11.64mins (96.8%), HRMS C₁₆H₂₄O₅N₂S₂Na requires *M*, 411.1024, found: MNa⁺, 411.1026. (δ+ 0.32 ppm), elemental analysis C₁₆H₂₄O₅N₂S₂ .1/4 TFA (req) %C 47.54, %H 5.86, %N 6.71, (fnd) %C 47.62, %H 5.98, %N 6.80. δ_{H} (500 MHz; CDCl₃); 0.79 (9H, s, C(CH₃)₃), 1. 40 (3H, d, *J* 6.1, 5S-CH₃), 1.45 (1H, q, CHCH₂C(CH₃)₃), 1.75 (1H, q, *J* 4.0, 14.3, CHCH₂C(CH₃)₃), 3.78 (2H, m, furanone CHα + *tert*-BuAla CHα), 4.06 / 4.19 (2H, d, *J* 17.3, furanone COCH₂O), 4.07 (1H, q, *J* 6.0, 5S-CHCH₃), 5.51 (1H, d, *J* 7.0, tert-BuAla, NH), 6.86 (1H, d, *J* 7.2, furanone, NH), 7.36 (1H, dd, *J* 1.3, 5.1, thiophene H4), 7.44 (1H, dd, *J* 5.1, 3.1, thiophene H5), 8.0 (1H, dd, *J* 3.1, 1.3, thiophene H2).
δ_{c} (125 MHz; CDCl₃); 18. 88 (5S-CH₃), 29.20 (C(CH₃)₃), 30.36 (C(CH₃)₃ ), 46.25 (CHCH₂C(CH₃)₃), 54.56 (*tert*-BuAla CHα), 61.40 (furanone CHα), 70.50 (furanone COCH₂O), 75.80 (5S-CHCH₃), 125.41, 128.17, 131.18 (thiophene C2 or 4 or 5), 138.82 (thiophene C3), 172.90 (tert-BuAla CO), 211.04 (furanone CO).

### Example 4. 4,4-Dimethyl-2S-(thiophene-2-sulfonylamino)pentanoic acid (2S-methyl-4-oxo-tetrahydrofuran-3S-yl)amide (7)

Prepared as detailed above for compound **(5)**, but using thiophene-2-sulphonyl chloride, to give **(7)** as a pale pink solid, lyophilised from 0.1%aq TFA / acetonitrile, yield 14mg, 0.036mmol, 36%. Electrospray-MS m/z 389.2 (MH⁺). Analytical HPLC Rt = 11.91mins (98.3%), HRMS C₁₆H₂₄O₅N₂S₂Na requires *M*, 411.1024, found: MNa⁺, 411.1015. (δ- 2.35 ppm), elemental analysis C₁₆H₂₄O₅N₂S₂ .1/2 TFA (req) %C 45.84, %H 5.54, %N 6.29, (fnd) %C 45.53, %H 5.61, %N 6.22. δ_{H} (500 MHz; CDCl₃) : 0.82 (9H, s, C(CH₃)₃), 1.41 (3H, d, *J* 6.0, 5S-CH₃), 1.45 (1H, dd, *J* 3.8, 14.7, CHCH₂C(CH₃)₃), 1.75 (1H, q, *J* 4.0, 14.3, CHCH₂C(CH₃)₃), 3.78 (1H, m, furanone CHα), 3.84 (1H, m, *tert*-BuAla CHα), 4.06 / 4.22 (2H, d, *J* 17.0, furanone COCH₂O), 4.07 (1H, m, 5S-CHCH₃), 5.42 (1H, d, *J* 6.8, *tert*-BuAla, NH), 6.72 (1H, d, *J* 7.0, furanone, NH), 7.10 (1H, q, *J* 3.8, 5.0, thiophene H4), 7.62 (1H, q, *J* 1.3, 5.0, thiophene H3 or H5), 7.66 (1H, q, *J* 1.3, 3.8, thiophene H3 or H5).
δ_{c} (125 MHz; CDCl₃); 18. 87 (5S-CH₃), 29.19 (C(CH₃)₃), 30.26 (C(CH₃)₃ ), 46.21 (CHCH₂C(CH₃)₃), 54.82 (*tert*-BuAla CHα), 61.43 (furanone CHα), 70.71 (furanone COCH₂O), 75.06 (5S-CHCH₃), 127.54, 132.49, 132.89 (thiophene C3 or 4 or 5), 139.49 (thiophene C3), 172.83 (tert-BuAla CO), 210.88 (furanone CO).

### General Synthesis of Chiral β-alkyl serine amino acids

Adapted from Blaskovich, M.A., Evinder, G., Rose, N. G. W., Wilkinson, S., Luo, Y. and Lajoie, G. A. *J. Org. Chem,* 63, 3631-3646, 1998. (Following scheme 2).

### Example 5. (2S, 3S)β-hydroxynorvaline (15)

### (a) N-Benzyloxycarbonyl-L-serine 3-methyl-3-(hydroxymethyl)oxetane ester (8)

N-Cbz-L-serine (10 g, 41.8 mmol) was dissolved in DCM (450 mL) and DMF (14 mL) and added dropwise over 2.5 h to a stirred solution of WSC. HCl (12 g, 62.7 mmol), N'N-dimethylaminopyridine (260 mg, 2.1 mmol) and 3-methyl-3-oxetane methanol (84 mL, 0.84 mmol) cooled to 0 °C. The reaction was warmed to room temperature and allowed to stir overnight. The mixture was washed with 0.1M HCl (200 mL), water (200 mL), 10 % Na₂CO₃ (200 mL x 2) and water (200mL x 2), dried (Na₂SO₄) and the solvent evaporated *in vacuo* to afford a pale yellow oil. Purification by column chromatography (4:1, EtOAc:heptane) and subsequent recrystallisation (1:1, EtOAc:heptane) yielded the target intermediate as a white crystalline solid, 8.07 g, 60 %; TLC (4:1, EtOAc:heptane), R*f* = 0.28, electrospray-MS m/z 324.1 (MH⁺).
δ_{H} (400 MHz; CDCl₃); 1.28 (3H, s, CH₃), 3.04 (1H, t, *J* 6.2, CHNH), 3.90-3.91 (1H, br m, OH), 4.10-4.13 (2H, m, CH₂OH), 4.41-4.55 (6H, m, 3 x CH₂), 5.13 (2H, s, OCH₂), 5.82 (1H, d, *J* 7.7, NH), 7.35-7.36 (5H, m, C₆H₅)·
δ_{c} (100 MHz; CDCl₃); 20.75 (CH₃), 39.67 (CH₂OH), 56.39 (CHNH), 63.37 (CH₂), 67.19 (CH₂), 68.94 (CH₂), 79.50 (OCH₂), 128.16 (C₆H₅), 128.27 (C₆H₅), 128.58 (C₆H₅), 136.14 (C₆H₅), 156.25 (CO₂NH), 170.74 (CO₂).

### (b) 1-[N-Benzyloxycarbonyl-(1S)-1-amino-2-hydroxyethyl]-4-methyl-2,6,7- trioxabicyclo[2.2.2]oxetane (9).

Compound **(8)** (10.23 g, 28.6 mmol) was dissolved in anhydrous DCM (150 mL) and cooled to 0 °C under N₂. A solution of boron trifluoride etherate (0.10 mL, 0.77 mmol) in anhydrous DCM (10 mL) was added and the mixture stirred for 30 minutes at 0 °C, then at room temperature overnight. Triethylamine (1.2 mL, 8.30 mmol) was added and the reaction mixture stirred for 30 minutes before being concentrated to a thick colourless oil.
Purification by column chromatography (4:1, EtOAc:heptane) and subsequent recrystallisation (1:1, EtOAc:heptane) yielded **(9)** as a white crystalline solid, 8.06 g, 80 %; TLC (4:1, EtOAc:heptane) R*f* = 0.27, electrospray-MS m/z 324.1 (MH⁺).
δ_{H} (400 MHz; CDCl₃); 0.78 (3H, s, CH₃), 2.67 (1H, m, CHNH), 3.64-3.69 (1H, m, CH₂OH), 3.80-3.83 (1H, m, CH₂OH), 3.88 (6H, s, CH₂ x 3), 5.09 (2H, dd, *J* 18.9, 12.3 , OCH₂), 5.38 (1H, d, *J* 8.7, NH), 7.26-7.34 (5H, m, C₆H₅). δ_{c} (100 MHz; CDCl₃); 14.11 (CH₃), 30.39 (CCH₃), 55.26 (CHNH), 61. 75 (CH₂OH), 66. 76 (CH₂O), 72.53 (CH₂ x 3), 108.29 (CO₃), 127.98 (C₆H₅), 128. 32 (C₆H₅), 136.31 (C₆H₅), 156.31 (CO₂NH).

### (c) 1-[N-Benzyloxycarbonyl-(1S)-1-amino-2-oxoethyl]-4-methyl-2,6,7- trioxabicyclo[2.2.2]oxetane (10).

Compound **(9)** (6.45 g, 20.0 mmol) was dissolved in anhydrous DCM (55 mL) under N₂ and cooled to -78 °C in flask 1. Oxalyl chloride (2.8 mL, 31.9 mmol) was added to anhydrous DCM (85 mL) in a separate flask (flask 2) under N₂ and cooled to -78 °C. Anhydrous dimethylsulphoxide (4.7 mL, 65.8 mmol) was added to the oxalyl chloride solution and the mixture stirred at -78 °C for 15 minutes. The alcohol solution was transferred over 20 minutes by cannula to flask 2 and rinsed with anhydrous DCM (35 mL). The resulting cloudy, white mixture was stirred for 1.5 hours at -78 °C. Diisopropylethylamine (17.4 mL, 99.7 mmol) was added and the solution stirred for 30 minutes at -78 °C and 10 minutes at 0 °C. Ice-cold DCM (140 mL) was added and the solution washed with ice-cold NH₄Cl (20 % saturated solution; 3 x 140 mL) and saturated NaCl (140 mL), dried (MgSO₄) and the solvent evaporated *in vacuo* to afford a yellow solid **(10)**, 5.08 g, 79 %; TLC (3:1, EtOAc:heptane), R*f* = 0.56, electrospray-MS m/z 322.1 (40%) (MH⁺), 340.2 (100%) (MH⁺+H₂O).
δ_{H} (400 MHz; CDCl₃); 0.82 (3H, s, CH₃), 3.93 (6H, s, CH₂ × 3), 4.60 (1H, d, *J* 8.8, CHNH), 5.12 (2H, dd, *J* 14.9, 12.4, OCH₂), 5.35 (1H, br d, *J* 8.0, NH), 7.30-7.36 (5H, m, C₆H₅), 9.68 (1H, s, HCO).
δ_{c} (100 MHz; CDCl₃); 14.25 (CH₃), 30.86 (CCH₃), 63.25 (CHNH), 67.22 (CH₂O), 72.88 (CH₂ x 3), 107.16 (CO₃), 128.13 (C₆H₅), 128.46 (C₆H₅), 136.13 (C₆H₅), 156.17 (CO₂NH), 195.66 (CHO).

### (d) 1-[N-(Benzyloxycarbonyl)-(1S,2R)-1-amino-2-hydroxybutyl]-4-methyl-2,6,7-trioxabicyclo[2.2.2]oxetane (11).

Compound **(10)** (2 g, 5.73 mmol) was dissolved in anhydrous DCM:Et₂O (1:1) under N₂. A solution of EtMgBr (3M solution in Et₂O; 7.6 mL, 22.9 mmol) was added quickly at - 78 °C and stirred vigorously. After 30 minutes the reaction was quenched by pouring into 5 % NH₄Cl (500 mL). DCM (500 mL) was added, the organic layer separated and washed with 3 % NH₄Cl (500 mL) and brine (500 mL), dried (Na₂SO₄) and the solvent evaporated *in vacuo* to afford a yellow oil. Purification by column chromatography (1:10, EtOAc:DCM) and subsequent recrystallisation (EtOAc:heptane) yielded a white crystalline solid **(11),** 1.32 g, 60 %; TLC (1:10, EtOAc:DCM) R*f* = 0.25, electrospray-MS m/z 352.2 (20%) (MH⁺), 370.3 (100%) (MH⁺+H₂O). δ_{H} (400 MHz; CDCl₃); 0.82 (3H, s, CH₃), 0.94 (3H, t, *J* 7.4, CH₂CH₃), 1.36-1.53 (2H, m, CH₂CH₃), 3.85 (1H, d, *J* 10.3, CH), 3.93 (6H, s, CH₂ x 3), 4.05 (1H, t, *J* 6.8, CH), 5.13-5.14 (2H, dd, *J* 16.4, 12.7, OCH₂), 5.32 (1H, d, *J* 10.2, NH), 7.30-7.36 (5H, m, C₆H₅).
δ_{c} (100 MHz; CDCl₃); 10.11 (CH₂CH₃), 14.34 (CH₃), 25.98 (CH₂CH₃) , 30.65 (CCH₃), 56.05 (CHOH) , 66.83 (CH₂O) , 70. 81 (CHNH) , 72.76 (CH₂ x 3), 108.93 (CO₃), 127.65 (C₆H₅) , 128.45 (C₆H₅), 136.65 (C₆H₅), 156.83 (CO₂NH).

### (e) 1-[N-Benzyloxycarbonyl-(1S)-1-amino-2-oxobutyl]-4-methyl-2,6,7- trioxabicyclo[2.2.2]oxetane (12).

Compound **(11)** (1.32 g, 3.8 mmol) was dissolved in anhydrous DCM (10 mL) under N₂ and cooled to -78 °C in flask 1. Oxalyl chloride (2M solution in DCM; 3 mL, 6.0 mmol) was diluted with anhydrous DCM (10 mL) in a separate flask (flask 2) under N₂ and cooled to -78 °C. Anhydrous dimethylsulphoxide (0.88 mL, 12.4 mmol) was added to the oxalyl chloride solution and the mixture stirred at -78 °C for 15 minutes. The alcohol solution was transferred over 20 minutes by cannula to flask-2 and rinsed with anhydrous DCM (10 mL). The resulting cloudy, white mixture was stirred for 2 hr 15 min at -78 °C. DIPEA (3.3 mL, 18.8 mmol) was added and the solution stirred for 30 minutes at - 78 °C and 10 minutes at 0 °C. Ice-cold DCM (25 mL) was added and the solution washed with ice-cold NH₄Cl (5 % saturated solution; 3 x 25 mL) and saturated NaCl (25 mL), dried (Na₂SO₄) and the solvent evaporated *in vacuo* to afford an orange oil. Purification by column chromatography (2:3, EtOAc:heptane) yielded a colourless oil **(12),** 556 mg, 45 %; TLC (2:3, EtOAc:heptane) R*f* = 0.25, electrospray-MS m/z 350.2 (60%) (MH⁺), 368.2 (100%) (MH⁺+H₂O).
δ_{H} (400 MHz; CDCl₃); 0.80 (3H, s, CH₃), 1.06 (3H, t, *J* 7.2, CH₂CH₃), 2.48-2.56 (1H, m, CHCH₃), 2.80-2.88 (1H, m, CHCH₃), 3.90 (6H, s, CH₂ × 3), 4.60 (1H, d, *J* 8.8, CHNH), 5.09 (2H, s, OCH₂), 5.66 (1H, d, *J* 8.5, NH), 7.30-7.35 (5H, m, C₆H₅). δ_{c} (100 MHz; CDCl₃); 7.53 (CH₂CH₃), 14.25 (CH₃), 30.57 (CCH₃), 35.74 (CH₂CH₃), 62.33 (CHNH), 67.05 (CH₂O), 72.94 (CH₂ x 3), 106-98 (CO₃), 128.10 (C₆H₅), 128.46 (C₆H₅), 136.31 (C₆H₅), 155.99 (CO₂NH).

### (f) 1-[N-(Benzyloxycarbonyl)-(1S,2S)-1-amino-2-hydroxybutyl]-4-methyl-2,6,7-trioxabicyclo[2.2.2]oxetane (13).

Compound **(12)** (2.77 g, 7.9 mmol) and LiBH4 (1.73 g, 79 mmol) were cooled to -78 °C under N2. A solution of DCM:CH30H (1.5:1; 332 mL cooled to -78 °C) was added and the solution stirred at -78 °C overnight. After being warmed to room temperature, the solution was poured into 5% NH4Cl solution (500 mL) and DCM (300 mL) added. The organic layer was separated, washed with 5 % NH4Cl solution (500 mL) and brine (400 mL), dried (Na2SO4) and the solvent evaporated in vacuo to afford a white solid **(13)**, 2.51 g, 90 %; TLC (1:1, EtOAc:heptane) Rf = 0.23, electrospray-MS m/z 352.2 (40%) (MH+), 370.3 (100%) (MH++H20).
δ_{H} (400 MHz; CDCl₃); 0.82 (3H, s, CH₃) , 0.97 (3H, t, *J* 7.4, CH₂CH₃), 1.44-1.45 (1H, m, CHCH₃), 1.63-1.68 (1H, m, CHCH₃), 3.44 (1H, d, *J* 4.0, CHOH), 3.66-3.69 (1H, m, CHNH), 3.92 (6H, s, CH₂ x 3), 5.04 (1H, d, *J* 9.8, NH), 5.16 (2H, d, *J* 6.1, OCH₂), 7.36 (5H, d, *J* 4.3, C₆H₅),
δ_{c} (100 MHz; CDCl₃); 9.79 (CH₂CH₃), 14.27 (CH₃), 26.10 (CH₂CH₃), 30.56 (CCH₃) , 57.57 (CHOH), 66.94 (CH₂O), 69.80 (CHNH), 72.66 (CH₂ x 3), 108.89 (CO₃), 128.06 (C₆H₅) , 128.47 (C₆H₅), 136.51 (C₆H₅), 156.49 (CO₂NH).

### (g) (1S,2S)-(1-amino-2-hydroxybutyl)-4-methyl-2,6,7-trioxabicyclo[2.2.2]oxetane (14).

Compound **(13)** (2.51g, 7.1mmol) was dissolved in ethanol (220 mL) and 10 % Pd/C (218 mg) added. The reaction mixture was stirred overnight in the presence of H₂. The catalyst was removed by filtration through celite and the solvent evaporated *in vacuo* to afford a thick oil. Purification by column chromatography (20:1, DCM:MeOH) yielded a pale yellow oil **(14)**, 1.24 g, 92 %, which crystallised on standing; TLC (5:1, DCM:MeOH) R*f* = 0.51, electrospray-MS m/z 218.1 (MH⁺). δ_{H} (400 MHz; CDCl₃); 0.83 (3H, s, CH₃), 0.98 (3H, t, *J* 7.4, CH₂CH₃), 1.38-1.48 (1H, m, CHCH₃), 1.71-1.78 (1H, m, CHCH₃), 2.77 (1H, d, *J* 7.1, CHNH₂), 3.62-3.66 (1H, m, CHOH), 3.93 (6H, s, CH₂ x 3).
δ_{c} (100 MHz; CDCl₃); 9.57 (CH₂CH₃), 14.37 (CH₃), 26.01 (CH₂CH₃), 30.52 (CCH₃), 58.52 (CHOH), 72.59 (CHNH₂), 72.67 (CH₂ x 3), 109.62 (CO₃).

### (h) (2S,3S)β-hydroxynorvaline (15)

Compound (**14**) (1.24 g, 5.5 mmol) was dissolved in DCM (68 mL) and trifluoroacetic acid (1.58 mL) and H₂O (1.13 mL) added. The resulting cloudy, white solution was stirred at room temperature for 30 minutes and the solvent evaporated *in vacuo.* The colourless residue was dissolved in MeOH (66 mL) and H₂O (17 mL) and 10 % Cs₂CO₃ (9.2 g in 92 mL H₂O) added. After stirring overnight at room temperature, the solution was acidified with 2 M HCl (~35 mL) to pH <3. The solution was loaded onto a cation exchange column (Bio-Rad AG 50W-X8 100-200 mesh, hydrogen form, 4.5 x 20 cm) washed with 0.01 M HCl (500 mL) and H₂O (500 mL) and eluted with 2M NH₄OH (2 L) then lyopholised to afford a pale yellow solid. The solid was washed with MeOH to yield an off-white solid **(15)**, 227 mg, 30 %; TLC (4:1:1, butan-2-ol : AcOH : H₂O) R*f* = 0.26, electrospray-MS m/z 134.1 (MH⁺), elemental analysis C₅H₁₁O₃N (req) %C 45.10, %H 8.33, %N 10.52, (fnd) %C 44.67, %H 8.03, %N 9.92.
δ_{H} (400 MHz; CDCl₃); 92:8 *erythro* (2S, 3S) : *threo* (2S, 3R), 1.00 (3H, t, *J* 7.4, CH₃), 1.40-1.54 (2H, m, CH₂), 3.41 (0.08H, d, *J* 4.2, CH), 3.61 (0.92H, d, *J* 4.2, CH), 3.60-3.65 (0.08H, m, CH), 3.66-3.69 (0.92H, m, CH).
δ_{c} (100 MHz; CDCl₃); 11.02 (CH₂CH₃), 25.26 (CH₂CH₃), 61.26 (CHOH) , 72.09 (CHNH₂), 172.03 (CO₂H).

### General method for the synthesis of Fmoc-3(2H)-furanones

Exemplified by dihydro-(4S-amino-[N-Fmoc])-5S-ethyl-3(2H)-furanone **(18)**, following the general chemistry detailed in scheme 1.

### (a) Preparation of Fmoc-(2S,3S)-β-ethylserine (16) (2S,3S)β-hydroxynorvaline (15) (277mg, 2.07mmol) and sodium carbonate (2.1eq, 460mg) were dissolved with stirring and ice-cooling in water (25mL) and THF (10mL).

9-fluorenylmethyl chloroformate (1.05eq, 560mg) in THF (15mL) was added over 45mins and the mixture stirred for a further 1hr at room temperature. Chloroform (100mL) and water (50mL) were added and the mixture acidified to pH2 with 0.1N HCl. The organic layer was collected and the aqueous washed with a further 2 x 100mL chloroform. The combined organics were backwashed with brine (1 x 300mL) and dried over magnesium sulphate. The chloroform was reduced *in vacuo* to yield a fine white solid. The solid was dissolved in *tert*-butyl methylether (25mL) with heating and heptane (75mL) added to give a cloudy solution. The mixture was cooled to -20°C and each 30mins further heptane (75mL) added for 4 cycles. The precipitate was filtered off and dried *in vacuo* to a fine white solid **(16)** 590mg, 80.6 %; TLC (CHCl₃; MeOH 3:1) R*f* = 0.40, electrospray-MS m/z 356.2 (MH⁺).

### (b) Preparation of (2S, 3S)-N-Fmoc-β-ethylserinydiazomethane (17)

Following the general method detailed in example 1. (a) for compound **(1),** Fmoc-(2S, 3S)-β-ethylserine **(16)** (560mg) was converted to a yellow solid (600mg) **(17)** used without purification.

### (c) Preparation of dihydro-(4S-amino-[N-Fmoc])-5S-ethyl-3(2H)-furanone (18)

A solution of lithium chloride (1.0g, 23.5mmol) in 80% aqueous acetic acid (10mL) was cooled to 5°C and added to crude (2S, 3S)-N-Fmoc-p-ethylserinydiazomethane **(17)** (0.6g) with stirring. The oil dissolved over 10mins and stirring continued for a further 1hr slowly warming to room temperature, with evolution of gas. The solvents were removed in vacuo and the residue taken into EtOAc (50mL) and washed successively with water (50mL), saturated aqueous sodium bicarbonate (2 x 100mL) and brine (75mL), then dried over sodium sulphate. The solvent was removed *in vacuo* and the crude product purified by flash chromatography over silica gel (25g) eluting with EtOAc / heptane (1:3, v/v). Desired fractions were pooled and reduced *in vacuo* to give dihydro-(4S-amino-[N-Fmoc])-5S-ethyl-3(2H)-furanone **(18)** as a white solid, yield 320mg, 0.91mmol, 58%. Electrospray-MS m/z 352 (MH⁺), HRMS C₂₁H₂₁O₄NNa requires *M*, 374.1368, found: MNa⁺, 374.1368. (δ-1.49 ppm), analytical HPLC Rt = 13.61mins (98.4%), elemental analysis C₂₁H₂₁O₄N (req) %C 71.78, %H 6.02, %N 3.99, (fnd) %C 70.95, %H 6.22, %N 3.81.
δ_{H} (500 MHz; CDCl₃); 1.05 (3H, m, CH₂CH₃), 1.76, 1.94 (2H, bm, CH₂CH₃), 3.83 (1H, bm, furanone CHβ), 3.88 (1H, bm, furanone CHα), 4.02 (1H, d, *J* 17.3, 1 x furanone COCH₂O), 4.23 (2H, m, 1 x furanone COCH₂O + Fmoc CHCH₂O), 4.42 (2H, b, Fmoc CHCH₂O), 5.05 (1H, b, furanone, NH), 7.35 (2H, t, *J* 7.4, Fmoc aromatic), 7.42 (2H, t, *J* 7. 3, Fmoc aromatic), 7.58 (2H, t, *J* 7.4, Fmoc aromatic), 7.77 (2H, t, *J* 7.4, Fmoc aromatic).
δ_{c} (125 MHz; CDCl₃); 8.90 (5S-CH₂CH₃), 26.14 (5S-CH₂CH₃), 46.90 (Fmoc CHCH₂O), 60.50 (furanone CHα), 66.99 (Fmoc CHCH₂O), 70.43 (furanone COCH₂O), 81.65 (furanone CHβ), 119.76 (Fmoc aromatic), 124.72 (Fmoc aromatic), 126.85 (Fmoc aromatic), 127.53 (Fmoc aromatic), 141.09 (Fmoc aromatic), 143.37 (Fmoc aromatic), 155.76 (OCONH), 211.72 (furanone CO).

### (d) Preparation of (2S, 3R)-N-Fmoc-O-t-butyl-L-threonyldiazomethane (19)

Following the general method detailed in example 1. (a) for compound **(1)**, Fmoc-(2S,3R)-*O*-t-butyl-L-threonine (1.99 g, 5 mmol) was converted to (2S, 3R)-N-Fmoc-O-t-butyl-L-threonyldiazomethane (**19)** (2.11g, 100%) as a pale yellow immobile oil. This compound was carried through to the next stage without further purification. Electrospray-MS m/z 444 (MNa⁺, 20%), 394 (MH⁺ - N₂, 70%) and 338 (MH⁺ - tbutyl - N₂, 100%).

### (e) Preparation of (2R, 3S)-N-Fmoc-O-t-butyl-D-threonyldiazomethane (20)

Following the general method detailed in example 1. (a) for compound **(1)**, Fmoc-(2R,3S)-*O-t*-butyl-D-threonine (0.4 g, 1 mmol) was converted to (2S, 3R)-N-Fmoc-*O-t*-butyl-L-threonyldiazomethane **(20)** (0.48g, 111%) as a pale yellow immobile oil. This compound was carried through to the next stage without further purification. Electrospray-MS m/z 394 (MH⁺ - N₂, 60%) and 338 (MH⁺ - tbutyl - N₂, 100%).

### (f) Preparation of (2S, 3S)-N-Fmoc-O-t-butyl-L-allo-threonyldiazomethane (21)

Following the general method detailed in example 1. (a) for compound **(1)**, Fmoc-(2S,3S)-*O*-*t*-butyl-L-*allo*-threonine (0.4 g, 1 mmol) was converted to (2S, 3S)-N-Fmoc-*O*-*t*-butyl-L-allo-threonyldiazomethane **(21)** (0.53g, 123%) as a pale yellow immobile oil.. Electrospray-MS m/z 394 (MH⁺-N₂, 90%) and 338 (MH⁺- tbutyl - N₂, 60%).

### (g) Preparation of (2S)-N-Fmoc-O-t-butyl-L-serinyldiazomethane (22)

Following the general method detailed in example 1. (a) for compound **(1)**, Fmoc-(2S)-*O*-t-butyl-L-serine (1.15 g, 3 mmol) was converted to (2S)-N-Fmoc-*O*-*t*-butyl-L-serinyldiazomethane **(22)** (1.67g, 136%) as a pale yellow immobile oil. This compound was carried through to the next stage without further purification. Electrospray-MS m/z 430 (MNa⁺, 5%), 380 (MH⁺ - N₂, 12%) and 324 (MH⁺ - *t*butyl - N₂, 28%).

### (h) Preparation of (2R)-N-Fmoc-O-t-butyl-D-serinyldiazomethane (23)

Following the general method detailed in example 1. (a) for compound **(1)**, Fmoc-(2R)-*O*-*t*-butyl-L-serine (0.38 g, 1 mmol) was converted to (2R)-N-Fmoc-*O*-*t*-butyl-D-serinyldiazomethane **(23)** (1.67g, 136%) as a pale yellow immobile oil. This compound was carried through to the next stage without further purification. Electrospray-MS m/z 380 (MH⁺ - N₂, 55%) and 324 (MH⁺ - *t*butyl - N₂, 52%).

### (i) Preparation of dihydro-(4S-amino-[N-Fmoc])-5R-methyl-3(2H)-furanone (24)

Following the general method detailed for cyclisation of **(17)** to **(18),** diazoketone **(19)** cyclised to give dihydro-(4S-amino-[N-Fmoc])-5R-methyl-3(2H)-furanone **(24)** isolated as a white solid, yield 69%, electrospray-MS m/z 338 (MH⁺, 100%), analytical HPLC Rt = 14.59 mins (97.7%).
δ_{H} (500 MHz; CDCl₃); 1.50 (3H, brd, CH₃), 3.80 (1H, brt, furanone CHα), 3.97 (1H, brm, furanone CHβ), 3.99 (1H, d, *J* 17.7, 1 x furanone COCH₂O), 4.22 (1H, t, *J* 6.7, Fmoc CHCH₂O), 4.25 (1H, d, *J* 17.7, 1 x furanone COCH₂O), 4.44 (2H, b, Fmoc CHCH₂O), 5.11 (1H, b, NH), 7.32 (2H, t, *J* 7.4, Fmoc aromatic), 7.41 (2H, t, *J* 7.4, Fmoc aromatic), 7.58 (2H, t, *J* 7.4, Fmoc aromatic), 7.76 (2H, t, *J* 7.4, Fmoc aromatic).
δ_{c} (125 MHz; CDCl₃) ; 19.1 (CH₃), 47.2 (Fmoc CHCH₂O), 62.7 (furanone CHα), 67.3 (Fmoc CHCH₂O), 70.8 (furanone COCH₂O), 77.4 (furanone CHβ), 120.1 (Fmoc aromatic), 125.0 (Fmoc aromatic), 127.1 (Fmoc aromatic), 127.8 (Fmoc aromatic), 141.4 (Fmoc aromatic), 143.7 (Fmoc aromatic), 156.1 (OCONH), 211.8 (furanone CO).

### (j) Preparation of dihydro-(4R-amino-[N-Fmoc])-5S-methyl-3(2H)-furanone (25)

Following the general method detailed for cyclisation of **(17)** to **(18),** diazoketone **(20)** cyclised to give dihydro-(4R-amino-[N-Fmoc])-5S-methyl-3(2H)-furanone **(25)** isolated as a white solid, yield 70%, electrospray-MS m/z 338 (MH⁺, 75%), analytical HPLC Rt = 14.62 mins (98.9%).

### (k) (k) Preparation of dihydro-(4S-amino-[N-Fmoc])-5S-methyl-3(2H)-furanone (26)

Following the general method detailed for cyclisation of **(17)** to **(18),** diazoketone **(21)** cyclised to give dihydro-(4S-amino-[N-Fmoc])-5S-methyl-3(2H)-furanone **(26)** isolated as a white solid, yield 64%, electrospray-MS m/z 338 (MH⁺, 100%), analytical HPLC Rt = 14.68 mins (97.5%).

### (1) (1) Preparation of dihydro-(4S-amino-[N-Fmoc])-3(2H)-furanone (27)

Following the general method detailed for cyclisation of **(17)** to **(18),** diazoketone **(22)** cyclised to give dihydro-(4S-amino-[N-Fmoc])-3(2H)-furanone **(27)** isolated as a white solid, yield 74%, electrospray-MS m/z 324 (MH⁺, 75%), analytical HPLC Rt = 13.97 mins (97.6%).
δ_{H} (500 MHz; CDCl₃); 3.78· (1H, t, *J* 9.5, 1 x CHβ), 3.94 (1H, d, *J* 17.5, 1 x furanone COCH₂O), 4.22 (3H, m, 1 x furanone COCH₂O + furanone CHα + Fmoc CHCH₂O), 4.43 (2H, d, *J* 6. 3, Fmoc CHCH₂O), 4.69 (1H, t, *J* 8.4, 1 x CHβ), 5.20 (1H, b, NH), 7.32 (2H, t, *J* 7.4, Fmoc aromatic), 7.41 (2H, t, *J* 7.4, Fmoc aromatic), 7.58 (2H, t, *J* 7.4, Fmoc aromatic), 7.77 (2H, t, *J* 7.4, Fmoc aromatic).
δ_{c} (125 MHz; CDCl₃); 47.1 (Fmoc CHCH₂O), 56.1 (furanone CHα), 67.4 ( Fmoc CHCH₂O), 70.0 (furanone COCH₂O), 70.7 (furanone CHβ), 120.1 (Fmoc aromatic), 125.0 (Fmoc aromatic), 127.1 (Fmoc aromatic), 127.8 (Fmoc aromatic), 141.4 (Fmoc aromatic), 143.6 (Fmoc aromatic), 156.0 (OCONH), 211.2 (furanone CO).

### (m) (m) Preparation of dihydro-(4R-amino-[N-Fmoc])-3(2H)-furanone (28)

Following the general method detailed for cyclisation of **(17)** to **(18),** diazoketone **(23)** cyclised to give dihydro-(4R-amino-[N-Fmoc])-3(2H)-furanone **(28)** isolated as a white solid, yield 78%, electrospray-MS m/z 324 (MH⁺, 100%), analytical HPLC Rt = 14.05 mins (98.2%).

### Preparation of Building Block-Linker Constructs

General method for the synthesis of Dihydro-3(2H)-Furanone - Linker Constructs (29-34), following scheme 7.

Dihydro-3(2H)-furanone (18, 24-28), (1.0eq) was dissolved in a mixture of ethanol / water (7:1 v/v, 10mL per mmole compound) containing sodium acetate trihydrate (1.5eq). 4-[[(hydrazinocarbonyl)amino]methyl]cyclohexanecarboxylic acid trifluoro acetate (mw 329.3, 1.0eq) (see Murphy, A. M., *et al, J. Am. Chem. Soc,* 114, 3156-3157, 1992) was added and the mixture heated under reflux for 2hrs. The mixture was then cooled, poured into dichloromethane (100mL per mmole compound) and water (100mL) added. The organic layer was separated, backwashed with saturated brine (100mL). The organic layer was dried (Na₂SO₄), filtered and evaporated *in vacuo* to yield a white solid. Yield 85 - 105% crude weight.
Constructs (29-34) were used without further purification

### Alternative Route Towards Chiral β-Alkyl Serines

Following the chemistry detailed in scheme 3. Exemplified by the synthesis of (2S, 3S)-β-hydroxynorvaline **(15)** (also termed of (2S, 3S)-β-ethylserine)

### (a) (a) Tri-acetone-D-mannitol

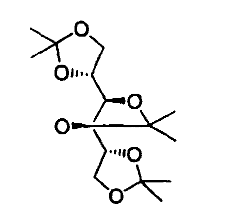

D-Mannitol (49.5g, 0.27mol) was suspended in acetone (600mL, 99.9% purity). To the suspension H₂SO₄ (4.95mL) was added and the mixture shaken at 21°C overnight. The solution was then filtered and the clear solution neutralised with a saturated solution of NaHCO₃ until pH=6. The solvent was concentrated in vacuo, affording tri-acetone-D-mannitol as a white solid, yield 78g, 96%. Electrospray-MS m/z 303 (MH⁺).

### (b) (b) 3,4-Isopropylidine-D-mannitol

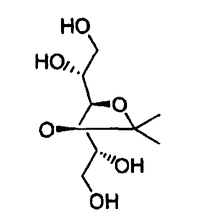

Tri-acetone-D-mannitol (78g, 0.26mol) was dissolved in the minimum amount of 70% acetic acid (400mL) and stirred in water bath at 42.7°C for 1.5hrs. The solvent was quickly evaporated *in vacuo* to give 3,4-Isopropylidine-D-mannitol as a colourless oil, yield 57.6g, 99.8%. Electrospray-MS m/z 223 (MH⁺).

### (c) (c) 1,2,5,6-tetra-O-benzyl-3,4-O-isopropylidine-D-mannitol (35)

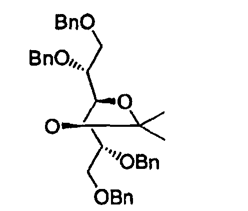

3,4-Isopropylidine-D-mannitol (57.64g, 0.26mol) was dissolved in benzylchloride (543mL). To the stirred solution powdered KOH (500g) was added and the solution heated in an oil bath at 133°C for 2hrs. The mixture was allowed to cool to room temperature and poured into a 3000mL beaker. Ice and water (1400mL) were carefully added, the mixture extracted with DCM (800mL) and the aqueous phase further extracted with DCM (300mL). The organic extracts were dried over sodium sulphate and the filtered solution concentrated *in vacuo.* The residue was purified by flash chromatography over silica gel eluting with EtOAc/heptane (1:15 to 1:10, v/v) to afford compound **(35)** as a colourless oil, yield 77g, 51%. Electrospray-MS m/z 583 (MH⁺). Analytical HPLC Rt = 29.16mins (91.8%).
δ_{H} (500 MHz, CDCl₃) 1.35 (6H, s, C(CH₃)₂), 3.62 (2H, dd, *J* 6, 10, 2 x CHOC), 3.75 ( 4H, m, 2 x CH₂OBn), 4.15-4.20
(1H, m, CHOBn), 4.46 (1H, dd, J 12.5, 14.5, CHOHn), 4.77 (4H, d, *J* 11.5, 4 x CH_{2A}C₆H₅), 4.73 (4H, d, *J* 11.5, 4 x CH_{2B}C₆H₅) and 7.25-7.34 (20H, m, 4 x C₆H₅).

### (d) (d) 1,2,5,6-Tetra-O-benzyl-D-mannitol (36)

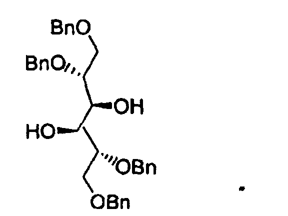

In a 2000mL flask fitted with a condenser compound **(35)** (41.11g, 0.071mol) was dissolved in 70% acetic acid (700mL) and the solution stirred at 100°C in an oil bath for 1.5hrs. After concentration *in vacuo,* the residue was purified by flash chromatography over silica gel eluting with EtOAc/heptane (3:7, v/v) to afford compound **(36)** as a pale yellow oil, yield 21.8g, 57%.
Electrospray-MS m/z 543 (MH⁺). Analytical HPLC Rt = 25.8 (100%).
δ_{H} (500 MHz, CDCl₃) 3.01 (2H, d, *J* 6.0, 2 x OH), 3.65 -3.70 (2H, m, 2 x CHOBn), 3.72-3.78 (4H, m, 2 x CH₂OBn), 3.93-3.97 (2H, m, 2 x CHOH), 4.55 (4H, s, 2 x CH₂C₆H₅), 4.73 (2H, d, *J* 11.5, 2 x CH_{2A}C₆H₅), 4.77 (2H, d, *J* 11.5, 2 x CH_{2B}C₆H₅) and 7.25-7.34 (20H, m, 4 x C₆H₅).

### (e) (2R)-2,3-Di-O-benzylglyceraldehyde (37)

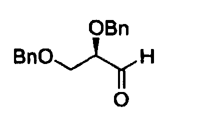

Compound **(36)** (10.78g, 0.02mol) was dissolved in anhydrous toluene (150mL). While vigorously stirring lead tetraacetate (9.83g, 0.023mol, 1.1eq) was added as a solid and the mixture stirred for 3hrs at room temperature. The mixture was then filtered and the filtered concentrated *in vacuo* to afford compound **(37)** as a colourless oil, yield 10.2g, 95%.
δ_{H} (500 MHz, CDCl₃) 3.75-3.83 (2H, m, CH₂OBn), 3.97 (1H, t, *J* 4, CHOBn), 4.55 (2H, d, *J* 5.5, 2 x CH_{2A}C₆H₅), 4.70 (2H, d, *J* 12, 2 x CH_{2B}C₆H₅), 7.20-7.40 (10H, m, 2 x C₆H₅) and 9.70 (1H, s, CHO).

### (f) (2S)-N-(2,3-Dibenzyloxypropylidene)benzylamine (38)

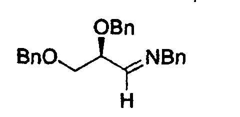

Benzylamine (4.06mL, 0.037mol, 1eq) was dissolved in anhydrous diethyl ether (150mL) and the solution cooled to 0°C. To a solution of compound **(37)** (9.9g, 0.037mol, 1eq) in anhydrous diethyl ether (100mL) at 0°C, was added anhydrous magnesium sulphate (7.3g) and the solution transferred *via* cannula under argon to the solution of the amine. After stirring for 3 hrs the reaction mixture was concentrated *in vacuo* to give compound **(38)** as a crude colourless oil, yield 12.2g, 96%.
δ_{H} (500 MHz, CDCl₃) 3.75-3.83 (2H, m, CH₂OBn), 4.17-4.25 (1H, m, CHOBn), 4.57 (2H, s, NCH₂C₆H₅), 4.62 (2H, m, 2 x CH_{2A}C₆H₅), 4.70 (2H, m, CH_{2B}C₆H₅), 7.20-7.40 (15H, m, 3 x C₆H₅) and 7.70 (1H, m, CHN).

### (g) (1R,2S)-N-Benzyl-2,3-dibenzyloxy-1-phenyl-1-propylamine (39)

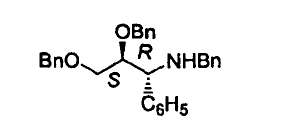

Phenylmagnesium bromide (29.17mL, 0.087mol, 3.0M, 2.5eq) was dissolved in anhydrous diethyl ether (124mL) and the solution cooled to 0°C under argon. A solution of compound **(38)** (12.5g, 0.035mol) in anhydrous diethyl ether (140mL) was transferred *via* cannula to the solution of the phenylmagnesium bromide and the reaction mixture stirred at room temperature for 2hrs. The solution was poured into an aqueous solution of NH₄Cl (200mL) and extracted with *tert*-butyl methyl ether (2 x 100mL). The combined extracts, dried over anhydrous sodium sulphate, were concentrated *in vacuo.* The crude oil obtained was purified by flash chromatography over silica gel eluting with EtOAc/heptane (1:4, v/v) to afford compound **(39)** as a pale yellow oil, yield 8.5g, 56%. Electrospray-MS m/z 438 (MH⁺). Analytical HPLC Rt = 24.0mins (98%).
δ_{H} (500 MHz, CDCl₃) 2.45 (1H, br s, NH), 3.32 (1H, dd, *J* 10, 4.5, CH_{2A}OBn), 3.43 (1H, d, *J* 13, C₆H₅CH_{2A}NH) 3.50-3.54 (1H, dd, *J* 10, 3, CH_{2B}OBn) , 3.55-3.61 (1H, d, *J* 13, C₆H₅CH_{2B}NH), 3.65-3.78 (1H, m, CHOBn), 3.90 (1H, d, *J* 7, C₆H₅CHNH), 4.40 (2H, s, OCH₂C₆H₅), 4.62 (1H, d, *J* 11, OCH_{2A}C₆H₅), 4.70 (1H, d, *J* 11, OCH_{2B}C₆H₅) and 7.18-7.40 (20H, m, 4 x C₆H₅).

### (h) (1R,2S)-N-Benzyl-tert-butoxycarbonyl-2,3-dibenzyloxy-1-phenyl-1-propylamine (40)

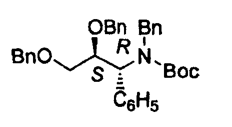

Compound **(39)** (9.26g, 0.02mol) was dissolved in dioxane (66mL) and diisopropylamine (0.37mL, 0.0021mol, 0.11eq) was added. To the stirred solution di-*tert*-butyl dicarbonate (11.25g, 0.0516mol, 2.6eq) was added as a solid and the solution stirred at 50°C in an oil bath overnight. The mixture was treated with text-butyl methyl ether (300mL), washed with 1.0M KHSO₄ aqueous solution (60mL) and the organic extracts were dried over anhydrous sodium sulphate and concentrated *in vacuo.* The crude oil was purified by flash chromatography over silica gel eluting with EtOAc/heptane (1:9, v/v) to afford compound **(40)** as a colourless oil, yield 7.7g, 71%. Electrospray-MS m/z 538 (MH⁺). Analytical HPLC Rt = 30.0mins (95%).
δ_{H} (500 MHz, CDCl₃) 1.30 (9H, s, C(CH₃)₃), 3.44 (1H, dd, *J* 10, 4.5, CH_{2A}OBn), 3.61 (1H, dd, *J* 10, 2, CH_{2B}OBn), 4.30 (1H, m, CH_{2A}N) 4.37 (2H, d, *J* 12, OCH_{2A}C₆H₅), 4.43 (2H, d, *J* 12, OCH_{2B}C₆H₅), 4.50-4.63 (1H, m, CH_{2B}N), 4.85 (1H, m, CHOBn) 5.25 (1H, d, *J* 9, C₆H₅CHN) and 7.00-7.45 (20H, m, 4 x C₆H₅).

### (i) (1R,2S)-N-tert-Butoxycarbonyl-2,3-hydroxy-1-phenyl-1-propylamine (41)

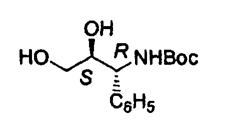

Compound **(40)** (7.67g, 0.014mol) was dissolved in anhydrous methanol (80mL). After having flushed the flask with argon, 20%Pd(OH)₂/C (10.00g, Degussa type, E101 NE/W, wet) was carefully added and the mixture stirred under H₂ for 48 hrs. The mixture was carefully filtered through a pad of Celite and the catalyst washed with a solution of aqueous methanol (10:100 H₂O:CH₃OH, v/v). The filtered solution was concentrated *in vacuo* and the residue purified by flash chromatography over silica gel eluting with EtOAc/heptane (3:1, v/v) to afford compound **(41)** as a colourless oil, yield 2.7g, 72%. Electrospray-MS m/z 268 (MH⁺). Analytical HPLC Rt = 15.3mins (100%).
δ_{H} (500 MHz, CDCl₃) 1.44 (9H, s, C(CH₃)₃), 2.6 (2H, br s, OH), 3.56 (2H, d, *J* 5.5, CH₂OH), 3.97 (1H, s, C₆H₅CHNH), 4.83 (1H, s, C₆H₅CHCHOH), 5.28 (1H, d, *J* 8, NH) and 7.20-7.45 (5H, m, C₆H₅).

### (j) (1R,2S)-N-tert-Butoxycarbonyl-3-tert-butyldimethylsilyloxy-2-hydroxy-1-phenyl-1-propylamine (42)

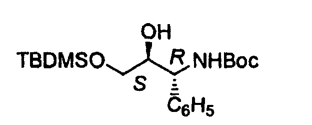

Compound **(41)** (2.67g, 0.01mol) was dissolved in anhydrous DMF (60mL) and stirred under argon. Imidazole (1.5g, 0.022mol, 2.2eq) was added followed by the addition of TBDMSCl (1.66g, 0.011mol, 1.1eq). The reaction mixture was stirred overnight at room temperature. The mixture was diluted with ether (240mL), washed with saturated NH₄Cl (120mL) and H₂O (40mL) and the aqueous layer extracted with ether (4 x 100mL). The combined extracts were dried over anhydrous sodium sulphate, filtered and concentrated *in vacuo.* Purification of the residue by flash chromatography over silica gel eluting with EtOAc/heptane (3:1, v/v) afforded compound **(42)** as a colourless oil, yield 3.31g, 87%. Electrospray-MS m/z 382 (MH⁺).
δ_{H} (500 MHz, CDCl₃) 0.05 (3H, s, CH_{3A}SiCH₃), 0.06 (3H, s, CH₃SiCH_{3B}), 0.89 (9H, s, Si(CH₃)₃), 1.39 (9H, br s, C(CH₃)₃), 2.45 (1H, br s, OH), 3.51 (1H, dd, J 10, 7, TBDMSOCH_{2A}), 3.65 (1H, dd, J 10, 4.5, TBDMSOCH_{2B}), 3.85 (1H, m, CHOH), 4.66 (1H, m, C₆H₅CHNH), 5.45 (1H, br s, NH) and 7.23-7.35 (5H, m, C₆H₅).

### (k) (1R,2S)-N-tert-butoxycarbonyl-3-tert-butyldimethylsilyloxy-2-mesyloxy-1-phenyl-1-propylamine (43)

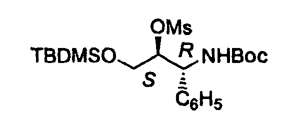

Compound **(42)** (1.30g, 3.40mmol, 1.0eq) was dissolved in anhydrous DCM (30mL). To the solution TEA (0.57mL, 4.09mmol, 1.2eq) was added and the mixture was cooled to 0°C in an ice-water bath. At this temperature and under argon, a solution of MsCl (0.32ml, 4.09mmol, 1.2eq) in anhydrous DCM (3mL) was added. The mixture was stirred for 1.5hrs. The reaction mixture was treated with water (20mL) and extracted with DCM (20mL). The aqueous phase was further extracted with DCM (4 x 60mL) and the combined organic layers were dried over anhydrous sodium sulphate and concentrated *in vacuo.* The residue was purified by flash chromatography over silica gel eluting with EtOAc/heptane (1:3, v/v) affording compound **(43)** as a colourless oil, yield 1.30g, 83%. Electrospray-MS m/z 460 (MH⁺). Analytical HPLC Rt: 27.1mins (98%).
δ_{H} (500 MHz, CDCl₃) 0.06 (3H, s, CH_{3A}SiCH₃), 0.07 (3H, s, CH₃SiCH_{3B}), 0.91 (9H, s, Si(CH₃)₃), 1.42 (9H, br s, C(CH₃)₃), 2.54 (3H, s, SO₂CH₃), 3.77 (2H, d, J6.0, TBDMSOCH₂), 4.7 (1H, m, CHOH), 5.1 (1H, m, C₆H₅CHNH), 5.4 (1H, br s, NH) and 7.26-7.38 (5H, m, C₆H₅).

### (1) (1R,2R)-N-tert-Butoxycarbonyl-2,3-epoxy-1-propylamine (44)

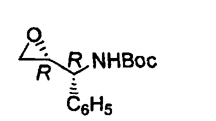

Compound **(43)** (3.79g, 8.26mmol, 1.0eq) was dissolved in THF anhydrous (78mL) and the solution cooled to 0°C in an ice water bath. TBAF (16.52mL, 1.0M sol in THF, 16.52mmol, 2eq) was added dropwise via syringe and once the addition was complete the ice bath was removed. The reaction mixture was stirred at room temperature overnight and then treated with water (40mL), extracted with diethyl ether (40mL) and the aqueous phase further extracted with diethyl ether (3 x 75mL). The combined extracts were dried over anhydrous sodium sulphate, filtered and concentrated *in vacuo.* The residue was purified by flash chromatography over silica gel eluting with TBME /heptane (1:6 to 2:1, v/v) affording compound **(44)** a white solid, yield 1.0g, 48%.Electrospray-MS m/z 250 (MH⁺).
δ_{H} (500 MHz, CDCl₃) 1.42 (9H, s, C(CH₃)₃), 2.50 (1H, dd, *J* 5, 2.2, CHCH_{2A}O), 2.76 (1H, dd, *J* 5, 4, CHCH_{2B}O), 3.20-3.30 (1H, m, CHCH₂O), 4.72 (1H, br s, C₆H₅CHCHO), 5.00 (1H, br s, NH) and 7.27-7.38 (5H, m, C₆Hₛ).

### (m) (1R,2S)-N-tert-butoxycarbonyl-2-hydroxy-1-phenyl-1-butylamine (45)

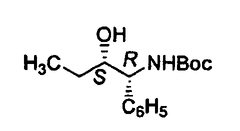

Copper(I)iodide (0.574g, 3.01mmol, 5eq) was dispersed in anhydrous diethyl ether (17mL). After cooling the suspension to -35°C under argon, CH₃Li in diethyl ether (3.76mL, 1.6M, 6.02mmol, 10eq) was added dropwise. After stirring at -35°C for 30 mins a solution of compound **(44)** (0.15g, 0.60mmol, 1.0eq) dissolved in diethyl ether (1.5mL) was added dropwise to the solution of the organocuprate and the reaction mixture was stirred at -35°C for 1.5 hrs. Ethyl acetate (12.5mL) was added followed by the careful addition of a saturated solution of NH₄Cl (10mL) and water (3mL). The mixture was allowed to warm up to room temperature and the organic phase extracted. The aqueous phase was further extracted with ethyl acetate (3 x 15mL) and the combined extracts dried over anhydrous sodium sulphate, filtered and concentrated *in vacuo.* The crude oil was purified by flash chromatography over silica gel eluting with TBME /heptane (2:3, v/v) affording compound **(45)** as a white solid, yield 0.14g, 88%. Electrospray-MS m/z 266 (MH⁺). Analytical HPLC Rt = 17.6mins (100%).
δ_{H} (500 MHz, CDCl₃) 0.97 (3H, t, *J* 7.5, CH₃CH₂), 1.10-1.25 (1H, m, CH₃CH_{2A}), 1.25-1.50 (1H, m, CH₃CH_{2B}), 1.50 (9H, s, C(CH₃)₃), 3.78 (1H, br s, CHOH), 4.73 (1H, br s, C₆H₅CHNH), 5.28 (1H, br s, NH) and 7.25-7.38 (5H, m, C₆H₅).

### (n) (1R,2S)-N-tert-Butoxycarbonyl-2-tert-butoxy-1-phenyl-1-butylamine (46)

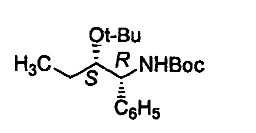

In a sealed tube, compound **(45)** (0.114g, 0.43mmol) was dissolved in anhydrous DCM (11mL). Whilst stirring was maintained, the tube was immersed in a dry ice-acetone bath and cooled to -60°C. Isobutylene (11mL) was condensed into the tube and methyltriflate (55µL) was carefully added. The tube was capped tightly and the bath removed to allow the reaction to proceed at room temperature for 4 days. The tube was cooled to -60°C, the lid removed and then the bath removed to allow the excess of isobutylene to slowly evaporate whilst warming up to room temperature. At about 10°C, TEA (0.7mL) was added to neutralise the excess acid. The residue obtained after removal of the solvents *in vacuo* was purified by flash chromatography over silica gel eluting with EtOAc/heptane (2:8, v/v) affording compound **(46)** as a white solid, yield 0.02g, 14.% Electrospray-MS m/z 322 (MH⁺). Analytical HPLC Rt = 24.1mins (90%).
δ_{H} (500 MHz, CDCl₃) 0.84 (3H, t, *J* 7.5, CH₃CH₂), 1.15-1.30 (1H, m, CH₃CH_{2A}) 1.24 (9H, s, CHOC(CH₃)₃), 1.35-1.40 (1H, m, CH₃CH_{2B}), 1.41 (9H, s, CO₂C(CH₃)₃), 3.72 (1H, m, CHO(CH₃)₃), 4.78 (1H, m, C₆H₅CHNH), 5.15 (1H, br s, NH) and 7.22-7.38 (5H, m, C₆H₅).

### (o) (2S,3S)-N-tert-Butoxycarbonyl-β-tert-butoxy-norvaline (47)

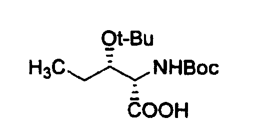

Compound **(46)** (0.024g, 0.074mmol, 1eq), was dissolved in a mixture of CCl₄/CH₃CN/H₂O (1:1:2, v/v/v, 2.4mL). To the stirred biphasic solution NaHO₃ (0.104g, 1.25mmol, 16.9eq) was added as a solid, followed by the careful addition of NaIO₄ (0.284g, 1.33mmol, 18eq). After 10 minutes RuCl₃.3H₂O (1.5mg, 7.23µmol, 0.1eq) was added and the reaction mixture stirred for 48hrs. The solution was treated with EtOAc (15mL) and acidified to pH =3 by dropwise addition of citric acid (10%). The organic phase was further extracted with EtOAc (3 x 15mL) and the combined extracts were dried over anhydrous magnesium sulphate, filtered and concentrated *in vacuo.* The crude residue was purified by flash chromatography over silica gel eluting with a gradient of MeOH /CH₃Cl (0.1:10 to 1.0:10, v/v) to give compound **(47)** as a white solid, yield 0.009g, 42%.
Electrospray-MS m/z 290 (MH⁺).

### (p) (2S,3S)-β-Hydroxy-norvaline (15)

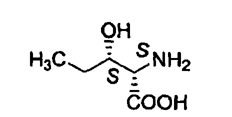

Compound **(47)** (9mg, 0.03mmol) was dissolved in a solution HCl in dioxane (1mL, 4.0M). After stirring for 3hrs at room temperature, the solvent was removed *in vacuo* and the residue was lyophilised using CH₃CN/H₂O (4:1, v/v) to yield (2S,3S)-β-hydroxynorvaline **(15)** as a white solid, 3.0 mg, 75%. Electrospray-MS m/z 134 (MH⁺).
δ_{H} (500 MHz; CD₃OD) 1.00 (3H, t, *J* 7.5, CH₃CH₂), 1.50-1.65 (2H, m, CH₃CH₂), 3.88-3.95 (1H, m, CHOH) and 3.98 (1H, d, *J* 3, C₆H₅CHNH₂).

### Threonine Chemistry Towards 2,4,5-trisubstituted Dihydro-3(2H)-furanones

Following the chemistry detailed in scheme 4.

### (a) N-benzyloxycarbonyl-O-tert-butyldimethylsilyl-L-threonine (48)

A solution of tert-butyldimethylsilyl chloride (65g, 0.431mol) in DCM (300ml) was added to a stirred solution of N-benzyloxycarbonyl-L-threonine (30.3g, 0.12mol) and imidazole (19.57g, 0.287mol). A precipitate formed immediately and stirring was continued for 60hr. The solvent was removed *in vacuo,* the residue taken up in THF and water (3:1, 400ml), and vigorously stirred for 15min. The THF was removed *in vacuo,* the product extracted into EtOAc (3 x 100ml), the combined organic layers washed with brine(2 x 100ml) and dried over MgSO₄. Purification by crystallisation from EtOAc and heptane gave compound **(48)** as a white solid, 39.15g (89%). Electrospray-MS m/z 368.2 (MH⁺).

### (b) N-Benzyloxycarbonyl-O-tert-butyldimethylsilyl-L-threonine N,O-dimethylhydroxyl amide (49)

Isobutyl chloroformate (15.07ml, 0.116mol) was added dropwise to a stirred solution of N-benzyloxycarbonyl-O-tert-butyldimethylsilyl-L-threonine **(48)** (38.65g, 0.105mol) and N-methylmorpholine (11.58ml, 0.106mmol) in THF (150ml). Another equivalent of N-methylmorpholine (11.58ml, 0.106mmol) was added and stirring continued for 30min before N,O-dimethylhydroxylamine hydrochloride (12.32g, 0.126mol) was added. After stirring overnight the solvent was removed *in vacuo* and the residue partitioned between EtOAc (3 x 180ml) and water / saturated brine (1:1, v/v, 180ml). The combined organic layers were washed with 0.1M HCl (2 x 180ml), 5% Na₂CO₃ (2 x 180ml), saturated brine (180ml) and dried over Na₂SO₄. Purification by flash silica chromatography eluting with EtOAc / heptane (3:7, v/v) yielded compound **(49)** as a colourless viscous oil, 32.29g (75%). Electrospray-MS m/z 411.2 (MH⁺).
δ_{H} (500 MHz; CDCl₃ at 398K) -0.02 (3H, s, Si(CH₃)₂), 0.01 (3H, s, Si(CH₃)₂), 0.85 (9H, s, SiC (CH₃)₃), 1.21 (3H, d, *J* 6.1, CHCH₃), 3.21 (3H, s, NCH₃), 3.76 (3H, s, OCH₃), 4.24 (1H, br m, CHCH₃), 4.56 (1H, br d, *J* 9.5, CHCHCH₃), 5.09 (1H, d, *J* 12.2, benzylic Hₐ), 5.13 (1H, d, *J* 12.2, benzylic H_{b}), 5.60 (1H, d, *J* 9.5, NH), 7.37 (5H, m, Ph). δ_{c} (125 MHz; CDCl₃ at 398K) -5.10 (Si (CH₃)₂), -4.67 Si(CH₃)₂), 17.95 (SiC(CH₃)₃), 21.24 (CHCH₃), 25.73 (SiC(CH₃)₃), 57.15 (NMe), 61.31 (CHCHCH₃), 66.92 (PhCH₂), 68.19 (OMe), 77.36 (CHCHCH₃), 128.09 (Ar), 128.50 (Ar), 136.43 (Ar 4°), 156.83 (NHCO), 170.47 (NMeCO).

### (c) N-Benzyloxycarbonyl-O-tert-butyldimethylsilyl-L-threonine aldehyde (50)

Lithium aluminium hydride (1M in Et₂O, 17.0ml, 17.1mmol) was added to a stirred solution of compound **(49)** (7g, 17.1mmol) in tBME (350ml) at -30°C. The reaction mixture was removed from the cooling bath and allowed to warm to room temperature. After stirring for 30min, a white precipitate had formed, and the reaction mixture was quenched by the careful dropwise addition of 0.1M H₂SO₄ until the effervescence stopped. The product was extracted into TBME (3 x 350ml) from ice cold 0.05M H₂SO₄ (600ml), the combined organic layers washed with brine (300ml) and dried over Na₂SO₉. Purification by flash silica chromatography eluting with EtOAc / heptane (2:8, v/v) yielded aldehyde **(50)** as a colourless viscous oil, 1.47g (25%). Electrospray-MS m/z 352.2 (MH⁺).

### (d) Ethyl trimethylsilyldiazoacetate (preparation obtained from Shuji Kanemasa, personal communication)

Trimethylsilyl trifluoromethanesulphonate (9.7ml, 53.6mmol) was slowly added via a syringe to a stirred solution of ethyl diazoacetate (5.3ml, 50.4mmol) and diisopropylethylamine (8.7ml, 49.9mmol) in diethyl ether (300ml) at 0°C. After 1hr the temperature was increased to room temperature and stirring continued for a further 1hr. The precipitate formed was removed by filtration and the solid washed with diethyl ether (50ml). The filtrate and washing was combined, washed with brine (70ml) and dried over Na₂SO₄. The solvent was removed *in vacuo* and the residue purified by vacuum distillation to yield ethyl trimethylsilyldiazoacetate as a yellow liquid, 2.48g (26%); bp 90-95°C at 25mmHg. υₘₐₓ cm⁻¹ 2091.9, 1688.9. δ_{H} (500 MHz; CDCl₃ at 398K) 0.25 (9H, s, Si(CH₃)₃), 1.25 (3H, t, *J* 7.1, CH₂CH₃), 4.12 (2H, q, *J* 7.2, CH₂CH₃).
δ_{c} (125 MHz; CDCl₃ at 398K) -1.51 (Si(CH₃)₃), 14.41 (CH₂CH₃), 60.59 (CH₂CH₃), 169.38 (CO₂Et).

### (e) 4R-Benzyloxycarbonylamino-3S-hydroxy-5R-methyltetrahydrofuran-2R-carboxylic acid ethyl ester (51)

Tetrabutylammonium fluoride (1M in THF, 5.03ml, 5.03mmol) was added over 30min to a vigorously stirred solution of aldehyde **(50)** (1.47g, 4.19mmol) and ethyl trimethylsilyldiazoacetate (1.56g, 8.38mmol) in diethyl ether (45ml) at 0°C. After3hr the product was extracted into tBME (2 x 70ml and 1 x 40ml) from water (70ml), the combined organic layers washed with brine (2 x 70ml) and dried over Na₂SO₄. Purification by repeated flash silica chromatography eluting with EtOAc / heptane (6:4, v/v) yielded 4R-benzyloxycarbonylamino-3S-hydroxy-5R-methyltetrahydrofuran-2R-carboxylic acid ethyl ester **(51)** as a pale yellow viscous oil that solidified on standing, 0.524g (39%); mp 90-92°C. Electrospray-MS m/z 324.2 (MH⁺). HRMS C₁₆H₂₁O₆NNa requires *M*, 346.1267, found: MNa⁺ 346.1272 (δ 1.48ppm).
δ_{H} (500 MHz; CDCl₃ at 398K) 1.17 (3H, d, *J* 6.3, CHCH₃), 1.25 (3H, t, *J* 7.1, CH₂CH₃), 3.18 (1H, br s, OH), 3.73 (1H, br dd, *J* 3.3, 9.2, furanone 4), 4.03 (1H, s br, furanone 2), 4.11 (1H, br m, furanone 5), 4.21 (2H, q, *J* 7, CH₂CH₃), 4.76 (1H, br s, furanone 3), 5.11 (1H, d, *J* 12.2, benzylic Hₐ), 5.13 (1H, d, *J* 12.2, benzylic H_{b}), 5.62 (1H, d, *J* 9.6, NH), 7.34 (5H, m, Ph).
δ_{c} (125 MHz; CDCl₃ at 398K) 14.43 (CH₂CH₃), 19.98 (CHCH₃), 58.99 (furanone 4), 61.29 (CH₂CH₃), 67.32 (PhCH₂), 68.70 (furanone 3), 68.85 (furanone 5), 77.39 (furanone 2), 128.07 (Ar), 128.26 (Ar), 128.57 (Ar), 136.18 (Ar 4°), 157.74 (NHCO), 166.79 (ester CO).

### Sugar Chemistry Route Towards Furanone Analogues

Following the chemistry detailed in scheme 5.

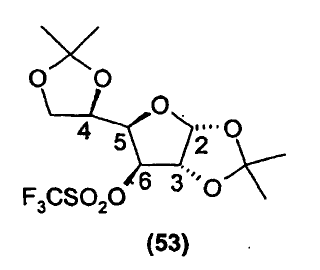

### (a) Trifluoro-methanesulfonic acid 5(R)-(2,2-dimethyl-[1,3]dioxolan-4(R)-yl)-2,2-dimethyl-tetrahydro-furo[2(R),3-d(R)][1,3]dioxol-6(S)-yl ester (53).

1,2,5,6-Diisopropylidene-*D*-glucose (5.00g, 19.21mmol) **(52)** was dissolved in dry dichloromethane (100mL) and stirred at 0 °C under a nitrogen atmosphere. Anhydrous pyridine (1.67g, 1.71mL, 21.13mmol) was added, followed by the dropwise addition of trifluoromethanesulfonic anhydride (5.96g, 3.55mL, 21.13mmol) which resulted in the formation of a yellow solution. The solution was stirred at 0 °C for a further 1 h and then at room temperature overnight. Tlc analysis indicated all the starting material had been consumed, therefore, the solvent was removed in *vacuo.*
The residue was dissolved in dichloromethane (40mL) was washed with 2.0M HCl solution and brine, separated, dried (MgSO₄), and evaporated *in vacuo.* Column chromatography eluting with heptane / ethyl acetate (8:1, v/v) afforded **(53)** as an unstable white solid (6.48g, 86%). R_{f} 0.51 heptane / ethyl acetate (8:1, v/v). Electrospray-MS m/z 392.3 (MH⁺).
δ_{H} (500 MHz, CDCl₃ at 298 K) 1.31 (3H, s, CH₃), 1.33 (3H, s, CH₃), 1.42 (3H, s, CH₃), 1.51 (3H, s, CH₃), 3.96 (1H, dd, *J* 3.7, 8.3, CH), 4.15 (1H, dd, *J* 5.5, 8.3, CH'), 4.19 (2H, m, H-4 and H-6), 4.76 (1H, app.d, *J* 3.6, H-3), 5.26 (1H, app.d, *J* 1.2, H-5), 5.98 (1H, d, *J* 3.6, H-2).
δ_{c} (125 MHz, CDCl₃ at 298 K) 24.8, 26.2, 26.5 and 26.8 (2xC(CH₃)₂), 67.6 (CH₂), 79.9 and 71.7 (C-4 and C-6), 83.2 (C-3), 88.2 (C-5), 105.0 (C-2), 109.8 and 113.1 (2xC(CH₃)₂), 118.1 (q, CF₃).
δ_{F}, (500 MHz, CDCl₃ at 298 K) -75.1 (CF₃).

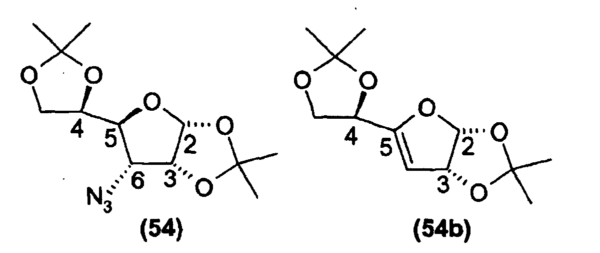

### (b) 6(R)-Azido-5(S)(2,2-dimethyl-[1,3]dioxolan-4(R)-yl)-2,2-dimethyl-tetrahydro-furo[2(R),3-d(R)][1,3]dioxole (54) and 5-(2,2-Dimethyl-[1,3]dioxolan-4(R)-yl)-2,2-dimethyl-3a,6a-dihydro-furo[2(R),3-d(R)][1,3]dioxole (54b).

Triflate **(53)** (2.59g, 6.61mmol) was dissolved in dry DMF (50mL) under a nitrogen atmosphere, while sodium azide (686mg, 10.55mmol) was carefully added. The solution was heated to 50 °C for 4 h and tlc analysis revealed the consumption of the starting material. The solvent was then removed *in vacuo*. Ethyl acetate (50mL) was added and the solution was washed with brine, separated, dried (MgSO₄) and evaporated *in vacuo.* The crude residue was purified by column chromatography using the eluent heptane / ethyl acetate (4:1, v/v) to afford **(54)** as a colourless oil (900mg, 48%), together with (54b) as a white solid (645mg, 40%).
**(54)**: R_{f} 0.28 heptane / ethyl acetate (4:1, v/v). HRMS C₁₂H₁₉O₅N₃Na requires *M,* 308.1217, found: MNa⁺, 308.1192. (δ - 2.49 ppm).
δ_{H} (500 MHz, CDCl₃ at 298 K) 1.34 (3H, s, CH₃), 1.36 (3H, s, CH₃), 1.47 (3H, s, CH₃), 1.56 (3H, s, CH₃), 3.50 (1H, dd, *J* 4.8, 9.1, H-6), 3.97 (1H, dd, *J* 5.5, 8.5, CH), 4.01 (1H, dd, *J* 6.0, 9.1, H-5), 4.12 (1H, app.t, *J* 8.5, CH'), 4.17 (1H, dd, *J* 6.0, 12.0, H-4), 4.71 (1H, app.t, *J* 4.2, H-3), 5.77 (1H, d, *J* 3.6, H-2).
δ_{c} (125 MHz, CDCl₃ at 298 K) 25.1, 26.3, 26.4 and 26.5 (2xC(CH₃)₂), 62.6 (C-6), 66.8 (CH₂), 75.8 (C-4), 78.1 (C-5), 80.6 (C-3), 103.4 (C-2), 110.1 and 113.2 (2xC(CH₃)₂). (54b): R_{f} 0.42 heptane / ethyl acetate (4:1, v/v).
δ_{H} (500 MHz, CDCl₃ at 298 K) 1.37 (3H, s, CH₃), 1.42 (3H, s, CH₃), 1.45 (6H, s, 2xCH₃), 3.95 (1H, dd, *J* 6.1, 7.8, CH), 4.13 (1H, app.t, *J* 7.8, CH'), 4.57 (1H, app.t, *J* 6.1, H-4), 5.23 (1H, d, *J* 0.8, C=CH), 5.28 (1H, app.d, *J* 4.4, H-3), 6.03 (1H, d, *J* 5.2, H-2).
δ_{c} (125 MHz, CDCl₃ at 298 K) 25.5, 26.2, 27.9 and 28.2 (2xC(CH₃)₂), 67.0 (CH₂), 71.3 (C-4), 83.4 (C-3), 99.0 (C=CH), 106.6 (C-2), 110.3 and 112.3 (2xC(CH₃)₂), 160.1 (C-5).

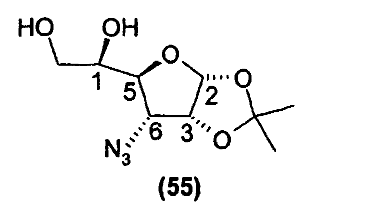

### (c) 1-(6(R)-Azido-2,2-dimethyl-tetrahydro-furo[2(R),3-d(R)][1,3]dioxol-5(S)-yl)-ethane-1(R),2-diol (55).

Compound **(54)** (1.03g, 3.61mmol) was dissolved in AcOH:MeOH:H₂O (90:50:60mL) and stirred at 50 °C for 17 h, then solvent was removed *in vacuo.* The residue was purified by column chromatography using heptane / ethyl acetate (1:1, v/v) as the eluent to afford **(55)** as a pale yellow solid (868mg, 98%). R_{f} 0.12 heptane / ethyl acetate (1:1, v/v).
δ_{H} (500 MHz, CD₃OD at 298 K) 1.52 (3H, s, CH₃), 1.98 (3H, s, CH₃), 3.50 (3H, m, CH₂ and H-6), 3.83 (1H, m, H-1), 4.13 (1H, dd, *J* 4.1, 9.4, H-5), 4.78 (1H, app.t, *J* 4.1, H-3), 5.78 (1H, d, *J* 3.6, H-2).
δ_{c} (125 MHz, CD₃OD at 298 K) 26.8 (C(CH₃)₂), 61.5 (C-6), 63.9 (CH₂), 72.8 (C-1), 79.1 (C-5), 82.3 (C-3), 105.6 (C-4), 114.0 (C(CH₃)₂).

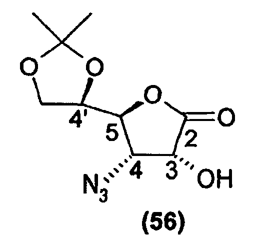

### (d) 4(R)-Azido-5(S)-(2,2-dimethyl-[1,3]dioxolan-4(R)-yl)-3(R)-hydroxy-dihydro-furan-2-one (56).

TFA (2mL) and water (40mL) were added to compound **(54)** (1.18g, 4.14mmol) and the solution was stirred at room temperature overnight. The solvent was then evaporated *in vacuo* to produce a colourless oil. The crude oil was then dissolved in water (34mL) and stirred at 0 °C, while barium carbonate (1.22g, 6.15mmol) was added, followed by the dropwise addition of bromine (727mg, 0.23mL, 4.55mmol). The yellow solution was stirred at 0 °C for 2 h and then for a further 2 h at room temperature. It was then filtered through celite and compressed air was bubbled through until the solution had been decolourised. The water was then removed *in vacuo* to afford a white solid. This white solid was pre-dried with acetone (2x50mL), before being suspended in acetone (100mL) and stirred at room temperature. 10-Camphorsulfonyl acid (192mg, 0.83mmol) was added and the mixture was stirred overnight. Tlc analysis indicated no change therefore another 0.2 equivalents of 10-Camphorsulfonyl acid (192mg, 0.83mmol) were added and the mixture was stirred at 50 °C for 4 h. The solution was filtered and the solvent was removed *in vacuo.* The crude residue was purified by column chromatography using heptane / ethyl acetate (2:1, v/v) as the eluent to afford **(56)** as a white solid (680mg, 68%). R_{f} 0.34 heptane / ethyl acetate (2:1, v/v). HRMS C₉H₁₃O₅N₃Na requires *M*, 266.0742, found: MNa⁺, 266.0700. (δ-4.21 ppm).
δ_{H} (500 MHz, DMSO at 298 K) 1.28 (3H, s, CH₃), 1.39 (3H, s, CH₃), 3.82 (1H, dd, *J* 5.5, 8.9, CH), 4.06 (1H, dd, *J* 6.9, 8.9, CH'), 4.24 (1H, app.d, *J* 5.1, H-4'), 4.29 (1H, dd, *J* 5.5, 12.0, H-5), 4.46 (1H, app.d, *J* 6.1, H-4), 4.82 (1H, app.t, *J* 6.5, H-3), 6.65 (1H, d, *J* 6.9, OH).
δ_{c} (125 MHz, DMSO at 298 K) 24.7 and 26.1 (C(CH₃)₂), 59.5 (C-4), 65.3 (CH₂), 68.5 (C-3), 73.6 (C-5), 81.2 (C-4'), 109.7 (C(CH₃)₂), 174.5 (C-2).

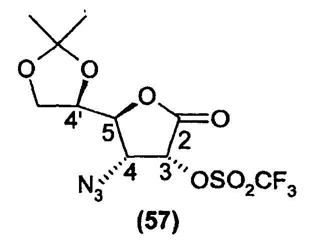

### (e) Trifluoro-methanesulfonic acid 4(R)-azido-5(S)-(2,2-dimethyl-[1,3]dioxolan-4(R)-yl)-2-oxo-tetrahydro-furan-3(R)-yl ester (57).

Compound **(56)** (500mg, 2.06mmol) was dissolved in dry dichloromethane (50mL) and stirred at 0 °C under a nitrogen atmosphere. Anhydrous pyridine (176mg, 0.18mL, 2.27mmol) was added, followed by the dropwise addition of trifluoromethane sulfonic anhydride (640mg, 0.38mL, 2.27mmol). The solution was stirred at room temperature overnight, during which time it turned dark yellow in colour. The solution was then washed with 2.0M HCl, brine, separated, dried (MgSO₄) and the solvent was evaporated *in vacuo.* The yellow oil was purified by column chromatography (2:1 heptane-ethyl acetate) to afford **(57)** (402 mg, 52%) which was immediately reacted on as it was found to be very unstable.

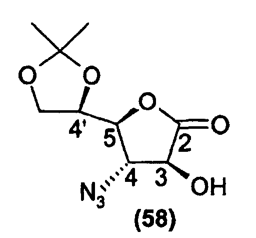

### (f) 4 (R)-Azido-5(S)-(2,2-dimethyl-[1,3]dioxolan-4 (R)-yl)-3(S)-hydroxy-dihydro-furan-2-one (58).

Triflate **(57)** (143mg, 0.38mmol) was dissolved in dry DMF (2.2mL) and stirred at room temperature. The sodium salt of trifluoroacetic acid (156mg, 1.15mmol) was added and the solution was stirred at room temperature overnight. Tlc analysis revealed no change therefore the solution was heated to 60 °C for 2 days. Then methanol (1mL) was added and the reaction was stirred overnight. The solvent was removed *in vacuo* and the residue was dissolved in dichloromethane (30mL), washed with water and brine, separated, dried (MgSO₄) and the solvent was removed *in vacuo.* The residue was purified by column chromatography using heptane / ethyl acetate (2:1, v/v) as the eluent to afford **(58)** as a white solid (24mg, 26%). R_{f} 0.24 heptane / ethyl acetate (2:1, v/v).
δ_{H} (500 MHz, DMSO at 298 K) 1.29 (3H, s, CH₃), 1.39 (3H, s, CH₃), 3.84 (1H, dd, *J* 4.6, 8.9, CH), 4.11 (1H, dd, *J* 6.9 and 8.9, CH'), 4.14 (2H, m, H-4' and H-5), 4.29 (1H, dd, *J* 4.6, 9.9, H-4), 4.44 (1H, m, H-3), 6.52 (1H, d, *J* 7.1, OH).
δ_{c} (125 MHz, DMSO at 298 K) 24.8 and 25.9 (C(CH₃)₂), 64.6 (C-4), 65.3 (CH₂), 71.7 (C-3), 74.4 (C-5), 76.8 (C-4'), 109.3 (C(CH₃)₂), 172.7 (C-2).

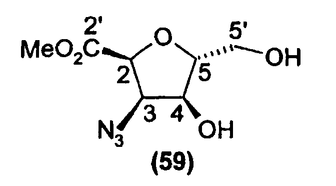

### (g) 3(R)-Azido-4(S)-hydroxy-5(R)-hydroxymethyl-tetrahydro-furan-2(S)-carboxylic acid methyl ester (59).

Acetyl chloride (46mg, 0.04mL, 0.59mmol) and dry methanol (4mL) were simultaneously added to the stirred triflate **(57)** (185mg, 0.49mmol) at room temperature under a nitrogen atmosphere. The resultant pale yellow solution was stirred for a further 20 h at room temperature and subsequent tlc analysis revealed the consumption of the starting material **(57)**. Sodium hydrogen carbonate (49mg, 0.58mmol) was added and the solution was then pre-absorbed onto silica. Purification by column chromatography using ethyl acetate / heptane (2:1, v/v) as the eluent afforded **(59)** as a white solid (73mg, 68%). R_{f} 0.13 ethyl acetate / heptane (2:1, v/v).
δ_{H} (500 MHz, CD₃OD at 298 K) 3.59 (1H, dd, *J* 3.6, 12.5, H-5'), 3.78 (3H, s, CO₂CH₃), 3.82 (1H, dd, *J* 1.7, 12.5, H-5'), 3.90 (1H, app.dt, *J* 3.0, 8.4, H-5), 4.35 (1H, app.t, *J* 4.8, H-3), 4.44 (1H, dd, *J* 5.1, 8.4, H-4), 4.70 (1H, d, *J* 4.6, H-2).
δ_{c} (125 MHz, CD₃OD at 298 K) 52.6 (CO₂CH₃), 61.8 (C-5'), 67.0 (C-3), 73.2 (C-4), 79.1 (C-5), 83.7 (C-2), 171.5 (C-2').

### Chemistry Towards P2 Hybrid Amino Acids

The general chemistry depicted in scheme 6 will shortly be published in full in the academic literature, by its inventors CS Dexter and RFW Jackson at the University of Newcastle, England.

### (a) General Procedure for the zinc coupling reactions

### (b) Zinc activation

Zinc dust (150mg, 2.3mmol, 3.0eq, Aldrich) was weighed into a 25mL round bottom flask with a side arm and fitted with a three way tap. The zinc powder was heated with a heat gun under vacuum and the flask was flushed with nitrogen and evacuated and flushed a further three times. With the flask filled with nitrogen, dry DMF (1mL) was added. Trimethylsilylchloride (30µl, 0.23mmol, 0.3eq) was added and the zinc slurry was vigorously stirred for a further 30mins.

### (c) Zinc insertion; N-(tert-Butoxycarbonyl)-3-iodozinc-L-alanine methyl ester (61)

N-(*tert*-Butoxycarbonyl)-3-iodo-L-alanine methyl ester (247mg, 0.75mmol, 1.0eq) dissolved in dry DMF (0.5mL) was added dropwise, *via* cannula, to the activated zinc slurry at 0°C prepared as described above. The reaction mixture was then allowed to warm up to room temperature and stirred for 1hr to give the organozinc reagent.

### (d) CuBr.SMe₂ preparation

Whilst the zinc insertion reaction was in progress, CuBr.SMe₂ (20mg, 0.1mmol, 0.13eq) was weighed into a 25ml round bottom flask fitted with a three way tap and dried "gently" with a heat gun under vacuum until CuBr.SMe₂ changed appearance from a brown powder to give a light green powder. Dry DMF (0.5mL) was then added followed by addition of the electrophile (either 1-bromo-2-methylbut-2-ene, toluene-4-sulfonic acid-(E)-2-methyl-but-2-enyl ester or 1-bromo-2,3-dimethylbut-2-ene) (1.0mmol, 1.3eq). The reaction mixture was then cooled to -15°C.

### (e) Coupling Reaction

Stirring of the organozinc reagent solution was stopped to allow the zinc powder to settle and the supernatant was carefully removed via cannula (care taken to avoid transferring too much zinc powder) and added dropwise to the solution of electrophile and copper catalyst. The cooling bath was removed and the solution was stirred at room temperature overnight. Ethyl acetate (20mL) was added and stirring was continued for a further 15mins. The reaction mixture was transferred to a separating funnel and a further aliquot of EtOAc (30mL) was added. The organic phase was washed successively with 1M Na₂S₂O₃ (20mL), water (2 x 20mL), brine (40mL), dried over sodium sulphate and filtered. The solvent was removed *in vacuo* and the crude product purified by flash chromatography on silica gel as described.

### (f) Hydrogenation of alkene

The alkene (1.0mmol) was dissolved in ethanol (10mL), 10% palladium on carbon (80mg) added and hydrogen introduced. Once the reaction had been deemed to have reached completion, the hydrogen was removed, the reaction filtered through Celite and the catalyst washed with ethanol (30mL). The combined organic filtrate was concentrated *in vacuo* and the alkane used directly in the subsequent reaction.

### (g) Saponification of methyl ester

The methyl ester (1.0mmol) was dissolved in THF (6mL) and whilst stirring, a solution of LiOH (1.2mmol, 1.2eq) in water (6mL) was added dropwise. Once the reaction was deemed to have reached completion, the THF was removed in *vacuo* and diethyl ether (10mL) added to the residue. The reaction mixture was then acidified with 1.0M HCl until pH =3. The organic phase was then removed and the aqueous layer extracted with diethyl ether (2 x 10mL). The combined organic extracts were dried over magnesium sulphate, filtered and the solvent removed *in vacuo* to give the carboxylic acid used directly in the subsequent reaction.

### (h) Removal of N-Boc protecting group

The N-Boc protected material (1.0mmol) was dissolved in DCM (2mL) and cooled to 0°C. Trifluoroacetic acid (2mL) was added dropwise and when the reaction was deemed to have reached completion, the solvents were removed *in vacuo* to yield the amine used directly in the subsequent reaction. Alternatively, the N-Boc protected material (1.0mmol) was cooled to 0°C and 4M HCl in dioxane (5mL) added dropwise and when the reaction was deemed to have reached completion, the solvents were removed *in vacuo* to yield the amine used directly in the subsequent reaction.

### (i) Fmoc protection of amine

The amine (1.0mmol) in 1,4-dioxane (2mL) was cooled to 0°C and 10% sodium carbonate (2.2mmol, 2.2eq, 2mL) added. The biphasic reaction mixture was stirred vigorously and Fmoc-Cl (1.1mmol, 1.1eq) added. Once the reaction was deemed to have reached completion, diethyl ether (10mL) added and the reaction mixture acidified to pH = 3 with 1M HCl. The organic phase was removed and the aqueous layer extracted with diethyl ether (2 x 10mL). The combined organic extracts were dried over sodium sulphate, filtered, the solvent removed *in vacuo* and the residue purified by flash chromatography over silica gel.

### Example Synthesis 1

### Preparation of 2S-2-(9H-fluoren-9-ylmethoxycarbonylamino)-4,4-dimethylhexanoic acid (68)

The following scheme explains how optically pure (S)-2-*tert*-Butoxycarbonylamino-4,4-dimethyl-hex-5-enoic acid methyl ester **(62)** was prepared and isolated.

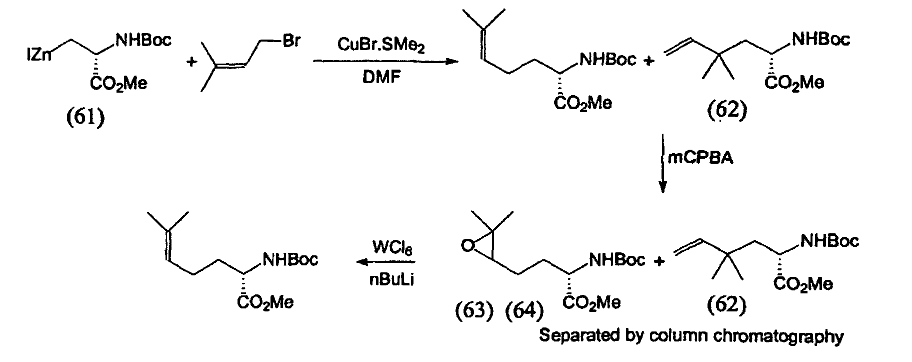

### (a) 2S-2-tert-Butoxycarbonylamino-4, 4-dimethyl-hex-5-enoic acid methyl ester (62), 2S-2-tert-butoxycarbonylamino-4-(2S-3,3-dimethyl-oxiranyl)-butyric acid methyl ester (63) and 2S-2-tert-butoxycarbonylamino-4-(2R-3,3-dimethyl-oxiranyl)-butyric acid methyl ester (64)

Following the general procedure for zinc coupling reactions, 1-bromo-3-methylbut-2-ene (115µL, 1.0mmol) was coupled to compound **(61)** (247mg, 0.75mmol) in the presence of CuBr.SMe₂ (20mg, 0.1mmol) to give a residue which was purified by flash column chromatography over silica gel eluting with EtOAc / 40:60 petroleum ether (1:9, v/v). Fractions were pooled and reduced *in vacuo* to give a mixture of regioisomers (2:1 formal SN2' *vs.* SN2), inseparable by column chromatography, as a colourless oil, yield 190mg, 93%. To a mixture of regioisomers (190mg, 0.7mmol) in chloroform (3mL) was added dropwise over 5mins, 3-chloroperbenzoic acid (156mg, 85% pure, 0.8mmol, 1.1eq) in chloroform (2mL). The reaction mixture was stirred at room temperature for a further 2hr. The reaction mixture was then washed successively with 1M Na₂S₂O₅ (5mL), saturated sodium bicarbonate solution (5mL) and brine (10mL). The organic phase was dried over sodium sulfate, filtered, the solvent removed *in vacuo* and the residue was purified by flash chromatography over silica gel eluting with EtOAc / 40:60 petroleum ether (2:8, v/v). Three products were obtained; compound (62) was eluted first and further elution afforded an inseparable mixture of compound **(63)** and compound **(64).** Fractions of the initial component were pooled and reduced *in vacuo* to give 2S-2-*tert*-butoxycarbonylamino-4,4-dimethyl-hex-5-enoic acid methyl ester **(62)** as a clear oil, yield 93mg, 49%. Electrospray-MS m/z 272 (MH⁺). Analytical HPLC Rt = 21.45mins (95%), HRMS C₁₀H₁₇O₄N requires *M*, 215.1158, found: M⁺-C₄H₈ 215.1152 (δ- 2.8 ppm); IR (cap. film)/cm⁻¹ 3369 (s), 3084 (m), 2965 (s), 1748 (s), 1715 (s), 1517 (s), 1167 (s), 1007 (s), 914 (s)
δ_{H} (500 MHz; CDCl₃) 1.06 (6H, s, CH₂=CHC(CH₃)₂), 1.42 (9H, s, C(CH₃)₃) 1.55 (1H, dd, *J* 14,9, CH₂=CHC(CH₃)₂CH₂A,), 1.82 (1H, dd, *J* 14, 3, CH₂=CHC(CH₃)₂CH_{2B}), 3.69 (3H, s, OCH₃), 4.30 (1H, m, NHCHCO₂CH₃), 4.83 (1H, br d, *J* 7, NH), 4.97 (2H, m, CH₂=CH) and 5.78 (1H, dd, *J*ₜᵣₐₙₛ 17.5, *J*_{cis} 11, CH₂=CH)
δ_{c} (125 MHz; CDCl₃) 26.93 (CH₂=CHC(CH₃)₂), 28.34 (C(CH₃)₃), 36.33 (CH₂=CHC(CH₃)₂CH₂), 45.06 (CH₂=CHC(CH₃)₂), 51.25 (NHCHCO₂CH₃), 52.15 (OCH₃), 79.77 (C(CH₃)₃), 111.39 (CH₂=CH), 146.87 (CH₂=CH), 154.97 (NHCO₂Bu^{t}) and 174.04 (CO₂CH₃).

### (b) 2S-2-tert-Butoxycarbonylamino-4,4-dimethyl-hexanoic acid methyl ester (65).

Following the general procedure for alkene hydrogenation, compound **(62)** (93mg, 0.3mmol) yielded compound **(65)** as a colourless oil, yield 90mg, 96% and used directly in the subsequent reaction. Electrospray-MS m/z 274 (MH⁺). Analytical HPLC Rt = 22.55mins (100%).

### (c) 2S-2-tert-Butoxycarbonylamino-4,4-dimethyl-hexanoic acid (66)

Following the general procedure for methyl ester saponification, compound **(65)** (90mg, 0.3mmol) gave compound **(66)** as crystals, yield 79mg, 92% and used directly in the subsequent reaction. Electrospray-MS m/z 260 (MH⁺). Analytical HPLC Rt = 20.90mins (100%).

### (d) 2S-2-Amino-4,4-dimethyl-hexanoic acid trifluoroacetic acid salt (67)

Following the general procedure of N-Boc removal using TFA, compound **(66)** (79mg, 0.3mmol) gave compound **(67)** as a solid, yield 80mg, 96% and used directly in the subsequent reaction. Electrospray-MS m/z 274 (MH⁺).

### (e) 2S-2-(9H-Fluoren-9-ylmethoxycarbonylamino)-4,4-dimethyl-hexanoic acid (68)

Following the general procedure for Fmoc protection of an amine, compound **(67)** (80mg, 0.3mmol) gave on purification by flash chromatography over silica gel eluting with CHCl₃/CH₃OH (100:0 to 96:4, v/v) 2S-2-(9*H*-fluoren-9-ylmethoxycarbonylamino)-4,4-dimethyl-hexanoic acid **(68)** as a solid, yield 60mg, 54%. Electrospray-MS m/z 382(MH⁺). Analytical HPLC Rt = 23.63mins (100%); [α]_{D}¹⁷ -18.4 (c 0.25 in EtOH)
δ_{H} (500MHz, CDCl₃) 0.88 (3H, t, *J* 7, CH₃CH₂), 0.95 (6H, s, CH₃CH₂C(CH₃)₂), 1.31 (2H, m, CH₃CH₂), 1.46 (1H, dd, *J* 14.5, 10, CH₃CH₂C(CH₃)₂CH_{2A}), 1.85 (1H, br d, *J* 14.5, CH₃CH₂C(CH₃)₂CH_{2B}), 4.21_(1H, t, *J* 6.5, CH-Fmoc), 4.41 (3H, m, NHCHCO₂H and CH₂O), 5.02 (1H, br d, *J* 8, NH-Fmoc), 7.29 (2H, m, H-2' and H-7'), 7.38 (2H, m, H-3' and H-6'), 7.58 (2H, m, H-1' and H-8') and 7.74 (2H, d, *J* 7, H-4' and H-5').

### Example Synthesis 2

### Preparation of 2S,4RS-2-(9H-Fluoren-9-ylmethoxycarbonylamino)-4,5-dimethyl-hexanoic acid (74)

Optically pure 2S,4S-2-*tert*-Butoxycarbonylamino-4,5-dimethyl-hex-5-enoic acid methyl ester **(69)** and 2S,4R-2-*tert*-Butoxy-carbonylamino-4,5-dimethyl-hex-5-enoic acid methyl ester **(70)** were obtained directly after zinc coupling reaction by flash chromatography.

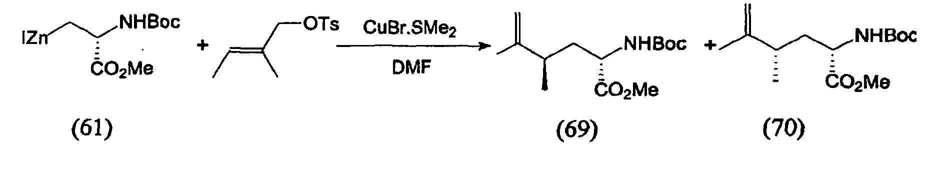

### (a) 2S,4S-2-tert-Butoxycarbonylamino-4,5-dimethyl-hex-5-enoic acid methyl ester (69) and 2S,4R-2-tert-butoxycarbonylamino-4,5-dimethyl-hex-5-enoic acid methyl ester (70)

Following the general procedure for zinc coupling reactions, toluene-4-sulfonic acid (E)-2-methyl-but-2-enyl ester (1.45mL, 1.0mmol) was coupled to compound **(61)** (247mg, 0.75mmol) in the presence of CuBr.SMe₂ (20mg, 0.10mmol) to give a residue which was purified by flash chromatography over silica gel eluting with EtOAc / 40:60 petroleum ether (1:9, v/v) to give two diastereoisomers. Analytical HPLC Rt = 22.49mins (60%) and Rt = 22.52mins (40%). Fractions of the first eluted component were pooled to give one of the diastereoisomers obtained as a colourless oil, yield 36mg, 18%. Next a mixture of the diastereomers as a colourless oil, yield 75mg, 37% was obtained. Pure fractions containing the later eluted component were pooled to give the other diastereoisomer as a colourless oil, yield 19mg, 9%. (The stereochemistry at the 4 position was not investigated). Spectral data obtained for the fast running diastereomer: Electrospray-MS m/z 272 (MH⁺); [α]_{D}²⁰ +12.3 (c 1.06 in CHCl₃); IR (cap. film)/cm⁻¹ 3382 (s), 3070 (m), 2966 (s), 1746 (s), 1716 (s), 1616 (w), 1507 (s), 886 (m)
δ_{H} (500 MHz, CDCl₃) 1.06 (3H, d, *J* 7, CH₃CH), 1.45 (9H, s, C(CH₃)₃), 1.58 (1H, m, CH₃CH), 1.68 (3H, s, CH₃C=CH₂), 1.85 (1H, m, CH_{2A}CH), 1.97 (1H, m, CH_{2B}CH), 3.73 (3H, s, OCH₃), 4.29 (1H, m, NHCHCO₂CH₃), 4.72 (1H, s, CH_{2A}=CH), 4.95 (1H, d, *J* 1.5, CH_{2B}=CH) and 5.04 (1H, d, *J* 7, NH) δ_{c} (125 MHz, CDCl₃) 18.61 (CH₃C=CH₂), 21.64 (CH₃CH), 28.32 (C(CH₃)₃), 30.79 (CH₃CHCH₂), 38.06 (CH₂CHNH), 52.00 (NHCHCO₂CH₃), 52.22 (OCH₃), 79.53 (C(CH₃)₃), 110.19 (CH₂=C(CH₃)), 144.62 (CH₂=C(CH₃)), 155.18 (OCONH) and 173.30 (CO₂CH₃).

Spectral data obtained for the slow running diastereoisomer: Electrospray-MS m/z 272 (MH⁺); [α]_{D}²⁰ +16.0 (c 0.60 in CHCl₃); IR (cap. film)/cm⁻¹ 3369 (s), 3073 (m), 2969 (s), 1747 (s), 1717 (s), 1617 (w), 1517 (s), 893 (m)
δ_{H} (500 MHz, CDCl₃) 1.04 (3H, d, *J* 7, CH₃CH), 1.44 (9H, s, C(CH₃)₃), 1.55 (1H, m, CH₃CH), 1.67 (3H, s, CH₃C=CH₂), 1.91 (1H, m, CH_{2A},CH), 2.37 (1H, m, CH_{2B}CH), 3.73 (3H, s, OCH₃), 4.26 (1H, m, NHCHCO₂CH₃), 4.75 (1H, d, *J* 1.5, CH_{2A}=CH), 4.79 (1H, d, *J* 1.5, CH_{2B}=CH) and 5.46 (1H, d, *J* 6.1, NH) δ_{c} (125 MHz, CDCl₃) 18.51 (CH₃C=CH₂), 20.14 (CH₃CH), 28.31 (C(CH₃)₃), 30.55 (CH₃CHCH₂), 37.64 (CH₂CHNH), 52.17 (NHCHCO₂CH₃), 52.22 (OCH₃), 79.74 (C(CH₃)₃), 111.27 (CH₂=C(CH₃)), 147.94 (CH₂=C(CH₃)), 155.36 (OCONH) and 173.83 (CO₂CH₃).

These diastereoisomers were not separated routinely and used as a mixture in subsequent reactions.

### (b) 2S,4RS-2-tert-Butoxycarbonylamino-4,5-dimethyl-hexanoic acid methyl ester (71)

Following the general procedure for alkene hydrogenation, compounds **(69)** and compound **(70)** (130mg, 0.48mmol) yielded a mixture of two diastereoisomers **(71)** which were not separated, obtained as a colourless oil, yield 128mg, 98%. Analytical HPLC Rt 22.49mins, electrospray-MS m/z 274 (MH⁺).

### (c) 2S,4RS-2-tert-Butoxycarbonylamino-4,5-dimethyl-hexanoic acid (72)

Following the general procedure for methyl ester saponification, compounds **(71)** (128mg, 0.47mmol) gave a inseparable mixture of compounds **(72)** as a colourless oil, yield 106mg, 87%. Electrospray-MS m/z 260 (MH⁺). Analytical HPLC Rt = 20.65mins (100%).

### (d) 2S,4RS-2-Amino-4,5-dimethyl-hexanoic acid trifluoroacetic acid salt (73)

Following the general procedure of N-Boc removal using TFA, compounds **(72)** (106mg, 0.41mmol) gave an inseparable mixture of compounds **(73)** as a solid, yield 107mg, 96% and used directly in the subsequent reaction. Electrospray-MS m/z 160 (MH⁺).

### (e) 2S,4RS-2-(9H-Fluoren-9-ylmethoxycarbonylamino)-4,5-dimethyl-hexanoic acid (74)

Following the general procedure for Fmoc protection of an amine, compounds **(73)** (107mg, 0.39mmol) gave on purification by flash chromatography over silica gel eluting with CHCl₃ / CH₃OH (100:0 to 95:5, v/v) 2S,4RS-2-(9H-fluoren-9-ylmethoxycarbonylamino)-4,5-dimethyl-hexanoic acid (**74**) as a solid, yield 60mg, 40% as a mixture of two diastereoisomers. Analytical HPLC Rt = 23.83mins (40%) and Rt = 24.06mins (60%). First eluted diastereomer: Electrospray-MS m/z 382 (MH⁺). Later eluted diastereomer: Electrospray-MS m/z 382 (MH⁺).

### Example Synthesis 3

### Preparation of 2S,5RS-2-(9H-Fluoren-9-ylmethoxycarbonylamino)-5,6-dimethyl-heptanoic acid (80) and 2S-2-(9H-Fluoren-9-ylmethoxycarbonylamino)-4,4,5-trimethyl-hexanoic acid (84)

(S)-2-*tert*-butyloxycarbonylamino-5,6-dimethyl-hept-5-enoic methyl ester **(75)** and (S)-2-*tert*-butyloxycarbonylamino-4,4,5-trimethyl-hex-5-enoic methyl ester **(76)** were obtained directly after zinc coupling reaction by flash chromatography.

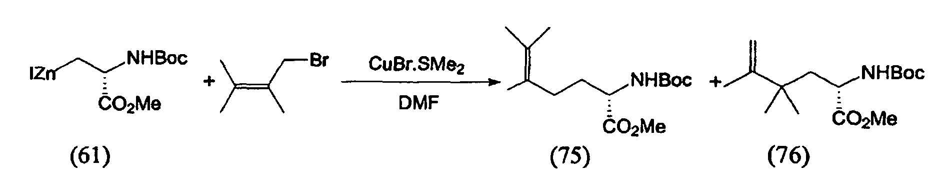

### (a) 2S-2-tert-Butyloxycarbonylamino-5,6-dimethyl-hept-5-enoic methyl ester (75) and 2S-2-tert-butyloxycarbonylamino-4,4,5-trimethyl-hex-5-enoic methyl ester (76)

Following the general procedure for zinc coupling reactions, 1-bromo-2,3-dimethylbut-2-ene (163mg, 1.0mmol) was coupled to compound **(61)** (247mg, 0.75mmol) in presence of CuBr.SMe₂ (20mg, 0.10mmol) to give a residue which on purification by flash chromatography over silica gel eluting with EtOAc/ 40:60 petroleum ether (1:9) gave two regioisomers. The first eluted component compound **(75)** as a colourless oil, yield 60mg, 28% and the second eluted component was compound **(76)** as a colourless oil, yield 51mg, 24%.

Spectral data obtained for compound **(75);** Electrospray-MS m/z 285 (MH⁺). Analytical HPLC Rt = 22.85mins (100%); HRMS C₁₅H₂₇NO₄ requires *M*, 285.1940, found: M⁺ 285.1954 (δ - 4.9 ppm); [α]_{D}²² +26.1 (c 1.01 in CH₂Cl₂); elemental analysis C₁₅H₂₇NO₄ (req) %C 63.1, %H 9.5, %N 4.9, (fnd) %C 62.4, %H 9.6, %N 5.3; IR (cap. film)/cm⁻¹ 3366 (s), 3154 (m), 2978 (s), 1744 (s), 1718 (s), 1506 (s), 1366 (s), 1164 (s)
δ_{H} (500 MHz, CDCl₃) 1.45 (9H, s, C(CH₃)₃), 1.62 (9H, m, (CH₃)₂=C(CH₃)), 1.87 (1H, m, CH_{2A}CH₂CH), 2.03 (1H, m, CH_{2B}CH₂CH), 2.09 (1H, dd, *J* 6, 10.5, CH₂CH_{2A}CH), 2.12 (1H, dd, *J* 6.5, 10.5, CH₂CH_{2B}CH), 3.74 (3H, s, OCH₃), 4.29 (1H, m, NHCHCO₂CH₃) and 5.02 (1H, d, *J* 7, NH)
δ_{c} (125 MHz, CDCl₃) 18.19 ((CH₃)₂C=C(CH₃)), 20.00 ((CH₃)_{2cis}C=C(CH₃)), 20.61 ((CH₃)₂ₜᵣₐₙₛC=C(CH₃)), 28.33 (C(CH₃)₃), 30.07 (CH₂CH₂CH), 30.92 (CH₂CH₂CH), 52.20 (NHCHCO₂CH₃), 53.47 (OCH₃), 80.00 (C(CH₃)₃), 95.90 ((CH₃)₂C=C(CH₃)), 96.49 ((CH₃)₂C=C(CH₃), 155.33 (OCONH) and 173.42 (CO₂CH₃).

Spectral data obtained for compound (76); Electrospray-MS m/z 285 (MH⁺). Analytical HPLC Rt = 22.91mins (100%); HRMS C₁₁H₁₉NO₄ requires *M* 229.1314, found: M⁺-C₄H₈ 229.1309 (δ - 2.2 ppm); [α]_{D}²³ +4.8 (c 1.01 in CH₂Cl₂); elemental analysis C₁₅H₂₇NO₄ (req) %C 63.1, %H 9.5, %N 4.9, (fnd) %C 62.5, %H 9.5, %N; IR (cap. film)/cm⁻¹ 3368 (s), 3091 (m), 2934 (s), 1748 (s), 1717 (s), 1516 (s)
δ_{H} (500 MHz, CDCl₃) 1.10 (3H, s, (CH₃)_{2A}C), 1.12 (3H, s, (CH₃)_{2B}C), 1.43 (9H, s, C(CH₃)₃), 1.60 (1H, m, CH_{2A}CH), 1.74 (3H, s, CH₃C=CH₂), 1.92 (1H, dd, *J* 14.5, 4, CH_{2B}CH), 3.70 (3H, s, OCH₃), 4.24 (1H, m, NHCHCO₂CH₃), 4.79 (1H, s, CH_{2A}=C(CH₃)), 4.82 (1H, s, CH_{2B}=C(CH₃)) and 4.83 (1H, br d, *J* 11, NH)
δ_{c} (125 MHz, CDCl₃) 19.38 (CH₃), 27.19 (CH₃), 27.61 (CH₃), 28.34 (C(CH₃)₃), 38.50 (CH₂CH), 38.95 ((CH₃)₂C) , 51.34 (NHCHCO₂CH₃), 52.13 (OCH₃), 79.71 (C(CH₃)₃), 110.95 (CH₂=C(CH₃)), 150.62 (CH₂=C(CH₃)), 155.00 (OCONH) and 174.24 (CO₂CH₃).

### (b) 2S,5RS-2-tert-Butoxycarbonylamino-5,6-dimethyl-heptanoic acid methyl ester (77)

Following the general procedure for alkene hydrogenation, 2S-2-*tert*-butyloxycarbonylamino-5,6-dimethyl-hept-5-enoic methyl ester **(75)** (60mg, 0.21mmol) yielded compound **(77)** as a colourless oil, yield 54mg, 89%. Electrospray-MS m/z 288 (MH⁺). Analytical HPLC Rt = 24.06mins (100%).

### (c) 2S,5RS-2-tert-Butoxycarbonylamino-5,6-dimethyl-heptanoic acid (78)

Following the general procedure for methyl ester saponification, compounds **(77)** (54mg, 0.19mmol) gave compounds **(78)** as a colourless oil, yield 54mg, 100%. Electrospray-MS m/z 274 (MH⁺). Analytical HPLC Rt = 21.44mins (100%).

### (d) 2S,5RS-2-Amino-5,6-dimethyl-heptanoic acid hydrochloride salt (79)

Following the general procedure of N-Boc removal using 4M HCl in dioxane, compounds **(78)** (54mg, 0.20mmol) gave compounds **(79)** as a solid, yield 40mg, 97%. Electrospray-MS m/z 174 (MH⁺).

### (e) 2S,5RS-2-(9H-Fluoren-9-ylmethoxycarbonylamino)-5,6-dimethyl-heptanoic acid (80)

Following the general procedure for Fmoc protection of an amine, compounds **(79)** (40mg, 0.19mmol) gave on purification by flash chromatography over silica gel eluting with CHCl₃ / CH₃OH (100:0 to 95:5, v/v) 2S,5RS-2-(9H-fluoren-9-ylmethoxycarbonylamino)-5,6-dimethyl-heptanoic acid **(80)** as a solid, yield 27mg, 36%.

Electrospray-MS m/z 395 (MH⁺). Analytical HPLC Rt = 24.52mins (100%), HRMS C₂₄H₂₉O₄NNa requires *M* 418.1994, found: MNa⁺, 418.1993. (δ - 0.38 ppm)
δ_{H} (500 MHz; CDCl₃) 0.73 (6H, m, (CH₃)₂CH), 0.82 (3H, d, *J* 6.5, (CH₃)₂CHCH(CH₃)), 1.23 (1H, m, (CH₃)₂CHCH(CH₃)CH_{2A}), 1.39 (1H, m, (CH₃)₂CHCH(CH₃)CH_{2B}), 1.55 (2H, m, (CH₃)₂CHCH(CH₃) and (CH₃)₂CHCH(CH₃)CH₂CH_{2A}), 1. 63 (1H, m, (CH₃)₂CHCH(CH₃)), 1. 90 (1H, m, (CH₃)₂CHCH(CH₃)CH₂CH_{2B}), 4.18 (1H, t, *J* 6.5, CH-Fmoc), 4.40 (3H, m, NHCHCO₂H and CH₂O 5.30 (1H, br d, *J* 8, NH-Fmoc), 7.27 (2H, m, H-2' and H-7'), 7.37 (2H, m, H-3' and H-6'), 7.56(2H, m, H-1' and H-8') and 7.75 (2H, d, J 7, H-4' and H-5')
δ_{c} (125 MHz; CDCl₃) 14.91 (CH₃)₂CHCH(CH₃)), 17.49 and 17.73 ((CH₃)_{2A}CH), 19.93 and 20.05 ((CH₃)_{2B}CH), 28.08 ((CH₃)₂CH), 29.26 and 29.44 ((CH₃)₂CHCH(CH₃)CH₂CH₂), 30.04 and 30.17 ((CH₃)₂CHCH(CH₃)CH₂CH₂), 31.38 and 31.68 ((CH₃)₂CHCH(CH₃)), 37.89 and 38.07 (NHCHCO₂H), 46.88 (CH-1'), 66.84 (CH₂O), 119.72 (CH-5' and CH-10'), 124.80 (CH-4' and CH-11'), 126.81 (CH-6' and CH-9'), 127.46 (CH-3' and CH-12'), 141.05 ( C-7' and C-8'), 143.47 (C-2' and C-13') and 155.89 (OCONH). The quaternary signal for the carboxylic acid was not observed.

### (f) 2S-2-tert-Butoxycarbonylamino-4,4,5-trimethyl-hexanoic acid methyl ester (81)

Following the general procedure for alkene hydrogenation, 2S-2-*tert*-butyloxycarbonylamino-4,4,5-trimethyl-hex-5-enoic methyl ester **(76)** (51mg, 0.18mmo1) yielded compound **(81)** as a colourless oil, yield 46mg, 90%. Electrospray-MS m/z 288 (MH⁺). Analytical HPLC Rt = 22.91mins (100%).

### (g) 2S-2-tert-Butoxycarbonylamino-4,4,5-trimethyl-hexanoic acid (82)

Following the general procedure for methyl ester saponification, compound **(81)** (46mg, 0.16mmol) gave compound **(82)** as a colourless oil, yield 44mg, 100%. Electrospray-MS m/z 274 (MH⁺).

### (h) 2S-2-Amino-4,4,5-trimethyl-hexanoic acid hydrochloride salt (83)

Following the general procedure of N-Boc removal using 4M HCl in dioxane, compound **(82)** (44mg, 0.16mmol) gave compound **(83)** as a solid, yield 33mg, 99%. Electrospray-MS m/z 174 (MH⁺) .

### (i) 2S-2-(9H-Fluoren-9-ylmethoxycarbonylamino)-4,4,5-trimethyl-hexanoic acid (84)

Following the general procedure for Fmoc protection of an amine, compound **(83)** (33mg, 0.16mmol) gave on purification by flash chromatography over silica gel eluting with CHCl₃/CH₃OH (100:0 to 95:5, v/v) 2S-2-(9H-fluoren-9-ylmethoxycarbonylamino)-4,4,5-trimethyl-hexanoic acid **(84)** as a solid, yield 20mg, 32%. Electrospray-MS m/z 396 (MH⁺). Analytical HPLC Rt = 24.28mins (100%), HRMS C₂₄H₂₉O₄NNa requires M 418.1994, found: MNa⁺, 418.1993. (δ-0.38 ppm)
δ_{H} (500 MHz; CDCl₃) 0.93 (9H, m, (CH₃)₂CHC(CH₃)_{2A}), 0.98 (3H, s, (CH₃)₂CHC(CH₃)_{2B}), 1.48 (1H, dd, *J* 14, 10, (CH₃)₂CHC(CH₃)₂CH_{2A}), 1.57 (1H, m, (CH₃)₂CH), 1.91 (1H, d, *J* 14, (CH₃)₂CHC(CH₃)₂CH_{2B}), 4.21 (1H, t, *J* 6.5, CH-Fmoc), 4.40 (3H, m, NHCHCO₂H and CH₂O), 5.10 (1H, br d, *J* 7.5, NH-Fmoc), 7.27 (2H, m, H-2' and H-7'), 7.36 (2H, m, H-3' and H-6'), 7.57 (2H, m, H-1' and H-8') and 7.74 (2H, d, *J* 7, H-4' and H-5')
δ_{c} (125 MHz; CDCl₃) 17.01 ((CH₃)_{2A}CH), 17.16 ((CH₃)_{2B}CH), 23.69 ((CH₃)₂CHC(CH₃)_{2A}), 24.27 ((CH₃)₂CHC(CH₃)_{2B}), 35.27 ((CH₃)₂CHC(CH₃)₂), 35.73 ((CH₃)₂CH), 41.88 ((CH₃)₂CHC(CH₃)₂CH₂), 46.93 (CH-1'), 54.20 (NHCHCO₂H) , 66.79 (CH₂O), 119.70 (CH-5' and CH-10'), 124.78 (CH-4' and CH-11'), 126.79 (CH-6' and CH-9'), 127.44 (CH-3' and CH-12'), 141.05 ( C-7' and C-8'), 143.61 (C-2' and C-13') and 155.68 (OCONH). The quaternary signal for the carboxylic acid was not observed.

### General Solid Phase procedures

Molecules were assembled using the furanone building blocks and novel protected amino acids described earlier, by solid phase procedures on Chiron multipins following the protocols detailed below.

### Preparation of Crown Assembly

The compounds were synthesised in parallel fashion using the appropriately loaded Fmoc-Building block-linker-DA/MDA derivatised macrocrowns (see above) loaded at approximately 3.5 - 9.1 µmoles per crown. Prior to synthesis each crown was connected to its respective stem and slotted into the 8 x 12 stem holder. Coupling of the amino acids employed standard Fmoc amino acid chemistry as described in 'Solid Phase Peptide Synthesis', E. Atherton and R.C. Sheppard, IRL Press Ltd, Oxford, UK, 1989.

### Removal of Nα-Fmoc Protection

A 250 mL solvent resistant bath is charged with 200 mL of a 20% piperidine/DMF solution. The multipin assembly is added and deprotection allowed to proceed for 30 minutes. The assembly is then removed and excess solvent removed by brief shaking. The assembly is then washed consecutively with (200 mL each), DMF (5 minutes) and MeOH (5 minutes, 2 minutes, 2 minutes) and left to air dry for 15 minutes.

### Quantitative UV Measurement of Fmoc Chromophore Release

A 1cm path length UV cell is charged with 1.2 mL of a 20% piperidine/DMF solution and used to zero the absorbance of the UV spectrometer at a wavelength of 290nm. A UV standard is then prepared consisting of 5.0 mg Fmoc-Asp(OBut)-Pepsyn KA (0.08 mmol/g) in 3.2 mL of a 20% piperidine/DMF solution. This standard gives Abs₂₉₀ = 0.55-0.65 (at room temperature). An aliquot of the multipin deprotection solution is then diluted as appropriate to give a theoretical AbS₂₉₀ = 0.6, and this value compared with the actual experimentally measured absorbance showing the efficiency of previous coupling reaction.

### Standard Coupling Of Amino Acid Residues

Coupling reactions are performed by charging the appropriate wells of a polypropylene 96 well plate with the pattern of activated solutions required during a particular round of coupling. Macrocrown standard couplings were performed in DMF (500 µl).

### Coupling of an Amino-acid Residue To Appropriate Well

Whilst the multipin assembly is drying, the appropriate N_{α}-Fmoc amino acid pfp esters (10 equivalents calculated from the loading of each crown) and HOBt (10 equivalents) required for the particular round of coupling are accurately weighed into suitable containers.

Alternatively, the appropriate N_{α}-Fmoc amino acids (10 equivalents calculated from the loading of each crown), desired coupling agent e.g. HBTU (9.9 equivalents calculated from the loading of each crown) and activation e.g. HOBt (9.9 equivalents calculated from the loading of each crown), NMM (19.9 equivalents calculated from the loading of each crown) are accurately weighed into suitable containers.
The protected and activated Fmoc amino acid derivatives are then dissolved in DMF (500 µl for each macrocrown e.g. for 20 macrocrowns, 20 x 10 eq. x 7 µmoles of derivative would be dissolved in 10 mL DMF). The appropriate derivatives are then dispensed to the appropriate wells ready for commencement of the 'coupling cycle'. As a standard, coupling reactions are allowed to proceed for 6 hours. The coupled assembly was then washed as detailed below.

### Washing Following Coupling

If a 20% piperidine/DMF deprotection is to immediately follow the coupling cycle, then the multipin assembly is briefly shaken to remove excess solvent washed consecutively with (200 mL each), MeOH (5 minutes) and DMF (5 minutes) and de-protected. If the multipin assembly is to be stored or reacted further, then a full washing cycle consisting brief shaking then consecutive washes with (200 mL each), DMF (5 minutes) and MeOH (5 minutes, 2 minutes, 2 minutes) is performed.

### Addition of Capping Group

Whilst the multipin assembly is drying, the appropriate acid capping group (10 equivalents calculated from the loading of each crown), desired coupling agent e.g. HBTU (9.9 equivalents calculated from the loading of each crown) and activation e.g. HOBt (9.9 equivalents calculated from the loading of each crown), NMM (19.9 equivalents calculated from the loading of each crown) are accurately weighed into suitable containers. The acid derivatives / coupling agents are then dissolved in DMF (500 µl for each macrocrown e.g. for 20 macrocrowns, 20 x 10 eq. of derivative would be dissolved in 10 mL DMF) and left to activate for 5 minutes. The appropriate derivatives are then dispensed to the appropriate wells ready for commencement of the 'capping cycle'. As a standard, capping reactions are allowed to proceed for 18 hours overnight. The capped assembly was then washed as detailed above.

### Acidolytic Mediated Cleavage of Molecule-Pin Assembly

Acid mediated cleavage protocols are strictly performed in a fume hood. A polystyrene 96 well plate (1 mL/well) is labelled and weighed to the nearest mg. Appropriate wells are then charged with a trifluoroacetic acid/water (95:5, v/v, 600 µl) cleavage solution, in a pattern corresponding to that of the multipin assembly to be cleaved.
The multipin assembly is added, the entire construct covered in tin foil and left for 2 hours. The multipin assembly in then added to another polystyrene 96 well plate (1 mL/well) containing trifluoroacetic acid/water (95:5, v/v, 600 µl) (as above) for 5 minutes.

### Work up of Cleaved Molecules

The primary polystyrene cleavage plate (2 hour cleavage) and the secondary polystyrene plate (5 minute wash) are then placed in the GeneVac evaporator and the solvents removed (minimum drying rate) for 90 minutes. The contents of the secondary polystyrene plate are transferred to their corresponding wells on the primary plate using an acetonitrile/water (50: 50 v/v/v) solution (3 x 150 µl) and the spent secondary plate discarded. Aliqouts (5-20µL) are taken for analysis. The plate was covered in tin foil, pin-pricked over wells containing compounds, placed into the freezer for 1hr, then lyophilised.

### Analysis and Purification of Molecules

The (5-20µL) aliquots are analysed by analytical HPLC and electrospray-MS. In virtually all cases, crude purities are >90% by HPLC with the desired m/z. Sample were purified by semi-preparative reverse phase HPLC, using Vydac C₄.Appropriate fractions are combined and lyophilised in tared 10mL glass vials, then re-weighed. Molecules were prepared on a 15-90µmole scale, yielding 2.0-26.0mg of purified products. The purity of each product was confirmed by analytical HPLC at >95% (215nm UV detection) and gave the appropriate [MH]⁺ by electrospray mass spectrometry analysis.

### Loading of Macrocrowns With Constructs

### General method for the loading of multipins with Dihydro-3(2H)-Furanone - Linker Constructs (29-34)

Amino functionalised DA/MDA macrocrowns (ex Chiron Mimotopes, Australia, 9.1µmole loading) or amino functionalised HEMA gears (ex Chiron Mimotopes, Australia, 1.3µmole loading) were used for all loadings and subsequent solid phase syntheses.
Dihydro-3(2H)-Furanone - Linker Construct (29-34) (3eq compared to total surface functionalisation of crowns / gears) was carboxyl activated with 2-(1H-benzotriazole-1-yl)-1,1,3,3-tetramethyluronium hexafluorophosphate (3eq), 1-hydroxybenzotriazole (3eq) and N-methylmorpholine (6eq) in dimethylformamide (5mL) for 5mins. This mixture was added to the crowns / gears, additional DMF added to cover the reaction surface and the mixture left overnight. Standard washing and Fmoc deprotection readings (see procedures above) indicated virtually quantitative loading.

Exemplar molecules prepared by the methods detailed above are shown in Tables 1 and 2, together with a Ki (µM) measurement of inhibition verses mammalian, murine and rat cathepsins S and mammalian L and K.

The moieties shown in the following Tables 1 and 2 use the terms "CAP", "P1" and "P2" which can be cross referenced to the generic formula (II) as follows: R1=CAP: R2=H; R3=the side chain of the amino acid indicated as P2; R4=H, R5=is the 5-substituent of the depicted furanone where the two right-most columns are comparative examples; and R6=H.

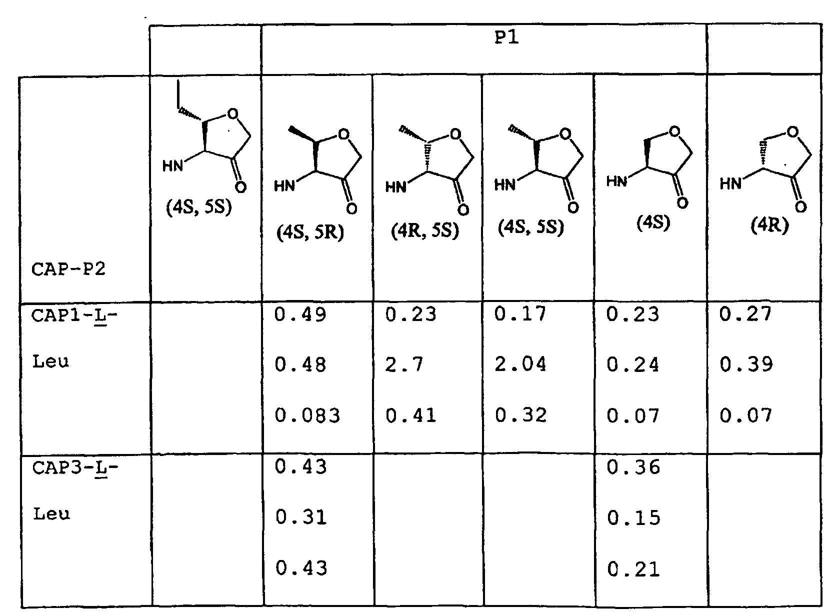

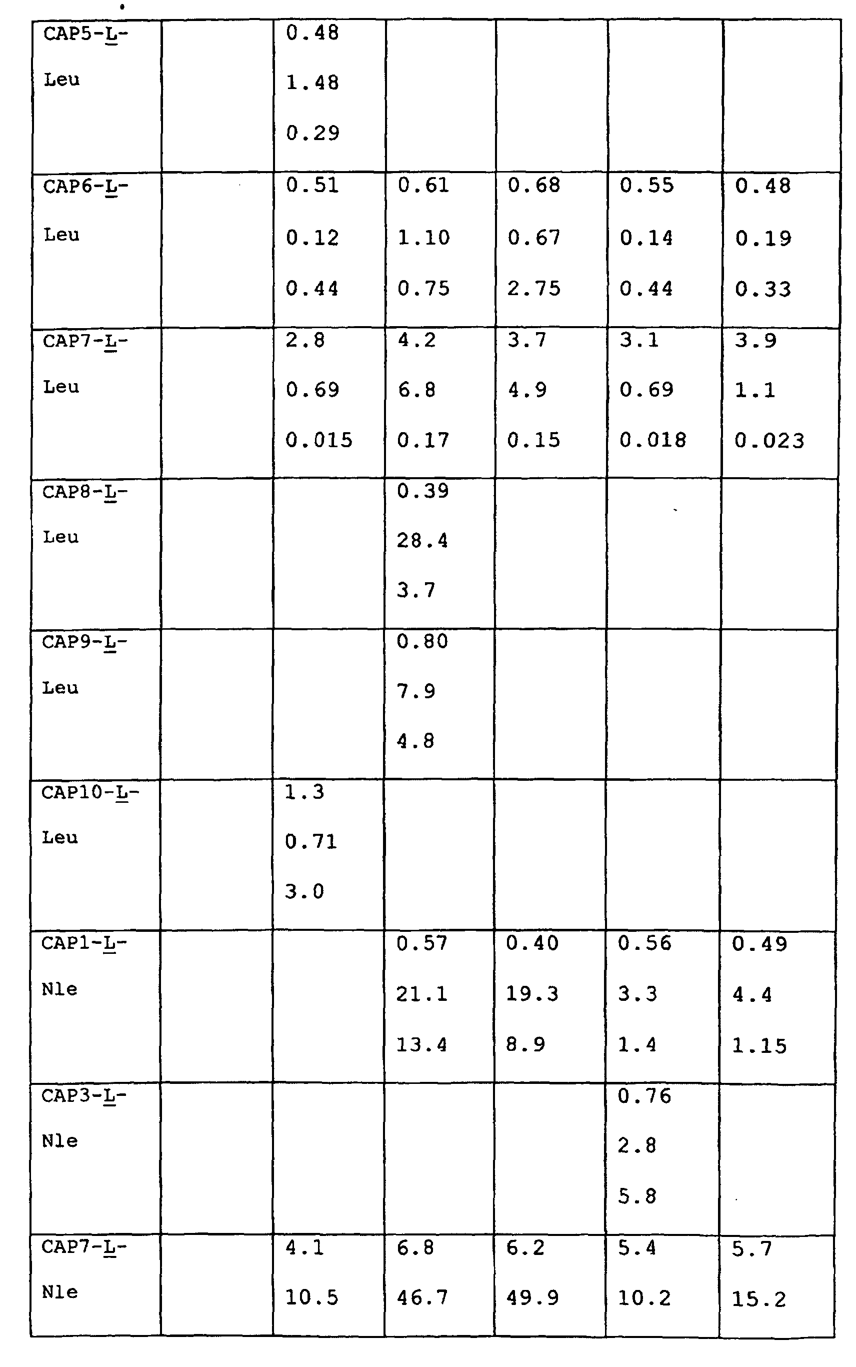

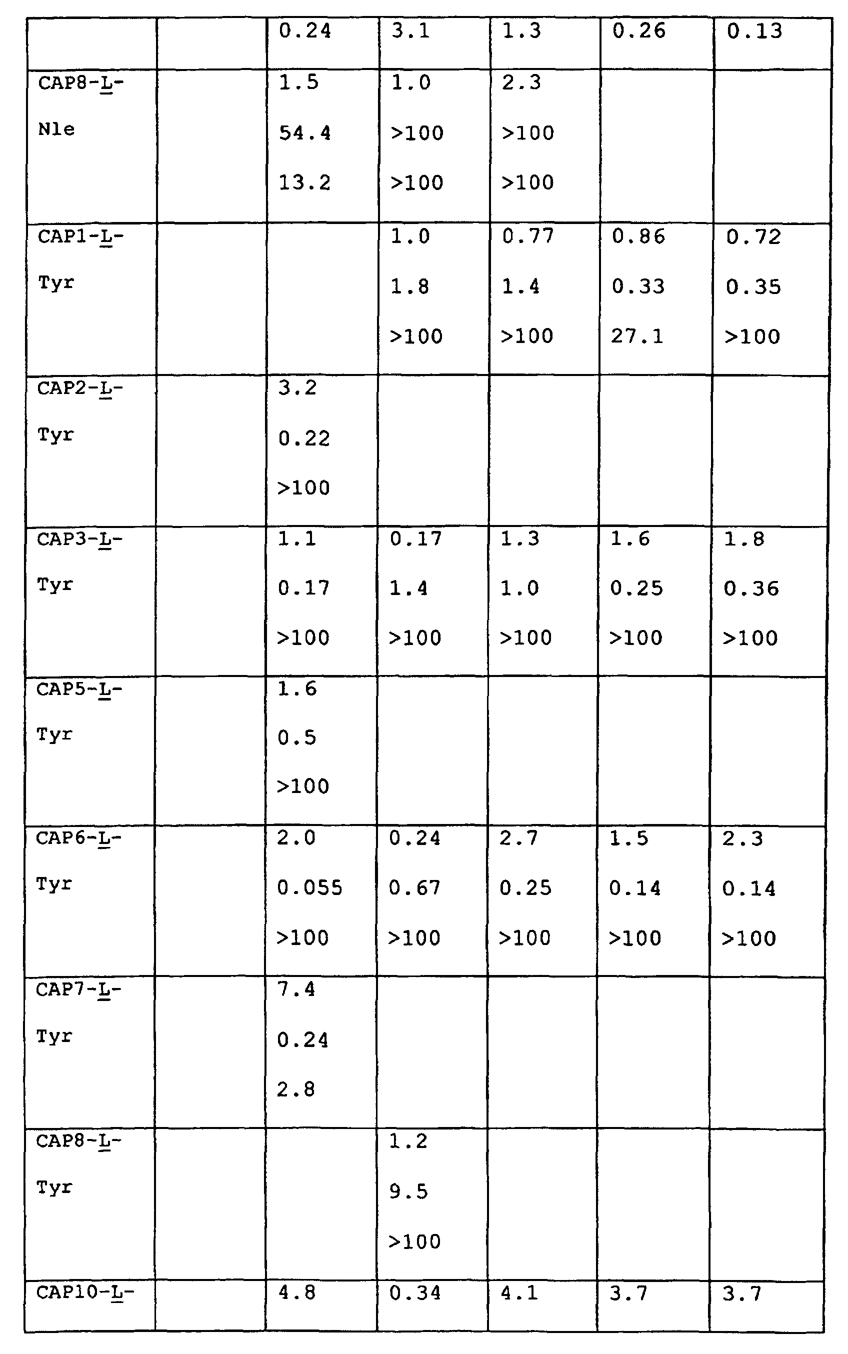

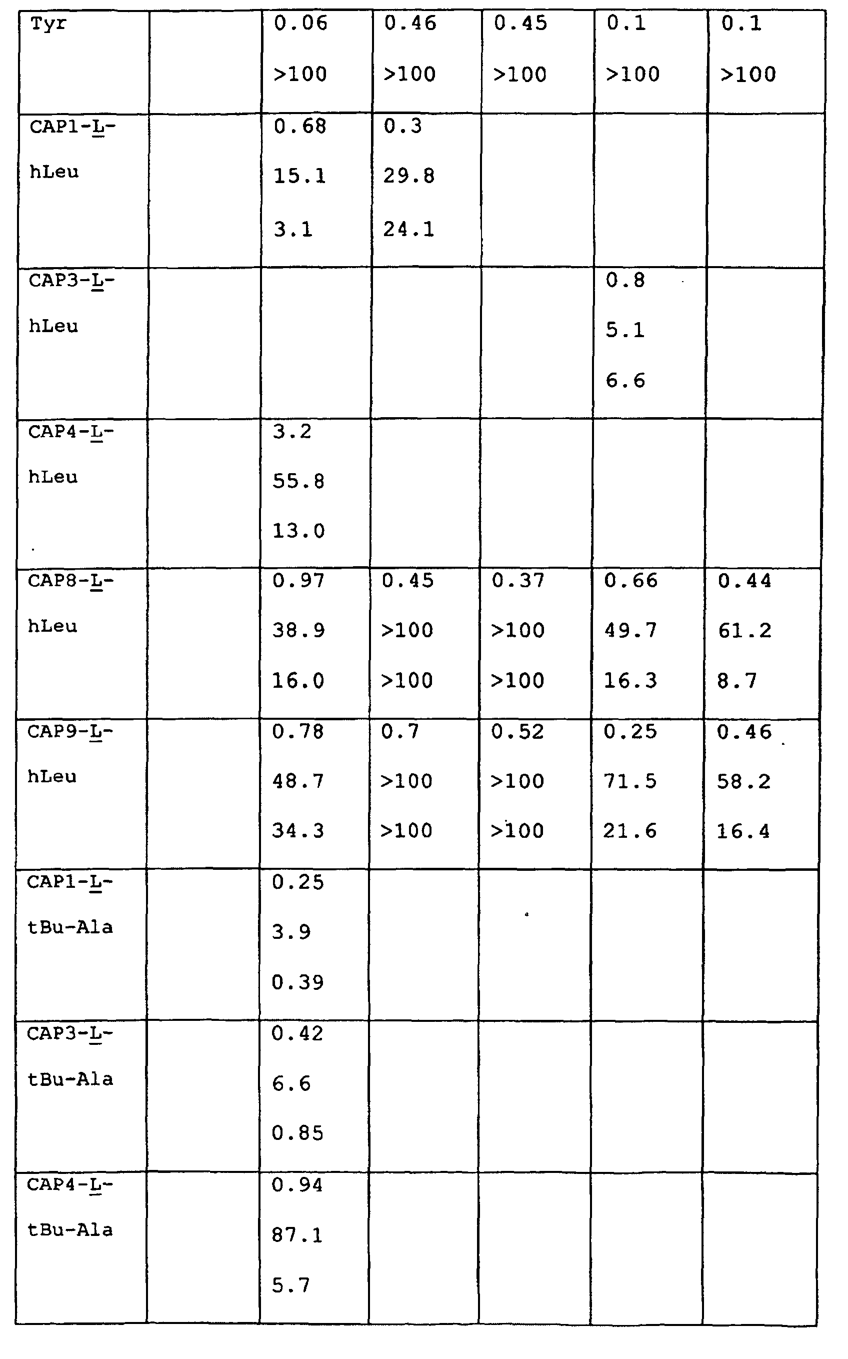

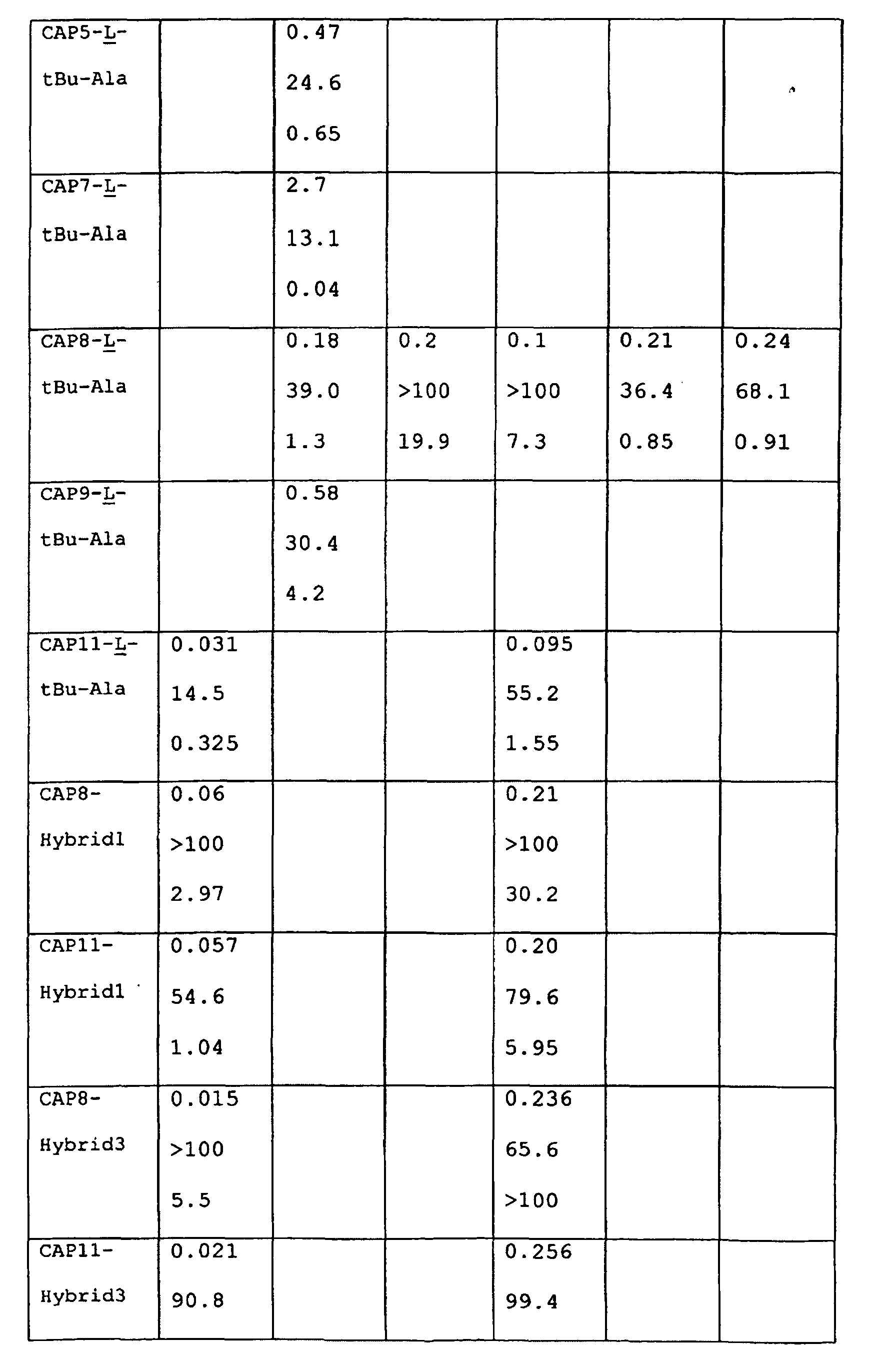

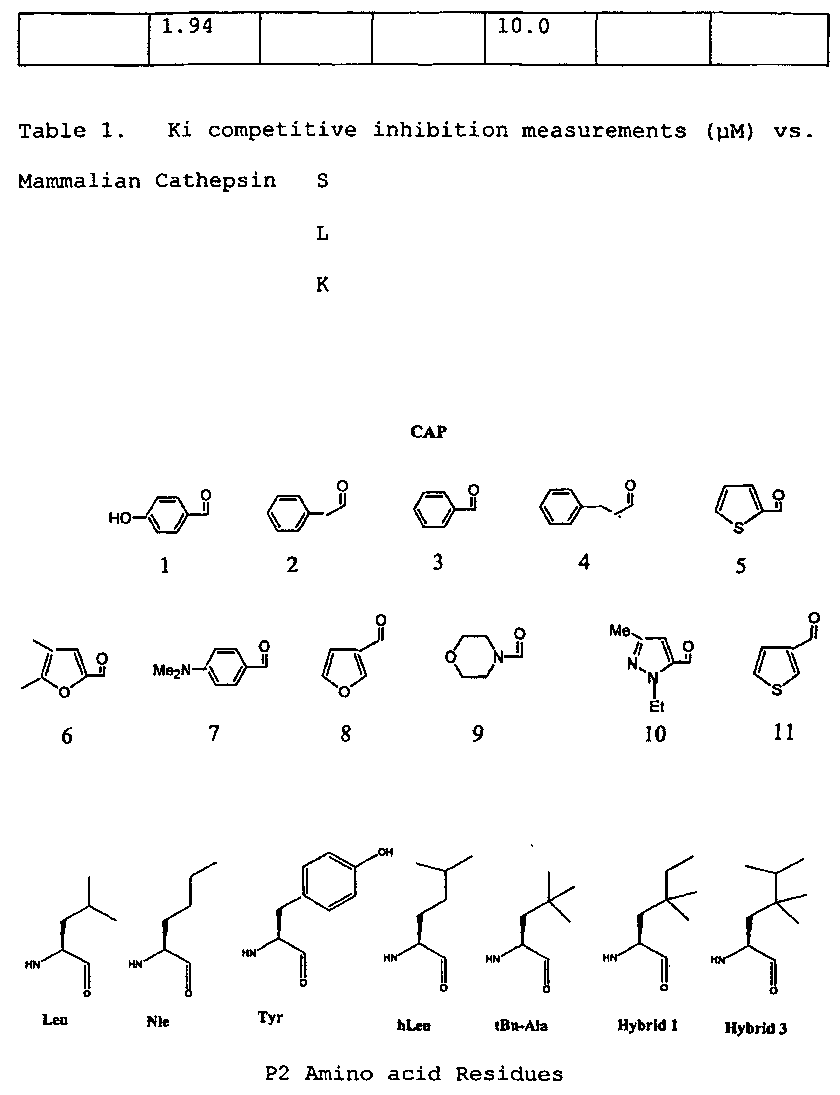

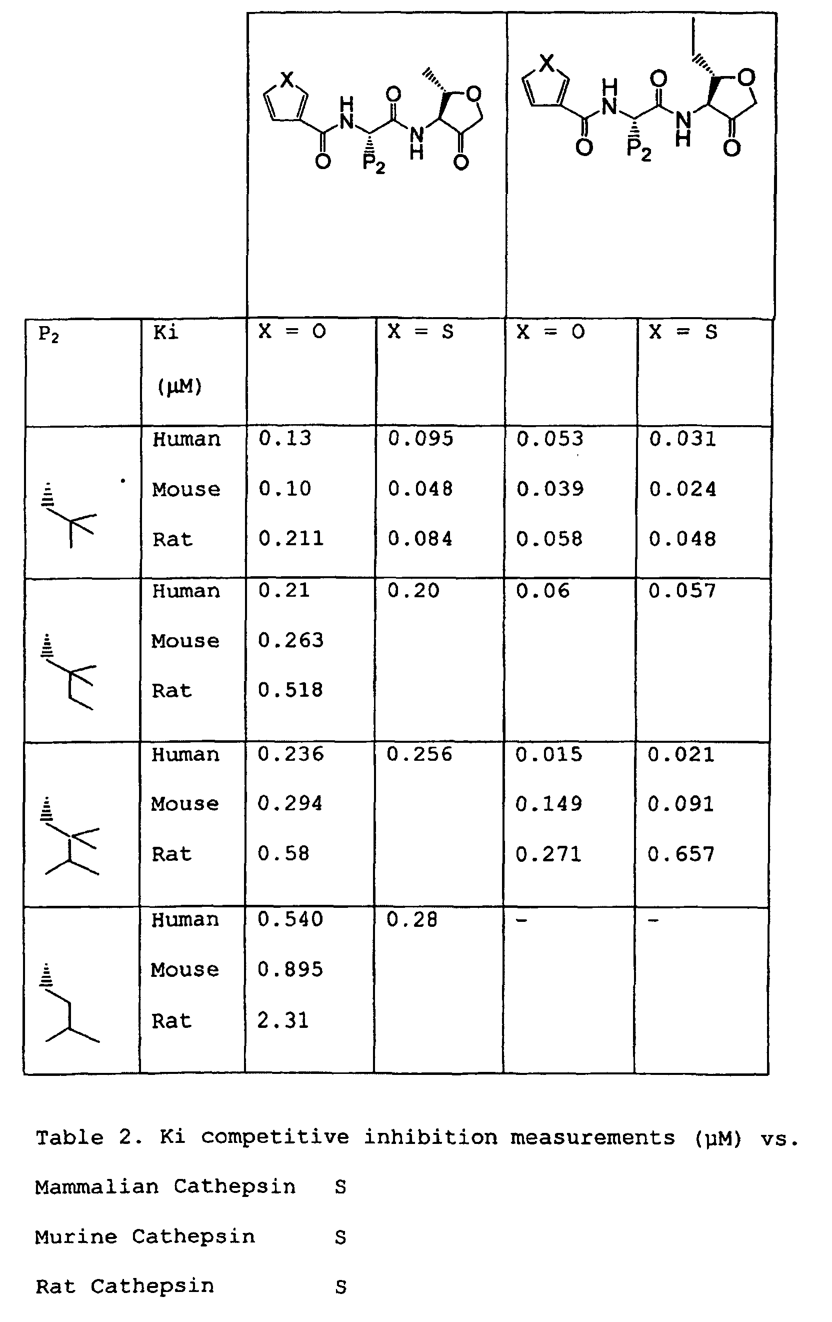

### Kᵢ determinations for cathepsins S, L, and K

### Cathepsin S (Mammalian, murine and rat)

### General

Assays were performed in 100 mM sodium phosphate, 100 mM NaCl, pH 6.5 (buffer) in white 384 well plates (Corning Costar). Eight inhibitors were assayed per plate.

### Inhibitor dilutions

Inhibitor dilutions were performed on a 96 well V-bottomed polypropylene plate (Corning Costar). 100 µl of buffer was placed in wells 2-5 and 7-12 of rows A, B, C and D. Sufficient of each inhibitor at 10 mM in DMSO was placed into wells A1-D1 and A6-D6 to give the desired final concentration when the volume in the well was made up to 200 µl with buffer. Column 1 was made up to 200 µl with buffer, mixed by aspirating and dispensing 100 µl in the well, and 100 µl transferred to column 2. The pipette tips were changed and the mixing and transferral repeated to column 5. This process was repeated for columns 6-10.

### Substrate dilutions

Substrate dilutions were performed on a 96 deep well polypropylene plate (Beckman Coulter). 280 µl of buffer was placed in wells B-H of columns 1 and 2. 70 µl of 10 mM boc-Val-Leu-Lys-AMC was placed in A1 and A2. 2 x 250 µl of buffer was added to wells A1 and A2, mixed by aspirating and dispensing 280 µl in the well, and 280 µl transferred to row B. The pipette tips were changed and the process repeated down the plate to row H.

### Assay

Column 1 of the substrate dilution plate was distributed at 10 µl per well into alternate rows beginning at row A. Column 2 was distributed to alternate rows beginning at row B. Row A of the inhibitor dilution plate was distributed at 10 µl per well to alternate rows and columns starting at A1. Row B was distributed to alternate rows and columns starting at A2. Row C was distributed to alternate rows and columns starting at B1 and row D was distributed to alternate rows and columns starting at B2. The assay was started by the addition of 30 µl per well of 20 nM cathepsin S in buffer containing 10 mM 2-mercaptoethanol.
Data were saved as text files and imported into Excel.
The initial rates were determined by linear regression and then fitted to the competitive inhibition equation using SigmaPlot.

### Cathepsins L and K

Assays were performed essentially as above. For cathepsin L, the buffer used was 100 mM sodium acetate, 1 mM EDTA, pH 5.5 and the substrate was *D*-Val-Leu-Lys-AMC with a highest concentration of 100 µM. For cathepsin K, the buffer used was 100 mM MES/Tris, 1 mM EDTA, pH 7.0 and the substrate was *D*-Ala-Leu-Lys-AMC with a highest concentration of 250 µM.

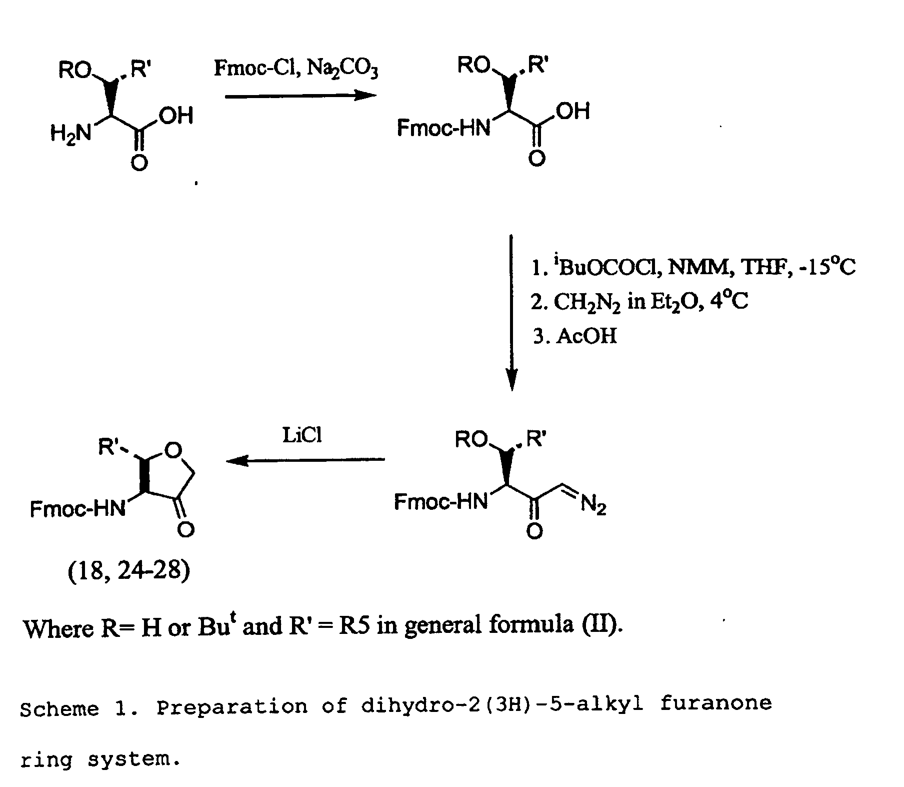

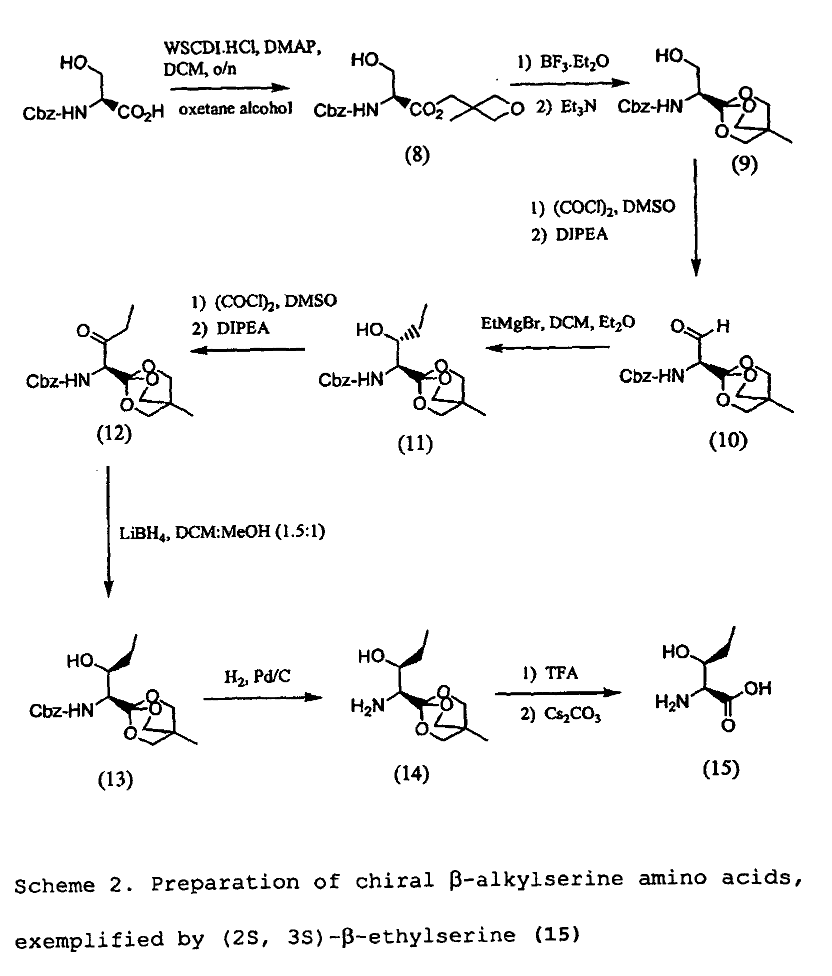

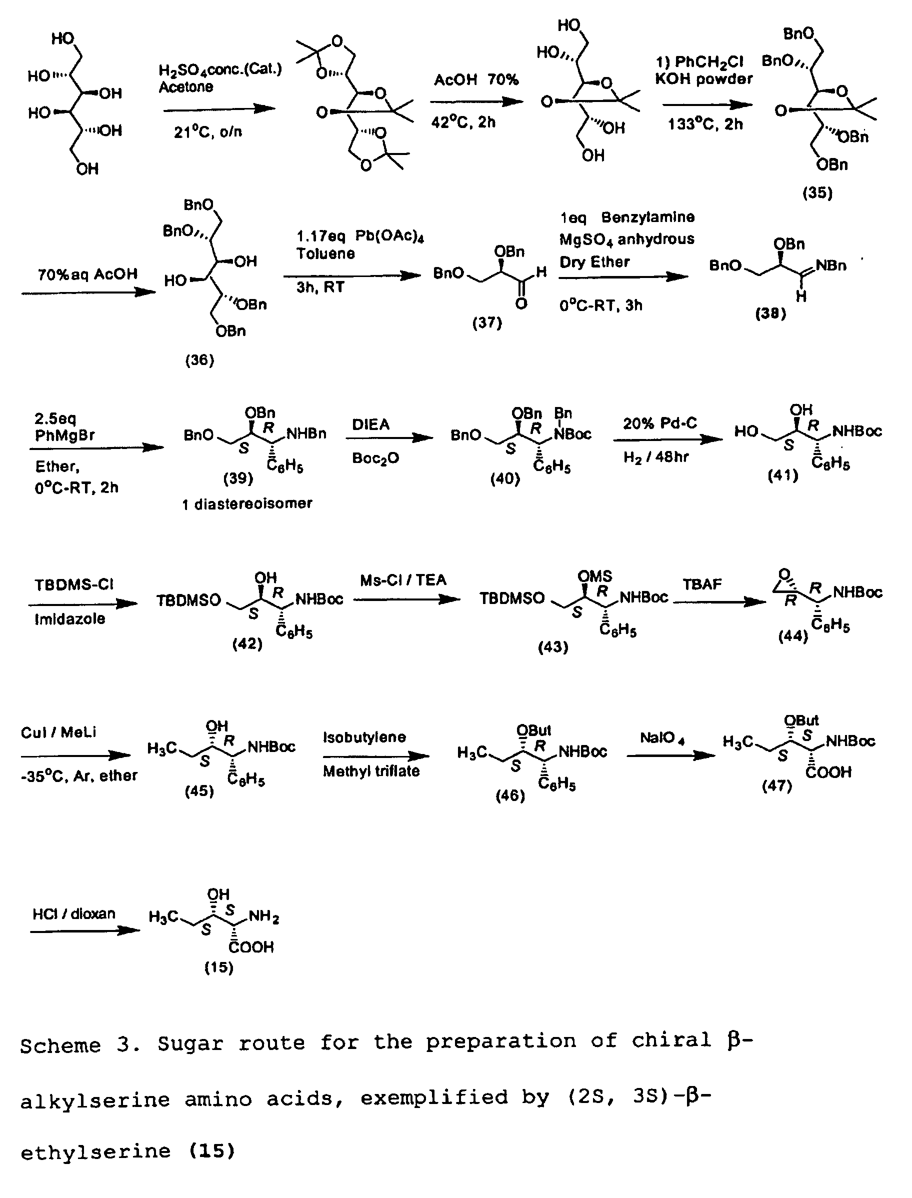

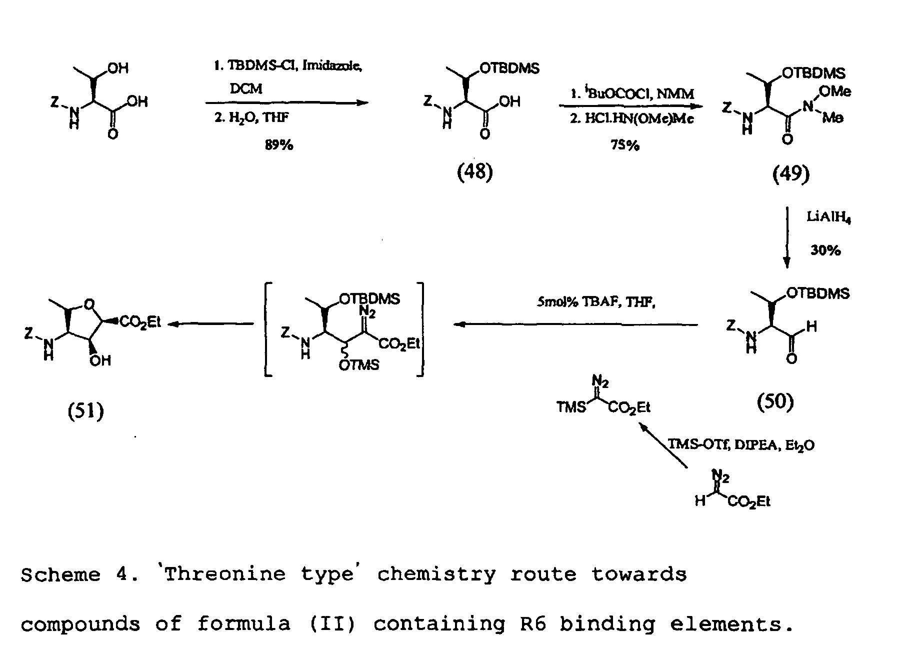

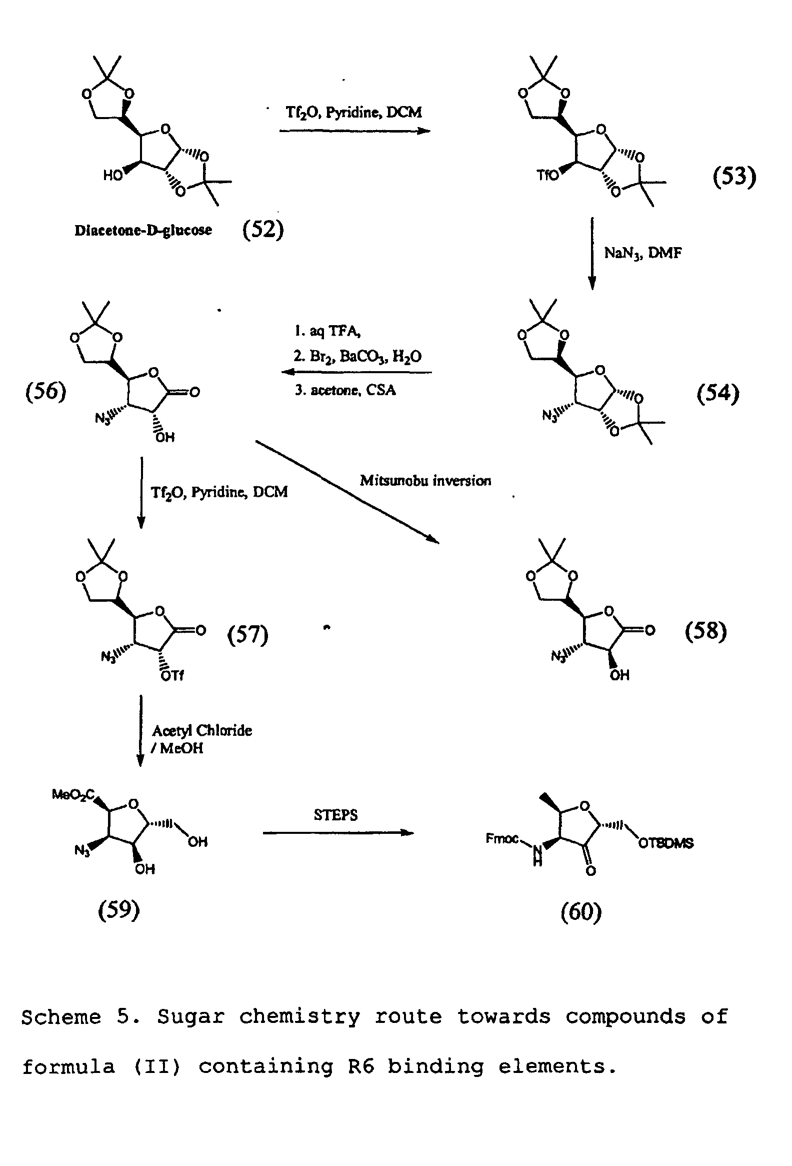

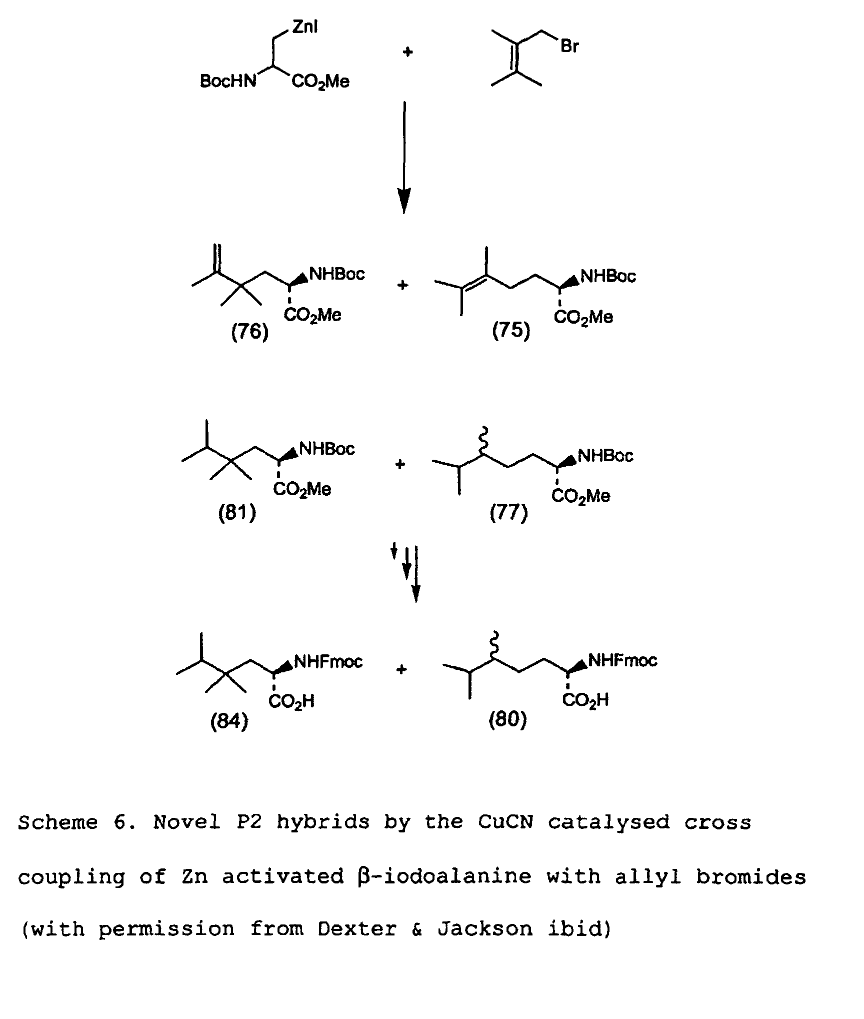

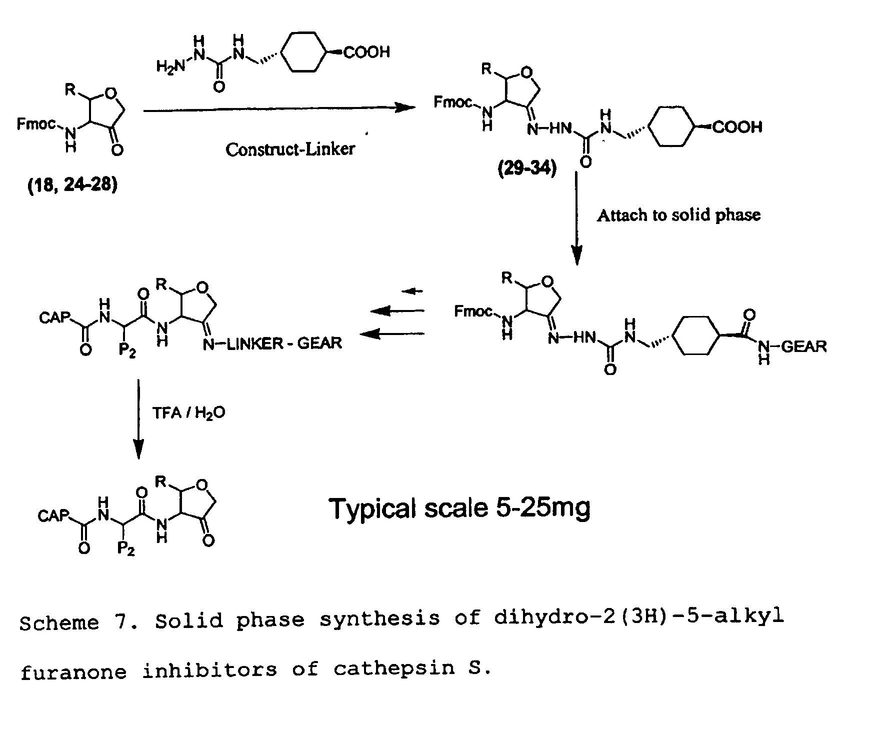

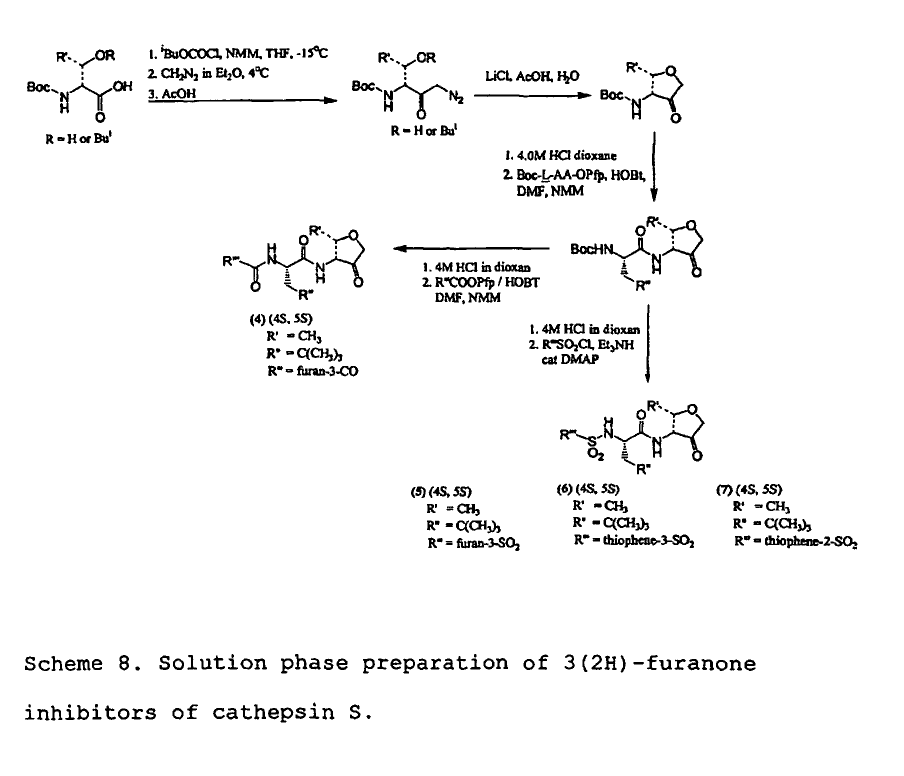

## Claims

1. A compound according to formula (II): 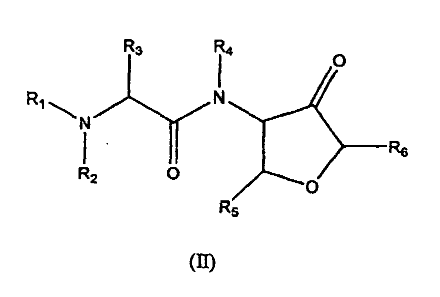 wherein:
R1 = R', R'C(O), R' C(S), R' SO₂, R' OC(O), R' NHC(O)
R'= 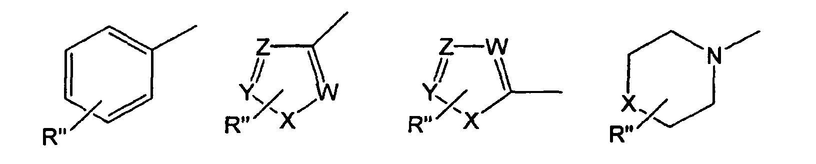
X,=O,S,NH;
W, Y, Z = CH, N;
R" = single or multiple ring substitution combinations taken from:
H, C₁₋₇-alkyl, C₁₋₇alkyl-C₃₋₆-cycloalkyl, OH, SH, amine, halogen;
R2,R4 = H, C₁₋₇-alky), C₁₋₇-alkyl-C₃₋₇-cycloalkyl;
R3 = C₁₋₇-alkyl, C₁₋₇-alkyl-C₃₋₇-cycloalkyl, Ar-C₁₋₇-alkyl;
R5 = C₁₋₇-alkyl, Ar-C₁₋₇-alkyl, C₁₋₃-alkyl-CONR''' or R^{iv};
R''' = H, methyl;
R^{iv} = 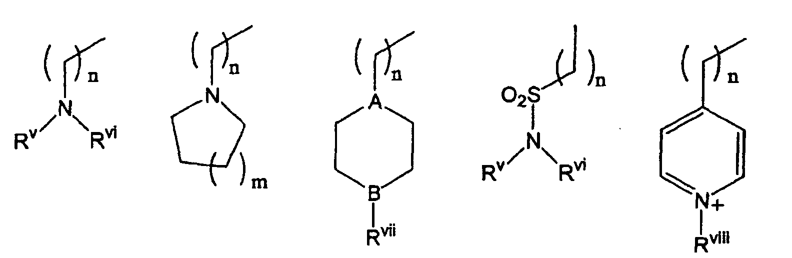
where n = 1-3, m = 1-3;
R^{v}, R^{vi} = H, C₁₋₇-alkyl;
A = N, CH;
B = N, O, S, CH;
R^{vii} = absent when B = O, S; or R^{vii} = H, C₁₋₇-alkyl when B = N, CH;
R^{viii} = O, C₁₋₇alkyl;
R6=H;
and wherein,
C₁₋₇alkyl or C₁₋₃ alkyl may be optionally substituted by one or two halogens and/or a heteroatom S, O, N, which if located at a chain terminus is substituted by one or two H atoms;
Ar is phenyl, pyrazolyl, imidazolyl, oxazolyl, thiazinolyl, isothiazinolyl, oxadiazolyl, 1,2,3-triazolyl, furanyl, or thienyl, which may be optionally substituted by halogen, C₁₋₃-alkyl,OH, OC₁₋₃-alkyl, SH, SC₁₋₃-alkyl, or amine; amine is -NH₂, -NHC₁₋₃-alkyl or -N(C₁₋₃alkyl)₂
and pharmaceutically acceptable salts thereof.

2. A compound according to claim 1, wherein R2 and/or R4 are hydrogen.

3. A compound according to claim 1 or 2, wherein R1 is R'C(O),
Where R' =

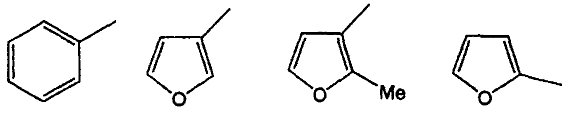

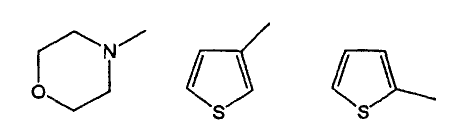

4. A compound according to claim 3, wherein R' is fur-3-yl or thien-3-yl

5. A compound according to any of claims 1-4, wherein R3 is n-butyl, t-butyl, 3-(2,2-dimethylpropyl), 4-(2-methylbutyl), 4-(3,3-dimethylbutyl), 4-(3,3-dimethyl-2-methylbutyl), 4-(3-methyl-2-methylbutyl) or 5-(2-methyl-3- methylpentyl).

6. A compound according to claim 5, wherein R3 is t-butyl, 3-(2,2-dimethylpropyl) or 4-(3,3-dimethyl-2-methylbutyl).

7. A compound according to any of claims 1-6, wherein R5 is CH₃, C₂H₅, CH₂Ar, CH₂CONH₂, (CH₂)₂CONH₂,

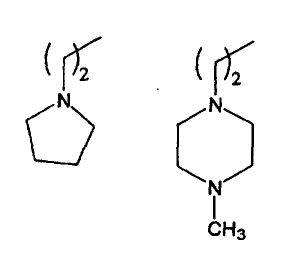

8. A compound according to claim 7, wherein R5 is CH₃ or CH₂CH₃.

9. A compound according to any of daims 1-8, wherein R5 has (S) stereochemistry.

10. A compound according to claim 1, wherein
R1 = R'C(O)
Where R' = 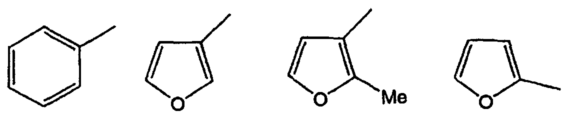 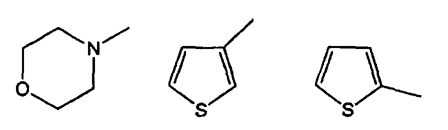 R2 and R4 = H;
R3 = n-butyl, t-butyl, 3-(2,2-dimethylpropyl), 4-(2-methylbutyl), 4-(3,3-dimethylbutyl), 4-(3,3-dimethyl-2-methylbutyl), 4-(3-methyl-2-methylbutyl), 5-(2-methyl-3-methylpentyl);
R5 = CH₃, C₂H₅, CH₂Ar, CH₂CONH₂, (CH₂)₂CONH₂, 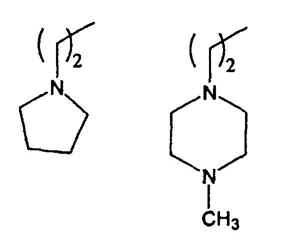 R6=H
or a pharmaceutically acceptable salt thereof.

11. A compound which is selected from the group consisting of:
Furan-3-carboxylic acid [3,3-dimethyl-1S-(2S-methyl-4-oxo-tetrahydrofuran-3S-ylcarbamoyl)butyl]amide,
Furan-3-carboxylic acid [3,3-dimethyl-1S-(2-ethyl-4-oxo-tetrahydrofuran-3-ylcarbamoyl)butyl]amide,
Furan-3-carboxylic acid [1S-(2-carbamoylmethyl-4-oxo-tetrahydrofuran-3-ylcarbamoyl)-3,3-dimethylbutyl]amide,
Furan-3-carboxylic acid {1S-[2-(2-dimethylaminoethyl)-4-oxo-tetrahydrofuran-3-ylcarbamoyl]-3,3-dimethylbutyl}amide,
Furan-3-carboxylic acid [3,3-dimethyl-1S-(4-oxo-2-pyrrolidln-1-ylmethyl-tetrahydrofuran-3-ylcarbamoyl)butyl]amide,
Furan-3-carboxylic acid [4-methyl-1S-(2S-methyl-4-oxo-tetrahydrofuran-3S-ylcarbamoyl)pentyl]amide,
Furan-3-carboxylic acid [4-methyl-1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoyl)-4-methylpentyl]amide,
Furan-3-carboxylic acid {1S-[2-(2-dimethylaminoethyl)-4-oxo-tetrahydrofuran-3-ylcarbamoyl]-4-methylpentyl}amide,
Furan-3-carboxylic acid [4-methyl-1S-(4-oxo-2-pyrrolidin-1-ylmethyl-tetrahydrofuran-3-ylcarbamoyl)pentyl]amide,
Furan-3-carboxylic acid [3,3-dimethyl-1S-(2S-methyl-4-oxo-tetrahydrofuran-3S-ylcarbamoyl)pentyl]amide,
Furan-3-carboxylic acid [3,3-dimethyl-1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoy)-3,3-dimethylpentyl]amide,
Furan-3-carboxylic acid {1S-[2-(2-dimethylaminoethyl)-4-oxo-tetrahydrofuran-3-ylcarbamoyl]-3,3-dimethylpentyl}amide,
Furan-3-carboxylic acid [3,3-dimethyl-1S-(4-oxo-2-pyrrolidin-1-ylmethyl-tetrahydrofuran-3-ylcarbamoyl)pentyl]amide,
Furan-3-carboxylic acid [3,3,4-trimethyl-1S-(2S-methyl-4-oxo-tetrahydrofuran-3S-ylcarbamoyl)pentyl]amide,
Furan-3-carboxylic acid [3,3,4-trimethyl -1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoyl)-3,3,4-trimethylpentyl]amide,
Furan-3-carboxylic acid {1S-[2-(2-dimethylaminoethyl)-4-oxo-tetrahydrofuran-3-ylcarbamoyl]-3,3,4-trimethylpentyl}amide,
Furan-3-carboxylic acid [3,3,4-trimethyl-1S-(4-oxo-2-pyrrolidin-1-ylmethyl-tetrahydrofuran-3-ylcarbamoyl)pentyl]amide,
Furan-3-carboxylic acid [3,4-dimethyl-1S-(2S-methyl-4-oxo-tetrahydrofuran-3S-ylcarbamoyl)pentyl]amide,
Furan-3-carboxylic acid [3,4-dimethyl-1S-(2-ethyl-4-oxo-tetrahydrofuran-3-ylcarbamoyl)pentyl]amide,
Furan-3-carboxylic acid [1S-(2-carbamoylmethyl-4-oxo-tetrahydrofuran-3-ylcarbamoyl)-3,4-dimethylpentyl]amide,
Furan-3-carboxylic acid {1S-[2-(2-dimethylaminoethyl)-4-oxo-tetrahydrofuran-3-ylcarbamoyl]-3,4-dimethylpentyl}amide,
Furan-3-carboxylic acid [3,4-dimethyl-1S-(4-oxo-2-pyrrolidin-1-ylmethyl-tetrahydrofuran-3-ylcarbamoyl)pentyl]amide,
Furan-3-carboxylic acid [4,5-dimethyl-1S-(2S-methyl-4-oxo-tetrahydrofuran-3S-ylcarbamoyl)hexyl]amide,
Furan-3-carboxylic acid [4,5-dimethyl-1S-(2-ethyl-4-oxo-tetrahydrofuran-3-ylcarbamoyl)hexyl]amide,
Furan-3-carboxylic acid [1S-(2-carbamoylmethyl-4-oxo-tetrahydrofuran-3-ylcarbamoyl)-4,5-dimethylhexyl]amide,
Furan-3-carboxylic acid {1S-[2-(2-dimethylaminoethyl)-4-oxo-tetrahydrofuran-3-ylcarbamoyl]-4,5-dimethylhexyl}amide,
Furan-3-carboxylic acid [4,5-dimethyl-1S-(4-oxo-2-pyrrolidin-1-ylmethyl-tetrahydrofuran-3-ylcarbamoyl)hexyl]amide,
Furan-3-carboxylic acid [3-methyl-1S-(2S-methyl-4-oxo-tetrahydrofuran-3S-ylcarbamoyl)-3-phenylbutyl]amide,
Furan-3-carboxylic acid [3-methyl-1S-(2-ethyl-4-oxo-tetrahydrofuran-3-ylcarbamoyl)-3-phenylbutyl]amide,
Furan-3-carboxylic acid [1S-(2-carbamoylmethyl-4-oxo-tetrahydrofuran-3-ylcarbamoyl)-3-methyl-3-phenylbutyl]amide,
Furan-3-carboxylic acid {1S-[2-(2-dimethylaminoethyl}-4-oxo-tetrahydrofuran-3-ylcarbamoyl]-3-methyl-3-phenylbutyl}amide,
Furan-3-carboxylic acid [3-methyl-1S-(4-oxo-2-pyrrolidin-1-ylmethyl-tetrahydrofuran-3-ylcarbamoyl)-3-phenylbutyl]amide,
Furan-3-carboxylic acid [3,3-dimethyl-1S-(2S-methyl-4-oxo-tetrahydrofuran-3S-ylcarbamoyl)-4-phenylbutyl]amide,
Furan-3-carboxylic acid [3,3-dimethyl-1S-(2-ethyl-4-oxo-tetrahydrofuran-3-ylcarbamoyl)-4-phenylbutyl]amide,
Furan-3-carboxylic acid [1S-(2-carbamoylmethyl-4-oxo-tetrahydrofuran-3-ylcarbamoyl)-3,3-dimethyl-4-phenylbutyl]amide,
Furan-3-carboxylic acid {1S-[2-(2-dimethylaminoethyl)-4-oxo-tetrahydrofuran-3-ylcarbamoyl]-3,3-dimethyl-4-phenylbutyl}amide,
Furan-3-carboxylic acid [3,3-dimethyl-1S-(4-oxo-2-pyrrolidin-1-ylmethyl-tetrahydrofuran-3-ylcarbamoyl)-4-phenylbutyl]amide,
Furan-3-carboxylic acid [3,3-dimethyl-1S-(2S-methyl-4-oxo-tetrahydrofuran-3S-ylcarbamoyl)-5-phenylpentyl]amide,
Furan-3-carboxylic acid [3,3-dimethyl-1S-(2-ethyl-4-oxo-tetrahydrofuran-3-ylcarbamoyl)-5-phenylpentyl]amide,
Furan-3-carboxylic acid [1S-(2-carbamoylmethyl-4-oxo-tetrahydrofuran-3-ylcarbamoyl)-3,3-dimethyl-5-phenylpentyl]amide,
Furan-3-carboxylic acid {1S-[2-(2-dimethylaminoethyl)-4-oxo-tetrahydrofuran-3-ylcarbamoyl]-3,3-dimethyl-5-phenylpentyl}amide,
Furan-3-carboxylic acid [3,3-dimethyl-1S-(4-oxo-2-pyrrolidin-1-ylmethyl-tetrahydrofuran-3-ylcarbamoyl)-5-phenylpentyl]amide,
Thiophene-3-carboxylic acid [3,3-dimethyl-1S-(2S-methyl-4-oxo-tetrahydrofuran-3S-ylcarbamoyl)butyl]amide,
Thiophene-3-carboxylic acid [3,3-dimethyl-1S-(2-ethyl-4-oxo-tetrahydrofuran-3-ylcarbamoyl)butyl]amide,
Thiophene-3-carboxylic acid [1S-(2-carbamoylmethyl-4-oxo-tetrahydrofuran-3-ylcarbamoyl)-3,3-dimethylbutyl]amide,
Thiophene-3-carboxylic acid {1S-[2-(2-dimethylaminoethyl)-4-oxo-tetrahydrofuran-3-ylcarbamoyl]-3,3-dimethylbutyl}amide,
Thiophene-3-carboxylic acid [3,3-dimethyl-1S-(4-oxo-2-pyrrolidin-1-ylmethyl-tetrahydrofuran-3-ylcarbamoyl)butyl]amide,
Thiophene-3-carboxylic acid [4-methyl-1S-(2S-methyl-4-oxo-tetrahydrofuran-3S-ylcarbamoyl)pentyl]amide,
Thiophene-3-carboxylic acid [4-methyl-1S-(2-ethyl-4-oxo-tetrahydrofuran-3-ylcarbamoyl)pentyl]amide,
Thiophene-3-carboxylic acid [1S-(2-carbamoylmethy)-4-oxo-tetrahydrofuran-3-ylcarbamoyl)-4-methylpentyl]amide,
Thiophene-3-carboxylic acid {1S-[2-(2-dimethylaminoethyl)-4-oxo-tetrahydrofuran-3-ylcarbamoyl]-4-methylpentyl}amide,
Thiophene-3-carboxylic acid [4-methyl-1S-(4-oxo-2-pyrrolidin-1-ylmethyl-tetrahydrofuran-3-ylcarbamoyl)pentyl]amide,
Thiophene-3-carboxylic add [3,3-dimethyl-1S-(2S-methyl-4-oxo-tetrahydrofuran-3S-ylcarbamoyl)pentyl]amide,
Thiophene-3-carboxylic acid [3,3-dimethyl-1S-(2-ethyl-4-oxo-tetrahydrofuran-3-ylcarbamoyl)pentyl]amide,
Thiophene-3-carboxylic acid [1S-(2-carbamoylmethyl-4-oxo-tetrahydrofuran-3-ylcarbamoyl)-3,3-dimethylpentyl]amide,
Thiophene-3-carboxylic acid {1S-[2-(2-dimethylaminoethy)-4-oxo-tetrahydrofuran-3-ylcarbamoyl]-3,3-dimethylpentyl}amide,
Thiophene-3-carboxylic acid [3,3-dimethyl-1S-(4-oxo-2-pyrrolidin-1-ylmethyl-tetrahydrofuran-3-ylcarbamoyl)pentyl]amide,
Thiophene-3-carboxylic acid [3,3,4-trimethyl-1S-(2S-methyl-4-oxo-tetrahydrofuran-3S-ylcarbamoyl)pentyl]amide,
Thiophene-3-carboxylic acid [3,3,4-trimethyl-1S-(2-ethyl-4-oxo-tetrahydrofuran-3-ylcarbamoyl)pentyl]amide,
Thiophene-3-carboxylic acid [1S-(2-carbamoylmethyl-4-oxo-tetrahydrofuran-3-ylcarbamoyl)-3,3,4-trimethylpentyl]amide,
Thiophene-3-carboxylic acid {1S-[2-(2-dimethylaminoethyl)-4-oxo-tetrahydrofuran-3-ylcarbamoyl]- 3,3,4-trimethylpentyl}amide,
Thiophene-3-carboxylic acid [3,3,4-trimethyl-1S-(4-oxo-2-pyrrolidin-1-ylmethyl-tetrahydrofuran-3-ylcarbamoyl)pentyl]amide,
Thiophene-3-carboxylic acid [3,4-dimethyl-1S-(2S-methyl-4-oxo-tetrahydrofuran-3S-ylcarbamoyl)pentyl]amide,
Thiophene-3-carboxylic acid [3,4-dimethyl-1S-(2-ethyl-4-oxo-tetrahydrofuran-3-ylcarbamoyl)pentyl]amide,
Thiophene-3-carboxylic acid [1S-(2-carbamoylmethyl-4-oxo-tetrahydrofuran-3-ylcarbamoyl)-3,4-dimethylpentyl]amide,
Thiophene-3-carboxylic acid {1S-[2-(2-dimethylaminoethyl)-4-oxo-tetrahydrofuran-3-ylcarbamoyl]-3,4-dimethylpentyl}amide,
Thiophene-3-carboxylic acid [3,4-dimethyl-1S-(4-oxo-2-pyrrolidin-1-ylmethyl-tetrahydrofuran-3-ylcarbamoyl)pentyl]amide,
Thiophene-3-carboxylic acid [4,5-dimethyl-1S-(2S-methyl-4-oxo-tetrahydrofuran-3S-ylcarbamoyl)hexyl]amide,
Thiophene-3-carboxylic acid [4,5-dimethyl-1S-(2-ethyl-4-oxo-tetrahydrofuran-3-ylcarbamoyl)hexyl]amide,
Thiophene-3-carboxylic acid [1S-(2-carbamoylmethyl-4-oxo-tetrahydrofuran-3-ylcarbamoyl)-4,5-dimethylhexyl]amide,
Thiophene-3-carboxylic acid {1S-[2-(2-dimethylaminoethyl)-4-oxo-tetrahydrofuran-3-ylcarbamoyl]-4,5-dimethylhexyl}amide,
Thiophene-3-carboxylic acid [4,5-dimethyl-1S-(4-oxo-2-pyrrolidin-1-ylmethyl-tetrahydrofuran-3-ylcarbamoyl)hexyl]amide,
Thiophene-3-carboxylic acid [3-methyl-1S-(2S-methyl-4-oxo-tetrahydrofuran-3S-ylcarbamoyl)-3-phenyl)butyl]amide,
Thiophene-3-carboxylic acid [3-methyl-1S-(2-ethyl-4-oxo-tetrahydrofuran-3-ylcarbamoyl)-3-phenylbutyl]amide,
Thiophene-3-carboxylic acid [1S-(2-carbamoylmethyl-4-oxo-tetrahydrofuran-3-ylcarbamoyl)-3-methyl-3-phenylbutyl]amide,
Thiophene-3-carboxylic acid {1S-[2-(2-dimethylaminoethyl)-4-oxo-tetrahydrofuran-3-ylcarbamoyl]-3-methyl-3-phenylbutyl}amide,
Thiophene-3-carboxylic acid [3-methyl-1S-(4-oxo-2-pyrrolidin-1-ylmethyl-tetrahydrofuran-3-ylcarbamoyl)-3-phenylbutyl]amide,
Thiophene-3-carboxylic acid [3,3-dimethyl-1S-(2S-methyl-4-oxo-tetrahydrofuran-3S-ylcarbamoyl)-4-phenylbutyl]amide,
Thiophene-3-carboxylic acid [3,3-dimethyl-1S-(2-ethyl-4-oxo-tetrahydrofuran-3-ylcarbamoyl)-4-phenylbutyl]amide,
Thiophene-3-carboxylic acid [1S-(2-carbamoylmethyl-4-oxo-tetrahydrofuran-3-ylcarbamoyl)-3,3-dimethyl-4-phenylbutyl]amide,
Thiophene-3-carboxylic acid {1S-[2-(2-dimethylaminoethyl)-4-oxo-tetrahydrofuran-3-ylcarbamoyl]-3,3-dimethyl-4-phenylbutyl}amide,
Thiophene-3-carboxylic acid [3,3-dimethyl-1S-(4-oxo-2-pyrrolidin-1-ylmethyl-tetrahydrofuran-3-ylcarbamoyl)-4-phenylbutyl]amide,
Thiophene-3-carboxylic acid [3,3-dimethyl-1S-(2S-methyl-4-oxo-tetrahydrofuran-3S-ylcarbamoyl)-5-phenylpentyl]amide,
Thiophene-3-carboxylic acid [3,3-dimethyl-1S-(2-ethyl-4-oxo-tetrahydrofuran-3-ylcarbamoyl)-5-phenylpentyl]amide,
Thiophene-3-carboxylic acid [1S-(2-carbamoylmethyl-4-oxo-tetrahydrofuran-3-ylcarbamoyl)-3,3-dimethyl-5-phenylpentyl]amide,
Thiophene-3-carboxylic acid {1S-[2-(2-dimethylaminoethyl)-4-oxo-tetrahydrofuran-3-ylcarbamoyl]-3,3-dimethyl-5-phenylpentyl}amide,
Thiophene-3-carboxylic acid [3,3-dimethyl-1S-(4-oxo-2-pyrrolidin-1-ylmethyl-tetrahydrofuran-3-ylcarbamoyl)-5-phenylpentyl]amide,
2-Methylfuran-3-carboxylic acid [3,3-dimethyl-1S-(2S-methyl-4-oxo-tetrahydrofuran-3S-ylcarbamoyl)butyl]amide,
2-Methylfuran-3-carboxylic acid [3,3-dimethyl-1S-(2-ethyl-4-oxo-tetrahydrofuran-3-ylcarbamoyl)butyl]amide,
2-Methylfuran-3-carboxylic acid [1S-(2-carbamoylmethyl-4-oxo-tetrahydrofuran-3-ylcarbamoyl)-3,3-dimethylbutyl]amide,
2-Methylfuran-3-carboxylic acid {1S-[2-(2-dimethylaminoethyl)-4-oxo-tetrahydrofuran-3-ylcarbamoyl]-3,3-dimethylbutyl}amide,
2-Methylfuran-3-carboxylic acid [3,3-dimethyl-1S-(4-oxo-2-pyrrolidin-1-ylmethyl-tetrahydrofuran-3-ylcarbamoyl)butyl]amide,
2-Methylfuran-3-carboxylic acid [4-methyl-1S-(2S-methyl-4-oxo-tetrahydrofuran-3S-ylcarbamoyl)pentyl]amide,
2-Methylfuran-3-carboxylic acid [4-methyl-1S-(2-ethyl-4-oxo-tetrahydrofuran-3-ylcarbamoyl)pentyl]amide,
2-Methylfuran-3-carboxylic acid [1S-(2-carbamoylmethyl-4-oxo-tetrahydrofuran-3-ylcarbamoyl)-4-methylpentyl]amide,
2-Methylfuran-3-carboxylic acid {1S-[2-(2-dimethylaminoethyl)-4-oxo-tetrahydrofuran-3-ylcarbamoyl]-4-methylpentyl}amide,
2-Methylfuran-3-carboxylic acid [4-methyl-1S-(4-oxo-2-pyrrolidin-1-ylmethyl-tetrahydrofuran-3-ylcarbamoyl)pentyl]amide,
2-Methylfuran-3-carboxylic acid [3,3-dimethyl-1S-(2S-methyl-4-oxo-tetrahydrofuran-3S-ylcarbamoyl)pentyl]amide,
2-Methylfuran-3-carboxylic acid [3,3-dimethyl-1S-(2-ethyl-4-oxo-tetrahydrofuran-3-ylcarbamoyl)pentyl]amide,
2-Methylfuran-3-carboxylic acid [1S-(2-carbamoylmethyl-4-oxo-tetrahydrofuran-3-ylcarbamoyl)-3,3-dimethylpentyl]amide,
2-Methylfuran-3-carboxylic acid {1S-[2-(2-dimethylaminoethyl)-4-oxo-tetrahydrofuran-3-ylcarbamoyl]-3,3-dimethylpentyl}amide,
2-Methylfuran-3-carboxylic acid [3,3-dimethyl-1S-(4-oxo-2-pyrrolidin-1-ylmethyl-tetrahydrofuran-3-ylcarbamoyl)pentyl]amide,
2-Methylfuran-3-carboxylic acid [3,3,4-trimethyl-1S-(2S-methyl-4-oxo-tetrahydrofuran-3S-ylcarbamoyl)pentyl]amide,
2-Methylfuran-3-carboxylic acid [3,3,4-trimethyl -1S-(2-ethyl-4-oxo-tetrahydrofuran-3-ylcarbamoyl)pentyl]amide,
2-Methylfuran-3-carboxylic acid [1S-(2-carbamoylmethyl-4-oxo-tetrahydrofuran-3-ylcarbamoyl)-3,3,4-trimethylpentyl)amide,
2-Methylfuran-3-carboxylic acid {1S-[2-(2-dimethylaminoethyl)-4-oxo-tetrahydrofuran-3-ylcarbamoyl]- 3,3,4-trimethylpentyl}amide,
2-Methylfuran-3-carboxylic acid [3,3,4-trimethyl-1S-(4-oxo-2-pyrrolidin-1-ylmethyl-tetrahydrofuran-3-ylcarbamoyl)pentyl]amide,
2-Methylfuran-3-carboxylic acid [3,4-dimethyl-1S-(2S-methyl-4-oxo-tetrahydrofuran-3S-ylcarbamoyl)pentyl]amide,
2-Methylfuran-3-carboxylic acid [3,4-dimethyl-1S-(2-ethyl-4-oxo-tetrahydrofuran-3-ylcarbamoyl)pentyl]amide,
2-Methylfuran-3-carboxylic acid [1S-(2-carbamoylmethyl-4-oxo-tetrahydrofuran-3-ylcarbamoyl)-3,4-dimethylpentyl]amide,
2-Methylfuran-3-carboxylic acid {1S-[2-(2-dimethylaminoethyl)-4-oxo-tetrahydrofuran-3-ylcarbamoyl]-3,4-dimethylpentyl}amide,
2-Methylfuran-3-carboxylic acid [3,4-dimethyl-1S-(4-oxo-2-pyrrolidin-1-ymethyl-tetra hydrofuran-3-ylcarbamoyl)pentyl]amide,
2-Methylfuran-3-carboxylic acid [4,5-dimethyl-1S-(2S-methyl-4-oxo-tetrahydrofuran-3S-ylcarbamoyl)hexyl]amide,
2-Methylfuran-3-carboxylic acid [4,5-dimethyl-1S-(2-ethyl-4-oxo-tetrahydrofuran-3-ylcarbamoyl)hexyl]amide,
2-Methylfuran-3-carboxylic acid [1S-(2-carbamoytmethyl-4-oxo-tetrahydrofuran-3-ylcarbamoyl)-4,5-dimethylhexyl]amide,
2-Methylfuran-3-carboxylic acid {1S-[2-(2-dimethylaminoethyl)-4-oxo-tetrahydrofuran-3-ylcarbamoyl]-4,5-dimethylhexyl}amide,
2-Methylfuran-3-carboxylic acid [4,5-dimethyl-1S-(4-oxo-2-pyrrolidin-1-ylmethyl-tetrahydrofuran-3-ylcarbamoyl)hexyl]amide,
2-Methylfuran-3-carboxylic acid [3-methyl-1S-(2S-methyl-4-oxo-tetrahydrofuran-3S-ylcarbamoyl)-3-pheny)butyl]amide,
2-Methylfuran-3-carboxylic acid [3-methyl-1S-(2-ethyl-4-oxo-tetrahydrofuran-3-ylcarbamoyl-3-phenylbutyl]amide,
2-Methylfuran-3-carboxylic acid [1S-(2-carbamoylmethyl-4-oxo-tetrahydrofuran-3-ylcarbamoyl)-3-methyl-3-phenylbutyl]amide,
2-Methylfuran-3-carboxylic acid {1S-[2-(2-dimethylaminoethyl)-4-oxo-tetrahydrofuran-3-ylcarbamoyl]-3-methyl-3-phenylbutyl}amide,
2-Methylfuran-3-carboxylic acid [3-methyl-1S-(4-oxo-2-pyrrolidin-1-ylmethyl-tetrahydrofuran-3-ylcarbamoyl)-3-phenylbutyl]amide.
2-Methylfuran-3-carboxylic acid [3,3-dimethyl-1S-(2S-methyl-4-oxo-tetrahydrofuran-3S-ylcarbamoyl)-4-phenylbutyl]amide.
2-Methylfuran-3-carboxylic acid [3,3-dimethyl-1S-(2-ethyl-4-oxo-tetrahydrofuran-3-ylcarbamoyl)-4-phenylbutyl]amide,
2-Methylfuran-3-carboxylic acid [1S-(2-carbamoylmethyl-4-oxo-tetrahydrofuran-3-ylcarbamoyl)-3,3-dimethyl-4-phenylbutyl]amide,
2-Methylfuran-3-carboxylic acid {1S-[2-(2-dimethylaminoethyl)-4-oxo-tatrahydrofuran-3-ylcarbamoyl]-3,3-dimethyl-4-phenylbutyl}amide,
2-Methylfuran-3-carboxylic acid [3,3-dimethyl-1S-(4-oxo-2-pyrrolidin-1-ytmethy)-tetrahydrofuran-3-ylcarbamoyl)-4-phenylbutyl]amide,
2-Methylfuran-3-carboxylic acid [3,3-dimethyl-1S-(2S-methyl-4-oxo-tetrahydrofuran-3S-ylcarbamoyl)-5-phenylpentyl]amide,
2-Methylfuran-3-carboxylic acid [3,3-dimethyl-1S-(2-ethyl-4-oxo-tetrahydrofuran-3-ycarbamoyl)-5-phenylpentyl]amide.
2-Methylfuran-3-carboxylic acid [1S-(2-carbamoylmethyl-4-oxo-tetrahydrofuran-3-ylcarbamoyl)-3,3-dimethyl-5-phenylpentyl]amide,
2-Methylfuran-3-carboxylic acid {1S-[2-(2-dimethytaminoethyl}-4-oxo-tetrahydrofuran-3-ylcarbamoyl]-3,3-dimethyl-5-phenylpentyl}amide,
2-Methylfuran-3-carboxylic acid [3,3-dimethyl-1S-(4-oxo-2-pyrrolidin-1-ylmethyl-tetrahydrofuran-3-ylcarbamoyl)-5-phenylpentyl]amide,
1*H*-Pyrrole-3-carboxylic acid [3,3-dimethyl-1S-(2S-methyl-4-oxo-tetrahydrofuran-3S-ylcarbamoyl)butyl]amide,
1*H*-Pyrrole-3-carboxylic acid [3,3-dimethyl-1S-(2-ethyl-4-oxo-tetrahydrofuran-3-ylcarbamoyl)butyl]amide,
1*H*-Pyrrole-3-carboxylic acid [1S-(2-carbamoylmethyl-4-oxo-tetrahydrofuran-3-ylcarbamoyl)-3,3-dimethylbutyl]amide,
1*H*-Pyrrole-3-carboxyilc acid {1S-[2-(2-dimethylaminoethyl)-oxo-tetrahydrofuran-3-ylcarbamoyl]-3,3-dimethytbutyl}amide,
1H-Pyrrole-3-carboxylic acid [3,3-dimethyl-1S-(4-oxo-2-pyrrolidin-1-ylmethyl-tetrahydrofuran-3-ylcarbamoyl)butyl]amide,
1*H*-Pyrrole-3-carboxylic acid [4-methyl-1S-(2S-methyl-4-oxo-tetrahydrofuran-3S-ylcarbamoyl)pentyl]amide,
1*H*-Pyrrole-3-carboxylic acid [4-methyl-1S-(2-ethyl-4-oxo-tetrahydrofuran-3-ylcarbamoyl)pentyl]amide,
1*H*-Pyrrole-3-carboxylic acid [1S-(2-carbamoylmethy)-4-oxo-tetrahydrofuran-3-ylcarbamoyl)-4-methylpentyl]amide,
1*H*-Pyrrole-3-carboxylic acid {1S-[2-(2-dimethylaminoethyl)-4-oxo-tetrahydrofuran-3-ylcarbamoyl]-4-methylpentyl}amide,
1*H*-Pyrrole-3-carboxylic acid [4-methyl-1S-(4-oxo-2-pyrrolidin-1-ylmethyl-tetrahydrofuran-3-ylcarbamoyl)pentyl]amide,
1*H*-Pyrrole-3-carboxylic acid [3,3-dimethyl-1S-(2S-methyl-4-oxo-tetrahydrofuran-3S-ylcarbamoyl)pentyl]amide,
1*H*-Pyrrole-3-carboxylic acid [3,3-dimethyl-1S-(2-ethyl-4-oxo-tetrahydrofuran-3-ylcarbamoyl)pentyl]amide,
1*H*-Pyrrole-3-carboxylic acid [1S-(2-carbamoylmethyl-4-oxo-tetrahydrofuran-3-ylcarbamoyl)-3,3-dimethylpentyl]amide,
1*H*-Pyrrole-3-carboxylic acid {1S-[2-(2-dimethytaminoethyl)-4-oxo-tetrahydrofuran-3-ylcarbamoyl]-3,3-dimethyipentyl}amide,
1*H*-Pyrrole-3-carboxylic acid [3,3-dimethyl-1S-(4-oxo-2-pyrrolidin-1-ylmethyl-tetrahydrofuran-3-ylcarbamoyl)pentyl]amide,
1*H*-pyrrole-3-carboxylic acid [3,3,4-trimethyl-1S-(2S-methyl-4-oxo-tetrahydrofuran-3S-ylcarbamoyl)pentyl]amide,
1*H*-Pyrrole-3-carboxylic acid [3,3,4-trimethyl-1S-(2-ethyl-4-oxo-tetrahydrofuran-3-ylcarbamoyl)pentyl]amide,
1*H*-Pyrrole-3-carboxylic acid [1S-(2-carbamoy)methyl-4-oxo-tetrahydrofuran-3-ylcarbamoyl)-3,3,4-trimethylpentyl]amide,
1*H*-Pyrrole-3-carboxylic acid {1S-[2-(2-dimethylaminoethyl)-4-oxo-tetrahydrofuran-3-ylcarbamoyl]-3,3,4-trimethylpentyl}amide,
1*H*-Pyrrole-3-carboxylic acid [3,3,4-trimethyl-1S-(4-oxo-2-pyrrolidin-1-ylmethyl-tetrahydrofuran-3-ylcarbamoyl)pentyl]amide,
1*H*-Pyrrole-3-carboxylic acid [3,4-dimethyl-1S-(2S-methyl-4-oxo-tetrahydrofuran-3S-ylcarbamoyl)pentyl]amide,
1*H*-Pyrrole-3-carboxylic acid [3,4-dimethyl-1S-(2-ethyl-4-oxo-tetrahydrofuran-3-ylcarbamoyl)pentyl]amide.
1*H*-Pyrrole-3-carboxylic acid [1S-(2-carbamoylmethyl-4-oxo-tetrahydrofuran-3-ylcarbamoyl)-3,4-dimethylpentyl]amide,
1*H*-Pyrrole-3-carboxylic acid {1S-[2-(2-dimethylaminoethyl)-4-oxo-tetrahydrofuran-3-ylcarbamoyl]-3,4-dimethylpentyl}amide,
1*H*-Pyrrole-3-carboxylic acid [3,4-dimethyl-1S-(4-oxo-2-pyrrolidin-1-yimethyl-tetrahydrofuran-3-ylcarbamoyl)pentyl]amide.
1*H-*Pyrrole-3-carboxylic acid [4,5-dimethyl-1S-(2S-methyl-4-oxo-tetrahydrofuran-3S-ylcarbamoyl)hexyl]amlde,
1*H*-Pyrrole-3-carboxylic acid [4,5-dimethyl-1S-(2-ethyl-4-oxo-tetrahydrofuran-3-ylcarbamoyl)hexyl]amide,
1*H*-Pyrrole-3-carboxylic acid [1S-(2-carbamoytmethyl-4-oxo-tetrahydrofuran-3-ylcarbamoyl)-4,5-dimethylhexyl]amide,
1*H*-Pyrrole-3-carboxylic acid {1S-[2-(2-dimethylaminoethyl)-4-oxo-tetrahydrofuran-3-ylcarbamoyl]-4,5-dimethylhexyl}amide,
1H-Pyrrole-3-carboxylic acid [4,5-dimethyl-1S-(4-oxo-2-pyrrolidin-1-ylmethyl-tetrahydrofuran-3-ylcarbamoyl)hexyl]amide,
1*H*-Pyrrole-3-carboxylic acid [3-methyl-1S-(2S-methy)-4-oxo-tetrahydrofuran-3S-ylcarbamoyl)-3-phenylbutyl]amide,
1*H*-Pyrrole-3-carboxylic acid [3-methyl-1S-(2-ethyl-4-oxo-tetrahydrofuran-3-ylcarbamoyl)-3-phenylbutyl]amide,
1*H*-Pyrrole-3-carboxylic acid [1S-(2-carbamoylmethyl-4-oxo-tetrahydrofuran-3-ylcarbamoyl)-3-methyl-3-phenylbutyl]amide,
1*H*-Pyrrole-3-carboxylic acid {1S-[2-(2-dimethylaminoethyl)-4-oxo-tetrahydrofuran-3-ylcarbamoyl]-3-methyl-3-phenylbutyl}amide,
1*H*-Pyrrole-3-carboxylic acid [3-methyl-1S-(4-oxo-2-pyrrolidin-1-ylmethyl-tetrahydrofuran-3-ylcarbamoyl)-3-phenylbutyl]amide,
1*H*-Pyrrole-3-carboxylic acid [3,3-dimethyl-1S-(2S-methyl-4-oxo-tetrahydrofuran-3S-ylcarbamoyl)-4-phenylbutyl]amide,
1*H*-Pyrrole-3-carboxylic acid [3,3-dimethyl-1S-(2-ethyl-4-oxo-tetrahydrofuran-3-ylcarbamoyl)-4-phenylbutyl]amide,
1*H*-Pyrrole-3-carboxylic acid [1S-(2-carbamoylmethyl-4-oxo-tetrahydrofuran-3-ylcarbamoyl)-3,3-dimethyl-4-phenylbutyl]amide,
1*H*-Pyrrole-3-carboxylic acid {1S-[2-(2-dimethylaminoethyl)-4-oxo-tetrahydrofuran-3-ylcarbamoyl]-3 ,3-dimethyl-4-phenylbutyl}amide,
1*H*-Pyrrole-3-carboxylic acid [3,3-dimethyl-1S-(4-oxo-2-pyrrolidin-1-ylmethyl-tetrahydrofuran-3-ylcarbamoyl)-4-phenylbutyl]amide,
1*H*-Pyrrole-3-carboxylic acid [3,3-dimethyl-1S-(2S-methyl-4-oxo-tetrahydrofuran-3S-ylcarbamoyl)-5-phenylpentyl]amide,
1*H*-Pyrrole-3-carboxylic acid [3,3-dimethyl-1S-(2-ethyl-4-oxo-tetrahydrofuran-3-ylcarbamoyl)-5-phenylpentyl]amide,
1*H*-Pyrrole-3-carboxylic acid [1S-(2-carbamoylmethyl-4-oxo-tetrahydrofuran-3-ylcarbamoyl)-3,3-dimethyl-5-phenylpentyl]amide,
1*H*-Pyrrole-3-carboxylic acid {1S-[2-(2-dimethylaminoethyl)-4-oxo-tetrahydrofuran-3-ylcarbamoyl]-3,3-dimethyl-5-phenylpentyl}amide,
1*H*-Pyrrole-3-carboxylic acid [3,3-dimethyl-1S-(4-oxo-2-pyrrolidin-1-ylmethyl-tetrahydrofuran-3-ylcarbamoyl)-5-phenylpentyl]amide,
*N*-[3,3-dimethyl-1S-(2S-methyl-4-oxo-tetrahydrofuran-3S-ylcarbamoyl)butyl]benzamide,
*N*-[3,3-dimethyl-1S-(2-ethyl-4-oxo-tetrahydrofuran-3-ylcarbamoyl)butyl]benzamide,
*N*-[1S-(2-carbamoylmethyl-4-oxo-tetrahydrofuran-3-ylcarbamoyl)-3,3-dimethylbutyl]benzamide,
*N*-{1S-[2-(2-dimethylaminoethyl)-4-oxo-tetrahydrofuran-3-ylcarbamoyl]-3,3-dimethylbutyl}benzamide,
*N*-[3,3-dimethyl-1S-(4-oxo-2-pyrrolidin-1-ylmethyl-tetrahydrofuran-3-ylcarbamoyl)butyl]benzamide,
*N*-[4-methyl-1S-(2S-methyl-4-oxo-tetrahydrofuran-3S-ylcarbamoyl)pentyl]benzamide,
*N*-[4-methyl-1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-4-methylpentyl]benzamide,
*N*-{1S-[2-(2-dimethylaminoethyl)-4-oxo-tetrahydrofuran-3-ylcarbamoyl]-4-methylpentyl}benzamide,
*N*-[4-methyl-1S-(4-oxo-2-pyrrolidin-1-ylmethyl-tetrahydrofuran-3-ylcarbamoyl)pentyl]benzamide,
*N*-[3,3-dimethyl-1S-(2S-methyl-4-oxo-tetrahydrofuran-3S-ylcarbamoyl)pentyl]benzamide,
*N*-[3,3-dimethyl-1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-3,3-dimethylpentyl]benzamide,
*N*-{1S-[2-(2-dimethylaminoethyl)-4-oxo-tetrahydrofuran-3-ylcarbamoyl]-3,3-dimethylpentyl}benzamide,
*N*-[3,3-dimethyl-1S-(4-oxo-2-pyrrolidin-1-ylmethyl-tetrahydrofuran-3-ylcarbamoyl)pentyl]benzamide,*N*-[3,3,4-trimethyl-1S-(2S-methyl-4-oxo-tetrahydrofuran-3S-ylcarbamoyl)pentyl]benzamide,
*N*-[3,3,4-trimethyl-1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-3,3,4-trimethylpentyl]benzamide.
*N*-{1S-[2-(2-dimethylaminoethyl)-4-oxo-tetrahydrofuran-3-ylcarbamoyl]-3,3,4-trimethylpentyl}benzamide,
*N*-[3,3,4-trimethyl-1S-(4-oxo-2-pyrrolidin-1-ylmethyl-tetrahydrofuran-3-ylcarbamoyl)pentyl]benzamide,
*N*-[3,4-dimethyl-1S-(2S-methyl-4-oxo-tetrahydrofuran-3S-ylcarbamoyl)pentyl]benzamide,
*N*-[3,4-dimethyl-1S-(2-ethyl-4-oxo-tetrahydrofuran-3-ylcarbamoyl)pentyl]benzamide,
*N*-[1S-(2-carbamoylmethyl-4-oxo-tetrahydrofuran-3-ylcarbamoyl)-3,4-dimethylpentyl]benzamide,
*N*-{1S-[2-(2-dimethylaminoethyl)-4-oxo-tetrahydrofuran-3-ylcarbamoyl]-3,4-dimethylpentyl}benzamide,
*N*-[3,4-dimethyl-1S-(4-oxo-2-pyrrolidin-1-ylmethyl-tetrahydrofuran-3-ylcarbamoyl)pentyl]benzamide,
*N*-[4,5-dimethyl-1S-(2S-methyl-4-oxo-tetrahydrofuran-3S-ylcarbamoyl)hexyl]benzamide,
*N*-[4,5-dimethyl-1S-(2-ethyl-4-oxo-tetrahydrofuran-3-ylcarbamoyl)hexyl]benzamide,
*N*-[1S-(2-carbamoylmethyl-4-oxo-tetrahydrofuran-3-ylcarbamoyl)-4,5-dimethylhexyl]benzamide,
*N*-{1S-[2-(2-dimethylaminoethyl)-4-oxo-tetrahydrofuran-3-ylcarbamoyl]-4,5-dimethylhexyl}benzamide,
*N*-[4,5-dimethyl-1S-(4-oxo-2-pyrrolidin-1-ylmethyl-tetrahydrofuran-3-ylcarbamoyl)hexyl]benzamide,
*N*-[3-methyl-1S-(2S-methyl-4-oxo-tetrahydrofuran-3S-ylcarbamoyl}-3-phenylbutyl]benzamide,
*N*-[3-methyl-1S-(2-ethyl-4-oxo-tetrahydrofuran-3-ylcarbamoyl)-3-phenylbutyl]benzamide,
*N*-[1S-(2-carbamoylmethyl-4-oxo-tetrahydrofuran-3-ylcarbamoyl)-3-methyl-3-phenylbutyl]benzamide,
*N*-{1S-[2-(2-dimethylaminoethyl)-4-oxo-tetrahydrofuran-3-ylcarbamoyl]-3-methyl-3-phenylbutyl}benzamide,
*N*-[3-methyl-1S-(4-oxo-2-pyrrolidin-1-ylmethyl-tetrahydrofuran-3-ylcarbamoyl)-3-phenylbutyl]benzamide,
*N*-[3,3-dimethyl-1S-(2S-methyl-4-oxo-tetrahydrofuran-3S-ylcarbamoyl)-4-phenylbutyl]benzamide,
*N*-[3,3-dimethyl-1S-(2-ethyl-4-oxo-tetrahydrofuran-3-ylcarbamoyl)-4-phenylbutyl]benzamide,
*N*-[1S-(2-carbamoylmethyl-4-oxo-tetrahydrofuran-3-ylcarbamoyl)-3,3-dimethyl-4-phenylbutyl]benzamide,
*N*-{1S-[2-(2-dimethylaminoethyl)-4-oxo-tetrahydrofuran-3-ylcarbamoyl]-3,3-dimethyl-4-phenylbutyl}benzamide,
*N*-[3,3-dimethyl-1S-(4-oxo-2-pyrrolidin-1-ylmethyl-tetrahydrofuran-3-ylcarbamoyl)-4-phenylbutyl]benzamide,
*N*-[3,3-dimethyl-1S-(2S-methyl-4-oxo-tetrahydrofuran-3S-ylcarbamoyl)-5-phenylpentyl]benzamide,
*N*-[3,3-dimethyl-1S-(2-ethyl-4-oxo-tetrahydrofuran-3-ylcarbamoyl)5-phenylpentyl]benzamide,
*N*-[1S-(2-carbamoylmethyl-4-oxo-tetrahydrofuran-3-ylcarbamoyl)-3,3-dimethyl-5-phenylpentyl]benzamide,
*N*-{1S-[2-(2-dimethylaminoethyl)-4-oxo-tetrahydrofuran-3-ylcarbamoyl]-3,3-dimethyl-5-phenylpentyl}benzamide,
*N*-[3,3-dimethyl-1S-(4-oxo-2-pyrrolidin-1-ylmethyl-tetrahydrofuran-3-ylcarbamoyl)-5-phenylpentyl]benzamide,
Morpholine-4-carboxylic acid [3,3-dimethyl-1S-(2S-methyl-4-oxo-tetrahydrofuran-3S-ylcarbamoyl)butyl]amide,
Morpholine-4-carboxylic acid [3,3-dimethyl-1S-{2-ethyl-4-oxo-tetrahydrofuran-3-ylcarbamoyl)butyl]amide,
Morpholine-4-carboxylic acid [1S-(2-carbamoylmethyl-4-oxo-tetrahydrofuran-3-ylcarbamoyl)-3,3-dimethylbutyl]amide,
Morpholine-4-carboxylic acid {1S-[2-(2-dimethylaminoethyl)-4-oxo-tetrahydrofuran-3-ylcarbamoyl]-3,3-dimethylbutyl}amide,
Morpholine-4-carboxylic acid [3,3-dimethyl-1S-(4-oxo-2-pyrrolidin-1-ylmethyl-tetrahydrofuran-3-ylcarbamoyl)butyl]amide,
Morpholine-4-carboxylic acid [4-methyl-1S-(2S-methyl-4-oxo-tetrahydrofuran-3S-ylcarbamoyl)pentyl]amide,
Morpholine-4-carboxylic acid [4-methyl-1S-(2-ethyl-4-oxo-tetrahydrofuran-3-ylcarbamoyl)pentyl]amide,
Morpholine-4-carboxylic acid [1S-(2-carbamoylmethyl-4-oxo-tetrahydrofuran-3-ylcarbamoyl)-4-methylpentyl]amide,
Morpholine-4-carboxylic acid {1S-[2-(2-dimethylaminoethyl)-4-oxo-tetrahydrofuran-3-ylcarbamoyl]-4-methylpentyl}amide,
Morpholine-4-carboxylic acid [4-methyl-1S-(4-oxo-2-pyrrolidin-1-ylmethyl-tetrahydrofuran-3-ylcarbamoyl)pentyl]amide,
Morpholine-4-carboxylic acid [3,3-dimethyl-1S-(2S-methyl-4-oxo-tetrahydrofuran-3S-ylcarbamoyl)pentyl]amide,
Morpholine-4-carboxylic acid [3,3-dimethyl-1S-(2-ethyl-4-oxo-tetrahydrofuran-3-ylcarbamoyl)pentyl]amide,
Morpholine-4-carboxylic acid [1S-(2-carbamoylmethyl-4-oxo-tetrahydrofuran-3-ylcarbamoyl)-3,3-dimethylpentyl]amide,
Morpholine-4-carboxylic acid {1S-[2-(2-dimethylaminoethyl)-4-oxo-tetrahydrofuran-3-ylcarbamoyl]-3,3-dimethylpentyl}amide,
Morpholine-4-carboxylic acid [3,3-dimethyl-1S-(4-oxo-2-pyrrolidin-1-ylmethyl-tetrahydrofuran-3-ylcarbamoyl)pentyl]amide,
Morpholine-4-carboxylic acid [3,3,4-trimethyl-1S-(2S-methyl-4-oxo-tetrahydrofuran-3S-ylcarbamoyl)pentyl]amide,
Morpholine-4-carboxylic acid [3,3,4-trimethyl-1S-(2-ethyl-4-oxo-tetrahydrofuran-3-ylcarbamoyl)pentyl]amide,
Morpholine-4-carboxylic acid [1S-(2-carbamoylmethyl-4-oxo-tetrahydrofuran-3-ylcarbamoyl)-3,3,4-trimethylpentyl]amide,
Morpholine-4-carboxylic acid {1S-[2-(2-dimethylaminoethyl)-4-oxo-tetrahydrofuran-3-ylcarbamoyl]- 3,3,4-trimethylpentyl}amide,
Morpholine-4-carboxylic acid [3,3,4-trimethyl-1S-(4-oxo-2-pyrrolidin-1-ylmethyl-tetrahydrofuran-3-ylcarbamoyl)pentyl]amide,
Morpholine-4-carboxylic acid [3,4-dimethyl-1S-(2S-methyl-4-oxo-tetrahydrofuran-3S-ylcarbamoyl)pentyl]amide,
Morpholine-4-carboxylic acid [3,4-dimethyl-1S-(2-ethyl-4-oxo-tetrahydrofuran-3-ylcarbamoyl)pentyl]amide,
Morpholine-4-carboxylic acid [1S-{2-carbamoylmethyl-4-oxo-tetrahydrofuran-3-ylcarbamoyl)-3,4-dimethylpentyl}amide,
Morpholine-4-carboxylic acid {1S-[2-(2-dimethylaminoethyl)-4-oxo-tetrahydrofuran-3-ylcarbamoyl]-3,4-dimethylpentyl}amide,
Morpholine-4-carboxylic acid [3,4-dimethyl-1S-(4-oxo-2-pyrrolidin-1-ylmethyl-tetrahydrofuran-3-ylcarbamoyl)pentyl]amide,
Morpholine-4-carboxylic acid [4,5-dimethyl-1S-(2S-methyl-4-oxo-tetrahydrofuran-3S-ylcarbamoyl)hexyl]amide,
Morpholine-4-carboxylic acid [4,5-dimethyl-1S-(2-ethyl-4-oxo-tetrahydrofuran-3-ylcarbamoyl)hexyl]amide,
Morpholine-4-carboxylic acid [1S-(2-carbamoylmethyl-4-oxo-tetrahydrofuran-3-ylcarbamoyl)-4,5-dimethylhexyl]amide,
Morpholine-4-carboxylic acid {1S-[2-(2-dimethylaminoethyl)-4-oxo-tetrahydrofuran-3-ylcarbamoyl]-4,5-dimethylhexyl}amide,
Morpholine-4-carboxylic acid [4,5-dimethyl-1S-(4-oxo-2-pyrrolidin-1-ylmethyl-tetrahydrofuran-3-ylcarbamoyl)hexyl]amide,
Morpholine-4-carboxylic acid [3-methyl-1S-(2S-methyl-4-oxo-tetrahydrofuran-3S-ylcarbamoyl)-3-phenylbutyl]amide,
Morpholine-4-carboxylic acid [3-methyl-1S-(2-ethyl-4-oxo-tetrahydrofuran-3-ylcarbamoyl)-3-phenylbutyl]amide,
Morpholine-4-carboxylic acid [1S-(2-carbamoylmethyl-4-oxo-tetrahydrofuran-3-ylcarbamoyl)-3-methyl-3-phenylbutyl]amide,
Morpholine-4-carboxylic acid {1S-[2-(2-dimethylaminoethyl)4-oxo-tetrahydrofuran-3-ylcarbamoyl]-3-methyl-3-phenylbutyl}amide,
Morpholine-4-carboxylic acid [3-methyl-1S-(4-oxo-2-pyrrolidin-1-ylmethyl-tetrahydrofuran-3-ylcarbamoyl)-3-phenylbutyl]amide.
Morpholine-4-carboxylic acid [3,3-dimethyl-1S-(2S-methyl-4-axo-tetrahydrofuran-3S-ylcarbamoyl)-4-phenylbutyl]amide,
Morpholine-4-carboxylic acid [3,3-dimethyl-1S-(2-ethyl-4-oxo-tetrahydrofuran-3-ylcarbamoyl)-4-phenylbutyl]amide,
Morpholine-4-carboxylic acid [1S-(2-carbamoylmethyl-4-oxo-tetrahydrofuran-3-ylcarbamoyl)-3,3-dimethyl-4-phenylbutyl]amide,
Morpholine-4-carboxylic acid {1S-[2-(2-dimethylaminoethyl)-4-oxo-tetrahydrofuran-3-ylcarbamoyl]-3,3-dimethyl-4-phenyibutyl}amide,
Morpholine-4-carboxylic acid [3,3-dimethyl-1S-(4-oxo-2-pyrrolidin-1-ytmethyl-tetrahydrofuran-3-ylcarbamoyl)-4-phenylbutyl]amide.
Morpholine-4-carboxylic acid [3,3-dimethyl-1S-(2S-methyl-4-oxo-tetrahydrofuran-3S-ylcarbamoyl)-5-phenylpentyl]amide,
Morpholine-4-carboxylic acid [3,3-dimethyl-1S-(2-ethyl-4-oxo-tetrahydrofuran-3-ylcarbamoyl)-5-phenylpentyl]amide,
Morpholine-4-carboxylic acid [1S-(2-carbamoylmethyl-4-oxo-tetrahydrofuran-3-ylcarbamoyl)-3,3-dimethyl-5-phenylpentyl]amide,
Morpholine-4-carboxylic acid {1S-[2-(2-dimethylaminoethyl)-4-oxo-tetrahydrofuran-3-ylcarbamoyl]-3,3-dimethyl-5-phenylpentyl}amide,
Morpholine-4-carboxylic acid [3,3-dimethyl-1S-(4-oxo-2-pyrrolidin-1-ylmethyl-tetrahydrofuran-3-ylcarbamoyl)-5-phenylpentyl]amide,
or a pharmaceutically acceptable salt thereof.

12. A pharmaceutical composition comprising a compound according to any preceding claim and a pharmaceutically acceptable carrier.

13. A compound according to any of claims 1 to 11 for use in a therapeutic method of inhibiting the cysteine protease Cathepsin S.

14. A composition according to claim 12 for use in a method of inhibiting the cysteine protease Cathepsin S.

15. Use of a compound according to any of claims 1 to 11 in the manufacture of a medicament for the treatment of a disease alleviated or moderated by inhibition of Cathepsin S cysteine protease activity.

## Patentansprüche

1. Verbindung gemäß Formel (II): 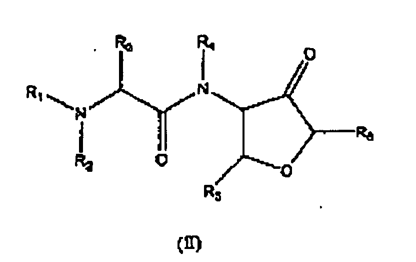 wobei:
R1 = R', R'C(O), R'C(S), R'SO₂, R'OC(O), R'NHC(O)
R' = 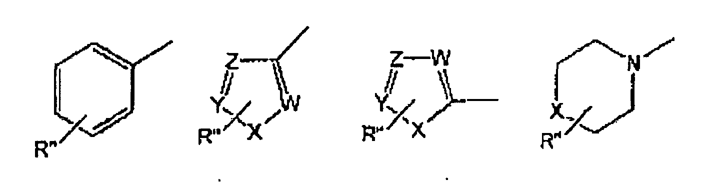
X = 0, S, NH;
W, Y, Z = CH, N;
R" = Einzel- oder Mehrfachringsubstitution-Kombinationen, die genommen wurden von:
H, C₁₋₇-alkyl, C₁₋₇-Alkyl-C₃₋₆-cycloalkyl, OH, SH, Amin, Halogen;
R2, R4 = H, C₁₋₇-Alkyl, C₁₋₇-Alkyl-C₃₋₇-cycloalkyl;
R3 = C₁₋₇-Alkyl, C₁₋₇-Alkyl-C₃₋₇-cycloalkyl, Ar-C₁₋₇-Alkyl;
R5 = C₁₋₇-Alkyl, Ar-C₁₋₇-alkyl, C₁₋₃-Alkyl-CONR"' oder R^{iv};
R"' = H, Methyl;
R^{iv} = 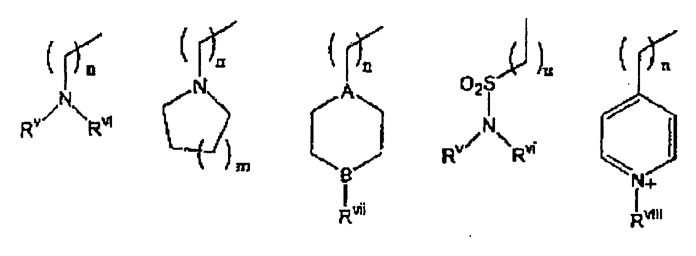
wobei:
n = 1-3, m = 1-3;
R^{v}, R^{vi} = H, C₁₋₇-Alkyl;
A = N, CH;
B = N, 0, S, CH;
R^{vii} = fehlt, wenn B = O, S; oder R^{vii} = H, C₁₋₇-Alkyl, wenn B = N, CH;
R^{viii} = O, C₁₋₇-Alkyl;
R6 = H;
und wobei:
C₁₋₇-Alkyl oder C₁₋₃-Alkyl optional durch ein oder zwei Halogene und/oder ein Heteroatom S, O, N substituiert sein kann, das, wenn es sich an einem Kettenende befindet, durch ein oder zwei H-Atome substituiert ist;
Ar Phenyl, Pyrazolyl, Imidazolyl, Oxazolyl, Thiazinolyl, Isothiazinolyl, Oxadiazolyl, 1,2,3-Triazolyl, Furanyl oder Thienyl ist, optional substituiert durch Halogen, C₁₋₃-Alkyl, OH, OC₁₋₃-Alkyl, SH, SC₁₋₃-Alkyl oder Amin;
Amin -NH₂, -NHC₁₋₃ -Alkyl oder -N(C₁₋₃-Alkyl)₂ ist,
und pharmazeutisch akzeptable Salze davon.

2. Verbindung nach Anspruch 1, wobei R2 und/oder R4 Wasserstoff sind.

3. Verbindung nach Anspruch 1 oder 2, wobei R1 R'C(O) ist, wobei R' =

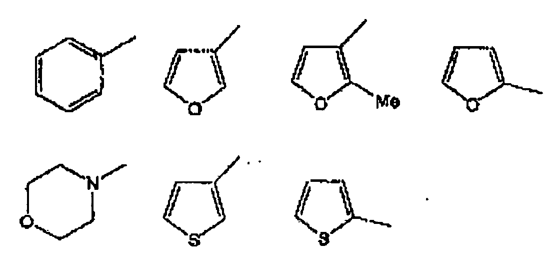

4. Verbindung nach Anspruch 3, bei der R' Fur-3-yl oder Thien-3-yl ist.

5. Verbindung nach einem der Ansprüche 1-4, bei der R3 n-Butyl, t-Butyl, 3-(2,2-Dimethylpropyl), 4-(2-Methylbutyl), 4-(3,3-Dimethylbutyl), 4-(3,3-Dimethyl-2-methylbutyl), 4-(3-Methyl-2-methylbutyl) oder 5-(2-Methyl-3-methylpentyl) ist.

6. Verbindung nach Anspruch 5, bei der R3 t-Butyl, 3-(2,2-Dimethylpropyl) oder 4-(3,3-Dimethyl-2-methylbutyl) ist.

7. Verbindung nach einem der Ansprüche 1-6, bei der R5 CH₃, C₂H₅, CH₂Ar, CH₂CONH₂, (CH₂)₂CONH₂ ist,

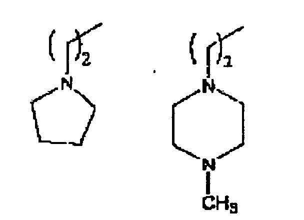

8. Verbindung nach Anspruch 7, bei der R5 CH₃ oder CH₂CH₃ ist.

9. Verbindung nach einem der Ansprüche 1-8, bei der R5 (S)-Stereochemie hat.

10. Verbindung nach Anspruch 1, bei der
RI = R'C(O)
wobei R' = 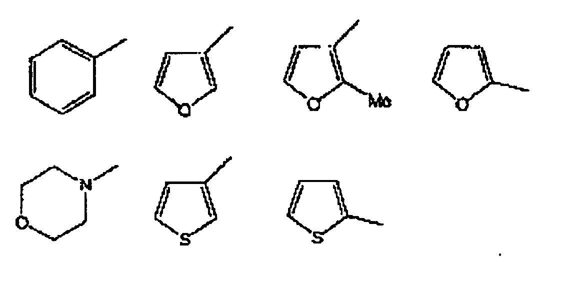 R2 und R4 = H;
R3 = n-Butyl, t-Butyl, 3-(2,2-Dimethylpropyl), 4-(2-Methylbutyl), 4-(3,3-Dimethylbutyl), 4-(3,3-Dimethyl-2-methylbutyl), 4-(3-Methyl-2-methylbutyl), 5-(2-Methyl-3-methylpentyl);
R5 = CH₃, C₂H₅, CH₂Ar, CH₂CONH₂, (CH₂)₂CONH₂, 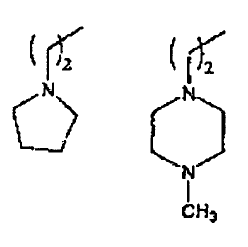 R6 = H
oder ein pharmazeutisch akzeptables Salz davon.

11. Verbindung, die ausgewählt wurde aus der Gruppe bestehend aus:
Furan-3-carbonsäure[3,3-dimethyl-1S-(2S-methyl-4-oxotetrahydrofuran-3S-ylcarbamoyl)butyl]amid,
Furan-3-carbonsäure[3,3-dimethyl-1S-(2-ethyl-4-oxotetrahydrofuran-3-ylcarbamoyl)butyl]amid,
Furan-3-carbonsäure[1S-(2-carbamoylmethyl-4-oxotetrahydrofuran-3-ylcarbamoyl)-3,3-dimethylbutyl]amid,
Furan-3-carbonsäure{1S-[2-(2-dimethylaminoethyl)-4-oxotetrahydrofuran-3-ylcabamoyl]-3,3-dimethylbutyl}amid,
Furan-3-carbonsäure(3,3-dimethyl-1S-(4-oxo-2-pyrrolidin-1-ylmethyl-tetrahydrofuran-3-ylcarbamoyl)butyl]amid,
Furan-3-carbonsäure[4-methyl-1S-(2S-methyl-4-oxotetrahydrofuran-3S-ylcarbamoyl)pentyl]amid,
Furan-3-carbonsäure[4-methyl-1S-(2-ethyl-4-oxotetrahydrofuran-3-ylcarbamoyl)pentyl]amid,
Furan-3-carbonsäure[1S-(2-carbamoylmethyl-4-oxotetrahydrofuran-3-ylcarbamoyl)-4-methylpentyl]amid,
Furan-3-carbonsäure{1S-[2-(2-dimethylaminoethyl)-4-oxotetrahydrofuran-3-ylcarbamoyl]-4-methylpentyl}amid,
Furan-3-carbonsäure[4-methyl-1S-(4-oxo-2-pyrrolidin-1-ylmethyl-tetrahydrofuran-3-ylcarbamoyl)pentyl] amid,
Furan-3-carbonsäure[3,3-dimethyl-1S-(2S-methyl-4-oxotetrahydrofuran-3S-ylcarbamoyl)pentyl]amid,
Furan-3-carbonsäure[3,3-dimethyl-1S-(2-ethyl-4-oxotetrahydrofuran-3-ylcarbamoyl)pentyl]amid,
Furan-3-carbonsäure[1S-(2-carbamoylmethyl-4-oxotetrahydrofuran-3-ylcarbamoyl)-3,3-dimethylpentyl]amid,
Furan-3-carbonsäure{1S-[2-(2-dimethylaminoethyl)-4-oxotetrahydrofuran-3-ylcarbamoyl]-3,3-dimethylpentyl}amid,
Furan-3-carbonsäure[3,3-dimethyl-1S-(4-oxo-2-pyrrolidin-1-ylmethyl-tetrahydrofuran-3-ylcarbamoyl)pentyl]amid,
Furan-3-carbonsäure[3,3,4-trimethyl-1S-(2S-methyl-4-oxotetrahydrofuran-3S-ylcarbamoyl)pentyl]amid,
Furan-3-carbonsäure[3,3,4-trimethyl-1S-(2-ethyl-4-oxotetrahydrofuran-3-ylcarbamoyl)pentyl]amid,
Furan-3-carbonsäure[1S-(2-carbamoylmethyl-4-oxotetrahydrofuran-3-ylcarbamoyl)-3,3,4-trimethylpentyl]amid,
Furan-3-carbonsäure{1S-[2-(2-dimethylaminoethyl)-4-oxotetrahydrofuran-3-ylcarbamoyl]-3,3,4-trimethylpentyl}amid,
Furan-3-carbonsäure[3,3,4-trimethyl-1S-(4-oxo-pyrrolidin-1-ylmethyl-tetrahydrofuran-3-ylcarbamoyl)pentyl]amid,
Furan-3-carbonsäure[3,4-dimethyl-1S-(2S-methyl-4-oxotetrahydrofuran-3S-ylcarbamoyl)pentyl]amid,
Furan-3-carbonsäure[3,4-dimethyl-1S-(2-ethyl-4-oxotetrahydrofuran-3-ylcarbamoyl)pentyl]amid,
Furan-3-carbonsäure[1S-(2-carbamoylmethyl-4-oxotetrahydrofuran-3-ylcarbamoyl)-3,4-dimethylpentyl]amid,
Furan-3-carboxysäure{1S-[2-(2-dimethylaminoethyl)-4-oxotetrahydrofuran-3-ylcarbamoyl]-3,4-dimethylpentyl}amid,
Furan-3-carbonsäure[3,4-dimethyl-1S-(4-oxo-2-pyrrolidin-1-ylmethyl-tetrahydrofuran-3-ylcarbamoyl)pentyl]amid,
Furan-3-carbonsäure[4,5-dimethyl-1S-(2S-methyl-4-oxotetrahydrofuran-3S-ylcarbamoyl)hexyl]amid,
Furan-3-carbonsäure[4,5-dimethyl-1S-(2-ethyl-4-oxotetrahydrofuran-3-ylcarbamoyl)hexyl]amid,
Furan-3-carbonsäure[1S-(2-carbamoylmethyl-4-oxotetrahydrofuran-3-ylcarbamoyl)-4,5-dimethylhexyl]amid,
Furan-3-carbonsäure{1S-[2-(2-dimethylaminoethyl)-4-oxotetrahydrofuran-3-ylcarbamoyl]-4,5-dimethylhexyl}amid,
Furan-3-carbonsäure[4,5-dimethyl-1S-(4-oxo-2-pyrrolidin-1-ylmethyl-tetrahydrofuran-3-ylcarbamoyl)hexyl]amid,
Furan-3-carbönsäure[3-methyl-1S-(2S-methyl-4-oxotetrahydrofuran-3S-ylcarbamoyl)-3-phenylbutyl]amid,
Furan-3-carbonsäure[3-methyl-1S-(2-ethyl-4-oxotetrahydrofuran-3-ylcarbamoyl)-3-phenylbutyl]amid,
Furan-3-carbonsäure[1S-(2-carbamoylmethyl-4-oxotetrahydrofuran-3-ylcarbamoyl)-3-methyl-3-phenylbutyl]amid,
Furan-3-carbonsäure{1S-[2-(2-carbamoylmethyl)-4-oxotetrahydrofuran-3-ylcarbamoyl]-3-methyl-3-phenylbutyl}amid,
Furan-3-carbonsäure[3-methyl-1S-(4-oxo-2-pyrrolidin-1-ylmethyl-tetrahydrofuran-3-ylcarbamoyl)-3-phenylbutyl]amid,
Furan-3-carbonsäure[3,3-dimethyl-1S-(2S-methyl-4-oxotetrahydrofuran-3S-ylcarbamoyl)-4-phenylbutyl]amid,
Furan-3-carbonsäure[3,3-dimethyl-1S-(2-ethyl-4-oxotetrahydrofuran-3-ylcarbamoyl)-4-phenylbutyl]amid,
Furan-3-carbonsäure[1S-(2-carbamoylmethyl-4-oxotetrahydrofuran-3-ylcarbamoyl)-3,3-dimethyl-4-phenylbutyl]amid,
Furan-3-carbonsäure{1S-[2-(2-dimethylaminoethyl)-4-oxotetrahydrofuran-3-ylcarbamoyl]-3,3-dimethyl-4-phenylbutyl}amid,
Furan-3-carbonsäure[3,3-dimethyl-1S-(4-oxo-2-pyrrolidin-1-ylmethyl-tetrahydrofuran-3-ylcarbamoyl)-4-phenylbutyl]amid,
Furan-3-carbonsäure[3,3-dimethyl-1S-(2S-methyl-4-oxotetrahydrofuran-3S-ylcarbamoyl)-5-phenylpentyl]amid,
Furan-3-carbonsäure[3,3-dimethyl-1S-(2-ethyl-4-oxotetrahydrofuran-3-ylcarbamoyl)-5-phenylpentyl]amid,
Furan-3-carbonsäure[1S-(2-carbamoylmethyl-4-oxotetrahydrofuran-3-ylcarbamoyl)-3,3-dimethyl-5-phenylpentyl]amid,
Furan-3-carbonsäure{1S-[2-(2-dimethylaminoethyl)-4-oxotetrahydrofuran-3-ylcarbamoyl]-3,3-dimethyl-5-phenylpentyl}amid,
Furan-3-carbonsäure[3,3-dimethyl-1S-(4-oxo-2-pyrrolidin-1-ylmethyl-tetrahydrofuran-3-ylcarbamoyl)-5-phenylpentyl]amid,
Thiophen-3-carbonsäure[3,3-dimethyl-1S-(2S-methyl-4-oxotetrahydrofuran-3S-ylcarbamoyl)butyl]amid,
Thiophen-3-carbonsäure[3,3-dimethyl-1S-(2-ethyl-4-oxo-tetrahydrofuran-3-ylcarbamoyl)butyl]amid,
Thiophen-3-carbonsäure[1S-(2-carbamoylmethyl-4-oxotetrahydrofuran-3-ylcarbamoyl]-3,3-dimethylbutyl]amid,
Thiophen-3-carbonsäure{1S-[2-(2-dimethylaminoethyl-4-oxotetrahydrofuran-3-ylcarbamoyl]-3,3-dimethylbutyl}amid,
Thiophen-3-carbonsäure[3,3-dimethyl-1S-(4-oxo-2-pyrrolidin-1-ylmethyl-tetrahydrofuran-3-ylcarbamoyl)butyl]amid,
Thiophen-3-carbonsäure(4-methyl-1S-(2S-methyl-4-oxotetrahydrofuran-3S-ylcarbamoyl)pentyl]amid,
Thiophen-3-carbonsäure[4-methyl-1S-(2-ethyl-4-oxotetrahydrofuran-3-ylcarbamoyl)pentyl]amid,
Thiophen-3-carbonsäure[1S-(2-carbamoylmethyl-4-oxotetrahydrofuran-3-ylcarbamoyl)-4-methylpentyl]amid,
Thiophen-3-carbonsäure{1S-[2-(2-dimethylaminoethyl)-4-oxotetrahydrofuran-3-ylcarbamoyl]-4-methylpentyl}amid,
Thiophen-3-carbonsäure[4-methyl-1S-(4-oxo-2-pyrrolidin-1-ylmethyl-tetrahydrofuran-3-ylcarbamoyl)pentyl]amid,
Thiophen-3-carbonsäure[3,3-dimethyl-1S-(2S-methyl-4-oxotetrahydrofuran-3S-ylcarbamoyl)pentyl]amid,
Thiophen-3-carbonsäure[3,3-dimethyl-1S-(2-ethyl-4-oxotetrahydrOfuran-3-ylcarbamoyl)pentyl]amid,
Thiophen-3-carbonsäure[1S-(2-carbymoylmethyl-4-oxotetrahydrofuran-3-ylcarbamoyl)-3,3-dimethylpentyl]amid,
Thiophen-3-carbonsäure{1S-[2-(2-dimethylaminoethyl)-4-oxotetrahydrofuran-3-ylcarbamoyl]-3,3-dimethylpentyl}amid,
Thiophen-3-carbonsäure[3,3-dimethyl-1S-(4-oxo-2-pyrrolidin-1-ylmethyl-tetrahydrofuran-3-ylcarbamoyl)pentyl]amid,
Thiophen-3-carbonsäure[3,3,4-trimethyl-1S-(2S-methyl-4-oxotetrahydrofuran-3S-ylcarbamoyl)pentyl]amid,
Thiophen-3-carbonsäure[3,3,4-trimethyl-1S-(2-ethyl-4-oxotetrahydrofuran-3-ylcarbamoyl)pentyl]amid,
Thiophen-3-carbonsäure[1S-(2-carbamoylmethyl-4-oxotetrahydrofuran-3-ylcarbamoyl)-3,3,4-trimethylpentyl]amid,
Thiophen-3-carbonsäure{1S-[2-(2-dimethylaminoethyl)-4-oxotetrahydrofuran-3-ylcarbamoyl]-3,3,4-trimethylpentyl}amid,
Thiophen-3-carbonsäure[3,3,4-trimethyl-1S-(4-oxo-2-pyrrolidin-1-ylmethyl-tetrahydrofuran-3-ylcarbamoyl)pentyl]amid,
Thiophene-3-carbonsäure[3,4-dimethyl-1S-(2S-methyl-4-oxotetrahydrofuran-3S-ylcarbamoyl)pentyl]amid,
Thiophen-3-carbonsäure[3,4-dimethyl-1S-(2-ethyl-4-oxotetrahydrofuran-3-ylcarbamoyl)pentyl]amid,
Thiophen-3-carbonsäure[1S-(2-carbamoylmethyl-4-oxotetrahydrofuran-3-ylcarbamoyl)-3,4-dimethylpentyl)amid,
Thiophen-3-carbonsäure{1S-[2-(2-dimethylaminoethyl)-4-oxotetrahydrofuran-3-ylcarbamoyl]-3,4-dimethylpentyl}amid,
Thiophen-3-carbonsäure[3,4-dimethyl-1S-(4-oxo-2-pyrrolidin-1-ylmethyl-tetrahydrofuran-3-ylcarbamoyl)pentyl]amid,
Thiophen-3-carbonsäure[4,5-dimethyl-1S-(2S-methyl-4-oxotetrahydrofuran-3S-ylcarbamoyl)hexyl]amid,
Thiophen-3-carbonsäure[4,5-dimethyl-1S-(2-ethyl-4-oxotetrahydrofuran-3-ylcarbamoyl)hexyl]amid,
Thiophen-3-carbonsäure[1S-(2-carbamoylmethyl-4-oxotetrahydrofuran-3-ylcarbamoyl)-4,5-dimethylhexyl]amid,
Thiophen-3-carbonsäure{1S-[2-(2-dimethylaminoethyl)-4-oxotetrahydrofuran-3-ylcarbamoyl]-4,5-dimethylhexyl}amid,
Thiophen-3-carbonsäure[4,5-dimethyl-1S-(4-oxo-2-pyrrolidin-1-ylmethyl-tetrahydrofuran-3-ylcarbamoyl)hexyl]amid,
Thiophen-3-carbonsäure[3-methyl-1S-(2S-methyl-4-oxotetrahydrofuran-3S-ylcarbamoyl)-3-phenylbutyl]amid,
Thiophen-3-carbonsäure[3-methyl-1S-(2-ethyl-4-oxotetrahydrofuran-3-ylcarbamoyl)-3-phenylbutyl]amid,
Thiophen-3-carbonsäure[1S-(2-carbamoylmethyl-4-oxotetrahydrofuran-3-ylcarbamoyl)-3-methyl-3-phenylbutyl]amid,
Thiophen-3-carbonsäure{1S-[2-(2-dimethylaminoethyl)-4-oxotetrahydrofuran-3-ylcarbamoyl]-3-methyl-3-phenylbutyl}amid,
Thiophen-3-carbonsäure[3-methyl-1S-(4-oxo-2-pyrrolidin-1-ylmethyl-tetrahydrofuran-3-ylcarbamoyl)-3-phenylbutyl]amid,
Thiophen-3-carbonsäure[3,3-dimethyl-1S-(2S-methyl-4-oxotetrahydrofuran-3S-ylcarbamoyl)-4-phenylbutyl]amid,
Thiophen-3-carbonsäure[3,3-dimethyl-1S-(2-ethyl-4-oxotetrahydrofuran-3-ylcarbamoyl)-4-phenylbutyl]amid,
Thiophen-3-carbonsäure[1S-(2-carbamoylmethyl-4-oxotetrahydrofuran-3-ylcarbamoyl)-3,3-dimethyl-4-phenylbutyl]amid,
Thiophen-3-carbonsäure{1S-[2-(2-dimethylaminoethyl)-4-oxotetrahydrofuran-3-ylcarbamoyl]-3,3-dimethyl-4-phyenylbutyl}amid,
Thiophen-3-carbonsäure[3,3-dimethyl-1S-(4-oxo-2-pyrrolidin-1-ylmethyl-tetrahydrofuran-3-ylcarbamoyl)-4-phenylbutyl]amid,
Thiophen-3-carbonsäure[3,3-dimethyl-1S-(2S-methyl-4-oxotetrahydrofuran-3S-ylcarbamoyl)-5-phenylpentyl]amid,
Thiophen-3-carbonsäure[3,3-dimethyl-1S-(2-ethyl-4-oxotetrahydrofuran-3-ylcarbamoyl)-5-phenylpentyl]amid,
Thiophen-3-carbonsäure[1S-(2-carbamoylmethyl-4-oxotetrahydrofuran-3-ylcarbamoyl)-3,3-dimethyl-5-phenylpentyl]amid,
Thiophen-3-carbonsäure{1S-[2-(2-dimethylaminoethyl)-4-oxotetrahydrofuran-3-ylcarbamoyl]-3,3-dimethyl-5-phenylpentyl}amid,
Thiophen-3-carbonsäure[3,3-dimethyl-1S-(4-oxo-2-pyrrolidin-1-ylmethyl-tetrahydrofuran-3-ylcarbamoyl)-5-phenylpentyl]amid,
2-Methylfuran-3-carbonsäure[3,3-dimethyl-1S-(2S-methyl-4-oxotetrahydrofuran-3S-ylcarbamoyl)butyl]amid,
2-Methylfuran-3-carbonsäure[3,3-dimethyl-1S-(2-ethyl-4-oxotetrahydrofuran-3-ylcarbamoyl)butyl]amid,
2-Methylfuran-3-carbonsäure[1S-(2-carbamoylmethyl-4-oxotetrahydrofuran-3-ylcarbamoyl)-3,3-dimethylbutyl]amid,
2-Methylfuran-3-carbonsäure{1S-[2-(2-dimethylaminoethyl)-4-oxo-tetrahydrofuran-3-ylcarbamoyl]-3,3-dimethylbutyl}amid,
2-Methylfuran-3-carbonsäure[3,3-dimethyl-1S-(4-oxo-2-pyrrolidin-1-ylmethyl-tetrahydrofuran-3-ylcarbamoyl)butyl]amid,
2-Methylfuran-3-carbonsäure[4-methyl-1S-(2S-methyl-4-oxotetrahydrofuran-3S-ylcarbamoyl)pentyl]amid,
2-Methylfuran-3-carbonsäure[4-methyl-1S-(2-ethyl-4-oxotetrahydrofuran-3-ylcarbamoyl)pentyl]amid,
2-Methylfuran-3-carbonsäure[1S-(2-carbamoylmethyl-4-oxotetrahydrofuran-3-ylcarbamoyl)-4-methylpentyl]amid,
2-Methylfuran-3-carbonsäure{1S-[2-(2-dimethylaminoethyl)-4-oxo-tetrahydrofuran-3-ylcarbamoyl]-4-methylpentyl}amid,
2-Methylfuran-3-carbonsäure[4-methyl-1S-(4-oxo-2-pyrrolidin-1-ylmethyl-tetrahydrofuran-3-ylcarbamoyl)pentyl]amid,
2-Methylfuran-3-carbonsäure[3,3-dimethyl-1S-(2S-methyl-4-oxotetrahydrofuran-3S-ylcarbamoyl)pentyl]amid,
2-Methylfuran-3-carbonsäure[3,3-dimethyl-1S-(2-ethyl-4-oxotetrahydrofuran-3-ylcarbamoyl)pentyl]amid,
2-Methylfuran-3-carbonsäure[1S-(2-carbamoylmethyl-4-oxotetrahydrofuran-3-ylcarbamoyl)-3,3-dimethylpentyl]amid,
2-Methylfuran-3-carbonsäure{1S-[2-(2-dimethylaminoethyl)-4-oxo-tetrahydrofuran-3-ylcarbamoyl]-3,3-dimethylpentyl}amid,
2-Methylfuran-3-carbonsäure[3,3-dimethyl-1S-(4-oxo-2-pyrrolidin-1-ylmethyl-tetrahydrofuran-3-ylcarbamoyl)pentyl]amid,
2-Methylfuran-3-carbonsäure[3,3,4-trimethyl-1S-(2S-methyl-4-oxo-tetrahydrofuran-3S-ylcarbamoyl)pentyl]amid,
2-Methylfuran-3-carbonsäure[3,3,4-trimethyl-1S-(2-ethyl-4-oxo-tetrahydrofuran-3-ylcarbamoyl)pentyl]amid,
2-Methylfuran-3-carbonsäure[1S-(2-carbamoylmethyl-4-oxotetrahydrofuran-3-ylcarbamoyl)-3,3,4-trimethylpentyl]amid,
2-Methylfuran-3-carbonsäure{1S-[2-(2-dimethylaminoethyl)-4-oxo-tetrahydrofuran-3-ylcarbamoyl]-3,3,4-trimethylpentyl}amid,
2-Methylfuran-3-carbonsäure[3,3,4-trimethyl-1S-(4-oxo-2-pyrrolidin-1-ylmethyl-tetrahydrofuran-3-ylcarbamoyl)pentyl]amid,
2-Methylfuran-3-carbonsäure[3,4-dimethyl-1S-(2S-methyl-4-oxotetrahydrofuran-3S-ylcarbamoyl)pentyl]amid,
2-Methylfuran-3-carbonsäure[3,4-dimethyl-1S-(2-ethyl-4-oxotetrahydrofuran-3-ylcarbamoyl)pentyl]amid,
2-Methylfuran-3-carbonsäure[1S-(2-carbamoylmethyl-4-oxotetrahydrofuran-3-ylcarbamoyl)-3,4-dimethylpentyl]amid,
2-Methylfuran-3-carbonsäure{1S-[2-(2-dimethylaminoethyl)-4-oxo-tetrahydrofuran-3-ylcarbamoyl]-3,4-dimethylpentyl}amid,
2-Methylfuran-3-carbonsäure[3,4-dimethyl-1S-(4-oxo-2-pyrrolidin-1-ylmethyl-tetrahydrofuran-3-ylcarbamoyl)pentyl]amid,
2-Methylfuran-3-carbonsäure[4,5-dimethyl-1S-(2S-methyl-4-oxotetrahydrofuran-3S-ylcarbamoyl)hexyl]amid,
2-Methylfuran-3-carbonsäure[4,5-dimethyl-1S-(2-ethyl-4-oxotetrahydrofuran-3-ylcarbamoyl)hexyl]amid,
2-Methylfuran-3-carbonsäure[1S-(2-carbamoylmethyl-4-oxotetrahydrofuran-3-ylcarbamoyl)-4,5-dimethylhexyl]amid,
2-Methylfuran-3-carbonsäure{1S-[2-(2-dimethylaminoethyl)-4-oxo-tetrahydrofuran-3-ylcarbamoyl]-4,5-dimethylhexyl}amid,
2-Methylfuran-3-carbonsäure[4,5-dimethyl-1S-(4-oxo-2-pyrrolidin-1-ylmethyl-tetrahydrofuran-3-ylcarbamoyl)hexyl]amid,
2-Methylfuran-3-carbonsäure[3-methyl-1S-(2S-methyl-4-oxotetrahydrofuran-3S-ylcarbamoyl)-3-phenylbutyl]amid,
2-Methylfuran-3-carbonsäure[3-methyl-1S-(2-ethyl-4-oxotetrahydrofuran-3-ylcarbamoyl)-3-phenylbutyl]amid,
2-Methylfuran-3-carbonsäure[1S-(2-carbamoylmethyl-4-oxotetrahydrofuran-3-ylcarbamoyl)-3-methyl-3-phenylbutyl]amid,
2-Methylfuran-3-carbonsäure{1S-[2-(2-dimethylaminoethyl)-4-oxo-tetrahydrofuran-3-ylcarbamoyl]-3-methyl-3-phenylbutyl}amid,
2-Methylfuran-3-carbonsäure[3-methyl-1S-(4-oxo-2-pyrrolidin-1-ylmethyl-tetrahydrofuran-3-ylcarbamoyl)-3-phenylbutyl]amid,
2-Methylfuran-3-carbonsäure[3,3-dimethyl-1S-(2S-methyl-4-oxotetrahydrofuran-3S-ylcarbamoyl)-4-phenylbutyl]amid,
2-Methylfuran-3-carbonsäure[3,3-dimethyl-1S-(2-ethyl-4-oxotetrahydrofuran-3-ylcarbamoyl)-4-phenylbutyl]amid,
2-Methylfuran-3-carbonsäure[1S-(2-carbamoylmethyl-4-oxotetrahydrofuran-3-ylcarbamoyl)-3,3-dimethyl-4-phenylbutyl]amid,
2-Methylfuran-3-carbonsäure{1S-[2-(2-dimethylaminoethyl)-4-oxo-tetrahydrofuran-3-ylcarbamoyl]-3,3-dimethyl-4-phenylbutyl}amid,
2-Methylfuran-3-carbonsäure[3,3-dimethyl-1S-(4-oxo-2-pyrrolidin-1-ylmethyl-tetrahydrofuran-3-ylcarbamoyl)-4-phenylbutyl]amid,
2-Methylfuran-3-carbonsäure[3,3-dimethyl-1S-(2S-methyl-4-oxotetrahydrofuran-3S-ylcarbamoyl)-5-phenylpentyl]amid,
2-Methylfuran-3-carbonsäure[3,3-dimethyl-1S-(2-ethyl-4-oxotetrahydrofuran-3-ylcarbamoyl)-5-phenylpentyl]amid,
2-Methylfuran-3-carbonsäure[1S-(2-carbamoylmethyl-4-oxotetrahydrofuran-3-ylcarbamoyl)-3,3-dimethyl-5-phenylpentyl]amid,
2-Methylfuran-3-carbonsäure{1S-[2-(2-dimethylaminoethyl)-4-oxo-tetrahydrofuran-3-ylcarbamoyl]-3,3-dimethyl-5-phenylpentyl}amid,
2-Methylfuran-3-carbonsäure[3,3-dimethyl-1S-(4-oxo-2-pyrrolidin-1-ylmethyl-tetrahydrofuran-3-ylcarbamoyl)-5-phenylbutyl]amid,
1*H*-Pyrrol-3-carbonsäure[3,3-dimethyl-1S-(2S-methyl-4-oxotetrahydrofuran-3S-ylcarbamoyl)butyl]amid,
1*H*-Pyrrol-3-carbonsäure[3,3-dimethyl-1S-(2-ethyl-4-oxotetrahydrofuran-3-ylcarbamoyl)butyl]amid,
1*H*-Pyrrol-3-carbonsäure[1s-(2-carbamoylmethyl-4-oxotetrahydrofuran-3-ylcarbamoyl)-3,3-dimethylbutyl]amid,
1*H*-Pyrrol-3-carbonsäure{1S-[2-(2-dimethylaminoethyl)-4-oxotetrahydrofuran-3-ylcarbamoyl]-3,3-dimethylbutyl}amid,
1*H*-Pyrrol-3-carbonsäure[3,3-dimethyl-1S-(4-oxo-2-pyrrolidin-1-ylmethyl-tetrahydrofuran-3-ylcarbamoyl)butyl]amid,
1*H*-Pyrrol-3-carbonsäure[4-methyl-1S-(2S-methyl-4-oxotetrahydrofuran-3S-ylcarbamoyl)pentyl]amid,
1*H*-Pyrrol-3-carbonsäure[4-methyl-1S-(2-ethyl-4-oxotetrahydrofuran-3-ylcarbamoyl)pentyl]amid,
1*H*-Pyrrol-3-carbonsäure[1S-(2-carbamoylmethyl-4-oxotetrahydrofuran-3-ylcarbamoyl)-4-methylpentyl]amid,
1*H*-Pyrrol-3-carbonsäure{1S-[2-(2-dimethylaminoethyl)-4-oxotetrahydrofuran-3-ylcarbamoyl]-4-methylpentyl}amid,
1*H*-Pyrrol-3-carbonsäure[4-methyl-1S-(4-oxo-2-pyrrolidin-1-ylmethyl-tetrahydrofuran-3-ylcarbamoyl)pentyl]amid,
1*H*-Pyrrol-3-carbonsäure[3,3-dimethyl-1S-(2S-methyl-4-oxotetrahydrofuran-3S-ylcarbamoyl)pentyl]amid,
1*H*-Pyrrol-3-carbonsäure[3,3-dimethyl-1S(2-ethyl-4-oxotetrahydrofuran-3-ylcarbamoyl)pentyl]amid,
1*H*-Pyrrol-3-carbonsäure[1S-(2-carbamoylmethyl-4-oxotetrahydrofuran-3-ylcarbamoyl)-3,3-dimethylpentyl]amid,
1*H*-Pyrrol-3-carbonsäure{1S-(2-(2-dimethylaminoethyl)-4-oxotetrahydrofuran-3-ylcarbamoyl]-3,3-dimethylpentyl}amid,
1*H*-Pyrrol-3-carbonsäure[3,3-dimethyl-1S-(4-oxo-2-pyrrolidin-1-ylmethyl-tetrahydrofuran-3-ylcarbamoyl)pentyl]amid,
1*H*-Pyrrol-3-carbonsäure[3,3,4-trimethyl-1S-(2S-methyl-4-oxotetrahydrofuran-3S-ylcarbamoyl)pentyl]amid,
1*H*-Pyrrol-3-carbonsäure[3,3,4-trimethyl-1S-(2-ethyl-4-oxotetrahydrofuran-3-ylcarbamoyl)pentyl]amid,
1H-Pyrrol-3-carbonsäure[1S-(2-carbamoylmethyl-4-oxotetrahydrofuran-3-ylcarbamol)-3,3,4-trimethylpentyl]amid,
1*H*-Pyrrol-3-carbonsäure{1S-[2-(2-dimethylaminoethyl)-4-oxotetrahydrofuran-3-ylcarbamoyl]-3,3,4-trimethylpentyl}amid,
1*H*-Pyrrol-3-carbonsäure[3,3,4-trimethyl-1S-(4-oxo-2-pyrrolidin-1-ylmethyl-tetrahydrofuran-3-ylcarbamoyl)pentyl]amid,
1*H*-Pyrrol-3-carbonsäure[3,4-dimethyl-1S-(2S-methyl-4-oxotetrahydrofuran-3S-ylcarbamoyl)pentyl]amid,
1*H*-Pyrrol-3-carbonsäure[3,4-dimethyl-1S-(2-ethyl-4-oxotetrahydrofuran-3-ylcarbamoyl)pentyl]amid,
1*H*-Pyrrol-3-carbonsäure[1S-(2-carbamoylmethyl-4-oxotetrahydrofuran-3-ylcarbamoyl)-3,4-dimethylpentyl]amid,
1*H*-Pyrrol-3-carbonsäure{1*S*-(2-(2-dimethylaminoethyl)-4-oxotetrahydrofuran-3-ylcarbamoyl]-3,4-dimethylpentyl}amid,
1*H*-Pyrrol-3-carbonsäure[3,4-dimethyl-1S-(4-oxo-2-pyrrolidin-1-ylmethyl-tetrahydrofuran-3-ylcarbamoyl)pentyl]amid,
1*H*-Pyrrol-3-carbonsäure[4,5-dimethyl-1S-(2S-methyl-4-oxotetrahydrofuran-3S-ylcarbamoyl)hexyl]amid,
1*H*-Pyrrol-3-carbonsäure[4,5-dimethyl-1S-(2-ethyl-4-oxotetrahydrofuran-3-ylcarbamoyl)hexyl]amid,
1*H*-Pyrrol-3-carbonsäure[1S-(2-carbamoylmethyl-4-oxotetrahydrofuran-3-ylcarbamoyl)-4,5-dimethylhexyl]amid,
1*H*-Pyrrol-3-carbonsäure{1S-(2-(2-dimethylaminoethyl)-4-oxotetrahydrofuran-3-ylcarbamoyl]-4,5-dimethylhexyl}amid,
1*H*-Pyrrol-3-carbonsäure[4,5-dimethyl-1S-(4-oxo-2-pyrrolidin-1-ylmethyl-tetrahydrofuran-3-ylcarbamoyl)hexyl]amid,
1*H*-Pyrrol-3-carbonsäure[3-methyl-1S-(2S-methyl-4-oxotetrahydrofuran-3S-ylcarbamoyl)-3-phenylbutyl]amid,
1*H*-Pyrrol-3-carbonsäure[3-methyl-1S-(2-ethyl-4-oxotetrahydrofuran-3-ylcarbamoyl)-3-phenylbutyl]amid,
1*H*-Pyrrol-3-carbonsäure[1S-(2-carbamoylmethyl-4-oxotetrahydrofuran-3-ylcarbamoyl)-3-methyl-3-phenylbutyl]amid,
1*H*-Pyrrol-3-carbonsäure{1S-[2-(2-dimethylaminoethyl)-4-oxotetrahydrofuran-3-ylcarbamoyl]-3-methyl-3-phenylbutyl}amid,
1*H*-Pyrrol-3-carbonsäure[3-methyl-1S-(4-oxo-2-pyrrolidin-1-ylmethyl-tetrahydrofuran-3-ylcarbamoyl)-3-phenylbutyl]amid,
1*H*-Pyrrol-3-carbonsäure[3,3-dimethyl-1S-(2S-methyl-4-oxotetrahydrofuran-3S-ylcarbamoyl)-4-phenylbutyl]amid,
1*H*-Pyrrol-3-carbonsäure[3,3-dimethyl-1S-(2-ethyl-4-oxotetrahydrofuran-3-ylcarbamoyl)-4-phenylbutyl]amid,
1*H*-Pyrrol-3-carbonsäure[1S-(2-carbamoylmethyl-4-oxotetrahydrofuran-3-ylcarbamoyl)-3,3-dimethyl-4-phenylbutyl]amid,
1*H*-Pyrrol-3-carbonsäure{1S-[2-(2-dimethylaminoethyl)-4-oxotetrahydrofuran-3-ylcarbamoyl]-3,3-dimethyl-4-phenylbutyl}amid,
1*H*-Pyrrol-3-carbonsäure[3,3-dimethyl-1S-(4-oxo-2-pyrrolidin-1-ylmethyl-tetrahydrofuran-3-ylcarbamoyl)-4-phenylbutyl]amid,
1*H*-Pyrrol-3-carbonsäure[3,3-dimethyl-1S-(2S-methyl-4-oxotetrahydrofuran-3S-ylcarbamoyl)-5-phenylpentyl]amid,
1*H*-Pyrrol-3-carbonsäure[3,3-dimethyl-1S-(2-ethyl-4-oxotetrahydrofuran-3-ylcarbamoyl)-5-phenylpentyl]amid,
1*H*-Pyrrol-3-carbonsäure[1S-(2-carbamoylmethyl-4-oxotetrahydrofuran-3-ylcarbamoyl)-3,3-dimethyl-5-phenylpentyl]amid,
1*H*-Pyrrol-3-carbonsäure{1S-[2-(2-dimethylaminoethyl)-4-oxotetrahydrofuran-3-ylcarbamoyl]-3,3-dimethyl-5-phenylpentyl}amid,
1*H*-Pyrrol-3-carbonsäure[3,3-dimethyl-1S-(4-oxo-2-pyrrolidin-1-ylmethyl-tetrahydrofuran-3-ylcarbamoyl)-5-phenylpentyl]amid,
*N-*[3,3-dimethyl-1S-(2S-methyl-4-oxo-tetrahydrofuran-3Sylcarbamoyl)butyl]benzamid,
*N*-[3,3-dimethyl-1S-(2-ethyl-4-oxo-tetrahydrofuran-3-ylcarbamoyl)butyl]benzamid,
*N*-[1S-(2-carbamoylmethyl-4-oxo-tetrahydrofuran-3-ylcarbamoyl)-3,3-dimethylbutyl]benzamid,
*N*-{1S-[2-(2-dimethylaminoethyl)-4-oxo-tetrahydrofuran-3-ylcarbamoyl]-3,3-dimethylbutyl}benzamid,
*N*-[3,3-dimethyl-1S-(4-oxo-2-pyrrolidin-1-ylmethyltetrahydrofuran-3-ylcarbamoyl)butyl]benzamid,
*N*-[4-methyl-1S-(2S-methyl-4-oxo-tetrahydrofuran-3S-ylcarbamoyl)pentyl]benzamid,
*N*-[4-methyl-1S-(2-ethyl-4-oxo-tetrahydrofuran-3-ylcarbamoyl)pentyl]benzamid,
*N*-[1S-(2-Carbamoylmethyl-4-oxo-tetrahydrofuran-3-ylcarbamoyl)-4-methylpentyl]benzamid,
*N*-{1S-[2-(2-dimethylaminoethyl)-4-oxo-tetrahydrofuran-3-ylcarbamoyl]-4-methylpentyl}benzamid,
*N*-[4-methyl-1S-(4-oxo-2-pyrrolidin-1-ylmethyl-tetrahydrofuran-3-ylcarbamoyl)pentyl]benzamid,
*N*-[3,3-dimethyl-1S-(2S-methyl-4-oxo-tetrahydrofuran-3S-ylcarbamoyl)pentyl]benzamid,
*N*-[3,3-dimethyl-1S-(2-ethyl-4-oxo-tetrahydrofuran-3-ylcarbamoyl)pentyl]benzamid,
*N*-[1S-(2-carbamoylmethyl-4-oxo-tetrahydofuran-3-ylcarbamoyl]-3,3-dimethylpentyl)benzamid,
*N*-{1S-[2-(2-dimethylaminoethyl)-4-oxo-tetrahydofuran-3-ylcarbamoyl]-3,3-dimethylpentyl}benzamid,
*N*-[3,3-dimethyl-1S-(4-oxo-2-pyrrolidin-1-ylmethyltetrahydrofuran-3-ylcarbamoyl)pentyl]benzamid,
*N*-[3,3,4-trimethyl-1S-(2S-methyl-4-oxo-tetrahydrofuran-3S-ylcarbamoyl)pentyl]benzamid,
*N*-[3,3,4-trimethyl-1S-(2-ethyl-4-oxo-tetrahydrofuran-3-ylcarbamoyl)pentyl]benzamid,
*N*-[1S-(carbamoylmethyl-4-oxo-tetrahydrofuran-3-ylcarbamoyl)-3,3,4-trimethylpentyl]benzamid,
*N*-*{1*S-[2-(2-dimethylaminoethyl)-4-oxo-tetrahydrofuran-3-ylcarbamoyl]-3,3,4-trimethylpentyl}benzamid,
*N*-[3,3,4-trimethyl-1S-(4-oxo-2-pyrrolidin-1-ylmethyl-tetrahydrofuran-3-ylcarbamoyl)pentyl]benzamid,
*N*-[3,4-dimethyl-1S-(2S-methyl-4-oxo-tetrahydrofuran-3S-ylcarbamoyl)pentyl]benzamid,
*N*-[3,4-dimethyl-1S-(2-ethyl-4-oxo-tetrahydrofuran-3-ylcarbamoyl)pentyl]benzamid,
*N*-[1S-(2-carbamoylmethyl-4-oxo-tetrahydrofuran-3-ylcarbamoyl)-3,4-dimethylpentyl]benzamid,
*N*-{1S-[2-(2-diemthylaminoethyl]-4-oxo-tetrahydrofuran-3-ylcarbamoyl]-3,4-dimethylpentyl}benzamid,
*N*-[3,4-dimethyl-1S-(4-oxo-2-pyrrolidin-1-ylmethyl-tetrahydrofuran-3-ylcarbamoyl)pentyl]benzamid,
*N*-[4,5-dimethyl-1S-(2S-methyl-4-oxo-tetrahydrofuran-3S-ylcarbamoyl)hexyl]benzamid,
*N*-[4,5-dimethyl-1S-(2-ethyl-4-oxo-tetrahydrofuran-3-ylcarbamoyl)hexyl]benzamid,
*N*-[1S-(2-carbamoylmethyl-4-oxo-tetrahydrofuran-3-ylcarbamoyl)-4,5-dimethylhexyl]benzamid,
*N*-{1S-[2-(2-dimethylaminoethyl)-4-oxo-tetrahydrofuran-3-ylcarbamoyl]-4,5-dimethylhexyl}benzamid,
*N*-[4,5-dimethyl-1S-(4-oxo-2-pyrrolidin-1-ylmethyl-tetrahydrofuran-3-ylcarbamoyl)hexyl]benzamid,
*N*-[3-methyl-1S-(2S-methyl-4-oxo-tetrahydrofuran-3S-ylcarbamoyl)-3-phenylbutyl]benzamid,
*N*-[3-methyl-1S-(2-ethyl-4-oxo-tetrahydrofuran-3-ylcarbamoyl)-3-phenylbutyl]benzamid,
*N*-[1S-(2-carbamoylmethyl-4-oxo-tetrahydrofuran-3-ylcarbamoyl)-3-methyl-3-phenylbutyl]benzamid,
*N*-{1S-[2-(2-dimethylaminoethyl)-4-oxo-tetrahydrofuran-3-ylcarbamoyl]-3-methyl-3-phenylbutyl}benzamid,
*N*-[3-methyl-1S-(4-oxo-2-pyrrolidin-1-ylmethyl-tetrahydrofuran-3-ylcarbamoyl)-3-phenylbutyl]benzamid,
*N*-[3,3-dimethyl-1S-(2S-methyl-4-oxo-tetrahydrofuran-3S-ylcarbamoyl)-4-phenylbutyl]benzamid,
*N*-[3,3-dimethyl-1S-(2-ethyl-4-oxo-teträhydrofuran-3-ylcarbamoyl)-4-phenylbutyl]benzamid,
*N*-[1S-(2-carbamoylmethyl-4-oxo-tetrahydrofuran-3-ylcarbamoyl)-3,3-dimethyl-4-phenylbutyl]benzamid,
*N*-{1S-[2-(2-dimethylaminoethyl)-4-oxo-tetrahydrofuran-3-ylcarbamoyl]-3,3-dimethyl-4-phenylbutyl}benzamid,
*N*-[3,3-dimethyl-1S-(4-oxo-2-pyrrolidin-1-ylmethyl-tetrahydrofuran-3-ylcarbamoyl)-4-phenylbutyl]benzamid,
*N*-[3,3-dimethyl-1S-(2S-methyl-4-oxo-tetrahydrofuran-3S-ylcarbamoyl)-5-phenylpentyl]benzamid,
*N*-[3,3-dimethyl-1S-(2-ethyl-4-oxo-tetrahydrofuran-3-ylcarbamoyl)-5-phenylpentyl]benzamid,
*N*-[1S-(2-carbamoylmethyl-4-oxo-tetrahydrofuran-3-ylcarbamoyl)-3,3-dimethyl-5-phenylpentyl]benzamid,
*N*-{1S-[2-(2-dimethylaminoethyl)-4-oxo-tetrahydrofuran-3-ylcarbamoyl]-3,3-dimethyl-5-phenylpentyl}benzamid,
*N*-[3,3-dimethyl-1S-(4-oxo-2-pyrrolidin-1-ylmethyl-tetrahydrofuran-3-ylcarbamoyl)-5-phenylpentyl]benzamid,
Morpholin-4-carbonsäure[3,3-dimethyl-1S-(2S-methyl-4-oxotetrahydrofuran-3S-ylcarbamoyl)butyl]amid,
Morpholin-4-carbonsäure[3,3-dimethyl-1S-(2-ethyl-4-oxotetrahydrofuran-3-ylcarbamoyl)butyl]amid,
Morpholin-4-carbonsäure[1S-(2-carbamoylmethyl-4-oxotetrahydrofuran-3-ylcarbamoyl)-3,3-dimethylbutyl]amid,
Morpholin-4-carbonsäure{1S-[2-(2-dimethylaminoethyl)-4-oxotetrahydrofuran-3-ylcarbamoyl]-3,3-dimethylbutyl}amid,
Morpholin-4-carbonsäure[3,3-dimethyl-1S-(4-oxo-2-pyrrolidin-1 - ylmethyl-tetrahydrofuran-3-ylcarbamoyl)butyl]amid,
Morpholin-4-carbonsäure[4-methyl-1S-(2S-methyl-4-oxotetrahydrofuran-3S-ylcarbamoyl)pentyl]amid,
Morpholin-4-carbonsäure[4-methyl-1S-(2-ethyl-4-oxotetrahydrofuran-3-ylcarbamoyl)pentyl]amid,
Morpholin-4-carbonsäure[1S-(2-carbamoylmethyl-4-oxotetrahydrofuran-3-ylcarbamoyl)-4-methylpentyl]amid,
Morpholin-4-carbonsäure{1S-[2-(2-dimethylaminoethyl)-4-oxotetrahydrofuran-3-ylcarbamoyl]-4-methylpentyl}amid,
Morpholin-4-carbonsäure[4-methyl-1S-(4-oxo-2-pyrrolidin-1-ylmethyl-tetrahydrofuran-3-ylcarbamoyl)pentyl]amid,
Morpholin-4-carbonsäure[3,3-dimethyl-1S-(2S-methyl-4-oxotetrahydrofuran-3S-ylcarbamoyl)pentyl]amid,
Morpholin-4-carbonsäure[3,3-dimethyl-1S-(2-ethyl-4-oxotetrahydrofuran-3-ylcarbamoyl)pentyl]amid,
Morpholin-4-carbonsäure(1S-(2-carbamoylmethyl-4-oxotetrahydrofuran-3-ylcarbamoyl)-3,3-dimethylpentyl]amid,
Morpholin-4-carbonsäure{1S-[2-(2-dimethylaminoethyl)-4-oxotetrahydrofuran-3-ylcarbamoyl]-3,3-dimethylpentyl}amid,
Morpholin-4-carbonsäure(3,3-dimethyl-1S-(4-oxo-2-pyrrolidin-1 - ylmethyl-tetrahydrofuran-3-ylcarbamoyl)pentyl] amid,
Morpholin-4-carbonsäure[3,3,4-trimethyl-1S-(2S-methyl-4-oxotetrahydrofuran-3S-ylcarbamoyl)pentyl]amid,
Morpholin-4-carbonsäure[3,3,4-trimethyl-1S-(2-ethyl-4-oxotetrahydrofuran-3-ylcarbamoyl)pentyl]amid,
Morpholin-4-carbonsäure[1S-(2-carbamoylmethyl-4-oxotetrahydrofuran-3-ylcarbamoyl)-3,3,4-trimethylpentyl]amid,
Morpholin-4-carbonsäure{1S-[2-(2-dimethylaminoethyl)-4-oxotetrahydrofuran-3-ylcarbamoyl]-3,3,4-trimethylpentyl}amid,
Morpholin-4-carbonsäure[3,3,4-trimethyl-1S-(4-oxo-2-pyrrolidin-1-ylmethyl-tetrahydrofuran-3-ylcarbämoyl)pentyl]amid,
Morpholin-4-carbonsäure[3,4-dimethyl-1S-(2S-methyl-4-oxotetrahydrofuran-3S-ylcarbamoyl)pentyl]amid,
Morpholin-4-carbonsäure[3,4-dimethyl-1S-(2-ethyl-4-oxotetrahydrofuran-3-ylcarbamoyl)pentyl]amid,
Morpholin-4-carbonsäure[1S-(2-carbamoylmethyl-4-oxotetrahydrofuran-3-ylcarbamoyl)-3,4-dimethylpentyl]amid,
Morpholin-4-carbonsäure{1S-[2-(2-dimethylaminoethyl)-4-oxotetrahydrofuran-3-ylcarbamoyl]-3,4-dimethylpentyl}amid,
Morpholin-4-carbonsäure[3,4-dimethyl-1S-(4-oxo-2-pyrrolidin-1-ylmethyl-tetrahydrofuran-3-ylcarbamoyl)pentyl]amid,
Morpholin-4-carbonsäure[4,5-dimethyl-1S-(2S-methyl-4-oxotetrahydrofuran-3S-ylcarbamoyl)hexyl]amid,
Morpholin-4-carbonsäure[4,5-dimethyl-1S-(2-ethyl-4-oxotetrahydrofuran-3-ylcarbamoyl)hexyl]amid,
Morpholin-4-carbonsäure[1S-(2-carbamoylmethyl-4-oxotetrahydrofuran-3-ylcarbamoyl)-4,5-dimethylhexyl]amid,
Morpholin-4-carbonsäure{1S-[2-(2-dimethylaminoethyl)-4-oxotetrahydrofuran-3-ylcarbamoyl]-4,5-dimethylhexyl}amid,
Morpholin-4-carbonsäure[4,5-dimethyl-1S-(4-oxo-2-pyrrolidin-1 - ylmethyl-tetrahydrofuran-3-ylcarbamoyl)hexyl]amid,
Morpholin-4-carbonsäure[3-methyl-1S-(2S-methyl-4-oxotetrahydrofuran-3S-ylcarbamoyl)-3-phenylbutyl]amid,
Morpholin-4-carbonsäure[3-methyl-1S-(2-ethyl-4-oxotetrahydrofuran-3-ylcarbamoyl)-3-phenylbutyl]amid,
Morpholin-4-carbonsäure[1S-(2-carbamoylmethyl-4-oxotetrahydrofuran-3-ylcarbamoyl)-3-methyl-3-phenylbutyl]amid,
Morpholin-4-carbonsäure{1S-[2-(2-dimethylaminoethyl)-4-oxotetrahydrofuran-3-ylcarbamoyl]-3-methyl-3-phenylbutyl}amid,
Morpholin-4-carbonsäure[3-methyl-1S-(4-oxo-2-pyrrolidin-1-ylmethyl-tetrahydrofuran-3-ylcarbamoyl)-3-phenylbutyl]amid,
Morpholin-4-carbonsäure[3,3-dimethyl-1S-(2S-methyl-4-oxotetrahydrofuran-3S-ylcarbamoyl)-4-phenylbutyl]amid,
Morpholin-4-carbonsäure[3,3-dimethyl-1S-(2-ethyl-4-oxotetrahydrofuran-3-ylcarbamoyl)-4-phenylbutyl]amid,
Morpholin-4-carbonsäure[1S-(2-carbamoylmethyl-4-oxotetrahydrofuran-3-ylcarbamoyl)-3,3-dimethyl-4-phenylbutyl]amid,
Morpholin-4-carbonsäure{1S-[2-(2-dimethylaminoethyl)-4-oxotetrahydrofuran-3-ylcarbamoyl]-3,3-dimethyl-4-phenylbutyl}amid,
Morpholin-4-carbonsäure[3,3-dimethyl-1S-(4-oxo-2-pyrrolidin-1 - ylmethyl-tetrahydrofuran-3-ylcarbamoyl)-4-phenylbutyl]amid,
Morpholin-4-carbonsäure[3,3-dimethyl-1S-(2S-methyl-4-oxotetrahydrofuran-3S-ylcarbamoyl)-5-phenylpentyl]amid,
Morpholin-4-carbonsäure[3,3-dimethyl-1S-(2-ethyl-4-oxotetrahydrofuran-3-ylcarbamoyl)-5-phenylpentyl]amid,
Morpholin-4-carbonsäure[1S-(2-carbamoylmethyl-4-oxotetrahydrofuran-3-ylcarbamoyl)-3,3-dimethyl-5-phenylpentyl]amid,
Morpholin-4-carbonsäure{1S-[2-(2-dimethylaminoethyl)-4-oxotetrahydrofuran-3-ylcarbamoyl]-3,3-dimethyl-5-phenylpentyl}amid,
Morpholin-4-carbonsäure[3,3-dimethyl-1S-(4-oxo-2-pyrrolidin-1 - ylmethyl-tetrahydrofuran-3-ylcarbamoyl)-5-phenylpentyl]amid,
oder ein pharmazeutisch akzeptables Salz davon.

12. Pharmazeutische Zusammensetzung, umfassend eine Verbindung nach einem der vorherigen Ansprüche und einen pharmazeutisch akzeptablen Träger.

13. Verbindung nach einem der Ansprüche 1 bis 11 für die Verwendung in einem therapeutischen Verfahren zum Hemmen der Cysteinprotease Cathepsin S.

14. Zusammensetzung nach Anspruch 12 für die Verwendung in einem Verfahren zum Hemmen der Cysteinprotease Cathepsin S.

15. Verwendung einer Verbindung nach einem der Ansprüche 1 bis 11 bei der Herstellung eines Medikaments für die Behandlung einer Erkrankung, die durch die Hemmung von Cathespin S Cysteinproteaseaktivität erleichtert oder gemildert wird.

## Revendications

1. Composé selon la formule (II): 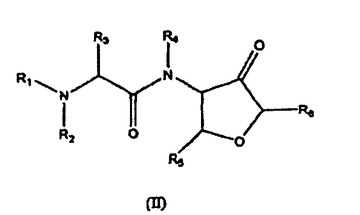 où:
R1 = R', R'C (O), R'C (S), R'SO₂, R'OC (O), R'NHC(O)
R' = 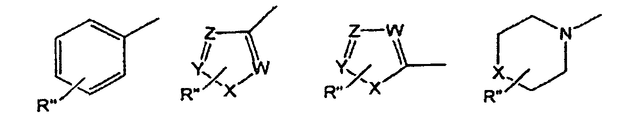
X, = O, S, NH;
W, Y, Z = CH, N;
R"= des combinaisons de substitutions cycliques simples ou multiples sélectionnées de :
H, alkyle C₁₋₇, alkyle C₁₋₇-cycloalkyle C₃₋₆, OH, SH, amine, halogène;
R2, R4 = H, alkyle C₁₋₇, alkyle C₁₋₇-cycloalkyle C₃₋₇;
R3 = alkyle C₁₋₇, alkyle C₁₋₇-cycloalkyle C₃₋₇; Ar-alkyle C₁₋₇;
R5 = alkyle C₁₋₇, Ar-alkyle C₁₋₇, alkyle C₁₋₃-CONR'" ou R^{iv};
R"' = H, méthyle;
R^{iv}= 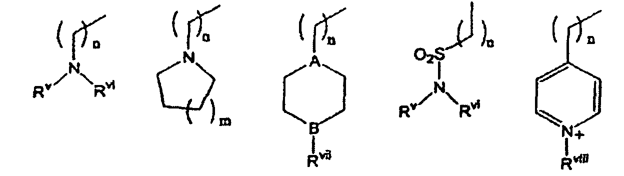
où n = 1-3, m = 1-3;
R^{v}, R^{vi} =H, alkyle C₁₋₇;
A=N,CH;
B=N,O,S,CH;
R^{vii} = absent lorsque B = O, S; ou R^{vii} = H, alkyle C₁₋₇ lorsque B = N, CH;
R^{viii}=O, alkyle C₁₋₇;
R6=H
Et où,
L'alkyle C₁₋₇ ou l'alkyle C₁₋₃ peut être optionnellement substitué par un ou deux halogènes et/ou un hétéro-atome S, O, N, qui, si situé à une terminaison de chaîne, est substitué par un ou deux atomes H;
Ar est un phényle, un pyrazolyle, un imidazolyle, un oxazolyle, un thiazinolyle, un isothiazinolyle, un oxadiazolyle, un 1,2,3-triazolyle, un furanyle, ou un thiényle, qui peut être optionnellement substitué par un halogène, un alkyle C₁₋₃, OH, un O-alkyle C₁₋₃, SH, un S-alkyle C₁₋₃ ou une amine;
l'amine est -NH₂, -NH-alkyle C₁₋₃ ou -N(C₁₋₃-alkyl)₂
et des sels pharmaceutiquement acceptables de ceux-ci.

2. Composé selon la revendication 1, où R2 et/ou R4 sont de l'hydrogène.

3. Composé selon la revendication 1 ou 2, où R1 est R'C (O),
OùR'=

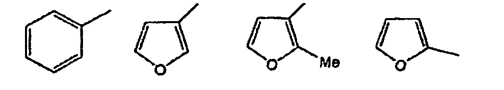

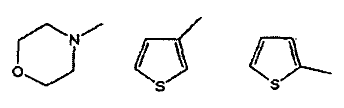

4. Composé selon la revendication 3, où R' est du fur-3-yl ou du thién-3-yl

5. Composé selon l'une quelconque des revendications 1-4, où R3 est du n-butyle, du t-butyle, du 3- (2,2-diméthylpropyle), du 4- (2-méthylbutyle), du 4- (3, 3-diméthylbutyle), du 4- (3,3-diméthyl-2- méthylbutyle), du 4- (3-méthyl-2-méthylbutyle) ou du 5- (2-méthyl-3- méthylpentyle).

6. Composé selon la revendication 5, où R3 est du t-butyle, du 3- (2,2-diméthylpropyle) ou du 4- (3,3-diméthyl-2-méthylbutyle).

7. Composé selon l'une quelconque des revendications 1-6, où R5 est CH₃, C₂H₅, CH₂AR, CH₂CONH₂, (CH₂)₂CONH₂,

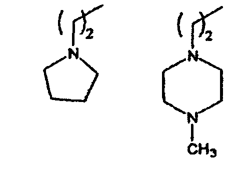

8. Composé selon la revendication 7, où R5 est CH₃, ou CH₂CH₃.

9. Composé selon l'une quelconque des revendications 1-8, où R5 a une stéréochimie (S).

10. Composé selon la revendication 1, où
R1 = R'C(O)
Où R' = 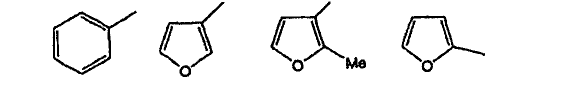 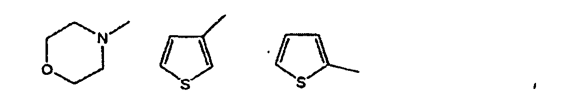 R2 et R4 = H;
R3 = du n-butyle, du t-butyle, du 3-(2,2-diméthylpropyle), du 4-(2-méthylbutyle), de 4-(3,3-diméthylbutyle), du 4-(3,3-diméthyl-2-méthylbutyle), du 4-(3-méthyl-2-méthylbutyle), du 5-(2-méthyl-3-méthylpentyle);
R5 = CH₃, C₂H₅, CH₂Ar, CH₂CONH₂, (CH₂)₂CONH₂, 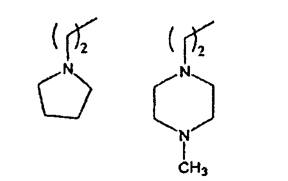 R6 = H.
ou un sel pharmaceutiquement acceptable de ceux-ci.

11. Composé qui est sélectionné parmi le groupe consistant en :
[3,3-diméthyl-1S- (2S-méthyl-4-oxo-tétrahydrofuran-3S-ylcarbamoyl) butyl] amide d'acide furan-3-carboxylique,
[3,3-diméthyl-1S-(2-éthyl-4-oxo-tétrahydrofuran-3-ylcarbamoyl) butyl] amide d'acide furan-3-carboxylique,
[1S-(2-carbamoylméthyl-4-oxo-tétrahydrofuran-3-ylcarbamoyl)-3,3-diméthylbutyl] amide d'acide furan-3-carboxylique,
{1S-[2-(2-diméthylaminoéthyl)-4-oxo-tétrahydrofuran-3-ylcarbamoyl]-3,3-diméthylbutyl} amide d'acide furan-3-carboxylique,
[3, 3-diméthyl-1S-(4-oxo-2-pyrrolidin-1-ylméthyl- tétrahydrofuran-3-ylcarbamoyl) butyl] amide d'acide furan-3-carboxylique,
[4-méthyl-1S-(2S-méthyl-4-oxo-tétrahydrofuran-3S-ylcarbamoyl) pentyl] amide d'acide furan-3-carboxylique,
[4-méthyl-1S- (2-éthyl-4-oxo-tétrahydrofuran-3-ylcarbamoyl) pentyl] amide d'acide furan-3-carboxylique,
[1S-(2-carbamoylméthyl-4-oxo-tétrahydrofuran-3-ylcarbamoyl)-4-méthylpentyl] amide d'acide furan-3-carboxylique,
{1S-[2-(2-diméthylaminoéthyl)-4-oxo-tétrahydrofuran-3-ylcarbamoyl]-4-méthylpentyl} amide d'acide furan-3-carboxylique,
[4-méthyl-1S-(4-oxo-2-pyrrolidin-1-ylméthyl-tétrahydrofuran- 3-ylcarbamoyl) pentyl] amide d'acide furan-3-carboxylique,
[3, 3-diméthyl-1S-(2S-méthyl-4-oxo-tétrahydrofuran-3S-ylcarbamoyl) pentyl] amide d'acide furan-3-carboxylique,
[3,3-diméthyl-1S-(2-éthyl-4-oxo-tétrahydrofuran-3-ylcarbamoyl) pentyl] amide d'acide furan-3-carboxylique,
[1S- (2-carbamoylméthyl-4-oxo-tétrahydrofuran-3-ylcarbamoyl)-3,3-diméthylpentyl] amide d'acide furan-3-carboxylique,
{1S- [2- (2-diméthylaminoéthyl)-4-oxo-tétrahydrofuran-3-ylcarbamoyl]-3, 3-diméthylpentyl} amide d'acide furan-3-carboxylique,
[3,3-diméthyl-1S- (4-oxo-2-pyrrolidin-1-ylméthyl-tétrahydrofuran-3-ylcarbamoyl) pentyl] amide d'acide furan-3-carboxylique,
[3,3,4-triméthyl-1S-(2S-méthyl-4-oxo-tétrahydrofuran-3S-ylcarbamoyl) pentyl] amide d'acide furan-3-carboxylique,
[3,3,4-triméthyl-1S-(2-éthyl-4-oxo-tétrahydrofuran-3-ylcarbamoyl) pentyl] amide d'acide furan-3-carboxylique,
[1S- (2-carbamoylméthyl-4-oxo-tétrahydrofuran-3-ylcarbamoyl)-3,3,4-triméthylpentyl] amide d'acide furan-3-carboxylique,
{1S- [2- (2-diméthylaminoéthyl)-4-oxo-tétrahydrofuran-3-ylcarbamoyl]-3,3,4-triméthylpentyl} amide d'acide furan-3-carboxylique,
[3, 3, 4-triméthyl-1S- (4-oxo-2-pyrrolidin-1-ylméthyl-tétrahydrofuran-3-ylcarbamoyl) pentyl] amide d'acide furan-3-carboxylique,
[3, 4-diméthyl-1S-(2S-méthyl-4-oxo-tétrahydrofuran-3S-ylcarbamoyl) pentyl] amide d'acide furan-3-carboxylique,
[3, 4-diméthyl-1S-(2-éthyl-4-oxo-tétrahydrofuran-3-ylcarbamoyl) pentyl] amide d'acide furan-3-carboxylique,
[1S-(2-carbamoylméthyl-4-oxo-tétrahydrofuran-3-ylcarbamoyl)-3,4-diméthylpentyl] amide d'acide furan-3-carboxylique,
{1S-[2-(2-diméthylaminoéthyl)-4-oxo-tétrahydrofuran-3-ylcarbamoyl]-3,4-diméthylpentyl} amide d'acide furan-3-carboxylique,
[3,4-diméthyl-1S- (4-oxo-2-pyrrolidin-1-ylméthyl-tétrahydrofuran-3-ylcarbamoyl) pentyl] amide d'acide furan-3-carboxylique,
[4,5-diméthyl-1S-(2S-méthyl-4-oxo-tétrahydrofuran-3S-ylcarbamoyl) hexyl] amide d'acide furan-3-carboxylique,
[4,5-diméthyl-1S-(2-éthyl-4-oxo-tétrahydrofuran-3-ylcarbamoyl) hexyl] amide d'acide furan-3-carboxylique,
[1S-(2-carbamoylméthyl-4-oxo-tétrahydrofuran-3-ylcarbamoyl)-4,5-diméthylhexyl] amide d'acide furan-3-carboxylique,
{1S- [2- (2-diméthylaminoéthyl)-4-oxo-tétrahydrofuran-3-ylcarbamoyl]-4,5-diméthylhexyl} amide d'acide furan-3-carboxylique,
[4,5-diméthyl-1S- (4-oxo-2-pyrrolidin-1-ylméthyl-tétrahydrofuran-3-ylcarbamoyl) hexyl] amide d'acide furan-3-carboxylique,
[3-méthyl-1S- (2S-méthyl-4-oxo-tétrahydrofuran-3S-ylcarbamoyl)-3-phenylbutyl] amide d'acide furan-3-carboxylique,
[3-méthyl-1 S- (2-éthyl-4-oxo-tétrahydrofuran-3-ylcarbamoyl)3-phenylbutyl] amide d'acide furan-3-carboxylique,
[1S- (2-carbamoylméthyl-4-oxo-tétrahydrofuran-3-ylcarbamoyl)-3-méthyl-3-phenylbutyl] amide d'acide furan-3-carboxylique,
{1S-[2- (2-diméthylaminoéthyl)-4-oxo-tétrahydrofuran-3-ylcarbamoyl]-3-méthyl-3-phenylbutyl}amide d'acide furan-3-carboxylique,
[3-méthyl-1S- (4-oxo-2-pyrrolidin-1-ylméthyl-tétrahydrofuran- 3-ylcarbamoyl)-3-phenylbutyl] amide d'acide furan-3-carboxylique,
[3,3-diméthyl-1S-{2S-méthyl-4-oxo-tétrahydrofuran-3S-ylcarbamoyl)-4-phenylbutyl] amide d'acide furan-3-carboxylique,
[3,3-diméthyl-1S-(2-éthyl-4-oxo-tétrahydrofuran-3-ylcarbamoyl)-4-phenylbutyl] amide d'acide furan-3-carboxylique,
[ 1S-(2-carbamoylméthyl-4-oxo-tétrahydrofuran-3-ylcarbamoyl)-3,3-diméthyl-4-phenylbutyl] amide d'acide furan-3-carboxylique,
{1S-[2-(2-diméthylaminoéthyl)-4-oxo-tétrahydrofuran-3-ylcarbamoyl]-3,3-diméthyl-4-phenylbutyl} amide d'acide furan-3-carboxylique,
[3,3-diméthyl-1S-(4-oxo-2-pyrrolidin-1-ylméthyl-tétrahydrofuran-3-ylcarbamoyl)-4-phenylbutyl] amide d'acide furan-3-carboxylique,
[3,3-diméthyl-1S-(2S-méthyl-4-oxo-tétrahydrofuran-3S-ylcarbamoyl)-5-phenylpentyl] amide d'acide furan-3-carboxylique,
[3,3-diméthyl-1S-(2-éthyl-4-oxo-tétrahydrofuran-3-ylcarbamoyl)-5-phenylpentyl] amide d'acide furan-3-carboxylique,
[1S-(2-carbamoylméthyl-4-oxo-tétrahydrofuran-3-ylcarbamoyl)-3,3-diméthyl-5-phenylpentyl] amide d'acide furan-3-carboxylique,
{1S-[2-(2-diméthylaminoéthyl)-4-oxo-tétrahydrofuran-3-ylcarbamoyl]-3,3-diméthyl-5-phenylpentyl} amide d'acide furan-3-carboxylique,
[3,3-diméthyl-1 (4-oxo-2-pyrrolidin-1-ylméthyl-tétrahydrofuran-3-ylcarbamoyl)-5-phenylpentyl] amide,
[3,3-diméthyl-1S- (2S-méthyl-4-oxo-tétrahydrofuran-3S-ylcarbamoyl) butyl] amide d'acide thiophène-3-carboxylique,
[3,3-diméthyl-1S-(2-éthyl-4-oxo-tétrahydrofuran-3-ylcarbamoyl) butyl] amide, d'acide thiophène-3-carboxylique,
[1S-(2-carbamoylméthyl-4-oxo-tétrahydrofuran-3-ylcarbamoyl)-3, 3-diméthylbutyl] amide d'acide thiophène-3-carboxylique,
{1S- [2- (2-diméthylaminoéthyl)-4-oxo-tétrahydrofuran-3-ylcarbamoyl]-3,3-diméthylbutyl} amide d'acide thiophène-3-carboxylique,
[3,3-diméthyl-1S-(4-oxo-2-pyrrolidin-1-ylméthyl-tétrahydrofuran-3-ylcarbamoyl) butyl] amide d'acide thiophène-3-carboxylique,
[4-méthyl-1 S- (2S-méthyl-4-oxo-tétrahydrofuran-3S-ylcarbamoyl) pentyl] amide d'acide thiophène-3-carboxylique,
[4-méthyl-1S- (2-éthyl-4-oxo-tétrahydrofuran-3-ylcarbamoyl) pentyl] amide d'acide thiophène-3-carboxylique,
[1S- (2-carbamoylméthyl-4-oxo-tétrahydrofuran-3-ylcarbamoyl)-4-méthylpentyl] amide d'acide thiophène-3-carboxylique,
{1S- [2-(2-diméthylaminoéthyl)-4-oxo-tétrahydrofuran-3-ylcarbamoyl]-4-méthylpentyl} amide d'acide thiophène-3-carboxylique,
[4-méthyl-1S-(4-oxo-2-pyrrolidin-1-ylméthyl-tétrahydrofuran-3-ylcarbamoyl)pentyl] amide d'acide thiophène-3-carboxylique,
[3,3-diméthyl-1S- (2S-méthyl-4-oxo-tétrahydrofuran-3S-ylcarbamoyl) pentyl] amide d'acide thiophène-3-carboxylique,
[3,3-diméthyl-1S- (2-éthyl-4-oxo-tétrahydrofuran-3-ylcarbamoyl) pentyl] amide d'acide thiophène-3-carboxylique,
[1S- (2-carbamoylméthyl-4-oxo-tétrahydrofuran-3-ylcarbamoyl)-3,3-diméthylpentyl] amide d'acide thiophène-3-carboxylique,
{1S-[2-(2-diméthylaminoéthyl)-4-oxo-tétrahydrofuran-3-ylcarbamoyl]-3,3-diméthylpentyl} amide d'acide thiophène-3-carboxylique,
[3,3-diméthyl-1S-(4-oxo-2-pyrrolidin-1-ylméthyl-tétrahydrofuran-3-ylcarbamoyl) pentyl] amide d'acide thiophène-3-carboxylique,
[3,3,4-triméthyl-1S- (2S-méthyl-4-oxo-tétrahydrofuran-3S-ylcarbamoyl) pentyl] amide d'acide thiophène-3-carboxylique,
[3,3,4-triméthyl-1S- (2-éthyl-4-oxo-tétrahydrofuran-3-ylcarbamoyl)pentyl] amide d'acide thiophène-3-carboxylique,
[1S-(2-carbamoylméthyl-4-oxo-tétrahydrofuran-3-ylcarbamoyl)-3,3,4-triméthylpentyl] amide d'acide thiophène-3-carboxylique,
{1S-[2-(2-diméthylaminoéthyl)-4-oxo-tétrahydrofuran-3-ylcarbamoyl]-3,3,4-triméthylpentyl} amide d'acide thiophène-3-carboxylique,
[3,3,4-triméthyl-1S-(4-oxo-2-pyrrolidin-1-ylméthyl-tétrahydrofuran-3-ylcarbamoyl) pentyl] amide d'acide thiophène-3-carboxylique,
[3,4-diméthyl-1S-(2S-méthyl-4-oxo-tétrahydrofuran-3S-ylcarbamoyl)pentyl] amide d'acide thiophène-3-carboxylique,
[3,4-diméthyl-1S-(2-éthyl-4-oxo-tétrahydrofuran-3-ylcarbamoyl) pentyl] amide d'acide thiophène-3-carboxylique,
[1S-(2-carbamoylméthyl-4-oxo-tétrahydrofuran-3-ylcarbamoyl)-3,4-diméthylpentyl] amide d'acide thiophène-3-carboxylique,
{1S- [2- (2-diméthylaminoéthyl)-4-oxo-tétrahydrofuran-3-ylcarbamoyl]-3,4-diméthylpentyl} amide d'acide thiophène-3-carboxylique,
[3,4-diméthyl-1S- (4-oxo-2-pyrrolidin-1-ylméthyl tétrahydrofuran-3-ylcarbamoyl) pentyl] amide d'acide thiophène-3-carboxylique,
[4,5-diméthyl-1S-(2S-méthyl-4-oxo-tétrahydrofuran-3S-ylcarbamoyl) hexyl] amide d'acide thiophène-3-carboxylique,
[4,5-diméthyl-1S- (2-éthyl-4-oxo-tétrahydrofuran-3-ylcarbamoyl) hexyl] amide d'acide thiophène-3-carboxylique,
[1S- (2-carbamoylméthyl-4-oxo-tétrahydrofuran-3-ylcarbamoyl)-4,5-diméthylhexyl] amide d'acide thiophène-3-carboxylique,
{1S-[2-(2-diméthylaminoéthyl)-4-oxo-tétrahydrofuran-3-ylcarbamoyl]-4,5-diméthylhexyl} amide d'acide thiophène-3-carboxylique,
[4,5-diméthyl-1S- {4-oxo-2-pyrrolidin-1-ylméthyl-tétrahydrofuran-3-ylcarbamoyl) hexyl] amide d'acide thiophène-3-carboxylique,
[3-méthyl-1S- (2S-méthyl-4-oxo-tétrahydrofuran-3S-ylcarbamoyl)-3-phenylbutyl] amide d'acide thiophène-3-carboxylique,
[3-méthyl-1S-(2-éthyl-4-oxo-tétrahydrofuran-3-ylcarbamoyl)-3-phenylbutyl] amide d'acide thiophène-3-carboxylique,
[1S-(2-carbamoylméthyl-4-oxo-tétrahydrofuran-3-ylcarbamoyl)-3-méthyl-3-phenylbutyl] amide d'acide thiophène-3-carboxylique,
{1S-[2-(2-diméthylaminoéthyl)-4-oxo-tétrahydrofuran-3-ylcarbamoyl]-3-méthyl-3-phenylbutyl} amide d'acide thiophène-3-carboxylique,
[3-méthyl-1S- (4-oxo-2-pyrrolidin-1-ylméthyl-tétrahydrofuran-3-ylcarbamoyl)-3-phenylbutyl] amide d'acide thiophène-3-carboxylique,
[3,3-diméthyl-1S- (2S-méthyl-4-oxo-tétrahydrofuran-3S-ylcarbamoyl)-4-phenylbutyl] amide d'acide thiophène-3-carboxylique,
[3,3-diméthyl-1S-(2-éthyl-4-oxo-tétrahydrofuran-3-ylcarbamoyl)-4-phenylbutyl] amide d'acide thiophène-3-carboxylique,
[1S-(2-carbamoylméthyl-4-oxo-tétrahydrofuran-3-ylcarbamoyl)-3,3-diméthyl-4-phenylbutyl] amide d'acide thiophène-3-carboxylique,
{1S-[2-(2-diméthylaminoéthyl)-4-oxo-tétrahydrofuran-3-ylcarbamoyl]-3,3-diméthyl-4-phenylbutyl}amide d'acide thiophène-3-carboxylique,
[3,3-diméthyl-1S- (4-oxo-2-pyrrolidin-1-ylméthyl-tétrahydrofuran-3-ylcarbamoyl)-4-phenylbutyl] amide d'acide thiophène-3-carboxylique,
[3,3-diméthyl-1S-(2S-méthyl-4-oxo-tétrahydrofuran-3S-ylcarbamoyl)-5-phenylpentyl] amide d'acide thiophène-3-carboxylique,
[3,3-diméthyl-1S- (2-éthyl-4-oxo-tétrahydrofuran-3-ylcarbamoyl)-5-phenylpentyl] amide d'acide thiophène-3-carboxylique,
[1S- (2-carbamoylméthyl-4-oxo-tétrahydrofuran-3-ylcarbamoyl)-3,3-diméthyl-5-phenylpentyl] amide d'acide thiophène-3-carboxylique,
{1S-[2-(2-diméthylaminoéthyl)-4-oxo-tétrahydrofuran-3-ylcarbamoyl]-3, 3-diméthyl-5-phenylpentyl} amide d'acide thiophène-3-carboxylique,
[3,3-diméthyl-1S- (4-oxo-2-pyrrolidin-1-ylméthyl-tétrahydrofuran-3-ylcarbamoyl)-5-phenylpentyl] amide d'acide thiophène-3-carboxylique,
[3,3-diméthyl-1S-(2S-méthyl-4-oxo-tétrahydrofuran-3S-ylcarbamoyl) butyl] amide d'acide 2-méthylfuran-3-carboxylique,
[3,3-diméthyl-1 S-(2-éthyl-4-oxo-tétrahydrofuran-3-ylcarbamoyl) butyl] amide d'acide 2-méthylfuran-3-carboxylique,
[1S- (2-carbamoylméthyl-4-oxo-tétrahydrofuran-3-ylcarbamoyl)-3,3-diméthylbutyl] amide d'acide 2-méthylfuran-3-carboxylique,
{1S-[2-(2-diméthylaminoéthyl)-4-oxo-tétrahydrofuran-3-ylcarbamoyl]-3,3-diméthylbutyl} amide d'acide 2-méthylfuran-3-carboxylique,
[3,3-diméthyl-1S- (4-oxo-2-pyrrolidin-1-ylméthyl-tétrahydrofuran-3-ylcarbamoyl) butyl] amide d'acide 2-méthylfuran-3-carboxylique,
[4-méthyl-1S- (2S-méthyl-4-oxo-tétrahydrofuran-3S-ylcarbamoyl) pentyl] amide d'acide 2-méthylfuran-3-carboxylique,
[4-méthyl-1S-(2-éthyl-4-oxo-tétrahydrofuran-3-ylcarbamoyl) pentyl] amide d'acide 2-méthylfuran-3-carboxylique,
[1S-(2-carbamoylméthyl-4-oxo-tétrahydrofuran-3-ylcarbamoyl)-4-méthylpentyl] amide d'acide 2-méthylfuran-3-carboxylique,
{1S- [2- (2-diméthylaminoéthyl)-4-oxo-tétrahydrofuran-3-ylcarbamoyl]-4-méthylpentyl} amide d'acide 2-méthylfuran-3-carboxylique,
[4-méthyl-1S-(4-oxo-2-pyrrolidin-1-ylméthyl-tétrahydrofuran-3-ylcarbamoyl) pentyl] amide d'acide 2-méthylfuran-3-carboxylique,
[3,3-diméthyl-1S-(2S-méthyl-4-oxo-tétrahydrofuran-3S-ylcarbamoyl) pentyl] amide d'acide 2-méthylfuran-3-carboxylique,
[3,3-diméthyl-1S-(2-éthyl-4-oxo-tétrahydrofuran-3-ylcarbamoyl) pentyl] amide d'acide 2-méthylfuran-3-carboxylique,
[1S-(2-carbamoylméthyl-4-oxo-tétrahydrofuran-3-ylcarbamoyl)-3,3-diméthylpentyl] amide d'acide 2-méthylfuran-3-carboxylique,
{1S-[2-(2-diméthylaminoéthyl)-4-oxo-tétrahydrofuran-3-ylcarbamoyl]-3,3-diméthylpentyl} amide d'acide 2-méthylfuran-3-carboxylique,
[3,3-diméthyl-1S- (4-oxo-2-pyrrolidin-1-ylméthyl-tétrahydrofuran-3-ylcarbamoyl) pentyl] amide d'acide 2-méthylfuran-3-carboxylique,
[3,3,4-triméthyl-1 S-(2S-méthyl-4-oxotétrahydrofuran-3S-ylcarbamoyl) pentyl] amide d'acide 2-méthylfuran-3-carboxylique,
[3,3,4-triméthyl-1S-(2-éthyl-4-oxo-tétrahydrofuran-3-ylcarbamoyl) pentyl] amide d'acide 2-méthylfuran-3-carboxylique,
[1S- (2-carbamoylméthyl-4-oxo-tétrahydrofuran-3-ylcarbamoyl)-3, 3, 4-triméthylpentyl] amide d'acide 2-méthylfuran-3-carboxylique,
{1S- [2- (2-diméthylaminoéthyl)-4-oxo-tétrahydrofuran-3-ylcarbamoyl]-3,3,4-triméthylpentyl} amide d'acide 2-méthylfuran-3-carboxylique,
[3,3,4-triméthyl-1 S- (4-oxo-2-pyrrolidin-1-ylméthyl-tétrahydrofuran-3-ylcarbamoyl) pentyl] amide d'acide 2-méthylfuran-3-carboxylique,
[3,4-diméthyl-1S- (2S-méthyl-4-oxo-tétrahydrofuran -3S-ylcarbamoyl) pentyl] amide d'acide 2-méthylfuran-3-carboxylique,
[3,4-diméthyl-1S- (2-éthyl-4-oxo-tétrahydrofuran-3-ylcarbamoyl) pentyl] amide d'acide 2-méthylfuran-3-carboxylique,
[1S-(2-carbamoylméthyl-4-oxo-tétrahydrofuran-3-ylcarbamoyl)-3,4-diméthylpentyl] amide d'acide 2-méthylfuran-3-carboxylique,
{1S-[2- (2-diméthylaminoéthyl)-4-oxo-tétrahydrofuran-3-ylcarbamoyl]-3,4-diméthylpentyl} amide d'acide 2-méthylfuran-3-carboxylique,
[3,4-diméthyl-1S-(4-oxo-2-pyrrolidin-1-ylméthyl-tétrahydrofuran-3-ylcarbamoyl) pentyl] amide d'acide 2-méthylfuran-3-carboxylique,
[4,5-diméthyl-1S- (2S-méthyl-4-oxo-tétrahydrofuran-3S-ylcarbamoyl) hexyl] amide d'acide 2-méthylfuran-3-carboxylique,
[4,5-diméthyl-1S- (2-éthyl-4-oxo-tétrahydrofuran-3-ylcarbamoyl) hexyl] amide d'acide 2-méthylfuran-3-carboxylique,
[1S- (2-carbamoylméthyl-4-oxo-tétrahydrofuran-3-ylcarbamoyl)-4,5-diméthylhexyl] amide d'acide 2-méthylfuran-3-carboxylique,
{1S-[2- (2-diméthylaminoéthyl)-4-oxo-tétrahydrofuran-3-ylcarbamoyl]-4,5-diméthylhexyl} amide d'acide 2-méthylfturan-3-carboxylique,
[4,5-diméthyl-1S-(4-oxo-2-pyrrolidin-1-ylméthyl-tétrahydrofuran-3-ylcarbamoyl) hexyl] amide d'acide 2-méthylfuran-3-carboxylique,
[3-méthyl-1S- (2S-méthyl-4-oxo-tétrahydrofuran-3S-ylcarbamoyl)-3-phenylbutyl] amide d'acide 2-méthylfuran-3 -carboxylique,
[3-méthyl-1S- (2-éthyl-4-oxo-tétrahydrofuran-3-ylcarbamoyl)-3-phenylbutyl] amide d'acide 2-méthylfuran-3-carboxylique,
[ 1S- (2-carbamoylméthyl-4-oxo-tétrahydrofuran-3-ylcarbamoyl)-3-méthyl-3-phenylbutyl] amide d'acide 2-méthylfuran-3-carboxylique,
{1S-[2-(2-diméthylaminoéthyl)-4-oxo-tétrahydrofuran-3-ylcarbamoyl]-3-méthyl-3-phenylbutyl} amide d'acide 2-méthylfuran-3-carboxylique,
[3-méthyl-1S- (4-oxo-2-pyrrolidin-1-ylméthyl-tétrahydrofuran-3-ylcarbamoyl)-3-phenylbutyl] amide d'acide 2-méthylfuran-3-carboxylique,
[3,3-diméthyl-1S-(2S-méthyl-4-oxo-tétrahydrofuran-3S-ylcarbamoyl)-4-phenylbutyl] amide d'acide 2-méthylfuran-3-carboxylique,
[3,3-diméthyl-1S-(2-éthyl-4-oxo-tétrahydrofuran-3-ylcarbamoyl)-4-phenylbutyl] amide d'acide 2-méthylfuran-3-carboxylique,
[1S- (2-carbamoylméthyl-4-oxo-tétrahydrofuran-3-ylcarbamoyl)-3,3-diméthyl-4-phenylbutyl] amide d'acide 2-méthylfuran-3-carboxylique,
{1S-[2-(2-diméthylaminoéthyl)-4-oxo-tétrahydrofuran-3-ylcarbamoyl]-3, 3-diméthyl-4-phenylbutyl} amide d'acide 2-méthylfuran-3-carboxylique,
[3, 3-diméthyl-1S- (4-oxo-2-pyrrolidin-1-ylméthyl-tétrahydrofuran-3-ylcarbamoyl)-4-phenylbutyl] amide d'acide 2-méthylfuran-3-carboxylique,
[3,3-diméthyl-1S-(2S-méthyl-4-oxo-tétrahydrofuran-3S-ylcarbamoyl)-5-phenylpentyl] amide d'acide 2-méthylfuran-3-carboxylique,
[3,3-diméthyl-1S-(2-éthyl-4-oxo-tétrahydrofuran-3-ylcarbamoyl)-5-phenylpentyl] amide d'acide 2-méthylfuran-3-carboxylique,
[1S- (2-carbamoylméthyl-4-oxo-tétrahydrofuran-3-ylcarbamoyl)-3,3-diméthyl-5-phenylpentyl] amide d'acide 2-méthylfuran-3-carboxylique,
{1S-[2-(2-diméthylaminoéthyl)-4-oxotétrahydrofuran-3-ylcarbamoyl]-3,3-diméthyl-5-phenylpentyl} amide d'acide 2-méthylfuran-3-carboxylique,
[3, 3-diméthyl-1S-(4-oxo-2-pyrrolidin-1-ylméthyl-tétrahydrofuran-3-ylcarbamoyl)-5-phenylpentyl] amide d'acide 2-méthylfuran-3-carboxylique,
[3,3-diméthyl-1S-(2S-méthyl-4-oxo-tétrahydrofuran-3S-ylcarbamoyl) butyl] amide d'acide 1H-pyrrole-3-carboxylique,
[3,3-diméthyl-1S-(2-éthyl-4-oxo-tétrahydrofuran-3-ylcarbamoyl) butyl] amide d'acide 1H-pyrrole-3-carboxylique,
[1S-(2-carbamoylméthyl-4-oxo-tétrahydrofuran-3-ylcarbamoyl)-3,3-diméthylbutyl] amide d'acide 1H-pyrrole-3-carboxylique,
{1S-[2-(2-diméthylaminoéthyl)-4-oxo-tétrahydrofuran-3-ylcarbamoyl]-3,3-diméthylbutyl} amide d'acide 1H-pyrrole-3-carboxylique,
[3,3-diméthyl-1S-(4-oxo-2-pyrrolidin-1-ylméthyl-tétrahydrofuran-3-ylcarbamoyl) butyl] amide d'acide 1H-pyrrole-3-carboxylique,
[4-méthyl-1S-(2S-méthyl-4-oxo-tétrahydrofuran-3S-ylcarbamoyl) pentyl] amide d'acide 1H-pyrrole-3-carboxylique,
[4-méthyl-1S- (2-éthyl-4-oxo-tétrahydrofuran-3-ylcarbamoyl) pentyl] amide d'acide 1H-pyrrole-3-carboxylique,
[1S-(2-carbamoylméthyl-4-oxo-tétrahydrofuran-3-ylcarbamoyl)-4-méthylpentyl] amide d'acide 1H-pyrrole-3-carboxylique,
{1S-[2-(2-diméthylaminoéthyl)-4-oxo-tétrahydrofuran-3-ylcarbamoyl]-4-méthylpentyl} amide d'acide 1H-pyrrole-3-carboxylique,
[4-méthyl-1S-(4-oxo-2-pyrrolidin-1-ylméthyl-tétrahydrofuran-3-ylcarbamoyl) pentyl] amide d'acide 1H-pyrrole-3-carboxylique,
[3,3-diméthyl-1S-(2S-méthyi-4-oxo-tétrahydrofuran-3S-ylcarbamoyl) pentyl] amide d'acide 1H-pyrrole-3-carboxylique,
[3,3-diméthyl-1S-(2-éthyl-4-oxo-tétrahydrofuran-3 ylcarbamoyl) pentyl] amide d'acide 1H-pyrrole-3-carboxylique,
[1S- (2-carbamoylméthyl-4-oxo-tétrahydrofuran-3-ylcarbamoyl)-3,3-diméthylpentyl] amide d'acide 1H-pyrrole-3-carboxylique,
{1S-[2-(2-diméthylaminoéthyl)-4-oxo-tétrahydrofuran-3-ylcarbamoyl)-3,3-diméthylpentyl}amide d'acide 1H-pyrrole-3-carboxylique,
[3, 3-diméthyl-1S- (4-oxo-2-pyrrolidirt-1-ylméthyl- tétrahydrofuran-3-ylcarbamoyl) pentyl] amide d'acide 1H-pyrrole-3-carboxylique,
[3,3,4-triméthyl-1S- (2S-méthyl-4-oxo-tétrahydrofuran-3S-ylcarbamoyl) pentyl] amide d'acide 1H-pyrrole-3 -carboxylique,
[3,3,4-triméthyl-1 S-(2-éthyl-4-oxo-tétrahydrofuran-3-ylcarbamoyl) pentyl] amide d'acide 1H-pyrrole-3-carboxylique,
[1S-(2-carbamoylméthyl-4-oxo-tétrahydrofuran-3-ylcarbamoyl)-3,3,4-triméthylpentyl] amide d'acide 1H-pyrrole-3-carboxylique, {1S-[2-(2-diméthylaminoéthyl)-4-oxo-tétrahydrofuran-3-ylcarbamoyl]-3,3,4-triméthylpentyl} amide d'acide 1H-pyrrole-3-carboxylique,
[3,3,4-triméthyl-1S-(4-oxo-2-pyrrolidin-1-ylméthyl-tétrahydrofuran-3-ylcarbamoyl) pentyl] amide d'acide 1H-pyrrole-3-carboxylique,
[3,4-diméthyl-1S-(2S-méthyl-4-oxo-tétrahydrofuran-3S-ylcarbamoyl) pentyl] amide d'acide 1H-pyrrole-3-carboxylique,
[3,4-diméthyl-1S-(2-éthyl-4-oxo-tétrahydrofuran-3-ylcarbamoyl) pentyl] amide d'acide 1H-pyrrole-3-carboxylique,
[1S-(2-carbamoylméthyl-4-oxo-tétrahydrofuran-3-ylcarbamoyl)-3,4-diméthylpentyl] amide d'acide 1H-pyrrole-3-carboxylique,
{1S- [2- (2-diméthylaminoéthyl)-4-oxo-tétrahydrofuran-3-ylcarbamoyl]-3, 4-diméthylpentyl} amide d'acide 1H-pyrrole-3-carboxylique,
[3, 4-diméthyl-1S-(4-oxo-2-pyrrolidin-1-ylméthyl-tétrahydrofuran-3-ylcarbamoyl)pentyl]amide d'acide 1H-pyrrole-3-carboxylique,
[4,5-diméthyl-1S-(2S-méthyl-4-oxo-tétrahydrofuran-3S-ylcarbamoyl) hexyl] amide d'acide 1H-pyrrole-3-carboxylique,
[4,5-diméthyl-1S-(2-éthyl-4-oxo-tétrahydrofuran-3-ylcarbamoyl) hexyl] amide d'acide 1H-pyrrole-3-carboxylique,
[1S-(2-carbamoylméthyl-4-oxo-tétrahydrofuran-3-ylcarbamoyl)-4,5-diméthylhexyl] amide d'acide 1H-pyrrole-3-carboxylique,
{1S-[2-(2-diméthylaminoéthyl)-4-oxo-tétrahydrofuran-3-ylcarbamoyl]-4,5-diméthylhexyl} amide d'acide 1H-pyrrole-3-carboxylique,
[4, 5-diméthyl-1S-(4-oxo-2-pyrrolidin-1-ylméthyl-tétrahydrofutan-3-ylcarbamoyl) hexyl] amide d'acide 1H-pyrrole-3-carboxylique,
[3-méthyl-1S-(2S-méthyl-4-oxo-tétrahydrofuran-3S-ylcarbamoyl)-3-phenylbutyl] amide d'acide 1H-pyrrole-3-carboxylique,
[3-méthyl-1S-(2-éthyl-4-oxo-tétrahydrofuran-3-ylcarbamoyl)-3-phenylbutyl] amide d'acide 1H-pyrrole-3-carboxylique,
[1S-(2-carbamoyhnéthyl-4-oxo-tétrahydrofuran-3-ylcarbamoyl)-3-méthyl-3-phenylbutyl] amide d'acide 1H-pyrrole-3-carboxylique,
{1S-[2-(2-diméthylaminoéthyl)-4-oxo-tétrahydrofuran-3-ylcarbamoyl]-3-méthyl-3-phenylbutyl} amide d'acide 1H-pyrrole-3-carboxylique,
[3-méthyl-1S-(4-oxo-2-pyrrolidin-1-ylméthyl-tétrahydrofuran-3-ylcarbamoyl)-3-phenylbutyl] amide d'acide 1H-pyrrole-3-carboxylique,
[3, 3-diméthyl-1S-(2S-méthyl-4-oxo-tétrahydrofuran-3S-ylcarbamoyl)-4-phenylbutyl] amide d'acide 1H-pyrrole-3-carboxylique,
[3,3-diméthyl-1S-(2-éthyl-4-oxo-tétrahydrofuran-3-ylcarbamoyl)-4-phenylbutyl] amide d'acide 1H-pyrrole-3-carboxylique,
[1S-(2-carbamoylméthyl-4-oxo-tétrahydrofuran-3-ylcarbamoyl)-3,3-diméthyl-4-phenylbutyl] amide d'acide 1H-pyrrole-3-carboxylique,
{1S-[2-(2-diméthylaminoéthyl)-4-oxo-tétrahydrofuran-3-ylcarbamoyl]-3,3-diméthyl-4-phenylbutyl} amide d'acide 1H-pyrrole-3-carboxylique,
[3, 3-diméthyl-1S- (4-oxo-2-pyrrolidin-1-ylméthyl-tétrahydrofuran-3-ylcarbamoyl)-4-phenylbutyl] amide d'acide 1H-pyrrole-3-carboxylique,
[3, 3-diméthyl-1S-(2S-méthyl-4-oxo-tétrahydrofuran-3S-ylcarbamoyl)-5-phenylpentyl] amide d'acide 1H-pyrrole-3-carboxylique,
[3,3-diméthyl-1S-(2-éthyl-4-oxo-tétrahydrofuran-3-ylcarbamoyl)-5-phenylpentyl] amide d'acide 1H-pyrrole-3-carboxylique,
[1S- (2-carbamoylméthyl-4-oxo-tétrahydrofuran-3-ylcarbamoyl)-3,3-diméthyl-5-phenylpentyl] amide d'acide 1H-pyrrole-3-carboxylique,
{1S-[2-(2-diméthylaminoéthyl)-4-oxo-tétrahydrofuran-3-ylcarbamoyl]-3,3-diméthyl-5-phenylpentyl}amide d'acide 1H-pyrrole-3-carboxylique,
[3,3-diméthyl-1S- (4-oxo-2-pyrrolidin-1-ylméthyl-tétrahydrofuran-3-ylcarbamoyl)-5-phenylpentyl] amide d'acide 1H-pyrrole-3-carboxylique,
N [3, 3-diméthyl-1S- (2S-méthyl-4-oxo-tétrahydrofuran-3S-ylcarbamoyl) butyl] benzamide,
N-[3,3-diméthyl-1S-(2-éthyl-4-oxo-tëtrahydrofuran-3-ylcarbamoyl)butyl]benzamide,
N-[1S-(2-carbamoylméthyl-4-oxo-tétrahydrofuran-3-ylcarbamoyl)-3,3diméthylbutyl] benzamide,
N-{1S-[2- {2-diméthylaminoéthyl)-4-oxo-tétrahydrofuran-3-ylcarbamoyl]-3,3-diméthylbutyl} benzamide,
N-[3,3-diméthyl-1S-(4-oxo-2-pyrrolidin-1-ylméthyl-tétrahydrofuran-3-ylcarbamoyl) butyl] benzamide,
N-[4-méthyl-1S-(2S-méthyl-4-oxo-tétrahydrofuran-3S-ylcarbamoyl) pentyl] benzamide,
N-[4-méthyl-1 S-{2-éthyl-4-oxo-tétrahydrofuran-3-ylcarbamoyl) pentyl] benzamide,
N-[1 S-(2-carbamoylméthyl-4-oxo-tétrahydrofuran-3-ylcarbamoyl) méthylpentyl] benzamide,
N- {1S-[2-(2-diméthylaminoéthyl)-4-oxo-tétrahydrofuran-3-ylcarbamoyl)-4-méthylpentyl] benzamide,
N-[4-méthyl-1S-(4-oxo-2-pyrrolidin-1-ylméthyl-tétrahydrofuran-3-ylcarbamoyl) pentyl] benzamide,
N-[3, 3-diméthyl-1S-(2S-méthyl-4-oxo-tétrahydrofuran-3S ylcarbamoyl) pentyl] benzamide,
N-[3,3-diméthyl-1S- (2-éthyl-4-oxo-tétrahydrofuran-3-ylcarbamoyl) pentyl] benzamide,
N-[1S- (2-carbamoylméthyl-4-oxo-tétrahydrofuran-3-ylcarbamoyl)-3,3-diméthylpentyl] benzamide,
N-{1S- [2- (2-diméthylaminoéthyl)-4-oxo-tétrahydrofuran-3-ylcarbamoyl]-3,3-diméthylpentyl} benzamide,
N-[3, 3-diméthyl-1S- (4-oxo-2-pyrrolidin-1-ylméthyl-tétrahydrofuran-3-ylcarbamoyl) pentyl] benzamide,
N-[3,3,4-triméthyl-1S-(2S-méthyl-4-oxo-tétrahydrofuran-3S-ylcarbamoyl) pentyl] benzamide,
N-[3,3,4-triméthyl-1S- (2-éthyl-4-oxo-tétrahydrofuran-3-ylcarbamoyl) pentyl] benzamide,
N-[1S-(2-carbamoylméthyl-4-oxo-tétrahydrofuran-3-ylcarbamoyl)-3,3,4-triméthylpentyl] benzamide,
N-{1S- [2- (2-diméthylaminoéthyl)-4-oxo-tétrahydrofuran-3-ylcarbamoyl]- 3,3,4-triméthylpentyl} benzamide,
N-[3,3,4-triméthyl-1S- (4-oxo-2-pyrrolidin-1-ylméthyl-tétrahydrofuran-3-ylcarbamoyl) pentyl] benzamide,
N-[3,4-diméthyl-1S-(2S-méthyl-4-oxo-tétrahydrofuran-3S-ylcarbamoyl) pentyl] benzamide,
N-[3,4-diméthyl-1S-(2-éthyl-4-oxo-tétrahydrofuran-3-ylcarbamoyl)-pentyl]benzamide,
N-[ 1 S-(2-carbamoylméthyl-4-oxo-tétrahydrofuran-3-ylcarbamoyl)-3,4-diméthylpentyl] benzamide,
N-{1S-[2-(2-diméthylaminoéthyl)-4-oxo-tétrahydrofuran-3-ylcarbamoyl]-3,4-diméthylpentyl} benzamide,
N-[3, 4-diméthyl-1S-(4-oxo-2-pyrrolidin-1-ylméthyl-tétrahydrofuran-3-ylcarbamoyl) pentyl] benzamide,
N-[4,5-diméthyl-1S-(2S-méthyl-4-oxo-tétrahydrofuran-3S- ylcarbamoyl) hexyl] benzamide,
N-[4,5-diméthyl-1S-(2-éthyl-4-oxo-tétrahydrofuran-3-ylcarbamoyl)hexyl]benzamide,
N-[1S-(2-carbamoylméthyl-4-oxo-tétrahydrofuran-3-ylcarbamoyl)-4, 5-diméthylhexyl] benzamide,
N- {1S-[2-(2-diméthylaminoéthyl)-4-oxo-tétrahydrofuran-3-ylcarbamoyl]-4, 5-diméthylhexyl} benzamide,
N-[4, 5-diméthyl-1S- (4-oxo-2-pyrrolidin-1-ylméthyl-tétrahydrofuran-3-ylcarbamoyl) hexyl] benzamide,
N-[3-méthyl-1S-(2S-méthyl-4-oxo-tétrahydrofuran-3S-ylcarbamonyl)-3-phenylbutyl] benzamide,
N [3-méthyl-1S- (2-éthyl-4-oxo-tétrahydrofuran-3-ylcarbamoyl)-3-phenylbutyl] benzamide,
N-[ 1 S-(2-carbamoylméthyl-4-oxo-tétrahydrofuran-3-ylcarbamoyl)-3-méthyl-3-phenylbutyl] benzamide,
N-{1S-[2- (2-diméthylaminoéthyl)-4-oxo-tétrahydrofuran-3-ylcarbamoyl]-3-méthyl-3- phenylbutyl} benzamide,
N-[3-méthyl-1S-(4-oxo-2-pyrrolidin-1-ylméthyl-tétrahydrofuran-3-ylcarbamoyl)-3-phenylbutyl] benzamide,
N-[3,3-diméthyl-1S-(2S-méthyl-4-oxo-tétrahydrofuran-3S-ylcarbamoyl)-4-phehylbutyl] benzamide,
N-[3,3-diméthyl-1S-(2-éthyl-4-oxo-tétrahydrofuran-3-ylcarbamoyl)-4-phenylbutyl] benzamide,
N-[1S-(2-carbamoylméthyl-4-oxo-tétrahydrofuran-3-ylcarbamoyl)-3, 3-diméthyl-4-phenylbutyl] benzamide,
N-[1S-[2-(2-diméthylaminoéthyl)-4-oxo-tétrahydrofuran-3-ylcarbamoyl]-3,3-diméthyl-4-phenylbutyl} benzamide,
N-[3, 3-diméthyl-1S-(4-oxo-2-pyrrolidin-1-ylméthyl-tétrahydrofuran-3-ylcarbamoyl)-4-phenylbutyl] benzamide,
N-[3, 3-diméthyl-1S-(2S-méthyl-4-oxo-tétrahydrofuran-3S-ylcarbamoyl)-5-phenylpentyl] benzamide,
N-[3,3-diméthyl-1S-(2-éthyl-4-oxo-tétrahydrofuran-3-ylcarbamoyl)-5-phenylpentyl] benzamide,
N-[1S-(2-carbamoylméthyl-4-oxo-tétrahydrofuran-3-ylcarbamoyl)-3,3-diméthyl-5-phenylpentyl] benzamide,
N- {1S- [2- (2-diméthylaminoéthyl)-4-oxo-tétrahydrofuran-3-ylcarbamoyl]-3, 3-diméthyl-5-phenylpentyl} benzamide,
N-[3,3-diméthyl-1S (4-oxo-2-pyrrolidin-1-ylméthyl-tétrahydrofuran-3-ylcarbamoyl)-5-phenylpentyl] benzamide,
[3,3-diméthyl-1S-(2S-méthyl-4-oxo-tétrahydrofuran-3S-ylcarbamoyl)butyl] amide d'acide morpholine-4-carboxylique,
[3,3-diméthyl-1S- (2-éthyl-4-oxo-tétrahydrofuran-3-ylcarbamoyl) butyl] amide d'acide morpholine-4-carboxylique,
[1S-(2-carbamoylméthyl-4-oxo-tétrahydrofuran-3-ylcarbamoyl)-3,3-diméthylbutyl] amide d'acide morpholine-4-carboxylique,
{1S-[2-(2-diméthylaminoéthyl)-4-oxo-tétrahydrofuran-3-ylcarbamoyl]-3,3-diméthylbutyl} amide d'acide morpholine-4-carboxylique,
[3,3-diméthyl-1S- (4-oxo-2-pyrrolidin-1-ylméthyl-tétrahydrofuran-3-ylcarbamoyl) butyl] amide d'acide morpholine-4-carboxylique,
[4-méthyl-1S-(2S-méthyl-4-oxo-tétrahydrofuran-3S-ylcarbamoyl) pentyl] amide d'acide morpholine-4-carboxylique,
[4-méthyl-1S-(2-éthyl-4-oxo-tétrahydrofuran-3-ylcarbamoyl) pentyl] amide d'acide morpholine-4-carboxylique,
[1S-(2-carbamoylméthyl-4-oxo-tétrahydrofuran-3-ylcarbamoyl)-4-méthylpentyl] amide d'acide morpholine-4-carboxylique,
{1S-[2-(2-diméthylaminoéthyl)-4-oxo-tétrahydrofuran-3-ylcarbamoyl]-4-méthylpentyl} amide d'acide morpholine-4-carboxylique,
[4-méthyl-1S- (4-oxo-2-pyirrolidin-1-ylméthyl-tétrahydrofuran-3-ylcarbamoyl) pentyl] amide d'acide morpholine-4-carboxylique,
[3,3-diméthyl-1S-(2S-méthyl-4-oxo-tétrahydrofuran-3S-ylcarbamoyl) pentyl] amide d'acide morpholine-4-carboxylique,
[3,3-diméthyl-1S-(2-éthyl-4-oxo-tétrahydrofuran-3-ylcarbamoyl) pentyl] amide d'acide morpholine-4-carboxylique,
[1S-(2-carbamoylméthyl-4-oxo-tétrahydrofuran-3-ylcarbamoyl)-3,3-diméthylpentyl] amide d'acide morpholine-4-carboxylique,
{1S-[2-(2-diméthylaminoéthyl)-4-oxo-tétrahydrofuran- 3-ylcarbamoyl]-3,3-diméthylpentyl} amide d'acide morpholine-4-carboxylique,
[3,3-diméthyl-1S- (4-oxo-2-pyrrolidin-1-ylméthyl-tétrahydrofuran-3-ylcarbamoyl)pentyl] amide d'acide morpholine-4-carboxylique,
[3,3,4-triméthyl-1 S-(2S-méthyl-4-oxo-tétrahydrofuran-3S-ylcarbamoyl) pentyl] amide d'acide morpholine-4-carboxylique,
[3,3,4-triméthyl-1S-(2-éthyl-4-oxo-tétrahydrofuran-3-ylcarbamoyl) pentyl] amide d'acide morpholine-4-carboxylique,
[1S-(2-carbamoylméthyl-4-oxo-tétrahydrofuran-3-ylcarbamoyl)-3,3,4-triméthylpentyl] amide d'acide morpholine-4-carboxylique,
{1S- [2- (2-diméthylaminoéthyl)-4-oxo-tétrahydrofuran- 3-ylcarbamoyl]-3,3,4-triméthylpentyl} amide d'acide morpholine-4-carboxylique,
[3,3,4-triméthyl-1 S-(4-oxo-2-pyrrolidin-1-ylméthyl-tétrahydrofuran-3-ylcarbamoyl) pentyl] amide d'acide morpholine-4-carboxylique,
[3,4-diméthyl-1S-(2S-méthyl-4-oxo-tétrahydrofuran-3S-ylcarbamoyl) pentyl] amide d'acide morpholine-4-carboxylique,
[3,4-diméthyl-1S-(2-éthyl-4-oxo-tétrahydrofuran-3-ylcarbamoyl) pentyl] amide d'acide morpholine-4-carboxylique,
[1S-(2-carbamoylméthyl-4-oxo-tétrahydrofuran-3-ylcarbamoyl)-3,4-diméthylpentyl] amide d'acide morpholine-4-carboxylique,
{1S- [2- (2-diméthylaminoéthyl)-4-oxo-tétrahydrofuran- 3-ylcarbamoyl]-3,4-diméthylpentyl} amide d'acide morpholine-4-carboxylique,
[3,4-diméthyl-1S- (4-oxo-2-pyrrolidin-1-ylméthyl-tétrahydrofuran-3-ylcarbamoyl) pentyl] amide d'acide morpholine-4-carboxylique,
[4,5-diméthyl-1S-(2S-méthyl-4-oxo-tétrahydrofuran-3S-ylcarbamoyl) hexyl] amide d'acide morpholine-4-carboxylique,
[4,5-diméthyl-1S-(2-éthyl-4-oxo-tétrahydrofuran-3-ylcarbamoyl) hexyl] amide d'acide morpholine-4-carboxylique,
[1S-(2-carbamoylméthyl-4-oxo-tétrahydrofuran-3-ylcarbamoyl)-4,5-diméthylhexyl] amide d'acide morpholine-4-carboxylique,
{1S-[2-(2-diméthylaminoéthyl)-4-oxo-tétrahydrofuran-3-ylcarbamoyl]-4,5-diméthylhexyl} amide d'acide morpholine-4-carboxylique,
[4,5-diméthyl-1S- (4-oxo-2-pyrrolidin-1-ylméthyl-tétrahydrofuran-3-ylcarbamoyl)hexyl] amide d'acide morpholine-4-carboxylique,
[3-méthyl-1S- (2S-méthyl-4-oxo-tétrahydrofuran-3S-ylcarbamoyl)-3-phenylbutyl] amide d'acide morpholine-4-carboxylique,
[3-méthyl-1S-(2-éthyl-4-oxo-tétrahydrofuran-3-ylcarbamoyl)-3-phenylbutyl] amide d'acide morpholine-4-carboxylique,
[1S- (2-carbamoylméthyl-4-oxo-tétrahydrofuran-3-ylcarbamoyl)-3-méthyl-3-phenylbutyl] amide d'acide morpholine-4-carboxylique,
{1S-[2-(2-diméthylaminoéthyl)-4-oxo-tétrahydrofuran-3-ylcarbamoyl]-3-méthyl-3-phenylbutyl} amide d'acide morpholine-4-carboxylique,
[3-méthyl-1S- (4-oxo-2-pyrrolidin-1-ylméthyl-tétrahydrofuran-3-ylcarbamoyl)-3-phenylbutyl] amide d'acide morpholine-4-carboxylique,
[3,3-diméthyl-1S-(2S-méthyl-4-oxo-tétrahydrofuran-3S-ylcarbamoyl)-4-phenylbutyl] amide d'acide morpholine-4-carboxylique,
[3,3-diméthyl-1S-(2-éthyl-4-oxo-tétrahydrofuran-3-ylcarbamoyl)-4-phenylbutyl]amide d'acide morpholine-4-carboxylique,
[1S- (2-carbamoylméthyl-4-oxo-tétrahydrofuran-3-ylcarbamoyl)-3,3-diméthyl-4-phenylbutyl] amide d'acide morpholine-4-carboxylique,
{1S- [2- (2-diméthylaminoéthyl)-4-oxo-tétrahydrofuran- 3-ylcarbamoyl]-3, 3-diméthyl-4-phenylbutyl} amide d'acide morpholine-4-carboxylique,
[3,3-diméthyl-1S- (4-oxo-2-pyrrolidin-1-ylméthyl-tétrahydrofuran-3-ylcarbamoyl)-4-phenylbutyl] amide d'acide morpholine-4-carboxylique,
[3,3-diméthyl-1S- (2S-méthyl-4-oxo-tétrahydrofuran-3S-ylcarbamoyl)-5-phenylpentyl] amide d'acide morpholine-4-carboxylique,
[3,3-diméthyl-1S-(2-éthyl-4-oxo-tétrahydrofuran-3-ylcarbamoyl)-5-phenylpentyl] amide d'acide morpholine-4-carboxylique,
[1S- (2-carbamoylméthyl-4-oxo-tétrahydrofuran-3-ylcarbamoyl)-3,3-diméthyl-5-phenylpentyl] amide d'acide morpholine-4-carboxylique,
{1S-[2- (2-diméthylaminoéthyl)-4-oxo-tétrahydrofuran- 3-ylcarbamoyl]-3,3-diméthyl-S-phenylpentyl} amide d'acide morpholine-4-carboxylique,
[3,3-diméthyl-1S-(4-oxo-2-pyrrolidin-1-ylméthyl-tétrahydrofuran-3-ylcarbamoyl)-5-phenylpentyl] amide d'acide morpholine-4-carboxylique,
ou un sel pharmaceutiquement acceptable de ceux-ci.

12. Composition pharmaceutique comprenant un composé selon l'une quelconque des revendications précédentes et un excipient pharmaceutiquement acceptable.

13. Composé selon l'une quelconque des revendications 1 à 11 à utiliser dans une méthode thérapeutique d'inhibition de la cystéine protéase cathepsine S.

14. Composition selon la revendication 12 à utiliser dans une méthode d'inhibition de la cystéine protéase cathepsine S.

15. Utilisation d'un composé selon l'une quelconque des revendications 1 à 11 dans la fabrication d'un médicament pour le traitement d'une maladie soulagée ou modérée par l'inhibition de l'activité de la cystéine protéase cathepsine S.
